(19) Europäisches Patentamt
European Patent Office
Office européen des brevets

(11) EP 1 333 824 B1

(12) EUROPEAN PATENT SPECIFICATION

(45) Date of publication and mention of the grant of the patent:
**07.09.2005 Bulletin 2005/36**

(21) Application number: **01994552.6**

(22) Date of filing: **14.11.2001**

(51) Int Cl.[7]: **A61K 31/165**, A61K 31/395, A61K 31/17, A61K 31/18, A61P 15/10

(86) International application number:
**PCT/GB2001/005018**

(87) International publication number:
**WO 2002/040008 (23.05.2002 Gazette 2002/21)**

# (54) TREATMENT OF SEXUAL DYSFUNCTION WITH BOMBESIN RECEPTOR ANTAGONISTS

BEHANDLUNG VON SEXUELLER FUNKTIONSSTÖRUNG MIT BOMBESIN REZEPTOR ANTAGONISTEN

TRAITEMENT DE DYSFONCTIONNEMENTS SEXUELS AVEC DES ANTAGONISTES DU RECEPTEUR DE LA BOMBESINE

(84) Designated Contracting States:
**AT BE CH CY DE DK ES FI FR GB GR IE IT LI LU MC NL PT SE TR**

(30) Priority: **17.11.2000 WOPCT/GB00/04380**
**23.04.2001 GB 0109910**
**04.05.2001 GB 0111037**

(43) Date of publication of application:
**13.08.2003 Bulletin 2003/33**

(73) Proprietor: **Warner-Lambert Company LLC**
**New Jersey 07950 (US)**

(72) Inventors:
- **GONZALEZ, Maria, Isabel**
  **Cambridge, Cambridgeshire CB1 2BX (GB)**
- **STOCK, Herman, Thijs**
  **NL-6602 WB Wijchen (NL)**
- **PINNOCK, Robert Denham**
  **Cambridge Cambridgeshire CB1 9YT (GB)**
- **PRITCHARD, Martyn Clive**
  **Huntingdon Cambridgeshire PE27 6WB (GB)**
- **WAYMAN, Christopher Peter**
  **Sandwich Kent CT13 9NJ (GB)**
- **VAN DER GRAAF, Pieter Hadewijn**
  **Sandwich Kent CT13 9NJ (GB)**
- **NAYLOR, Alisdair Mark**
  **Sandwich Kent CT13 9NJ (GB)**
- **HIGGINBOTTOM, Michael**
  **Cambridge CB2 1PD (GB)**

(74) Representative: **England, Peter Michael**
**Pfizer Limited**
**European Pharma Patent Department (I.P.C. 748)**
**Ramsgate Road**
**Sandwich**
**Kent CT13 9NJ (GB)**

(56) References cited:
**WO-A-00/37450** **WO-A-00/37462**
**WO-A-00/42955** **WO-A-96/28214**
**WO-A-98/07718** **WO-A-99/21562**
**WO-A-99/30697**

- **ASHWOOD V ET AL: "PD 176252 - The First High Affinity Non-peptide Gastrin-Releasing Peptide (BB2) Receptor Antagonist" BIOORGANIC & MEDICINAL CHEMISTRY LETTERS, OXFORD, GB, vol. 8, no. 18, 22 September 1998 (1998-09-22), pages 2589-2594, XP004138276 ISSN: 0960-894X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

• DATABASE BIOSIS [Online] BIOSCIENCES INFORMATION SERVICE, PHILADELPHIA, PA, US; September 2000 (2000-09) KE H Z ET AL: "Co-treatment of lasofoxifene (CP-336,156) and estrogen inhibits estrogen's effect in the uterus but maintains the bone protective effects in ovariectomized rats." Database accession no. PREV200000405569 XP002192429 & JOURNAL OF BONE AND MINERAL RESEARCH, vol. 15, no. Suppl. 1, September 2000 (2000-09), page S310 Twenty-Second Annual Meeting of the American Society for Bone and Mineral Research;Toronto, Ontario, Canada; September 22-26, 2000 ISSN: 0884-0431

## Description

### FIELD OF THE INVENTION

The present invention relates to methods for the treatment of sexual dysfunction and to the preparation of medicaments for the treatment of sexual dysfunction.

### BACKGROUND TO THE INVENTION

Both males and females can suffer from sexual dysfunction. Sexual dysfunctions are relatively common in the general population (see O'Donohue, 1997). The disorder may relate to seeking sexual behaviour (proceptivity) and/or to acceptance of sexual behaviour, accompanied by sexual arousal (receptivity). The prevalence of sexual problems is higher in populations receiving medicaments, in particular antidepressants and antihypertensives. A need for pharmacotherapy for sexual dysfunction is increasing, but there has been very little research effort directed at finding drugs to treat sexual dysfunction.

Sexual dysfunctions include erectile dysfunctions of organic and psychogenic origin (Benet, 1995) as well as hypoactive sexual desire disorders, sexual arousal disorders, anorgasmy and sexual pain disorders (Berman, 1999, *Urology*).

In males, impotence can be defined as an inability to achieve penile erection or ejaculation. Its prevalence is claimed to be between 2% and 7% of the human male population, increasing with age up to 50 years and between 18% and 80% between 55 and 80 years of age. In the USA alone, for example, it has been estimated that there are up to 10 million impotent males, with the majority suffering from problems of organic rather than of psychogenic origin. Although many different drugs have been shown to induce penile erection, they were only effective after direct injection into the penis e.g. intraurethrally or intracavernosally (i.c.) and were not approved for erectile dysfunction. US-A-5576290 discloses peptides which are stated to induce erection, but they have to be given subcutaneously e.g. by injection, and if an excessive dose is given they produce an exaggerated erectile response and stomach discomfort. Impotence treatment was revolutionized by the unexpected discovery that cGMP PDE inhibitors, e.g. pyrazolo[4,3-d]pyrimidin-7-ones were useful in the treatment of erectile dysfunction and could be administered orally, therefore obviating the disadvantages associated with i.c. administration. One such compound that is currently being manufactured is sildenafil (Viagra).

Thirty to 50% of American women complain of sexual dysfunction. Ageing, menopause, and decline in circulating oestrogen levels significantly increase the incidence of sexual complaints. In a recent publication, Berman J.R. et al. (1999, *Int. J. Impot. Res.*) describe methodology for evaluating physiologic and subjective components of the female sexual response in the clinical setting and determine the effects of age and oestrogen status on them. Low or absent sexual drive/desire constitutes the commonest problem in the female population (Laumann *et al*., 1999), but no therapy is available other than psychotherapy or empirical approaches. In a further publication (Bonney R.C et al., 2000) the causes and management of female sexual dysfunction are discussed, including the use of tibolone (Livial; Organon) which is a synthetic steroid that mimics the effects of oestrogen and has been reported to have mild androgenic properties, and the use of testosterone. WO 0037462 discloses the use of NK1-receptor antagonists for the treatment of diseases including sexual dysfunction.

So far in the UK and the USA no drug has been licensed by the Department of Health specifically for the treatment of female sexual dysfunction, hence there is an unmet medical need in the treatment of female sexual dysfunction, especially sexual drive problems.

### SUMMARY OF THE INVENTION

This invention is based on the realisation that substances that act as bombesin receptor antagonists have utility in the treatment of sexual dysfunction, including the behavioural component thereof, in both male and female subjects. In other words, they can provide a treatment, in males, for erectile dysfunctions of organic and psychogenic origin as well as hypoactive sexual desire disorders, sexual arousal disorders, anorgasmy and sexual pain disorders in females.

The invention therefore provides the use in the preparation of a medicament for the treatment of sexual dysfunction of a pharmaceutical combination comprising an effective amount of a bombesin (BB) receptor antagonist.

The invention further provides the use of a bombesin receptor antagonist in the manufacture of a medicament for preventing or treating male sexual dysfunction or female sexual dysfunction.

Furthermore, many of the compounds that can be used in this invention have both the property of binding to bombesin receptors and the property that an effective dose can be administered orally.

The bombesin antagonists preferably have a Ki against the bombesin receptor of less than 1000nM, preferably

less than 500nM, more preferably less than 100 nM, preferably less than 50 nM and most preferably less than 10 nM. Preferably the bombesin antagonists are selective for $BB_1$ over the other bombesin receptor subtypes (preferably a selectivity of greater than 10, and more preferably a selectivity greater than 30 and most preferably greater than 100 measurable *in vitro* by the ratio of their IC50 or Ki values against the BB1 and BB2 receptors respectively) and has a Ki against the $BB_1$ receptor of less than 1000nM, preferably less than 500nM, more preferably less than 100 nM, preferably less than 50 nM and most preferably less than 10 nM. Compounds having the potencies set out above can be identified by the *in vitro* screen described below.

Thus the invention provides the use in the preparative of a medicament for the treatment of drug induced sexual dysfunction (particularly but not exclusively dysfunction induced by antidepressants) in a male of a pharmaceutical combination comprising an effective amount of a bombesin BB1 antagonist or a mixed BB1/BB2 antagonist .

The invention further provides the use in the preparation of a medicament for the treatment of drug induced sexual dysfunction (particularly but not exclusively disfunction induced by antidepressants) in a female of a pharmaceutical combination comprising an effective amount of a bombesin BB1 antagonist or a mixed BB1/BB2 antagonist.

The invention also provides the use in the preparation of a medicament for the treatment of erectile dysfunction in a male of a pharmaceutical combination comprising an effective amount of a bombesin BB1 antagonist or a mixed BB1/BB2 antagonist.

The invention also provides the use in the preparation of a medicament for the treatment of a female patient suffering from hypoactive sexual desire disorder of a pharmaceutical combination comprising an effective amount of a bombesin BB1 antagonist or a mixed BB1/BB2 antagonist.

The invention also provides the use in the preparation of a medicament for the treatment of sexual arousal disorder and/or orgasmic disorder of a pharmaceutical combination comprising an effective amount of a bombesin BB1 antagonist or a mixed BB1/BB2 antagonist.

The combination provides a treatment for erectile dysfunctions of organic, neurogenic and/or psychogenic origin as well as hypoactive sexual desire disorders, sexual arousal disorders, anorgasmic and sexual pain disorders

The pharmaceutical combination (for simultaneous, separate or sequential administration) of a bombesin receptor antagonist further comprises one or more materials selected from (1) to (33) below:

(1) naturally occurring or synthetic prostaglandins or esters thereof;
(2) $\alpha$ - adrenergic receptor antagonist compounds also known as $\alpha$ - adrenoceptor antagonists or $\alpha$-receptor antagonists or $\alpha$-blockers;
(3) NO-donor (NO-agonist) compounds;
(4) potassium channel openers or modulators;
(5) dopaminergic agents;
(6) nimodepine, pinacidil, cyclandelate, isoxsuprine, chloropromazin halo peridol, Rec 15/2739, trazodone, alprostadil or phentolamine
(7) thromboxane A2 agonists;
(8) ergot alkaloids;
(9) angiotensin receptor antagonists such as losartan;
(10) substrates for NO-synthase;
(11) calcium channel blockers;
(12) cholesterol lowering agents;
(13) antiplatelet and antithrombotic agents;
(14) insulin sensitising agents and hypoglycacmic agents;
(15) L-DOPA or carbidopa;
(16) acetylcholinesterase inhibitors;
(17) steroidal or non-steroidal anti-inflammatory agents;
(18) estrogen receptor modulators and/or estrogen agonists and/or estrogen antagonists, and pharmaceutically acceptable salts thereof;
(19) PDE inhibitors;
(20) NPY (neuropeptide Y) inhibitors;
(21) NEP inhibitors;
(22) vasoactive intestinal proteins (VIP), VIP mimetics, VIP analogues,acting through one or more of the VIP receptor subtypes VPAC1, VPAC2 or PACAP (pituitory adenylate cyclase activating peptide), VIP analogue Ro-125-1553, VIP receptor agonists or VIP analogues or VIP fragments, or $\alpha$-adrenoceptor antagonists with VIP combinations;
(23) melanocortin receptor agonists or modulators or melanocortin enhancers;
(24) serotonin receptor agonists, antagonists or modulators;
(25) testosterone replacement agents, testosternone, dihydrotestosterone or a testosterone implant;

(26) estrogen, estrogen and medroxyprogesterone or medroxyprogesterone acetate (MPA) (i.e. as a combination), or estrogen and methyl testosterone hormone replacement therapy agents;
(27) monoamine metabolism or uptake modifiers that inhibit catecholamine metabolism or reuptake;
(28) purinergic receptor agonists and/or modulators;
(29) neurokinin (NK) receptor antagonists;
(30) opioid receptor agonists, antagonists or modulators;
(31) agonists or modulators for oxytocin/vasopressin receptors; and
(32) modulators of cannabinoid receptors.

In particular the invention includes a pharmaceutical composition or a unit dosage form comprising an effective amount of a bombesin receptor antagonist and an effective amount of any of the materials selected from (1) to (33) above.

In the above methods, and in the above combination, composition or dosage form, said antagonists preferably have a Ki against BB1, of of less than 1000nM, preferably less than 500nM, more preferably less than 100 nM, preferably less than 50 nM and most preferably less than 10 nM and/or a selectivity for $BB_1$ over the other bombesin receptor subtypes greater than 10, and more preferably greater than 30 and most preferably greater than 100 measurable *in vitro* by the ratio of their IC50 or Ki values against the BB1 and BB2 receptors respectively.

**BRIEF DESCRIPTION OF FIGURES**

**Figure 1**: Effect of (S) 3-(1H-Indol-3-yl)-N-[1-(5-methoxy-pyridin-2-yl)-cyclohexylmethyl]-2-methyl-2-[3-(4-nitro-phenyl)-ureido]-propionamide (Compound (1)) on female rat sexual proceptivity.

**Figure 2**: Effect of Compound (1) on female rat sexual receptivity.

**Figure 3:** Effect of repeated administration of Compound (1) on female rat proceptivity.

**Figure 4:** Effect of intracerebroventricular administration of Compound (1) on female rat sexual proceptivity.

**Figure 5:** Inhibitory effect of NMB on female rat sexual proceptivity and antagonism of this effect by Compound (1).

**Figure 6:** Results of an investigation to show whether the effect of Compound (1) on female sexual behaviour is mediated through progesterone.

**Figure 7:** Results of an investigation to show whether the effect of Compound (1) on female sexual behaviour is mediated through oestradiol.

**Figure 8:** Results of an investigation to show whether the effect of Compound (1) on female sexual behaviour is mediated through prolactin.

**Figure 9:** Results of an investigation to show whether the effect of Compound (1) on female sexual behaviour is mediated through LH.

**Figure 10:** Results of an investigation to show whether the effect of Compound (1) on female sexual behaviour is mediated through FSH.

**Figure 11:** Effect of Compound (1) on the sexual behaviour of normal male rats (Mount Latency).

**Figure 12:** Effect of Compound (1) on the sexual behaviour of normal male rats (Intromission Latency).

**Figure 13:** Effect of Compound (1) on the sexual behaviour of normal male rats (Number of Mounts + Intromission).

**Figure 14:** Effect of Compound (1) on the sexual behaviour of normal male rats (Ejaculation Latency).

**Figure 15:** Effect of Compound (1) on the sexual behaviour of normal male rats (Refractory Period).

**Figure 16:** Effect of Compound (1) on the sexual behaviour of sexually dysfunctional male rats (Mount Latency).

**Figure 17:** Effect of Compound (1) on the sexual behaviour of sexually dysfunctional male rats (Ejaculation Latency).

**Figure 18:** Effect of Compound (1) on the sexual behaviour of sexually dysfunctional male rats (% animals ejaculating).

**Figure 19:** Effect of (S)-3-(1*H*-Indol-3-yl)-*N*-[1-(5-methoxy-pyridin-2-yl)-cyclohexylmethyl]-2-methyl-2-[4-(4-nitrophenyl)-oxazol-2-ylamino]-propionamide (Compound (2)) in PEG 200 on female rat sexual proceptivity.

**Figure 20:** Effect of Compound (2) in methylcellulose on female rat sexual proceptivity.

**Figure 21:** Effect of Compound (2) in PEG 200 on female rat sexual receptivity.

**Figure 22:** Effect of compound **1** on basal and pelvic nerve-stimulated increases in female genital blood flow in the anaesthetised rabbit model of female sexual arousal.

**Figure 23:** Effect of (2*S*)-*N*-{[1-(4-aminophenyl)cyclohexyl]methyl}-3-(1*H*-indol-3-yl)-2-methyl-2-{[(4-nitroanilino) carbonyl]amino}propanamide (Compound **3**) on basal and pelvic nerve-stimulated increases female genital blood flow in the anaesthetised rabbit model of female sexual arousal.

**Figure 24:** Effect of compound **1** on penile intracavemosal pressure in the conscious male rat.

**Figure 25:** Effect of compound **3** on penile intracavemosal pressure in the conscious male rat model of penile erection.

**Figure 26:** Effect of compound **3** alone and in combination with a phosphodiesterase type five inhibitor on basal and pelvic nerve-stimulated increases penile intracavemosal pressure in the anaesthetised rabbit model of penile erection.

## DESCRIPTION OF PREFERRED EMBODIMENTS

### Suitable subjects

As previously explained the invention provides combinations, compositions and methods for the treatment of male sexual dysfunction or female sexual dysfunction. The inventors believe that there are common mechanisms underlying the pathologies of male and female psychogenic sexual dysfunctions.

Male sexual dysfunction includes male erectile dysfunction (MED). Patients with mild to moderate MED should benefit from treatment with a bombesin antagonist and patients with severe MED should also respond.the ability of bombesin antagonists to return intracavernosal pressure to normal levels in a conscious rat model of penile erection (Example 170, Figs. 24 and 25) and in a pelvic nerve stimulation model (Example 171, Fig 26) has been demonstrated using telemetry. However, early investigations suggest that the response rate of patients with mild, moderate and severe MED will be greater with a bombesin antagonist/PDE5 inhibitor combination (see Example 171 and Fig. 26). Mild, moderate and severe MED will be terms known to the man skilled in the art, but guidance can be found in *The Journal of Urology,* vol 151, 54-61 (Jan 1994).

Our investigations suggest the below mentioned male sexual dysfunction/MED patient groups should benefit from treatment with a bombesin antagonist and/or a bombesin antagonist with a phosphoesterase type 5 inhibitor (PDE5i) or other combination set out herein. These patient groups which are described in more detail in *Clinical Andrology* vol 23,no.4, p773-782, and chapter 3 of the book by I. Eardley and K. Sethia *"Erectile Dysfunction-Current Investigation and Management"*, published by Mosby-Wolfe are as follows: psychogenic, endocrinologic, neurogenic, arteriogenic, drug-induced sexual dysfunction and sexual dysfunction related to cavernosal factors, particularly venogenic causes. The invention finds application in the following sub-populations of patients with sexual dysfunction/MED: the young, the elderly including ageing-related decline in sexual arousability. More particularly, the invention finds application in patients with male sexual dyfunction such as MED arising from:-

(i) Arteriogenic/vasculogenic etiologies eg cardiovascular or atherosclerotic diseases, hypercholesterolemia, cigarette smoking, diabetes, hypertension, radiation and perineal trauma, traumatic injury to the iliohypogastric pudendal vacular system.

(ii) Neurogenic etiologies such as spinal cord injuries or diseases of the central nervous system including multiple sclerosis, diabetes, Parkinsonism, cerebrovascular accidents, peripheral neuropathies, trauma or radical pelvic surgery.

(iii) Hormonal/endocrine etiologies such as dysfunction of the hypothalamic/pituitary/gonadal axis, dysfunction of the pancreas, surgical or medical castration, androgen deficiency, high circulating levels of prolactin eg hyperprolactinemia, hyper and hypothyroidism.

(iv) Psychogenic etiologies such as depression, obsessive-compulsive disorder, anxiety disorder, emotional and relational issues, performance anxiety, marital discord, dysfunctional attitudes, sexual phobias, religious inhibition or a traumatic past experiences.

(v) Drug-induced sexual dysfunction resulting from therapy with selective serotonin reuptake inhibitors (SSRis) and other antidepressant therapies (tricyclics and major tranquillizers), anti-hypertensive therapies, and sympatholytic drugs.

Drug-induced sexual disfunction in males includes patients whose drug treatment/therapy leads to delayed ejaculation/orgasm, reduced libido and/or erectile dysfunction. The bombesin antagonists of the invention (more particularly BB1 antagonists) restore ejaculatory/orgasmic, libido and erectile function to normal physiological "levels". This is supported by the experiments described in Example 8 and Figs 16-18 below.

When the erectile dysfunction is other than drug induced, the bombesin antagonists (more particularly BB1 antagonists) can also be used to treat the erectile dysfunction by potentiating the normal endogenous erectiogenic mechanisms of the male (during normal sexual stimulation) and restoring the erectile function to normal levels during sexual stimulation. Thus Examples 170 and 171 and Figures 24, 25 and 26 hereinafter illustrate that bombesin antagonists (more particularly BB1 antagonists) or a bombesin antagonist with a phosphodiesterase type 5 inhibitor potentiate erectiogenic mechanisms in animal models by enhancing intercavemosal pressure and potentiating the effect of the pelvic nerve stimulation-induced increases in intracavemosal pressure.

Early investigations also show that that the invention will help restore the libido/desire in males to normal levels where the desire dysfunction is not drug induced (e.g. psychogenic).

The psychogenic component of male sexual dysfunction has been classified by the nomenclature committee of the International Society for Impotence Research (and is illustrated by Sachs B. D., 2000) as generalised type, characterised by a general unresponsiveness or primary lack of sexual arousal, and ageing-related decline in sexual arousability, characterised by generalised inhibition or chronic disorders of sexual intimacy.

The compounds of this invention are useful in the treatment of male sexual dysfunction, especially drug-induced male sexual dysfunction and psychogenic male sexual dysfunction associated with generalised unresponsiveness and ageing-related decline in sexual arousability.

Female sexual dysfunction can be grouped into four classes (Scrip's Complete Guide to Women's Healthcare, p.194-205, 2000), which include:

- Hypoactive sexual desire disorders, which can be characterised as persistent or recurrent lack of sexual thoughts/fantasies and lack of receptivity to sexual activity, causing personal distress.
- Sexual arousal disorders, which can be can be characterised as persistent or recurrent inability to achieve or maintain adequate sexual excitement, causing personal distress. The normal sexual arousal response consists of a number of physiological responses that are observed during sexual excitement. These changes such as vaginal, labial and clitoral engorgement result from increases in genital blood flow. Engorgement leads to increased vaginal lubrication via plasma transudation, increased vaginal compliance (relaxation of vaginal smooth muscle) and increases in vaginal and clitoral sensitivity. Female sexual arousal disorder (FSAD) is a highly prevalent sexual disorder affecting up to 40% of pre-, peri- and postmenopausal (±HRT) women. The primary consequence of FSAD is reduced genital engorgement or swelling which manifests itself as a lack of vaginal lubrication and a lack of pleasurable genital sensation. Secondary consequences include reduced sexual desire, pain during intercourse and difficulty in achieving orgasm. The most common cause of FSAD is decreased genital blood flow resulting in reduced vaginal, labial and clitoral engorgement. (Park, 1997; Goldstein, 1998; Berman, 1999, Werbin, 1999).
- Orgasmic disorders can be characterised as persistent or recurrent difficulty or delay in attaining orgasm after adequate sexual stimulation and arousal, causing personal distress.
- Sexual pain disorders can be characterised by dyspareunia, (characterised by recurrent or persistent genital pain associated with sexual intercourse), vaginismus (characterised by recurrent or persistent involuntary spasm of the muscles of the outer third of the vagina which interferes with vaginal penetration, causing personal distress) and other pain disorders (characterised by recurrent or persistent genital pain induced by non coital sexual stimulation).

The compounds of this invention are useful in the treatment of female sexual dysfunction (FSD), and this

includes pre-, peri- and post-menopausal female sexual dysfunction associated with hypoactive sexual desire disorders, sexual arousal disorders, orgasmic disorders or anorgasmy, or sexual pain disorders.

Early investigations suggest the below mentioned female sexual dysfunction (FSD) patient groups should benefit from treatement with a bombesin antagonist or a bombesin antagonist and a PDE5i (or other combination set out hereinafter). These patient groups are described in more detail in Berman *et al* (Urology, 1999). The invention finds application in the following sub-populations of patients with FSD: the young, the elderly (ageing-related sexual dysfunction), pre-menopausal, peri-menopausal, post-menopausal women with or without hormone replacement therapy. More particularly the invention finds application in patients with FSD arising from:-

(i) Arteriogenic/vasculogenic etiologies eg cardiovascular or atherosclerotic diseases, hypercholesterolemia, cigarette smoking, diabetes, hypertension, radiation and perineal trauma, traumatic injury to the iliohypogastric pudendal vacular system.

(ii) Neurogenic etiologies such as spinal cord injuries or diseases of the central nervous system including multiple sclerosis, diabetes, Parkinsonism, cerebrovascular accidents, peripheral neuropathies, trauma or radical pelvic surgery.

(iii) Hormonal/endocrine etiologies such as dysfunction of the hypothalamic/pituitary/gonadal axis, or dysfunction of the ovaries, dysfunction of the pancreas, surgical or medical castration, androgen deficiency, high circulating levels of prolactin eg hyperprolactinemia, natural menopause, premature ovarian failure, hyper and hypothyroidism.

(iv) Psychogenic etiologies such as depression, obsessive compulsive disorder, anxiety disorder, postnatal depression/"Baby Blues", emotional and relational issues, performance anxiety, marital discord, dysfunctional attitudes, sexual phobias, religious inhibition or a traumatic past experiences.

(v) Drug-induced sexual dysfunction resulting from therapy with selective serotonin reuptake inhibitors (SSRis) and other antidepressant therapies (tricyclics and major tranquillizers), anti-hypertensive therapies, sympatholytic drugs, chronic oral contraceptive pill therapy.

By drug-induced sexual dysfunction in females we mean to include cases where the drug treatment/therapy leads to delayed orgasm or inability to achieve an orgasm, reduced libido and FSD. The bombesin antagonists (more particularly BB1 antagonists) help restore orgasm, libido and female sexual function to normal physiological "levels". Furthermore, since bombesin antagonists have been shown to have beneficial effects on sexual function in ovary intact and in ovarectomised animals, it is apparent that bombesin antagonists (more particularly BB1 antagonists) can also be used to treat female sexual arousal disorders (FSAD), hypoactive sexual desire disorders (HSDD) and anorgasmy (FOD) and also sexual pain disorders, especially where these are secondary to arousal disorders. In particular, Example 2 and Figures 2 and 21 and Example 169 and Figures 22 and 23 illustrate that the combinations and methods of treatment of the invention can enhance receptive behaviour and arousal via increased genital blood flow in women with FSAD and FOD respectively. Also, Examples 1, 4 and 5 and Figures 1,3, 4 and 5 illustrate that the combinations and methods of treatment of the invention can increase proceptive behaviour and restore normal desire/libido in women with HSDD.

**Bombesin receptor antagonists - General**

Bombesin receptors are present in hypothalamic areas. We have found that they can exert a neuromodulatory effect on sexual behaviour.

We have tested compounds that are bombesin receptor antagonists using animal models that we have refined and believe are reliable and predictive, in particular with the capacity to make predictions for females. In rodents proceptive behaviour is under hormonal control, progesterone being essential for induction of proceptive behaviour in combination with oestrogen (Johnson M, 1988). The evidence for the hormonal control of proceptive behaviour in primates is conflicting, but on the whole oestrogens and/or androgens appear to enhance proceptive behaviour (Baum M.J, 1983). The behavioural manifestations of proceptive behaviour in the rat include "hopping and darting" movement, with rapid vibration of the ears. Tests to assess the eagerness to seek sexual contact (sexual motivation) have been reported as the most appropriate way to measure proceptivity (Meyerson, 1973). Receptivity, in the rat, is demonstrated when the female assumes a lordotic position. This occurs when, on mounting, the male exerts pressure with his forepaws on the flanks of the receptive female. The main sites of neuronal control for this behaviour are the ventromedial nucleus (VMN) and the midbrain central grey area (MCG) (for review, see Wilson C.A., 1993).

Bombesin is a 14-amino acid peptide originally isolated from the skin of the European frog *Bombina bombina* (Anastasi A., 1971). It belongs to a class of peptides which share structural homology in their C-terminal decapeptide region (Dutta A.S., *Small Peptides; Chemistry, Biology, and Clinical Studies*). At present, two mammalian bombesin-like peptides have been identified, the decapeptide neuromedin B (NMB) and a 23-residue amino acid, gastrin-releasing

peptide (GRP).

Bombesin evokes a number of central effects through actions at a heterogeneous population of receptors. The $BB_1$ receptor binds neuromedin B (NMB) with higher affinity than gastrin-related peptide (GRP) and neuromedin C (NMC) and $BB_2$ receptors bind GRP and NMC with greater affinity than NMB. More recently evidence has emerged of two more receptor subtypes denoted $BB_3$ and $BB_4$ but due to limited pharmacology, little is known of their function at present. $BB_1$ and $BB_2$ receptors have a heterogeneous distribution within the central nervous system indicating that the endogenous ligands for these receptors may differentially modulate neurotransmission. Among other areas, $BB_1$ receptors are present in the ventromedial hypothalamus (Ladenheim E.E, 1990).

Bombesin-like immunoreactivity and mRNA have been detected in mammalian brain (Braun M., et al., 1978, Battey J., et al.1991). NMB and GRP are believed to mediate a variety of biological actions (for a review, see WO 98/07718).

The following patent applications disclose compounds capable of antagonising the effects of NMB and/or GRP at bombesin receptors: CA 2030212, EP 0309297, EP 0315367, EP 0339193, EP 0345990, EP 0402852, EP 0428700, EP 0438519, EP 0468497, EP 0559756, EP 0737691, EP 0835662, JP 07258081, UK 2231051, US 4943561, US 5019647, US 5028692, US 5047502, US 5068222, US 5084555, US 5162497, US 5244883, US 5439884, US 5620955, US 5620959, US 5650395, US 5723578, US 5750646, US 5767236, US 5877277, US 5985834, WO 88/07551, WO 89/02897, WO 89/09232, WO 90/01037, WO 90/03980, WO 91/02746, WO 91/04040, WO 91/06563, WO 92/02545, WO 92/07830, WO 92/09626, WO 92/20363, WO 92/20707, WO 93/16105, WO 94/02018, WO 94/02163, WO 94/21674, WO 95/00542, WO 96/17617, WO 96/28214, WO 97/09347, WO 98/07718, WO 00/09115, WO 00/09116. We believe that compounds disclosed in these applications can be used in the prevention or treatment of male and/or female sexual dysfunction, which is an indication that is not disclosed or suggested by the aforesaid applications, or indeed in any previous scientific publication concerning bombesin receptors.

Bombesin receptor antagonists to which this invention is applicable include both non-peptide compounds and peptide compounds. Compounds that can be formulated into compositions for oral administration, especially human oral administration, without substantial loss of activity are preferred. Many non-peptide compounds having the desired properties fall into this category.

**A) Non-peptide bombesin receptor antagonists**

One preferred genus of compounds for use in the invention comprises bombesin receptor antagonists of the formula (I)

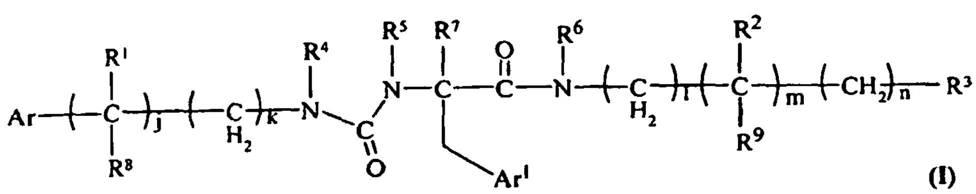

**(I)**

and pharmaceutically acceptable salts thereof, wherein:

- j is 0 or 1;
- k is 0 or 1;
- l is 0, 1, 2, or 3;
- m is 0 or 1;
- n is 0, 1 or 2;
- Ar is phenyl, pyridyl or pyrimidyl, each unsubstituted or substituted by from 1 to 3 substituents selected from alkyl, halogen, alkoxy, acetyl, nitro, amino, - $CH_2NR^{10}R^{11}$, cyano, $-CF_3$, $-NHCONH_2$, and $-CO_2R^{12}$;
- $R^1$ is hydrogen or straight, branched, or cyclic alkyl of from 1 to 7 carbon atoms;
- $R^8$ is hydrogen or forms a ring with $R^1$ of from 3 to 7 carbon atoms;
- $R^2$ is hydrogen or straight, branched, or cyclic alkyl of from 1 to 8 carbon atoms which can also contain 1 to 2 oxygen or nitrogen atoms;
- $R^9$ is hydrogen or forms with $R^2$ a ring of from 3 to 7 carbon atoms which can contain an oxygen or nitrogen atom; or $R^2$ and $R^9$ can together be a carbonyl;
- $Ar^1$ can be independently selected from Ar and can also include pyridyl-N-oxide, indolyl, imidazolyl, and pyridyl;
- $R^4$, $R^5$, $R^6$, and $R^7$ are each independently selected from hydrogen and lower alkyl; $R^4$ can also form with $R^5$ a covalent link of 2 to 3 atoms which may include an oxygen or a nitrogen atom;

- $R^3$ can be independently selected from Ar or is hydrogen, hydroxy, $-NMe_2$, N-methyl-pyrrolyl, imidazolyl, N-methyl-imidazolyl, tetrazolyl, N-methyl-tetrazolyl, thiazolyl, $-CONR^{13}R^{14}$, alkoxy,

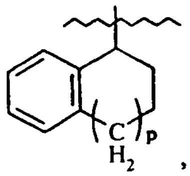 , 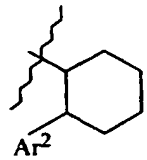 , 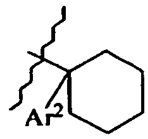 ,

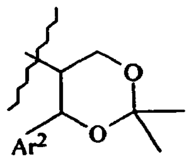 , or 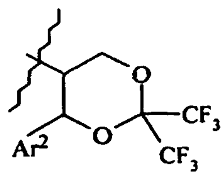 ,

wherein p is 0, 1 or 2 and $Ar^2$ is phenyl or pyridyl;

- $R^{10}$, $R^{11}$, $R^{12}$, $R^{13}$ and $R^{14}$ are each independently selected from hydrogen or straight, branched, or cyclic alkyl of from 1 to 7 carbon atoms.

Preferred compounds are those of Formula (Ia)

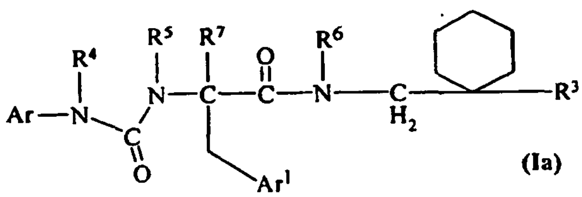

(Ia)

wherein

- Ar is phenyl unsubstituted or substituted with 1 or 2 substituents selected from isopropyl, halo, nitro, and cyano;
- $R^4$, $R^5$, and $R^6$ are hydrogen;
- $R^7$ is methyl or hydrogen;
- $R^3$ is 2-pyridyl or hydroxy; and
- $Ar^1$ is indolyl, pyridyl, pyridyl-N-oxide, or imidazolyl.

Other preferred compounds are those of Formula I wherein

- ; Ar is unsubstituted phenyl;
- ; $R^1$ is cyclopentyl or *tert*-butyl;
- ; $R^4$ and $R^5$ are hydrogen;
- ; $R^7$ is methyl;
- ; $R^6$ is hydrogen;
- ; $R^3$ is phenyl with two isopropyl substituents, unsubstituted phenyl, or

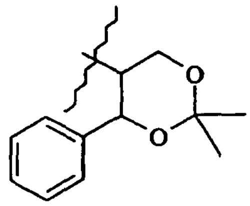
;

and

Ar$^1$ is indolyl.

Other preferred compounds are those of Formula I wherein

- ; Ar is 2,6-diisopropyl-phenyl, 4-nitro-phenyl, and 4-cyano-phenyl;
- ; $R^4$, $R^5$, and $R^6$ are hydrogen;
- ; $R^7$ is methyl;
- ; $R^2$ is hydrogen or cyclohexyl; and
- ; $R^3$ is hydroxyl, pyridyl,

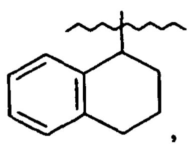
,
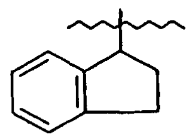
, or
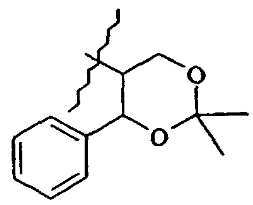
.

At present, most preferred of the compounds of formula (I) are (S) 3-(1H-Indol-3-yl)-N-[1-(5-methoxy-pyridin-2-yl)-cyclohexylmethyl]-2-methyl-2-[3-(4-nitrophenyl)-ureido]-propionamide (also referred to as Compound **1**) and its pharmacologically acceptable salts and (2*S*)-*N*-{[1-(4-aminophenyl)cyclohexyl]methyl}-3-(1*H*-indol-3-yl)-2-methyl-2-{[(4-nitroanilino)-carbonyl]amino}propanamide (also referred to as Compound **3**) and its pharmacologically acceptable salts.

Other preferred compounds of Formula (I) are set out below and included also are their pharmaceutically acceptable salts:

(S) *N*-cyclohexylmethyl-2-[3-(2,6-diisopropyl-phenyl)-ureido]-3-(1*H*-indol-3-yl)-2-methyl-propionamide;
*N*-cyclohexylmethyl-2-[3-(2,6-diisopropyl-phenyl)-ureido]-3-(1*H*-indol-3-yl)-*N*-methyl-propionamide;
*N*-cyclohexylmethyl-2-[3-(2,6-diisopropyl-phenyl)-1-methyl-ureido]-3-(1*H*-indol-3-yl)-propionamide;
2-[3-(2,6-diisopropyl-phenyl)-ureido]-2-methyl-3-(1-oxy-pyridin-2-yl)-*N*-(1-pyridin-2-yl-cyclohexylmethyl)-propionamide;
2-[3-(2,6-diisopropyl-phenyl)-ureido]-2-methyl-3-pyridin-2-yl-*N*-(1-pyridin-2-yl-cyclohexylmethyl)-propionamide;
2-[3-(2-*tert*-butyl-phenyl)-ureido]-*N*-cyclohexylmethyl-3-(1*H*-indol-3-yl)-2-methyl-propionamide;
*N*-cyclohexylmethyl-2-[3-(2,6-dichloro-phenyl)ureido]-3-(1*H*-indol-3-yl)-2-methyl-propionamide;
*N*-cyclohexylmethyl-2-[3-(2,6-dimethoxy-phenyl)ureido]-3-(1*H*-indol-3-yl)-2-methyl-propionamide;
*N*-cyclohexylmethyl-2-[3-(2,6-dimethylamino-phenyl)-ureido]-3-(1*H*indol-3-yl)-2-methyl-propionamide;
(S) *N*-cyclohexylmethyl-3-(1*H*-indol-3-yl)-2-methyl-2-[3-(4-nitro-phenyl)-ureido]-propionamide;
*N*-cyclohexylmethyl-2-[3-(2,2-dimethyl-1-phenyl)propyl)-ureido]-3-(1*H*-indol-3-yl)-2-methyl-propionamide;
[S-(R*, R*)] 3-(1*H*-indol-3-yl)-2-methyl-2-{3-[1-(4-nitro-phenyl)-ethyl]-ureido}-*N*-(1-pyridin-2-yl-cyclohexylmethyl)-propionamide;
*N*-(2,2-dimethyl-4-phenyl-[1,3]dioxan-5-yl)-3-(1*H*-indol-3-yl)-2-methyl-2-[3-(1-phenyl-cyclopentylmethyl)ureido]-propionamide;
(S)-*N*-(2,6-diisopropyl-phenyl)-2-[3-(2,2-dimethyl-1-phenyl-propyl)-ureido]-3-(1*H*-indol-3-yl)-propionamide;
(R)-*N*-(2,6-diisopropyl-phenyl)-2-[3-(2,2-dimethyl-1-phenyl-propyl)-ureido]-3-(1*H*-indol-3-yl)-propionamide;
2-[3-(2,6-diisopropyl-phenyl)-ureido]-*N*-(2,2-dimethyl-4-phenyl-[1,3]dioxan-5-yl)-3-(1*H*-indol-3-yl)-2-methyl-propionamide;
*N*-cyclohexyl-2-[3-(2,6-diisopropyl-phenyl)-ureido]-3-(1*H*-indol-3-yl)-2-methyl-propionamide;
*N*-(2-cyclohexyl-ethyl)-2-[3-(2,6-diisopropyl-phenyl)-ureido]-3-(1*H*-indol-3-yl)-2-methyl-propionamide;
2-[3-(2,6-diisopropyl-phenyl)-ureido]-3-(1*H*-indol-3-yl)-2-methyl-propionamide;

2-[3-(2,6-diisopropyl-phenyl)-ureido]-3-(1*H*-indol-3-yl)-2-methyl-*N*-(3-methyl-butyl)-propionamide;
2-[3-(2,6-diisopropyl-phenyl)-ureido]-3-(1*H*-indol-3-yl)-2-methyl-*N*-(3-phenyl-propyl)-propionamide;
2-[3-(2,6-diisopropyl-phenyl)-ureido]-3-(1*H*-indol-3-yl)-2-methyl-*N*-(1,2,3,4-tetrahydro-naphthalen-1-yl)-propionamide;
2-[3-(2,6-diisopropyl-phenyl)-ureido]-3-(1*H*-indol-3-yl)-2-methyl-*N*-(2-phenyl-cyclohexyl)-propionamide;
2-[3-(2,6-diisopropyl-phenyl)-ureido]-*N*-indan-1-yl-3-(1*H*-indol-3-yl)-2-methyl-propionamide;
2-[3-(2,6-diisopropyl-phenyl)-ureido]-*N*-(1-hydroxy-cyclohexylmethyl)-3-(1*H*-indol-3-yl)-2-methyl-propionamide;
2-[3-(2,6-diisopropyl-phenyl)-ureido]-3-(1*H*-indol-3-yl)-2-methyl-*N*-(1-pyridin-2-yl-cyclohexylmethyl)-propionamide;
2-[3-(2,6-diisopropyl-phenyl)-ureido]-3-(1*H*-indol-3-yl)-2-methyl-*N*-(6,7,8,9-tetrahydro-5*H*-benzocyclohepten-5-yl)-propionamide;
2-[3-(2,6-diisopropyl-phenyl)-ureido]-3-(1*H*-indol-3-yl)-2-methyl-*N*-phenyl-propionamide;
*N*-(1-hydroxy-cyclohexylmethyl)-3-(1*H*-indol-3-yl)-2-methyl-2-[3-(4-nitrophenyl)-ureido]-propionamide;
2-[3-(4-cyano-phenyl)-ureido]-3-(1*H*-indol-3-yl)-2-methyl-*N*-(1-pyridin-2-yl-cyclohexylmethyl)-propionamide;
(S) 3-(1*H*-indol-3-yl)-2-methyl-2-[3-(4-nitro-phenyl)-ureido]-*N*-(1-pyridin-2-yl-cyclohexylmethyl)-propionamide;
(S) 3-(1*H*-indol-3-yl)-2-methyl-*N*-(1-pyridin-2-yl-cyclohexylmethyl)-2-[3-(4-trifluoromethyl-phenyl)-ureido]-propionamide;
(S) 4-(3-{2-(1*H*-indol-3-yl)-1-methyl-1-[(1-pyridin-2-yl-cyclohexylmethyl)-carbamoyl]-ethyl}-ureido)-benzoic acid ethyl ester;
2-[3-(2,6-diisopropyl-phenyl)-ureido]-3-(1*H*-imidazol-4-yl)-*N*-(1-pyridin-2-yl-cyclohexylmethyl)-propionamide;
2-[3-(2,6-diisopropyl-phenyl)-ureido]-2-methyl-*N*-(1-pyridin-2-yl-cyclohexylmethyl)-3-(2-trifluoromethyl-phenyl)-propionamide;
2-[3-(2,6-diisopropyl-phenyl)-ureido]-2-methyl-3-(2-nitro-phenyl)-*N*-(1-pyridin-2-yl-cyclohexylmethyl)-propionamide;
(S) 3-(1*H*-indol-3-yl)-*N*-[1-(5-methoxy-pyridin-2-yl)-cyclohexylmethyl]-2-methyl-2-[3-(4-nitro-phenyl)-ureido]-propionamide; and
*N*-cyclohexylmethyl-2-[3-(2,6-diisopropyl-phenyl)-ureido]-2-methyl-3-pyridin-2-yl-propionamide.

Another preferred genus of compounds which can be used for the present purpose is of formula (II) and includes pharmaceutically acceptable salts thereof:

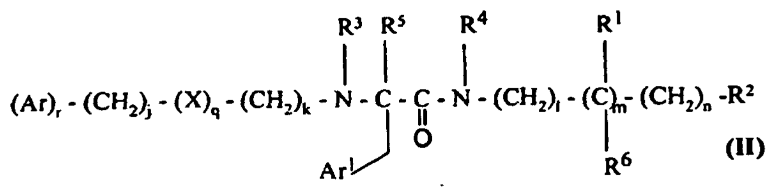

(II)

wherein:

- j is 0, 1 or 2;
- k is 0 or 1;
- l is 0, 1, 2, or 3;
- m is 0 or 1;
- n is 0, 1 or 2;
- q is 0 or 1;
- r is 0 or 1; when r is 0, Ar is replaced by hydrogen;
- Ar is phenyl, pyridyl, pyrimidyl, thienyl, furyl, imidazolyl, pyrrolyl or thiazolyl each unsubstituted or substituted by from 1 to 3 substituents selected from acetyl, alkoxy, alkyl, amino, cyano, halo, hydroxy, nitro, sulfonamido, sulfonyl, $-CF_3$, $-OCF_3$, $-CO_2H$, $-CH_2CN$, $-SO_2CF_3$, $-CH_2CO_2H$ and $-(CH_2)_SNR^7R^8$ wherein s is 0, 1, 2 or 3 and $R^7$ and $R^8$ are each independently selected from H, straight or branched alkyl of up to 6 carbon atoms, or $R^7$ and $R^8$ together with the nitrogen atom to which they are linked can form a 5- to 7-membered aliphatic ring which may contain 1 or 2 oxygen atoms;
- $R^1$ is hydrogen, straight or branched alkyl of up to 6 carbon atoms or cycloalkyl of between 5 and 7 carbon atoms which may contain 1 or 2 nitrogen or oxygen atoms;
- $R^6$ is hydrogen, methyl, or forms with $R^1$ an aliphatic ring of from 3 to 7 atoms which can contain an oxygen or

nitrogen atom, or together with $R^1$ is a carbonyl group;

- $Ar^1$ is independently selected from Ar or is indolyl or pyridyl-*N*-oxide;
- $R^3$, $R^4$, and $R^5$ are each independently selected from hydrogen and lower alkyl;
- $R^2$ is independently selected from Ar or is hydrogen, hydroxy, alkoxy, $-NMe_2$, $-CONR^9R^{10}$ wherein $R^9$ and $R^{10}$ are each independently selected from hydrogen, straight or branched alkyl of up to 6 carbon atoms, or $R^9$ and $R^{10}$ together with the nitrogen atom to which they are linked can form a 5-to 7-membered aliphatic ring which may contain 1 or 2 oxygen or nitrogen atoms, or $R^2$ is

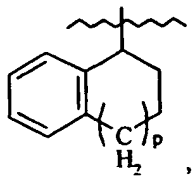,
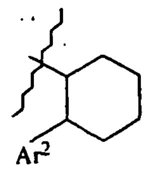,
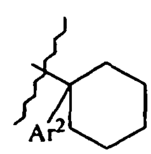,

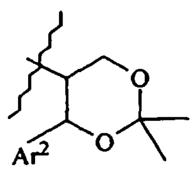, or
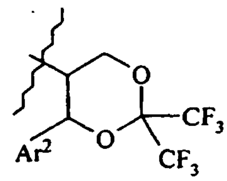

wherein p is 0, 1 or 2 and $Ar^2$ is phenyl or pyridyl;

- X is a divalent radical derived from any of the following

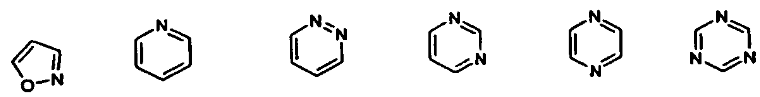

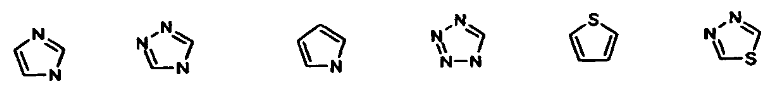

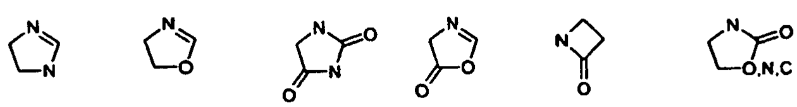

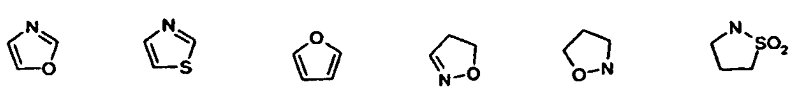

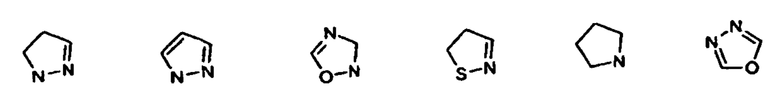

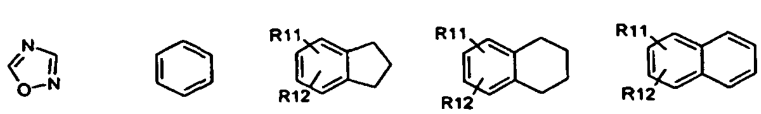

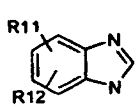 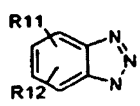 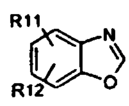 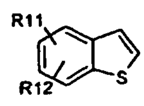 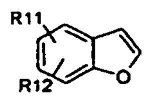

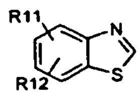 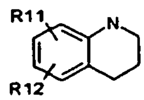 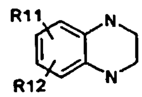 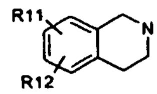

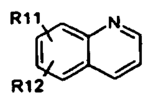 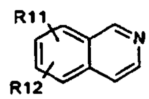

where the ring nitrogen atoms may have lower alkyl groups attached thereto, $R^{11}$ and $R^{12}$ are independently selected from H, halogen, hydroxy, alkoxy, acetyl, nitro, cyano, amino, $CF_3$ and $-(CH_2)_tNR^{13}R^{14}$ where t can be 0 or 1, $R^{13}$ and $R^{14}$ are each independently selected from hydrogen, straight or branched alkyl of up to 6 carbon atoms or cycloalkyl of 5 to 7 carbon atoms, containing up to 2 oxygen or nitrogen atoms.

A preferred species of compounds within the genus defined by formula (II) is represented by the formula **(IIa),** and includes pharmaceutically acceptable salt thereof:

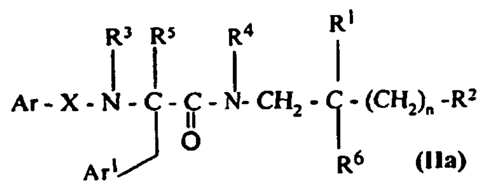

**(IIa)**

wherein:

- n is 0 or 1;
- Ar is phenyl or pyridyl which may be unsubstituted or substituted with from 1 to 3 substituents selected from halogen, alkoxy, nitro and cyano;
- $Ar^1$ is independently selected from Ar or is pyridyl-*N*-oxide or indolyl;
- $R^6$ forms with $R^1$ an aliphatic ring of from 3 to 7 atoms which can contain an oxygen or nitrogen atom, or together with $R^1$ is a carbonyl group;
- $R^2$ is independently selected from Ar or is hydrogen, hydroxy, alkoxy, dimethylamino, tetrazolyl or $-CONR^9R^{10}$ wherein $R^9$ and $R^{10}$ are each independently selected from hydrogen or methyl or $R^2$ is any of

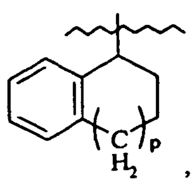 , 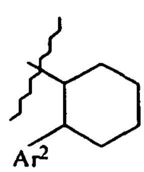 , 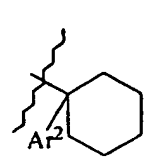 ,

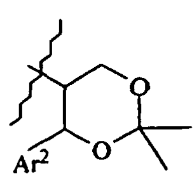

, or

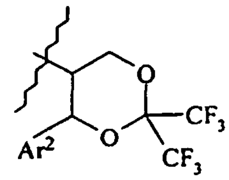

wherein p is 0, 1 or 2 and $Ar^2$ is phenyl or pyridyl;

- $R^3$, $R^4$ and $R^5$ are each independently selected from hydrogen and methyl; and
- X is selected from:

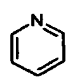
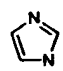
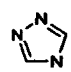
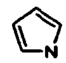
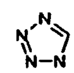
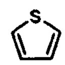

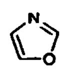
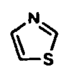
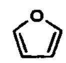
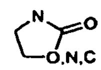
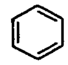
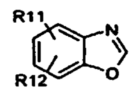

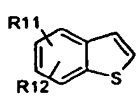
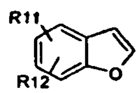
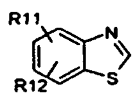
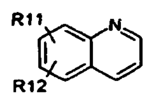
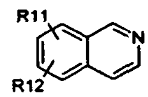

$R^{11}$ and $R^{12}$ being independently selected from H, halogen, hydroxy, alkoxy, acetyl, nitro, cyano, amino, $CF_3$ and $(CH_2)_tNR^{13}R^{14}$ wherein t is 0 or 1 and $R^{13}$ and $R^{14}$ are independently selected from hydrogen and methyl.

A sub-species of preferred compounds within the general formula **(II)** has the formula **(IIb)** or **(IIc):**

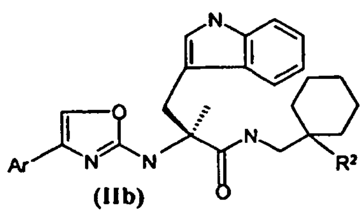

(IIb)

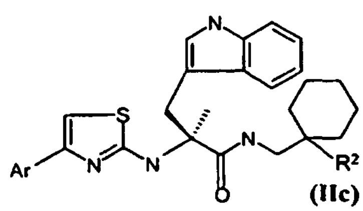

(IIc)

wherein Ar and $R^2$ independently represent phenyl or pyridyl which may be unsubstituted or substituted with from 1 to 3 substituents selected from halogen, alkoxy, nitro and cyano, and pharmaceutically acceptable salts thereof.

A particularly preferred compound falling within formula **(II)** is (S)-3-(1*H*-indol-3-yl)-*N*-[1-(5-methoxy-pyridin-2-yl)-cyclohexylmethyl]-2-methyl-2-[4-(4-nitrophenyl)-oxazol-2-ylamino]-propionamide (also referred to as Compound **2**) and its pharmaceutically acceptable salts.

Other preferred compounds falling within formula **(II)** are described below in Examples 10-27 and are included within the invention, as also are their pharmaceutically acceptable salts.

A third genus of bombesin receptor antagonists according to the invention has the formula **(III)** and include pharmaceutically acceptable salts thereof:

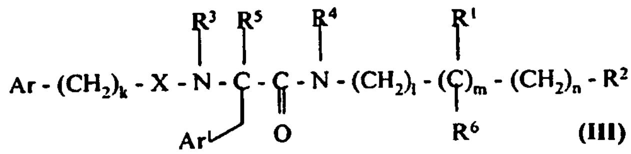

(III)

wherein:

- k is 0, 1 or 2;
- l is 0, 1, 2 or 3;
- m is 0 or 1;
- n is 0, 1 or 2;
- X is -CO-, -OCO, -SO- and $-SO_2-$;
- Ar is benzimidazolyl, benzofuryl, benzothiadiazolyl, benzothiazolyl, benzothienyl, benzopyrazinyl, benzotriazolyl, benzoxadiazolyl, furyl, imidazolyl, indanyl, indolyl, isoquinolyl, isoxazolyl, naphthyl, oxazolyl, phenyl, pyrazinyl, pyrazolyl, pyridyl, pyridazinyl, pyrimidyl, pyrrolyl, quinolinyl, tetralinyl, tetrazolyl, thiazolyl, thienyl or triazolyl each unsubstituted or substituted with from 1 to 3 substituents selected from amino, acetyl, alkyl (straight chain or branched with from 1 to 6 carbon atoms), alkoxy, cyano, halogen, hydroxy, nitro, phenyl, pyridyl, pyrrolyl, isoxazolyl, phenoxy, tolyloxy, $-CF_3$, $-OCF_3$, $-SO_2CF_3$, $-NHCONH_2$, $-CO_2H$, $-CH_2CO_2H$, $-CH_2CN$, $SO_2Me$, $SO_2NH_2$, $SO_2Ph$, $-(CH_2)_qNR^7R^8$, $-CONR^9R^{10}$, and $CO_2R^{11}$, wherein q is 0, 1 or 2 and $R^7$, $R^8$, $R^9$, $R^{10}$, $R^{11}$ are each independently selected from hydrogen or straight or branched alkyl of up to 6 carbon atoms or cyclic alkyl of between 5 to 7 atoms which may contain 1 or 2 oxygen or nitrogen atoms or $R^7$ and $R^8$ or $R^9$ and $R^{10}$ together with the nitrogen atom to which they are linked can form a 5- to 7-membered aliphatic ring which may contain 1 or 2 oxygen or nitrogen atoms;
- $Ar^1$ is independently selected from Ar and can also be pyridyl-*N*-oxide;
- $R^1$ is hydrogen or straight or branched alkyl of up to 6 carbon atoms or cyclic alkyl of between 5 and 7 atoms which may contain 1 or 2 oxygen or nitrogen atoms;
- $R^2$ is independently selected from Ar or is hydrogen, hydroxy, alkoxy, $-NMe_2$, $-CONR^{12}R^{13}$,

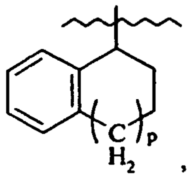 , 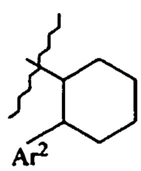 , 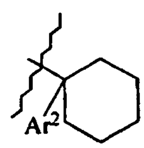 ,

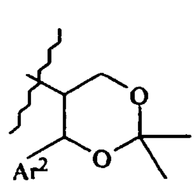 , or 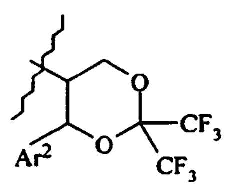 ,

wherein p is 0, 1 or 2, $Ar^2$ is phenyl or pyridyl; and, $R^{12}$ and $R^{13}$ are each independently selected from hydrogen, straight or branched alkyl of up to 6 carbon atoms or cyclic alkyl of between 5 and 7 carbon atoms;
- $R^3$, $R^4$ and $R^5$ are each independently selected from hydrogen and lower alkyl; and
- $R^6$ is hydrogen, methyl or forms with $R^1$ a ring of from 3 to 7 carbon atoms which can contain an oxygen or nitrogen atom, or $R^1$ and $R^6$ can together be carbonyl.

In a preferred group of the compounds of formula (III):

- k is 0 or 1;
- l is 1;
- m is 0 or 1;

- n is 0 or 1;
- X is -C(O)-, -OC(O)-, or $-SO_2-$;
- Ar is benzofuryl, furyl, indolyl, isoquinolyl, naphthyl, phenyl, pyridyl, quinolyl or thienyl each unsubstituted or substituted with 1 or 2 substituents selected from alkoxy, cyano, halogen, nitro, phenyl, phenoxy, $-CF_3$, $-(CH_2)_qNR^7R^8$, wherein $R^7$ and $R^8$ can form a ring of between 5 to 7 atoms which may contain 1 or 2 oxygen or nitrogen atoms, or $R^7$ and $R^8$ can be independently selected from hydrogen, straight or branched alkyl of up to 4 carbon atoms or cyclic alkyl of 5 carbon atoms;
- $Ar^1$ is independently selected from Ar, preferably indolyl, and can also be pyridyl-*N*-oxide;
- $R^1$ and $R^6$ can form a cyclic alkyl of from 5 to 7 carbon atoms or $R^1$ and $R^6$ together are carbonyl;
- $R^2$ is independently selected from unsubstituted or substituted pyridyl or is hydrogen, hydroxy, alkoxy, $-NMe_2$, $-CONR^{12}R^{13}$ wherein $R^{12}$ and $R^{13}$ are each independently selected from H and $CH_3$;
- $R^3$, $R^4$ and $R^5$ are each independently selected from hydrogen and methyl.

  In another preferred group of the compounds of Formula (**III**),

- l is 1;
- m is 1;
- n is 0;
- $R^2$ is 2-pyridyl;
- $R^6$ forms a cyclohexyl with $R^1$.

A particularly preferred group of compounds is of formula **(IIIa):**

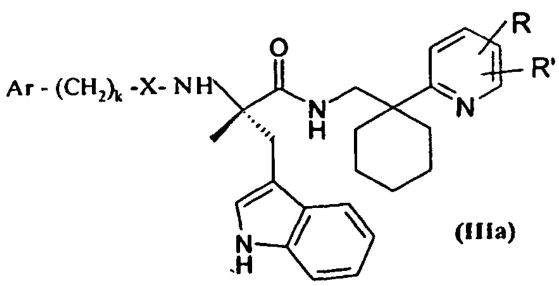

**(IIIa)**

wherein Ar, k and X have the meanings given above in first, and the pyridine ring is optionally substituted by with 1 or 2 substituents, R and R', independently selected from alkoxy, cyano, halogen, nitro, phenyl, phenoxy, $-CF_3$, $-(CH_2)_qNR^7R^8$, wherein $R^7$ and $R^8$ together with the nitrogen atom to which they are linked can form a 5- to 7-membered aliphatic ring which may contain 1 or 2 oxygen or nitrogen atoms, or $R^7$ and $R^8$ can be independently selected from hydrogen or cyclic alkyl of between 5 to 7 carbon atoms, and their pharmaceutically acceptable salts thereof.

In a further set of compounds (IIIa), Ar is benzofuryl, furyl, indolyl, isoquinolyl, naphthyl, phenyl, pyridyl, quinolyl or thienyl each unsubstituted or substituted with 1 or 2 substituents selected from alkoxy, cyano, halogen, nitro, phenyl, phenoxy, $-CF_3$, $-(CH_2)_qNR^7R^8$, wherein $R^7$ and $R^8$ can form a ring of between 5 to 7 atoms which may contain 1 or 2 oxygen or nitrogen atoms, or $R^7$ or $R^8$ can be a independently selected from hydrogen or cyclic alkyl of 5 carbon atoms, and X is - C(O)-, -OC(O)- or $-SO_2$.

**Preferred *N*-terminal amide derivatives of the compounds of formula (III)**

Amongst *N*-terminal amide derivatives, i.e. compounds of formula (III) wherein X is -C(O)-, the following compounds are most preferred:

*N*-{(S)-2-(1*H*-indol-3-yl)-1-methyl-1-[(1-pyridin-2-yl-cyclohexylmethyl)-carbamoyl]-ethyl}-4-nitro-benzamide;
C-dimethylamino-*N*-{(S)-2-(1*H*-indol-3-yl)-1-methyl-1-[(1-pyridin-2-yl-cyclohexylmethyl)-carbamoyl]-ethyl}-benzamide;
1*H*-indole-2-carboxylic acid {(S)-2-(1*H*-indol-3-yl)-1-methyl-1-[(1-pyridin-2-yl-cyclohexylmethyl)-carbamoyl]-ethyl}-amide;
benzo[b]thiophene-2-carboxylic acid {(S)-2-(1*H*-indol-3-yl)-1-methyl-1-[(1-pyridin-2-yl-cyclohexylmethyl)-carbamoyl]-ethyl}-amide;
N-{(S)-2-(1H-Indol-3-yl)-1-methyl-1-[(1-pyridin-2-yl-cyclohexylmethyl)-carbamoyl]-ethyl}-2-pyrrol-1-yl-benzamide
1*H*-indole-5-carboxylic acid {(S)-2-(1*H*-indol-3-yl)-1-methyl-1-[(1-pyridin-2-yl-cyclohexylmethyl)-carbamoyl]-ethyl}-amide; and
1*H*-indole-2-carboxylic acid ((S)-2-(1*H*-indol-3-yl)-1-{[1-(5-methoxy-pyridin-2-yl)-cyclohexylmethyl]-carbamoyl}-1-methyl-ethyl)-amide.

Other preferred *N*-terminal amide derivatives of formula (III) include the compounds of Examples 32-35, 37-47, 49-60, 62-80, 82-85 and *N*-{(S)-2-(1*H*-indol-3-yl)-1-methyl-1-[(1-pyridin-2-yl-cyclohexylmethyl)-carbamoyl]-ethyl}-2-pyrrol-1-yl-benzamide.

**Preferred *N*-terminal urethane derivatives of the compounds of formula (III)**

Amongst *N*-terminal urethane derivatives, i.e. compounds of formula III wherein X is -OC(=O)- , the following compounds and theor pharmaceutically acceptable salts are particularly preferred:

{(S)-2-(1H-Indol-3-yl)-1-methyl-1-[(1-pyridin-2-yl-cyclohexylmethyl)-carbamoyl]-ethyl}-carbamic acid naphthalen-1-ylmethyl ester;
{(S)-2-(1H-Indol-3-yl)-1-methyl-1-[(1-pyridin-2-yl-cyclohexylmethyl)-carbamoyl]-ethyl}-carbamic acid 3,4-dichloro-benzyl ester;
{(S)-2-(1H-Indol-3-yl)-1-methyl-1-[(1-pyridin-2-yl-cyclohexylmethyl)-carbamoyl]-ethyl}-carbamic acid 4-nitro-benzyl ester;
{(S)-2-(1H-Indol-3-yl)-1-methyl-1-[(1-pyridin-2-yl-cyclohexylmethyl)-carbamoyl]-ethyl}-carbamic acid 3-nitro-benzyl ester;
{(S)-2-(1H-Indol-3-yl)-1-methyl-1-[(1-pyridin-2-yl-cyclohexylmethyl)-carbamoyl]-ethyl}-carbamic acid 3-cyano-benzyl ester;
{(S)-2-(1H-Indol-3-yl)-1-methyl-1-[(1-pyridin-2-yl-cyclohexylmethyl)-carbamoyl]-ethyl}-carbamic acid 3-trifluoromethyl-benzyl ester;
{(S)-2-(1H-Indol-3-yl)-1-methyl-1-[(1-pyridin-2-yl-cyclohexylmethyl)-carbamoyl]-ethyl}-carbamic acid 2,3-dichloro-benzyl ester; and
{(S)-2-(1H-Indol-3-yl)-1-methyl-1-[(1-pyridin-2-yl-cyclohexylmethyl)-carbamoyl]-ethyl}-carbamic acid quinolin-6-ylmethyl ester.

Other preferred *N*-terminal urethane derivatives of formula (III) include the compounds of Examples 88-90, 92-95, 97-98, 100-102, 104-106 and 108.

**Preferred *N*-terminal sulfonamide derivatives of the compounds of formula (III)**

Amongst *N*-terminal sulfonamide derivatives of formula (III) (compounds of formula (III) wherein X is $-SO_2-$) the following compounds are particularly preferred:

(S)-3-(1*H*-indol-3-yl)-2-methyl-2-phenylmethanesulfonylamino-*N*-(1-pyridin-2-yl-cyclohexylmethyl)-propionamide;
(S)-2-(2-chloro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(naphthalene-1-sulfonylamino)-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(quinoline-8-sulfonylamino)-propionamide;
(S)-3-(1*H*-indol-3-yl)-2-methyl-*N*-(1-pyridin-2-yl-cyclohexylmethyl)-2-(2-trifluoromethyl-benzcnesulfonylamino)-propionamide;
(S)-2-(biphenyl-2-sulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(5-methyl-2-phenoxy-benzenesulfonylamino)-*N*-(1-pyridin-2-yl-cyclohexylmethyl)-propionamide; and
(S)-3-(1*H*-indol-3-yl)-2-methyl-*N*-(1-pyridin-2-yl-cyclohexylmethyl)-2-(2-*p*-tolyloxy-benzenesulfonylamino)-propionamide.

Further preferred *N*-terminal sulfonamide derivatives of formula (III) include the compounds of Examples 112, 114, 116-119, 121-128, 130-151, 155-168 and the following:

(S)-3-(1*H*-indol-3-yl)-2-methanesulfonylamino-2-methyl-*N*-(1-pyridin-2-yl-cyclohexylmethyl)-propionamide; and
(S)-3-(1*H*-indol-3-yl)-2-methyl-*N*-(1-pyridin-2-yl-cyclohexylmethyl)-2-(2,2,2-trifluoro-ethanesulfonylamino)-propionamide.

The compounds of the general formulae above are optically active. The scope of the invention therefore also includes:

- All stereoisomers of the compounds of the above general formulae.
- The solvates, hydrates and polymorphs (different crystalline lattice descriptors) of the above compounds.
- Pharmaceutical compositions of the above compounds.
- Prodrugs of the above compounds such as would occur to a person skilled in the art; see Bundgaard et al (1987).

The alkyl groups contemplated by the invention include straight, branched, or cyclic carbon chains of from 1 to 8 carbon atoms except where specifically stated otherwise. Representative groups are methyl ethyl, propyl, isopropyl, n-propyl, n-butyl, iso-butyl, sec-butyl, tert-butyl, 2-methylhexyl, n-pentyl, 1-methylbutyl, 2,2-dimethylbutyl, 2-methylpentyl, 2,2-dimethylpropyl, n-hexyl, and the like.

The lower alkyl groups include carbon chains of up to 6 carbon atoms. The cycloalkyl groups contemplated by the invention comprise those having 3 to 7 carbon atoms including cyclopentyl and cyclohexyl. They may be substituted with from 1 to 3 groups selected from halogens, nitro, alkyl, and alkoxy.

The alkoxy groups contemplated by the invention comprise both straight and branched carbon chains of from 1 to 6 carbon atoms unless otherwise stated. Representative groups are methoxy, ethoxy, propoxy, i-propoxy, t-butoxy, and hexoxy.

The term "halogen" is intended to include fluorine, chlorine, bromine, and iodine.

The term "amine" is intended to include free amino, alkylated amines, and acylated amines.

The term "subject" includes animals, particularly mammals and more particularly humans.

**Optical isomers and salts**

The compounds of the above general formulae all have at least one chiral centre and some have multiple chiral centres depending on their structure. In particular, the compounds of the present invention may exist as diastereoisomers, mixtures of diastereoisomers, or as the mixed or the individual optical enantiomers. The present invention contemplates all such forms of the compounds. The mixtures of diastereoisomers are typically obtained as a result of the reactions described more fully below. Individual diastereoisomers may be separated from mixtures of the diastereoisomers by conventional techniques such as column chromatography or repetitive recrystallization. Individual enantiomers may be separated by conventional methods well known in the art such as conversion to a salt with an optically active compound, followed by separation by chromatography or recrystallization and reconversion to the non-salt form.

Where it is appropriate to form a salt, the pharmaceutically acceptable salts include acetate, benzenesulfonate, benzoate, bicarbonate, bitartrate, bromide, calcium acetate, camsylate, carbonate, chloride, citrate, dihydrochloride, edetate, edisylate, estolate, esylate, fumarate, gluceptate, gluconate, glutamate, glycoloylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, mucate, napsylate, nitrate, pamoate (embonate), pantothenate, phosphate/diphosphate, polygalacturonate, salicylate, stearate, subacetate, succinate, sulfate, tannate, tartrate, theoclate, triethiodide, benzathine, chloroprocaine, choline, diethanolamine, ethylenediamine, meglumine, procaine, aluminum, calcium, lithium, magnesium, potassium, sodium, and zinc.

Preferred salts are made from strong acids. Such salts include hydrochloride, mesylate, and sulfate.

**Other non-peptide bombesin antagonists**

Other non-peptide bombesin antagonists which are believed to be suitable for use in the present invention are described and claimed in the following documents, the contents of which are incorporated herein by reference: WO 00/09115, WO 00/09116, WO 92/07830, JP 07258081 and WO 98/07718.

**Preparative methods for the compounds of formula (I)**

Preparation of the compounds of formula (I) is described in WO 98/07718, the disclosure of which is incorporated herein by reference.

Preparation of the closest reported analogues of Compound **3** - (2*S*)-*N*-{[1-(4-aminophenyl)cyclohexyl]methyl}-3-(1*H*-indol-3-yl)-2-methyl-2-{[(4-nitroanilino)-carbonyl]amino}propanamide are described in Ashwood, V. Brownhill, M. Higginbottom, D. C. Horwell, J. Hughes, R. A. Lewthwaite, A. T. McKnight, R. D. Pinnock, M. C. Pritchard, N. Suman-Chauhan, C. Webb and S. C. Williams. *Bioorg. Med. Chem. Lett.*, 1998, **8,** 2589-2594.; J. E. Eden, M. D. Hall,

D. C. Horwell, W. Howson, J. Hughes, R. E. Jordan, R. A. Lewthwaite, K. Martin, A. T. McKnight, J. O'Toole, R. D. Pinnock, M. C. Pritchard, N. Suman-Chauhan and S. C. Williams. *Bioorg. Med. Chem. Lett.*, 1996, **6**, 2617-2622. and in WO98/07718. Compound 3 can be synthesized using methods disclosed in the above publications.

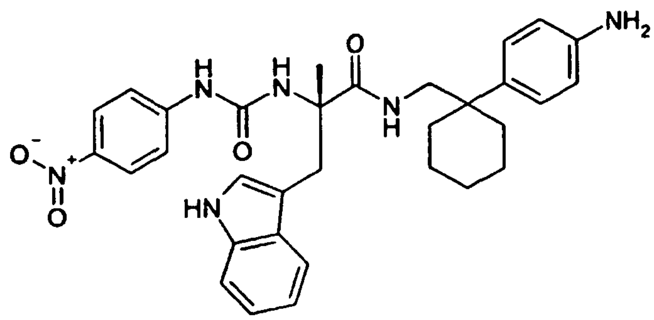

**Preparative methods for compounds of formula (II)**

Throughout this application the following abbreviations have the meanings listed below:

| | |
|---|---|
| $NEt_3$ | triethylamine |
| THF | tetrahydrofuran |
| HBTU | *O*-benzotriazol-1-yl-*N,N, N',N'*-tetramethyluronium hexafluorophosphate |
| DIPEA | *N,N*-diisopropylethylamine |
| DMF | *N,N*-dimethylformamide |
| TEBA | benzyltriethylammonium chloride |
| BOC2O | di-*tert*-butyl dicarbonate |
| TFA | trifluoroacetic acid |
| DMA | *N,N*-dimethylacetamide |
| EtOAc | ethyl acetate |
| MeOH | methanol |
| Trp | tryptophan |
| Ph | phenyl |
| HPLC | high pressure liquid chromatography |
| NP | normal phase |
| RP | reverse phase |
| DMAP | *N,N*-dimethyl-4-amino pyridine |
| OAc | acetate |
| OB | oestradiol benzoate |

The production of compounds of the formula **(II)** in which X is oxazolyl is shown in Scheme 1 which illustrates the synthesis of the compounds of **Examples 9** to 12 in four steps via Intermediates **4a** or **4b.** The steps are:

- Formation of the *p*-nitrophenylcarbamate of the methyl ester (Intermediate 1) and subsequent treatment with aqueous ammonia to give a primary urea (Intermediate **2).**
- Cyclisation of the primary urea with 2-bromo-1-(4-nitro-phenyl)-ethanone to form an oxazole ring (Intermediate **3).**
- Hydrolysis of the methyl-ester-protecting group gives Intermediates **4a** or **4b.**
- Reaction of Intermediate **4a** or **4b** with the amine **Z2,** using HBTU to form an amide linkage, affords the desired compounds.

**Scheme 1:**

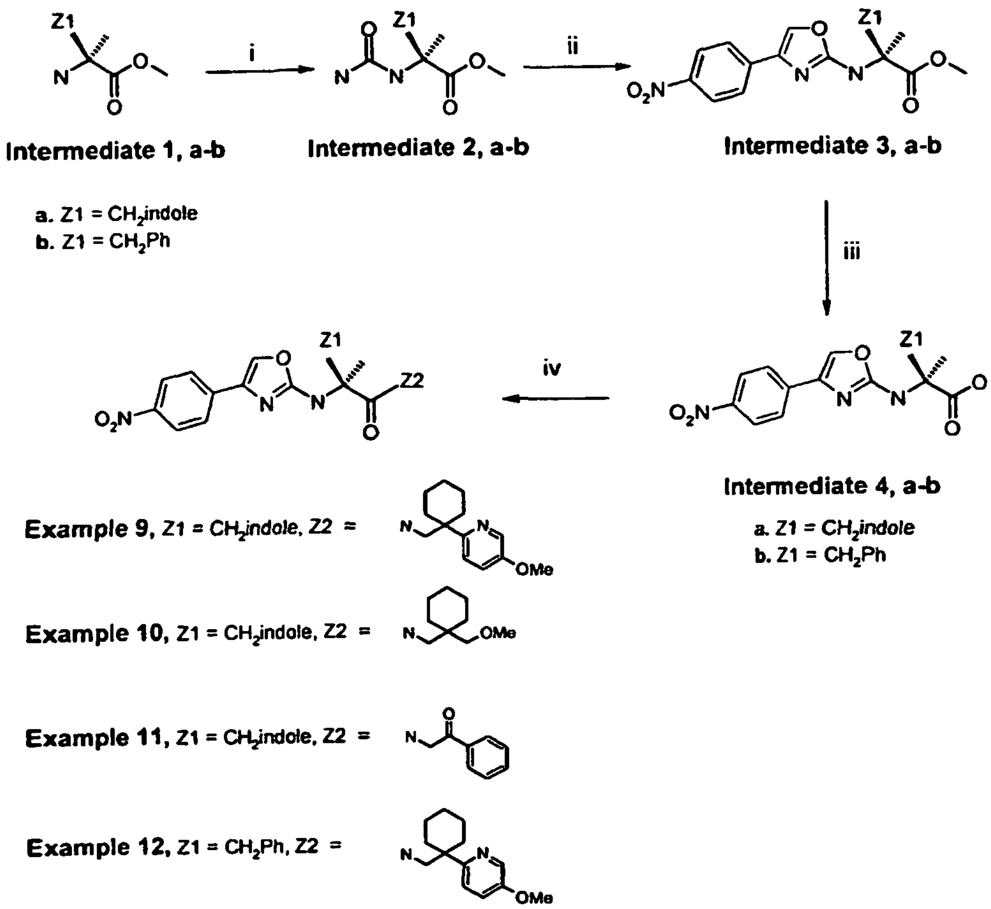


Intermediate 1, a-b

Intermediate 2, a-b

Intermediate 3, a-b

a. Z1 = $CH_2$indole
b. Z1 = $CH_2$Ph

Intermediate 4, a-b

a. Z1 = $CH_2$indole
b. Z1 = $CH_2$Ph

Example 9, Z1 = $CH_2$indole, Z2 =

Example 10, Z1 = $CH_2$indole, Z2 =

Example 11, Z1 = $CH_2$indole, Z2 =

Example 12, Z1 = $CH_2$Ph, Z2 =

In the above scheme:

i) a) 4-Nitrophenylchloroformate, $NEt_3$, THF b) $NH_3$ aq.
ii) 2-bromo-1-(4-nitro-phenyl)-ethanone in either toluene/dioxan at reflux (**3a**) or 1,2-dichloroethane at reflux (**3b**)
iii) LiOH, dioxan, $H_2O$
iv) HBTU, DIPEA, DMF, Z2

Scheme 2 describes the synthesis of the compounds of **Examples 13** to **15** from **intermediate 2a**.

- A primary urea **2a** is cyclised with an appropriate bromomethyl ketone containing the group Z3 to form an oxazole ring (Intermediate **5).**
- ; Hydrolysis of the methyl ester protecting group of the resulting Intermediate **5a, 5b** or **5c** gives the Intermediates **6 a-c.**
- ; Reaction of an Intermediate **6a, 6b** or **6c** with [1-(5-methoxy-2-pyridyl)cyclohexyl]methanamine in the presence of HBTU to form an amide bond affords the desired compounds.

## Scheme 2:

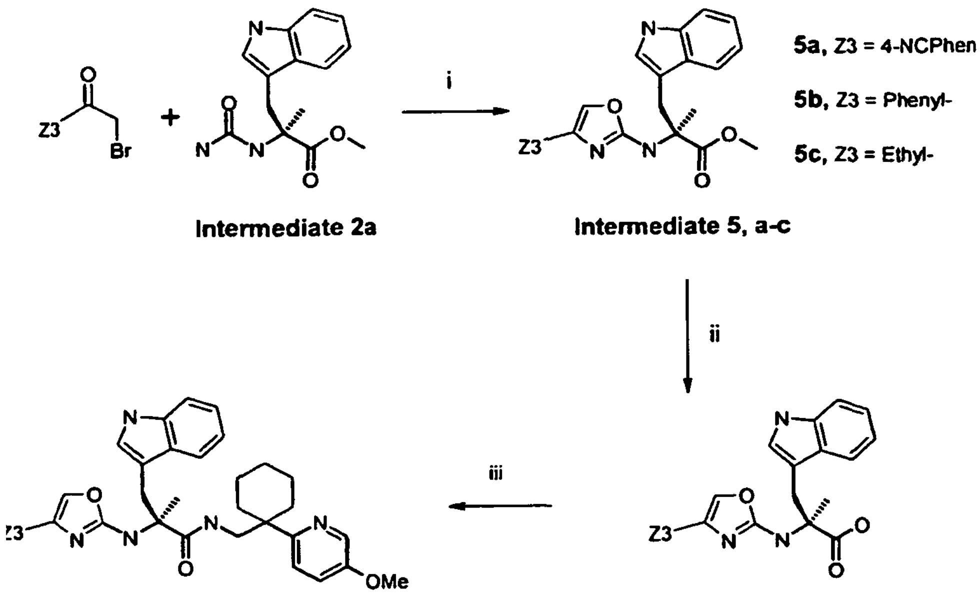


5a, Z3 = 4-NCPhen
5b, Z3 = Phenyl-
5c, Z3 = Ethyl-

Intermediate 2a

Intermediate 5, a-c

Intermediate 6, a-c

In the above scheme:

i) DMF at 30°C
ii) LiOH, dioxan, $H_2O$
iii) HBTU, DIPEA, DMF, [1-(5-methoxy-2-pyridyl)cyclohexyl]methanamine (described in WO 98/07718)

Scheme 3 describes a two step synthesis for the compounds of **Examples 16-23.** The reactions are preferentially carried out as a "one-pot" process in which:

- An aromatic ring of a compound Z5-Br or Z5-Cl is appended onto the N-terminal of the illustrated amino acid using a copper catalysed reaction.
- Formation of an amide linkage between the resulting acid and [1-(5-methoxy-2-pyridyl)cyclohexyl]methanamine or [1-(2-pyridyl)cyclohexyl]methylamine in the presence of HBTU affords the desired compounds.

## Scheme 3:

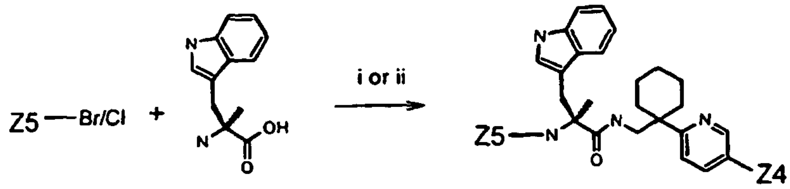


**Intermediate 7**

**Example 16** Z4=OMe Z5 = 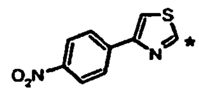

**Example 17** Z4=H Z5 = 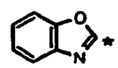

**Example 18** Z4=H Z5= 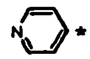

**Example 19** Z4=H Z5 = 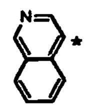

**Example 20** Z4=H Z5= 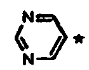

**Example 21** Z4=H Z5 = 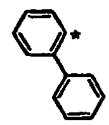

**Example 22** Z4=H Z5 = 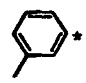

**Example 23** Z4=H Z5 = 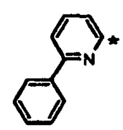

In the above scheme:

i)

a) 10% CuI, $K_2CO_3$, DMF, 130°C
b) HBTU, DIPEA, DMF, and [1-(5-methoxy-2-pyridyl)cyclohexyl]methanamine (described in WO 98/07718) or [1-(2-pyridyl)cyclohexyl]methylamine (described in WO 98/07718)

ii)

a) 5-10% CuI, $K_2CO_3$, TEBA, Pd(P(*o*-tolyl)$_3$)$Cl_2$, DMF, 130°C
b) HBTU, DIPEA, DMF, and [1-(5-methoxy-2-pyridyl)cyclohexyl]methanamine (described in WO 98/07718) or [1-(2-pyridyl)cyclohexyl]methylamine (described in WO 98/07718)

* represents the attachment point.

Scheme 4 describes the two step one-pot synthesis of the compound of **Example 24:**

- The aromatic ring is appended onto the N-terminal of the amino acid (Intermediate **8**) using a copper catalysed

reaction and then an in situ HBTU amide bond formation reaction affords the desired compound.

**Scheme 4:**

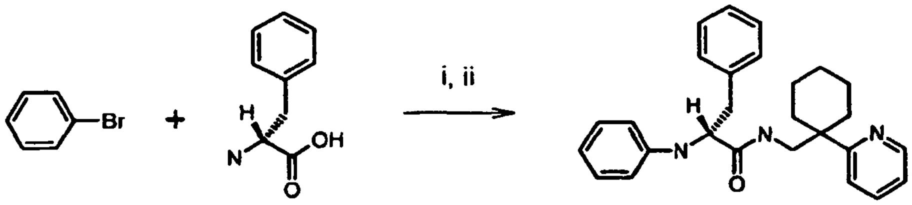


**Intermediate 8** **Example 24**

In the above scheme:

i) 10% CuI, $K_2CO_3$, DMA, 90°C
ii) HBTU, $NEt_3$, DMA, [1-(2-pyridyl)cyclohexyl]methylamine (described in WO 98/07718)

Scheme 5 describes the synthesis of the compounds of **Examples 25 - 27** via **Intermediate 10** by the steps of:

- N-BOC protection of the amino acid **(Intermediate 7**) which provides the groups $R^5$ and $Ar^1$.
- Reaction of the protected amino acid with an amine that provides the groups $R^1$, $R^2$, $R^4$ and $R^6$ using HBTU to form an amide linkage, and thereby give the **Intermediate 9.**
- N-BOC deprotection of the **Intermediate 9** to give **Intermediate 10.**
- Reductive amination of **Intermediate 10** with the appropriate aldehyde Z6CHO to give the desired compounds.

**Scheme 5:**

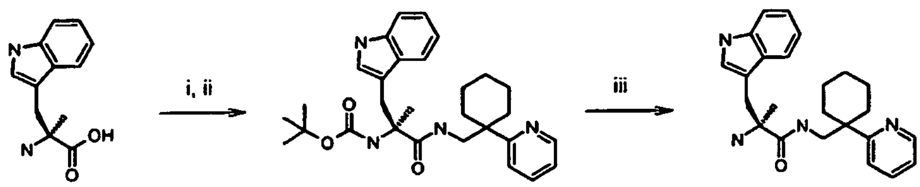


**Intermediate 7** **Intermediate 9** **Intermediate 10**

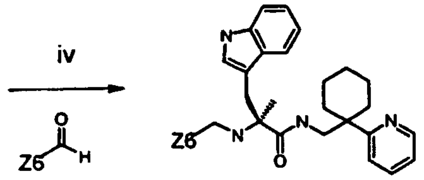


**Exemple 25, Z6=** 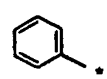

**Exemple 26, Z6=** 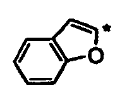

**Exemple 27, Z6=** 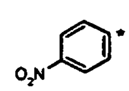

In the above scheme:

i) $BOC_2O$, $K_2CO_3$, dioxane, water

ii) HBTU, DIPEA, [1-(2-pyridyl)cyclohexyl]methylamine (described in WO 98/07718), DMF
iii) TFA, $CH_2Cl_2$
iv) $NaBH(OAc)_3$, 1,2-dichloroethane.

* represents the attachment point.

Scheme 6 describes the synthesis of **Intermediate 13.**

- The alcohol **11** is methylated using sodium hydride.
- The resulting nitrile is reduced using Raney nickel under an atmosphere of hydrogen.

**Scheme 6:**

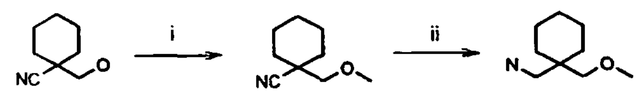


**Intermediate 11** **Intermediate 12** **Intermediate 13**

In the above scheme:

i) NaH, $CH_3I$, THF
ii) Raney nickel, ethanolic ammonia, $H_2$, 345 kPa

**Intermediate 13**

***C*-(1-methoxymethyl-cyclohexyl)-methylamine**

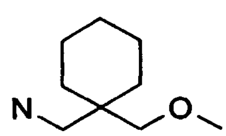

**Intermediate 13**

The above compound was prepared as shown in Scheme 6.

1. Sodium hydride (862mg, 21.5mmol, 60% in oil) was taken up in THF (50ml) under argon at 0°C. To this was added a solution of methyl iodide (1.34ml, 21.6mmol) and 1-hydroxy-cyclohexanecarbonitrile (1.0g, 7.18mmol; see J. Fröhlich *et al*., 1994) in THF (30ml) dropwise over 45 minutes. Once addition was complete the reaction mixture was stirred at room temperature overnight, and then quenched with *i*-propanol followed by water (100ml). The mixture was then extracted with dichloromethane (2x 150ml). The combined organic phases were dried ($MgSO_4$) and solvent removed under reduced pressure. Residue was purified by chromatography using heptane/ ethyl acetate (4:1). Removal of solvent under reduced pressure gave 1-methoxymethyl-cyclohexanecarbonitrile (1.1g, 88%) as a pale yellow oil.
IR (film): 2934, 2861, 2832, 2235, 1476, 1452, 1385, 1211, 1187, 1185, 1126, 1102, 978, 932, 901, 849 $cm^{-1}$;
$^1H$ NMR ($CDCl_3$): $\delta$ = 1.13-1.33 (3H, m), 1.57-1.78 (5H, m), 1.94-2.02 (2H, m), 3.36 (1H, s), 3.42 (3H, s);

2. To the 1-methoxymethyl-cyclohexanecarbonitrile (1.1g, 7.2mmol) in ethanolic ammonia (60ml) was added Raney nickel catalyst (0.55g, pre-washed with water and ethanol). Reaction mixture was shaken for 16 hours under hydrogen (345 kPa) at 30°C. The catalyst was filtered off catalyst with extreme caution through a bed of

Kieselguhr and washed with ethanol. Removal of the solvent under reduced pressure gave **Intermediate 13** (1.12g, 99%) as a yellow oil.

MS m/e (ES+): 158.2 ($M^+$ + H, 100%);

IR (film): 2926, 2857, 1572, 1452, 1378, 1316, 1190, 1140, 966 $cm^{-1}$;

$^1$H NMR ($CDCl_3$): $\delta$ = 1.20-1.60 (12H, m), 2.62 (2H, s), 3.23 (2H, s), 3.32 (3H, s)

**Preparative methods for compounds of formula (III)**

Compounds of the formula (III) in which X is -CO- can be prepared by condensing an acid of the formula (III-1)

$$Ar - (CH_2)_k\text{-}COOH \qquad \textbf{(III-1)}$$

or a derivative thereof with an amine of the formula (III-2)

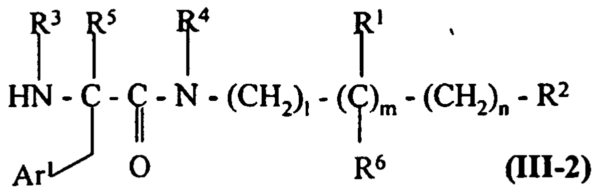

**(III-2)**

in an aprotic polar solvent in the presence of an appropriate catalyst, the values of the substituents Ar, $Ar^1$ and $R^1$ to $R^6$ and the parameters k to n being as defined above with reference to formula (III), and optionally converting the resulting product to a pharmaceutically acceptable salt. For example, the condensation may be carried out in dimethylformamide using *O*-benzotriazol-1-yl-*N,N,N',N'*-tetramethyluronium hexafluorophosphate (HBTU) and *N,N*-diisopropyl-ethylamine (DIPEA) as catalyst.

Compounds of the formula (III) in which X is -OC(=O)- can be prepared by forming a carbonate from an alcohol of the formula (III-3)

$$Ar - (CH_2)_k\text{-}OH \qquad \textbf{(III-3)}$$

and reacting the carbonate with an amine of the formula (III-2)

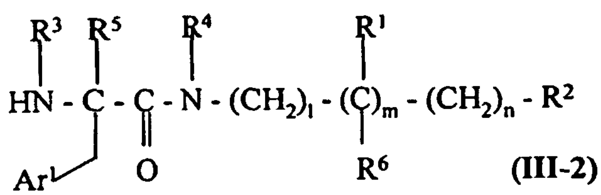

**(III-2)**

in an aprotic polar solvent in the presence of a base, the values of the substituents Ar, $Ar^1$ and $R^1$ to $R^6$ and the parameters k to n being as defined above with reference to formula (III), and optionally converting the resulting product to a pharmaceutically acceptable salt. For example, the compound of formula (III-3) may be reacted with 4-nitrophenyl chloroformate in dichloromethane using pyridine as catalyst, and the resulting carbonate may be reacted with the amine of formula (III-2) in dimethyl formamide using *N,N*-dimethyl-4-amino pyridine as catalyst.

Compounds of the formula (III) in which X is $-SO_2-$ can be prepared by condensing a sulfonyl chloride of the formula (III-4)

$$Ar - (CH_2)_k\text{-}SO_2Cl \qquad \textbf{(III-4)}$$

with an amine of the formula (III-2)

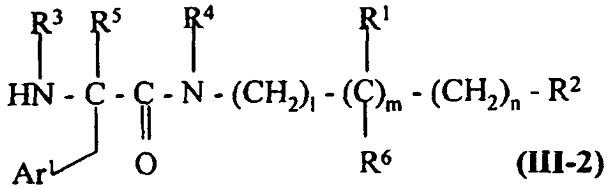

(III-2)

in an aprotic polar solvent in the presence of a base as catalyst, the values of the substituents Ar, $Ar^1$ and $R^1$ to $R^6$ and the parameters k to n being as defined above with reference to formula (III), and optionally converting the resulting product to a pharmaceutically acceptable salt. For example, the condensation may be carried out in dimethylformamide in the presence of *N,N*-diisopropylethylamine and *N,N*-dimethyl-4-aminopyridine.

In the above methods, the amine of formula (III-2) is preferably a chiral amine of formula (III-5)

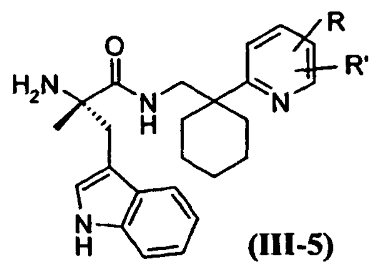

(III-5)

wherein the pyridine ring is optionally substituted by with 1 or 2 substituents R and R' selected from alkoxy, cyano, halogen, nitro, phenyl, phenoxy, - $CF_3$, - $(CH_2)_qNR^7R^8$, wherein $R^7$ and $R^8$ can form a ring of between 5 to 7 atoms, which may contain 1 or 2 oxygen or nitrogen atoms, or $R^7$ and $R^8$ can be independently selected from hydrogen or cyclic alkyl of from 1 to 5 carbon atoms, methoxy being a particularly preferred substituent, as in the chiral amine (III-6):

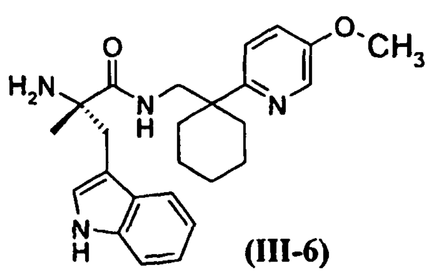

(III-6)

**B) Peptide bombesin receptor antagonists**

Bombesin antagonists which are peptides and which are believed to be suitable for use in the present invention are described in the following documents, the contents of which are incorporated herein by reference:

| Publication number | Publication number |
|---|---|
| WO 97/09347 | EP 0835662 |
| US 5650395 | US 5439884 |
| WO 96/28214 | WO 95/00542 |
| EP 0737691 | US 5620955 |
| US 5767236 | WO 92/02545 |
| WO 91/04040 | EP 0468497 |

(continued)

| Publication number | Publication number |
|---|---|
| EP 0309297 | CA 2030212 |
| EP 0438519 | WO 92/20707 |
| EP 0559756 | WO 93/16105 |
| WO 89/02897 | US 4943561 |
| WO 90/03980 | US 5019647 |
| WO 91/02746 | US 5028692 |
| WO 92/09626 | US 5047502 |
| WO 92/20363 | WO 94/02018 |
| WO 94/02163 | WO 88/07551 |
| WO 94/21674 | WO 89/09232 |
| WO 96/17617 | EP 0315367 |
| US 5084555 | EP 0345990 |
| US 5162497 | US 5068222 |
| US 5244883 | US 5620959 |
| US 5723578 | UK 2231051 |
| US 5750646 | EP 0339193 |
| US 5877277 | WO 90/01037 |
| US 5985834 | WO 91/06563 |
| EP 0428700 | EP 0402852 |

**Pharmaceutical compositions**

For preparing pharmaceutical compositions from the compounds of this invention, inert, pharmaceutically acceptable carriers can be either solid or liquid. Solid form preparations include powders, tablets, dispersible granules, capsules, sachets, and suppositories.

A solid carrier can be one or more substances which may also act as diluents, flavouring agents, solubilizers, lubricants, suspending agents, binders, or tablet disintegrating agents; it can also be an encapsulating material. In powders, the carrier is a finely divided solid which is in a mixture with the finely divided active component. In tablets, the active component is mixed with the carrier having the necessary binding properties in suitable proportions and compacted in the shape and size desired. The powders and tablets preferably contain 5% to about 70% of the active component. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Liquid form preparations include solutions, suspensions, and emulsions. Sterile water or water-propylene glycol solutions of the active compounds may be mentioned as an example of liquid preparations suitable for parenteral administration. Liquid preparations can also be formulated in solution in aqueous polyethylene glycol solution. Aqueous solutions for oral administration can be prepared by dissolving the active component in water and adding suitable colorants, flavouring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Preferably the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, sachet, or tablet itself, or it can be the appropriate number of any of these packaged forms.

For preparing suppository preparations, a low-melting wax such as a mixture of fatty acid glycerides and

cocoa butter is first melted and the active ingredient is dispersed therein by, for example, stirring. The molten homogeneous mixture is then poured into convenient sized moulds and allowed to cool and solidify.

Compositions that are adapted for oral administration to humans are preferred, especially such compositions in unit dosage form.

**Combination therapy**

Without wishing to be bound by any particular theory or teaching, the inventors believe that bombesin receptor antagonists could be used as part of a medicament in combination with one or more vasodilator, hormone therapy or neurotransmitter modulator. Such products are used or tested in the treatment of sexual dysfunction.

Vasodilators for the treatment of sexual dysfunctions of organic (rather than psychogenic) origin, act at the penis, clitoris or vagina level on local blood flow or lubricant secretions. Vasodilators useful for the treatment of sexual dysfunction include alprostadil or phentolamine, NO (nitric oxide) enhancers such as L-arginine, and PDE5 inhibitors such as sildenafil or a pharmaceutically acceptable salt thereof (*Scrip's Complete Guide to Women's Healthcare,* p. 194-205, 2000)(Sachs B.D., 2000, Benet and Melman, 1995), VIP (Vaso Intestinal Peptide) enhancers (*Scrip's Complete Guide to Women's Healthcare,* p.194-205, 2000) or angiotensin-2 receptor antagonists such as losartan (American Heart Association meeting, New Orleans, 2000).

Hormone therapies useful in the treatment of sexual dysfunction of organic and psychogenic nature include modulators of steroid hormones, steroid hormones or hormone product (including synthetic hormones) including oestrogen (*Scrip's Complete Guide to Women's Healthcare,* p.194-205, 2000), or androgens such as testosterone (*Scrip's Complete Guide to Women's Healthcare,* p.194-205, 2000, Sachs B.D., 2000), which act in areas of the CNS associated with sexual desire and sexual arousal (Wilson CA., 1993).

Neurotransmitter modulators useful in the treatment of both psychogenic and organic sexual dysfunction include neurotransmitter agonists and antagonists such as catecholamine agonists such as the $D_2$ agonist quinelorane, $5HT_2$ antagonists such as ritanserin, monoamine synthesis modifiers such as treatments that reduce endogenous 5HT activity, including inhibition of 5HT synthesis using para-chlorophenylalanine, monoamine metabolism or uptake modifiers that inhibit catecholamine metabolism or reuptake, such as tricyclic antidepressants, e.g. imipramine (Wilson CA., 1993).

The use of this combination therapy includes the preparation of therapies that would allow administration of both components of the medicament, i.e. bombesin receptor antagonists and a vasodilator, hormone therapy medicament or neurotransmitter modulator medicament in a single dose. A preferred formulation would allow oral administration. However, administration by suppository, cream, transdermal patch or injection is also part of this invention. Alternatively the inventors envisage formulations that allow administration of the bombesin receptor antagonist via a separate route to that of the vasodilator, hormone therapy medicament or neurotransmitter modulator medicament. Such routes could include for example oral administration of the bombesin receptor antagonist and transdermal patch application of the vasodilator. Thus there may be provided a kit in which unit doses of bombesin receptor antagonist occur in association with unit doses of the vasodilator, hormone therapy medicament or neurotransmitter modulator medicament. For example, in the case of a kit where bombesin receptor antagonist is formulated as a tablet capsule or other unit dosage form for oral administration and the vasodilator is provided as a transdermal patch, the two dosage forms could be provided in the form of a two-row tear-off strip in which compartments containing the tablets, etc. occur above compartments containing the transdermal patches. Other forms of packaging in which the two dosage forms are spatially associated so as to make it easy for patients to take them together and to be reminded when they have done so will readily occur to those skilled in the art. The kit will also contain instructions as to when and how the individual components of the kit should be administered.

More generally, the invention provides the use in the preparation of a medicament of a pharmaceutical combination (for simultaneous, separate or sequential administration) of a bombesin receptor antagonist and one or more materials selected from (1) to (33) below:

(1) One or more naturally occurring or synthetic prostaglandins or esters thereof. Suitable prostaglandins for use herein include compounds such as alprostadil, prostaglandin $E_1$, prostaglandin $E_0$, 13, 14 - dihydroprostaglandin $E_1$, prostaglandin $E_2$, eprostinol, natural synthetic and semi-synthetic prostaglandins and derivatives thereof including those described in WO-00033825 and/or US 6,037,346 issued on 14th March 2000 all incorporated herein by reference, $PGE_0$, $PGE_1$, $PGA_1$, $PGB_1$, $PGF_1$ $\alpha$, 19-hydroxy $PGA_1$, 19-hydroxy - $PGB_1$, $PGE_2$, $PGB_2$, 19-hydroxy-$PGA_2$, 19-hydroxy-$PGB_2$, $PGE_3\alpha$, carboprost, tromethamine, dinoprost, dinoprostone, iloprost, gemeprost, metenoprost, sulprostune, tiaprost and moxisylate.

(2) One or more $\alpha$ - adrenergic receptor antagonist compounds also known as $\alpha$ - adrenoceptor antagonists or $\alpha$-receptor antagonists or $\alpha$-blockers. Suitable compounds for use herein include: the $\alpha$-adrenergic receptor blockers

as described in PCT application WO99/30697 published on 14th June 1998, the disclosure of which relating to α-adrenergic receptors incorporated herein by reference and include, selective $\alpha_1$-adrenoceptor or $\alpha_2$-adrenoceptor blockers and non-selective adrenoceptor blockers, Suitable $\alpha_1$-adrenoceptor blockers include: phentolamine, phentolamine mesylate, trazodone, alfuzosin, indoramin, naftopidil, tamsulosin, dapiprazole, phenoxybenzamine, idazoxan, efarxan, yohimbine, rauwolfa alkaloids, Recordati 15/2739, SNAP 1069, SNAP 5089, RS17053, SL 89.0591, doxazosin, terazosin, abanoquil and prazosin; $\alpha_2$-blocker blockers from US 6,037,346 [14th March 2000] dibenarnine, tolazbline, trimazosin and dibenamine; α-adrenergic receptor antagonists as described in US patents: 4,188,390; 4,026,894; 3,511,836; 4,315,007; 3,527,761; 3,997,666; 2,503,059; 4,703,063; 3,381,009; 4,252,721 and 2,599,000 each of which is incorporated herein by reference: $\alpha_2$-Adrenoceptor blockers include; clonidine, papaverine, papaverine hydrochloride, optionally in the presence of a cardiotonic agent such as pirxamine.

(3) One or more NO-donor (NO-agonist) compounds. Suitable NO-donor compounds for use herein include organic nitrates, such as mono- di or tri-nitrates or organic nitrate esters including glyceryl tririnitrate (also known as nitroglycerin), isosorbide 5-mononitrate, isosorbide dinitrate, pentaerythritol tetranitrate, erythrityl tetranitrate, sodium nitroprusside (SNP), 3-morpholinosydnonimine, molsidomine, S-nitroso- N-acetyl penicillamine (SNAP) S-nitroso-N-glutathione (SNO-GLU), N-hydroxy - L-arginine, amylnitrate, linsidomine, linsidomine hydrochloride, (SIN-1) S-nitroso - N-cysteine, diazenium diolates,(NONOates), 1,5-pentanedinitrate, L-arginine, ginseng, zizphi fructus, molsidomine, Re - 2047, nitrosylatcd maxisylyte derivatives such as NMI-678-11 and NMI-937 as described in published PCT application WO 0012075 ; and/or

(4) One or more potassium channel openers or modulators. Suitable potassium channel openers/modulators for use herein include nicorandil, cromokalim, leveromakalim (lemakalim), pinacidil, diazoxide, minoxidil, charybdotoxin, glyburide, 4-aminopyridine, $BaCl_2$.

(5) One or more dopaminergic agents, preferably apomorphine or a selective D2, D3 or D2/$D_3$agonist such as pramipexole and ropirinol (as claimed in WO-0023056),PNU95666 (as claimed in WO-0040226).

(6) One or more vasodilator agents. Suitable vasodilator agents for use herein include nimodepine, pinacidil, cyclandelate, isoxsuprine, chloropromazine, halo pcridol, Rec 15/2739, trazodonc.

(7) One or more thromboxane A2 agonists.

(8) One or more ergot alkaloids. Suitable ergot alkaloids are described in US patent 6,037,346 issued on 14th March 2000 and include acetergamine, brazergoline, bromerguride, cianergoline, delorgotrile, disulergine, ergonovine maleate, ergotamine tartrate, etisulergine, lergotrile, lysergide, mesulergine, metergoline, metergotamine, nicergoline, pergolide, propisergide, proterguride, terguride.

(9) One or more compounds which modulate the action of natriuretic factors in particular atrial natriuretic factor (also known as atrial natriuretic paptide), B type and C type natriuretic factors.

(10) One or more angiotensin receptor antagonists such as losartan.

(11) One or more substrates for NO-synthase, such as L-arginine.

(12) One or more calcium channel blockers such as amlodipine.

(13) One or more cholesterol lowering agents such as statins (e.g. atorvastatin/Lipitor- trade mark) and fibrates.

(14) One or more antiplatelet and antithrombotic agents, e.g. tPA, uPA, warfarin, hirudin and other thrombin inhibitors, heparin, thromboplastin activating factor inhibitors.

(15) One or more insulin sensitising agents such as triglitazone (rezulin) and hypoglycaemic agents such as glipizide.

(16) L-DOPA or carbidopa.

(17) One or more acetylcholinesterase inhibitors such as donepezil (Aricept).

(18) One or more steroidal or non-steroidal anti-inflammatory agents.

(19) One or more estrogen receptor modulators (SERM) and/or estrogen agonists and/or estrogen antagonists, preferably raloxifene or lasofoxifene, (-)-cis-6-phenyl-5-[4-(2-pyrrolidin-1-yl-ethoxy)-phenyl]-5,6,7,8-tetrahydronaphthalene-2-ol and pharmaceutically acceptable salts thereof (compound A below) the preparation of which is detailed in WO 96/21656.

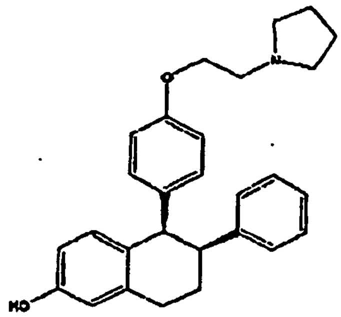

**Compound A**

(20) One or more PDE inhibitors, more particularly a PDE 2,5,7 or 8 inhibitor (for oral or local administration), preferably PDE2 or PDE5 inhibitor and most preferably a PDE5 inhibitor (see hereinafter), said inhibitors preferably having an IC50 against the respective enzyme of less than 100nM; and PDE 3 , 4 inhibitor for local administation (e.g. intracavemosal injection).

(21) In the case where the combination is for the treatment or prophylaxis of female sexual dysfunction, one or more of an NPY (neuropeptide Y) inhibitor, more panicularly NPY1 or NPY5 inhibitor, preferably NPY1 inhibitor. Preferably said NPY inhibitors (including NPYY1 and NPYY5) have an IC50 of less than 100 nM, more preferably less than 50 nM.

(22) One or more of a neutral endopeptidase (NEP) inhibitor preferably having an IC50 for NEP of less than 100nM. Preferably the NEP inhibitor is selective for NEP and has a selectivity over the endothelin converitin enzyme (ECE) and angiotensin converting enzyme (ACE) of greater than 100. However, mixed/dual NEP/ECE and NEP/ACE inhibitors (such as ompatrilat) are still within the scope of the invention.

(23) One or more of vasoactive intestinal protein (VIP), VIP mimetic, VIP analogue, more particularly acting through one or more of the VIP receptor subtypes VPAC1,VPAC or PACAP (pituitory adenylate cyclase activating peptide), one or more of a VIP receptor agonist or a VIP analogue (eg Ro-125-1553) or a VIP fragment, one or more of a $\alpha$-adrenoceptor antagonist with VIP combination (eg Invicorp, Aviptadil).

(24) One or more of a melanocortin receptor agonist or modulator or melanocortin enhancer, such as melanotan II, PT-14, PT-141 or compounds claimed in WO-09964002, WO-00074679, WO-09955679, WO-00105401, WO-00059361, WO-00114879, WO-00113112, WO-09954358.

(25) One or more of a serotonin receptor agonist, antagonist or modulator, more particularly agonists, antagonists or modulators for 5HT1A (including VML 670), 5HT2A, 5HT2C, 5HT3 and/or 5HT6 receptors, including those described in WO-09902159, WO-00002550 and/or WO-00028993..

(26) One or more of a testosterone replacement agent (including dehydroandrostendione), testosterone (Tostrelle), dihydrotestosterone or a testosterone implant.

(27) One or more of estrogen, estrogen and medroxyprogesterone or medroxyprogesterone acetate (MPA) (i.e. as a combination), or estrogen and methyl testosterone hormone replacement therapy agent (e.g. HRT especially Premarin, Cenestin, Oestrofeminal, Equin, Estrace, Estrofem, Elleste Solo, Estring, Eastraderm TTS, Eastraderm Matrix, Dermestril, Premphase, Preempro, Prempak, Premique, Estratest, Estratest HS, Tibolone).

(28) One or more of a modulator of transporters for noradrenaline, dopamine and/or serotonin, such as bupropion,

GW-320659.

(29) One or more of a purinergic receptor agonist and/or modulator.

(30) One or more of a neurokinin (NK) receptor antagonist, including those described in WO-09964008.

(31) One or more of an opioid receptor agonist, antagonist or modulator, preferably agonists for the ORL-1 receptor.

(32) One or more of an agonist or modulator for oxytocin/vasopressin receptors, preferably a selective oxytocin agonist or modulator.

(33) One or more modulators of cannabinoid receptors.

**Auxiliary Agent PDE5 inhibitor (I:PDE5) :**

**PDE5 Inhibitors**

Suitable PDE5i's for use in the pharmaceutical combinations according to the present invention are the cGMP PDE5i's hereinafter detailed. Particularly preferred for use herein are potent and selective cGMP PDE5i's. Suitable cGMP PDE5 inhibitors for the use according to the present invention include:

- pyrazolo [4,3-d]pyrimidin-7-ones disclosed in EP-A-0463756;
- pyrazolo [4,3-d]pyrimidin-7-ones disclosed in EP-A-0526004;
- pyrazolo [4,3-d]pyrimidin-7-ones disclosed in published international patent application WO 93/06104;
- isomeric pyrazolo [3,4-d]pyrimidin-4-ones disclosed in published international patent application WO 93/07149;
- quinazolin-4-ones disclosed in published international patent application WO 93/12095;
- pyrido [3,2-d]pyrimidin-4-ones disclosed in published international patent application WO 94/05661;
- purin-6-ones disclosed in published international patent application WO 94/00453;
- pyrazolo [4,3-d]pyrimidin-7-ones disclosed in published international patent application WO 98/49166;
- pyrazolo [4,3-d]pyrimidin-7-ones disclosed in published international patent application WO 99/54333;
- pyrazolo [4,3-d]pyrimidin-4-ones disclosed in EP-A-0995751;
- pyrazolo [4,3-d]pyrimidin-7-ones disclosed in published international patent application WO 00/24745;
- pyrazolo [4,3-d]pyrimidin-4-ones disclosed in EP-A-0995750;
- the compounds disclosed in published international application WO95/19978;
- the compounds disclosed in published international application WO 99/24433 and
- the compounds disclosed in published international application WO 93/07124.

It is to be understood that the contents of the above published patent applications, and in particular the general formulae and exemplified compounds therein are incorporated herein in their entirety by reference thereto.

Preferred type V phosphodiesterase inhibitors for the use according to the present invention include:

5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);
5-(2-ethoxy-5-morpholinoacetylphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo [4,3-d]pyrimidin-7-one (see WO98/49166);
3-ethyl-5-[5-(4-ethylpiperazin-1-ylsulphonyl)-2-(2-meihoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);
5-[2-ethoxy-5-(4-ethylpiperazin-1-ylsulphonyl)pyridin-3-yl]-3-ethyl-2-[2-methoxyethyl]-2,6-dihydro-7H-pyrazolo [4,3-d]pyrimidin-7-one, also known as 1-{6-ethoxy-5-[3-ethyl-6,7-dihydro-2-(2-methoxyethyl)-7-oxo-2H-pyrazolo [4,3-d]pyrimidin-5-yl]-3-pyridylsulphonyl}-4-ethylpiperazine (see WO 01/27113, Example 8);
5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see WO 01/27113, Example 15);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO 01/27113, Example 66);
5-(5-Acetyl-2-propoxy-3-pyridinyl)-3-ethyl-2-(1-isopropyl-3-azetidinyl)-2,6-dihydro-7*H*-pyrazolo[4,3-*d*]pyrimidin-7-one (seeWO 01/27112, Example 124);
5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one (see WO 01/27112, Example 132);
(6R,12aR)-2,3,6,7,12,12a-hexahydro-2-methyl-6-(3,4-methylenedioxyphenyl) -pyrazino[2',1':6,1]pyrido[3,4-b]indole-1,4-dione (IC-351), i.e. the compound of examples 78 and 95 of published international application WO95/19978, as well as the compound of examples 1, 3, 7 and 8;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e. the compound of examples 20, 19, 337 and 336 of published international application WO99/24433; and
the compound of example 11 of published international application WO93/07124 (EISAI); and
compounds 3 and 14 from Rotella D P, *J. Med. Chem.,* 2000, 43, 1257.

Still other type cGMP PDE5 inhibitors useful in conjunction with the present invention include:

4-bromo-5-(pyridylmethylamino)-6-[3-(4-chlorophenyl)-propoxy]-3(2H)pyridazinone;
1-[4-[(1,3-benzodioxol-5-ylmethyl)amiono]-6-chloro-2-quinozolinyl]-4-piperidine-carboxylic acid, monosodium salt;
(+)-cis-5,6a,7,9,9,9a-hexahydro-2-[4-(trifluoromethyl)-phenylmethyl-5-methyl-cyclopent-4,5]imidazo[2,1-b]purin-4(3H)one;
furazlocillin;
cis-2-hexyl-5-methyl-3,4,5,6a,7,8,9,9a- octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate;
4-bromo-5-(3-pyridylmethylamino)-6-(3-(4-chlorophenyl) propoxy)-3-(2H)pyridazinone;
1-methyl-5(5-morpholinoacetyl-2-n-propoxyphenyl)-3-n-propyl-1,6-dihydro-7H-pyrazolo(4,3-d)pyrimidin-7-one;
1-[4-[(1,3-benzodioxol-5-ylmethyl)arnino]-6-chloro-2- quinazolinyl]-4-piperidinecarboxylic acid, monosodium salt;
Pharmaprojects No. 4516 (Glaxo Wellcome);
Pharmaprojects No. 5051 (Bayer);
Pharmaprojects No. 5064 (Kyowa Hakko; see WO 96/26940);
Pharmaprojects No. 5069 (Schering Plough);
GF-196960 (Glaxo Wellcome);
E-8010 and E-4010 (Eisai); Bay-38-3045 & 38-9456 (Bayer); and
Sch-51866.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice.

Preferably, the cGMP PDE5 inhibitors have an $IC_{50}$ at less than 100 nanomolar, more preferably, at less than 50 nanomolar, more preferably still at less than 10 nanomolar. $IC_{50}$ values for the cGMP PDE5 inhibitors may be determined using the PDE5 assay in the Test Methods Section hereinafter.

Preferably the cGMP PDE5 inhibitors used in the pharmaceutical combinations according to the present invention are selective for the PDE5 enzyme. Preferably they are selective over PDE3, more preferably over PDE3 and PDE4. Preferably, the cGMP PDE5 inhibitors of the invention have a selectivity ratio greater than 100 more preferably greater than 300, over PDE3 and more preferably over PDE3 and PDE4. Selectivity ratios may readily be determined by the skilled person. $IC_{50}$ values for the PDE3 and PDE4 enzyme may be determined using established literature methodology, see S A Ballard *et al* (1998) and as detailed hereinafter.

**Auxiliary Agent : NEP inhibitor (I:NEP)**

NEP EC3.4.24.11 (*FEBS Lett*., 229(1), 206-210 (1988)), also known as enkephalinase or neprilysin, is a zinc-dependent neutral endopeptidase. This enzyme is involved in the breakdown of several bioactive oligopeptides, cleaving peptide bonds on the amino side of hydrophobic amino acid residues (Reviewed in Turner et al., 1997). The key neuronally released bioactive agents or neuropeptides metabolised by NEP include natriuretic peptides such as atrial natriuretic peptides (ANP) as well as brain natriuretic peptide and C-type natriuretic peptide, bombesin, bradykinin,

calcitonin gene-related peptide, endothelins, enkephalins, neurotensin, substance P and vasoactive intestinal peptide. Some of these peptides have potent vasodilatory and neurohormone functions, diuretic and natriuretic activity or mediate behaviour effects. Background teachings on NEP have been presented by Victor A. McKusick et al on http://www3.ncbi.nlm.nih.gov/Omim/searchomim.htm. The following information concerning NEP has been extracted from that source.

"Common acute lymphocytic leukemia antigen is an important cell surface marker in the diagnosis of human acute lymphocytic leukemia (ALL). It is present on leukemic cells of pre-B phenotype, which represent 85% of cases of ALL. CALLA is not restricted to leukemic cells, however, and is found on a variety of normal tissues. CALLA is a glycoprotein that is particularly abundant in kidney, where it is present on the brush border of proximal tubules and on glomerular epithelium. Letarte et al. (1988) cloned a cDNA coding for CALLA and showed that the amino acid sequence deduced from the cDNA sequence is identical to that of human membrane-associated neutral endopeptidase (NEP; EC 3.4.24.11), also known as enkephalinase. NEP cleaves peptides at the amino side of hydrophobic residues and inactivates several peptide hormones including glucagon, enkephalins, substance P, neurotensin, oxytocin, and bradykinin. By cDNA transfection analysis, Shipp et al. (1989) confirmed that CALLA is a functional neutral endopeptidase of the type that has previously been called enkephalinase. Barker et al. (1989) demonstrated that the CALLA gene, which encodes a 100-kD type II transmembrane glycoprotein, exists in a single copy of greater than 45 kb which is not rearranged in malignancies expressing cell surface CALLA. The gene was located to human chromosome 3 by study of somatic cell hybrids and in situ hybridization regionalized the location to 3q21-q27. Tran-Paterson et al. (1989) also assigned the gene to chromosome 3 by Southern blot analysis of DNA from human-rodent somatic cell hybrids. D'Adamio et al. (1989) demonstrated that the CALLA gene spans more than 80 kb and is composed of 24 exons."

Preferred for NEPi's for use as auxiliary agents in combination with bombesin receptor antagonists according to the present invention are:

(*2R*)-2-[(1-{[(5-ethyl-1,3,4-thiadiazol-2-yl)amino]carbonyl}cyclopentyl) methyl]pentanoic acid

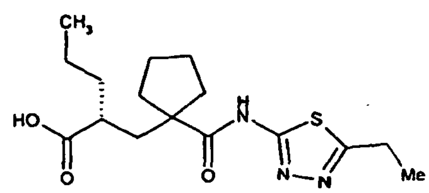

and

**(2*S*)-2-[(1-{[(5-Ethyl-1,3,4-thiadiazol-2-yl)amino]carbonyl}cyclopentyl)-methyl]pentanoic acid**

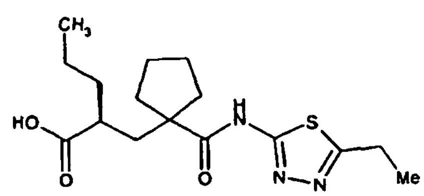

The title product from stage c) below (824mg) was further purified by HPLC using an AD column and using hexane:*iso*-propanol:trifluoroacetic acid (85:15:0.2) as elutant to give (*2R*)-2-[(1-{[(5-ethyl-1,3,4-thiadiazol-2-yl)amino]carbonyl}-cyclopentyl) methyl]pentanoic acid as a white foam, 400mg, 99.5% ee,

$^1$H NMR ($CDCl_3$, 400MHz) δ: 0.90 (t, 3H), 1.36 (m, 6H), 1.50-1.80 (m, 9H), 2.19 (m, 1H), 2.30 (m, 1H), 2.44 (m, 1H), 2.60 (m, 1H), 2.98 (q, 2H), 12.10-12.30 (bs, 1H), LRMS : m/z 338 ($MH^-$), $[\alpha]_D$ = -9.0° (c = 0.1, methanol), and (*2S*)-2-[(1-{[(5-ethyl-1,3,4-thiadiazol-2-yl)amino]carbonyl}cyclopentyl)-methyl]pentanoic acid as a white foam, 386mg, 99% ee, $^1$H NMR ($CDCl_3$, 400MHz) δ: 0.90 (t, 3H), 1.38 (m, 6H), 1.50-1.79 (m, 9H), 2.19 (m, 1H), 2.30 (m, 1H), 2.44 (m, 1H), 2.60 (m, 1H), 2.98 (q, 2H), 12.10-12.27 (bs, 1H); LRMS: m/z 338 ($MH^-$); and $[\alpha]_D$ = +3.8° (c = 0.1, methanol)

**Preparation of Starting Materials**

**a) 1-[2-(*tert*-Butoxycarbonyl)-4-pentyl]-cyclopentane carboxylic acid**

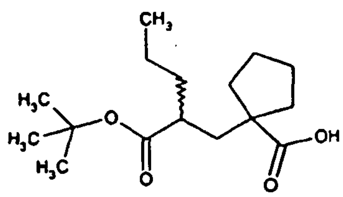

A mixture of 1-[2-(*tert*-butoxycarbonyl)-4-pentenyl]-cyclopentane carboxylic acid (EP 274234) (23g, 81.5mmol) and 10% palladium on charcoal (2g) in dry ethanol (200ml) was hydrogenated at 30psi and room temperature for 18 hours. The reaction mixture was filtered through Arbocel®, and the filtrate evaporated under reduced pressure to give a yellow oil. The crude product was purified by column chromatography on silica gel, using ethyl acetate:pentane (40:60) as the eluant, to provide the desired product as a clear oil, 21g, 91%; $^1$H NMR ($CDCl_3$, 0.86 (t, 3H), 1.22-1.58 (m, 15H), 1.64 (m, 4H), 1.78 (dd, 1H), 2.00-2.18 (m, 3H), 2.24 (m, 1H); LRMS : m/z 283 $(M-H)^-$

**b) tert-Butyl 2-[(1-{[(5-ethyl-1,3,4-thiadiazol-2-yl)amino]carbonyl}-cyclopentyl)methyl]pentanoate.**

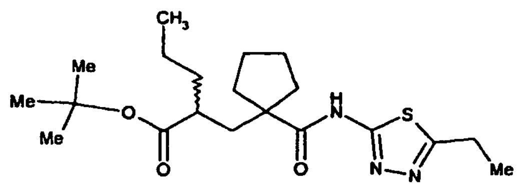

1-(3-Dimethylaminopropyl)-3-ethylcarbodiimide hydrochloride (0.21mmol), 1-hydroxybenzotriazole hydrate (0.2mmol), N-methylmorpholine (0.3 1mmol) and 2-amino-5-ethyl-1,3,4-thiadiazole (0.22mmol) were added to a solution of the product from stage a) above (150mg, 0.53mmol) in N,N-dimethylformamide (3ml), and the reaction stirred at 90°C for 18 hours. The cooled solution was diluted with ethyl acetate (90ml), washed with water (3x25ml), and brine (25ml), then dried ($MgSO_4$) and evaporated under reduced pressure. The crude product was purified by chromatography on silica gel, using ethyl acetate:pentane (30:70) as the eluant to afford the title compound, 92%; $^1$H NMR ($CDCl_3$, 300MHz) δ: 0.82 (t, 3H), 1.20-1.80 (m, 22H), 1.84 (m, 1H), 2.20 (m, 4H), 3.04 (q, 2H), 9.10 (bs, 1H); LRMS : m/z 396.2 $(MH^+)$.

**c) 2-[(1-{[(5-ethyl-1,3,4-thiadiazol-2-yl)amino]carbonyl}cyclopentyl) methyl]pentanoic acid.**

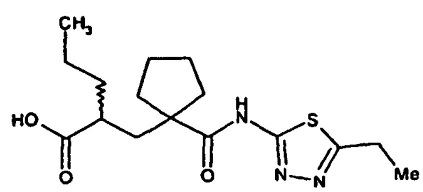

Trifluoroacetic acid (5ml) was added to a solution of the title product from stage b) above (0.31 mmol) in dichloromethane (5ml), and the solution stirred at room temperature for 4 hours. The reaction mixture was concentrated under reduced pressure and the residue azeotroped with toluene and dichloromethane to afford the title compound as a clear oil, 81%, $^1$H NMR ($CDCl_3$, 400MHz) δ: 0.92 (t, 3H), 1.35 (t, 3H), 1.25-1.80 (m, 11H), 2.20-2.50 (m, 4H), 2.95

(q, 2H), 12.10 (bs, 1H); LRMS : m/z 339.8 ($MH^+$); Anal. Found: C, 56.46; H, 7.46; N, 12.36. $C_{16}H_{25}N_3O_3S$ requires C, 56.62; H, 7.44; N, 12.37%.

Details on a suitable assay system for identifying and/or studying an I:NEP are presented in the hereinafter in the section entitled NEP Assay. Further examples of NEP inhibitors are disclosed and discussed in the following review articles:

Pathol. Biol., 46(3), 1998, 191.
Current Pharm. Design, 2(5), 1996, 443.
Biochem. Soc. Trans., 21(3), 1993, 678.
Handbook Exp. Pharmacol., 104/1, 1993, 547.
TiPS, 11, 1990, 245.
Pharmacol. Rev., 45(1), 1993, 87.
Curr. Opin. Inves. Drugs, 2(11), 1993, 1175.
Antihypertens. Drugs, (1997), 113.
Chemtracts, (1997), 10(11), 804.
Zinc Metalloproteases Health Dis. (1996), 105.
Cardiovasc. Drug Rev., (1996), 14(2), 166.
Gen. Pharmacol., (1996), 27(4), 581.
Cardiovasc. Drug Rev., (1994), 12(4), 271.
Clin. Exp. Pharmacol. Physiol., (1995), 22(1), 63.
Cardiovasc. Drug Rev., (1991), 9(3), 285.
Exp. Opin. Ther. Patents (1996), 6(11), 1147.

Yet further examples of NEPi's are disclosed in the following documents:

**EP-509442A**
US-192435
US-4929641
EP-599444B
US-884664
EP-544620A
US-798684
J. Med. Chem. 1993, 3821.
Circulation 1993, 88(4), 1.
EP-136883
JP-85136554
US-4722810
Curr. Pharm. Design, 1996,2,443.
EP-640594
J. Med. Chem. 1993, 36(1), 87.
EP-738711-A
JP-270957
CAS # 115406-23-0
DE-19510566
DE-19638020
EP-830863
JP-98101565
EP-733642
WO9614293
JP-08245609
JP-96245609
WO9415908
JP05092948
WO-9309101
WO-9109840
EP-519738
EP-690070
J. Med. Chem. (1993), 36, 2420.

JP-95157459
Bioorg. Med. Chem. Letts., 1996, 6(1), 65.

Further I:NEPs are disclosed in the following documents:

EP-A-0274234
JP-88165353
Biochem.Biophys.Res. Comm.,1989, 164, 58
EP-629627-A
US-77978
Perspect. Med. Chem. (1993), 45.
EP-358398-B

Further examples of I:NEPs are selected from the following structures:

| **Compound** | Structure | Mode of Action<br>References |
|---|---|---|
| FXII | 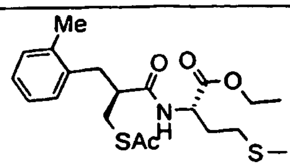 | **I:NEP**<br>**EP-509442A**<br>US-192435<br>US-4929641 |
| FXIII | 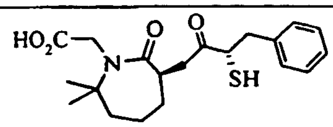 | I:NEP<br>(also an ACE inhibitor)<br>EP-599444B<br>US-884664 |
| FXIV | 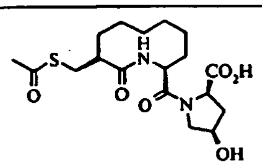 | I:NEP<br>EP-544620A<br>US-798684<br>J. Med. Chem.<br>1993, 3821. |
| FXV | 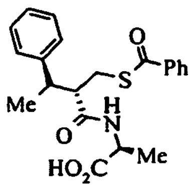 | I:NEP<br>(also an ACE inhibitor)<br>Mixanpril<br>Circulation<br>1993, 88(4), 1. |
| FXVI | 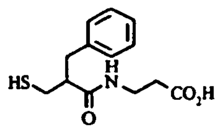 | I:NEP<br>EP-136883<br>JP-85136554<br>US-4722810 |
| FXVII | 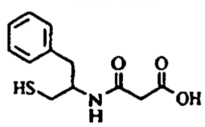 | I:NEP<br>Retrothiorphan<br>Curr. Pharm. Design,<br>1996, 2, 443. |

| | | |
|---|---|---|
| FXVIII | 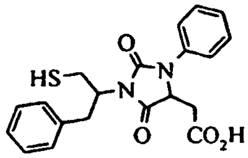 | I:NEP<br>(also an ACE inhibitor)<br>EP-640594 |
| FXIX | 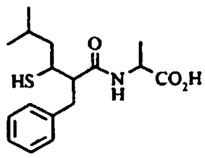 | I:NEP<br>J. Med. Chem.<br>1993, 36(1), 87. |
| FXX | 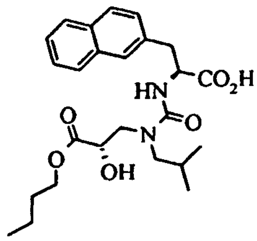 | I:NEP<br>(also an ACE inhibitor)<br>EP-738711-A<br>JP-270957 |
| FXXI | 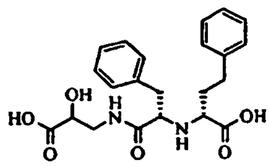 | I:NEP<br>CAS #<br>115406-23-0 |
| FXXII | 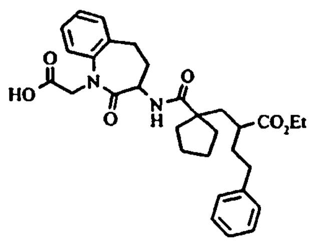 | I:NEP<br>(also an ECE inhibitor)<br>DE-19510566<br>DE-19638020<br>EP-830863<br>JP-98101565 |
| FXXIII | 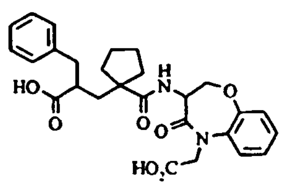 | I:NEP<br>(also an ECE inhibitor)<br>EP-733642 |
| FXXIV | 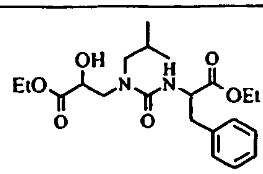 | I:NEP<br>WO96/14293 |

| | | |
|---|---|---|
| FXXV | 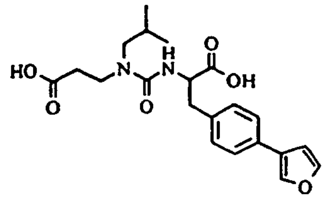 | I:NEP<br>JP-08245609<br>JP-96245609 |
| FXXVI | 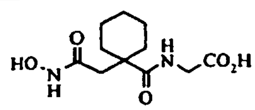 | I:NEP<br>WO9415908 |
| FXXVII | 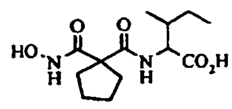 | I:NEP<br>JP05092948 |
| FXXVIII | 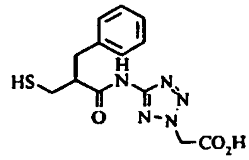 | I:NEP<br>WO-9309101 |
| FXXIX | 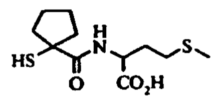 | I:NEP<br>WO-9109840 |
| FXXXI | 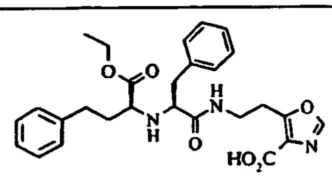 | I:NEP<br>EP-519738<br>EP-690070 |
| FXXXII | 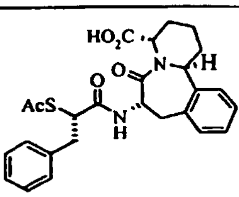 | I:NEP<br>(also an ACE inhibitor)<br>J. Med. Chem. (1993), 36, 2420. |
| FXXXIII | 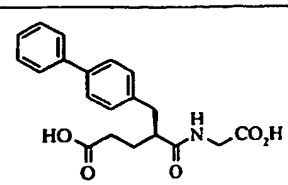 | I:NEP<br>JP-95157459<br>Bioorg. Med. Chem. Letts., 1996, 6(1), 65. |

Preferred additional I:NEPs are selected from the following structures:

| Compound | Structure | Mode of Action References |
|---|---|---|
| FV | 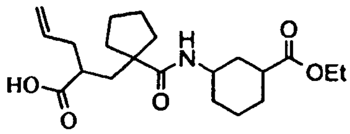 | I:NEP<br>EP-A-0274234<br>(Example 300) |
| FVI | 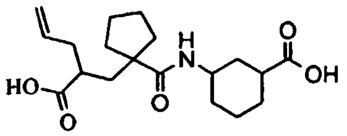 | I:NEP<br>EP-A-0274234<br>(Example 379) |
| FVII | 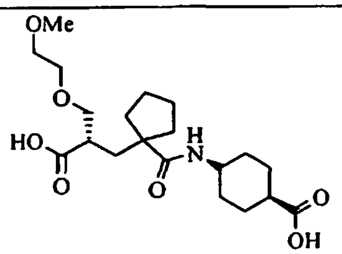 | I:NEP<br>Candoxatrilat<br>EP-274234<br>JP-88165353<br>Biochem.Biophys.Res. Comm.,1989, 164, 58 |
| FVIII | 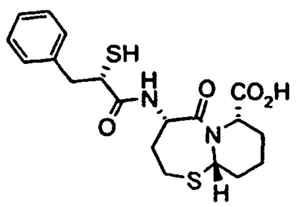 | I:NEP<br>Omapatrilat<br>(also an inhibitor of ACE)<br>EP-0629627-A<br>US-77978 |
| FIX | 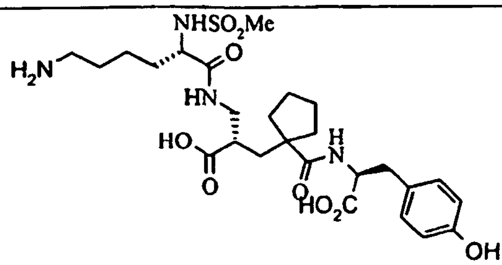 | I:NEP<br>Sampatrilat<br>(also an inhibitor of ACE)<br>Perspect. Med. Chem. (1993), 45.<br>EP-0358398-B |

| | | |
|---|---|---|
| FX | 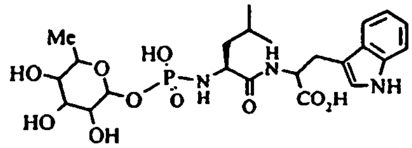 | I:NEP Phosphoramidon (which is commercially available) |
| FXI | 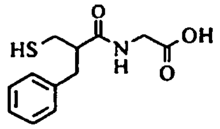 | I:NEP Thiorphan (which is commercially available) |

More preferred additional I:NEPs are selected from the following structures:

| COMPOUND | STRUCTURE |
|---|---|
| F57 | 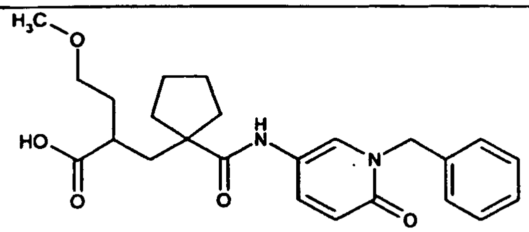 |
| F58 | 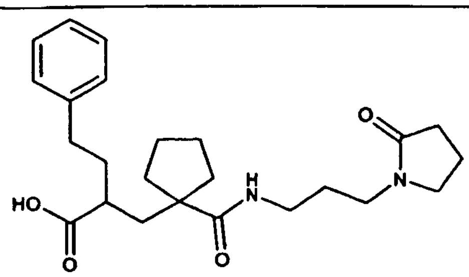 |
| F59 | 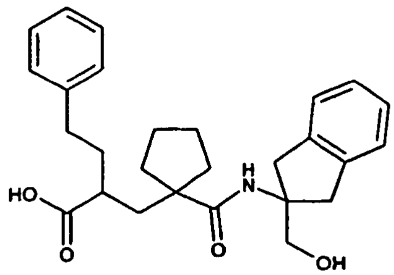 |

| | |
|---|---|
| F60 | 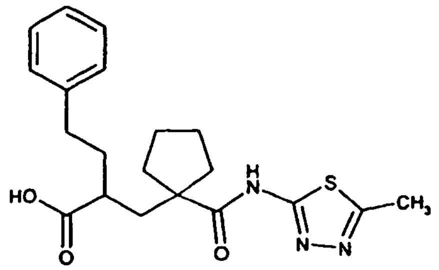 |
| F61 | 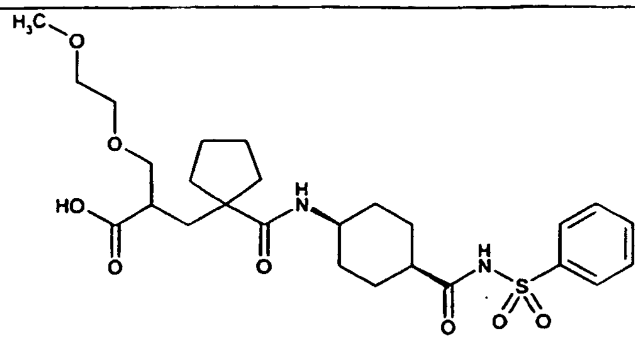 |
| F62 | 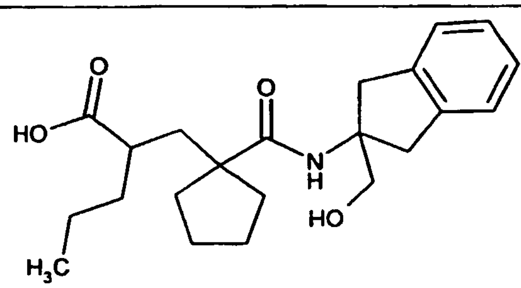 |
| F63 | 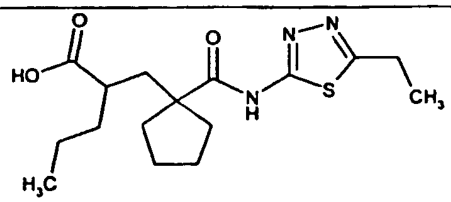 |
| F64 | 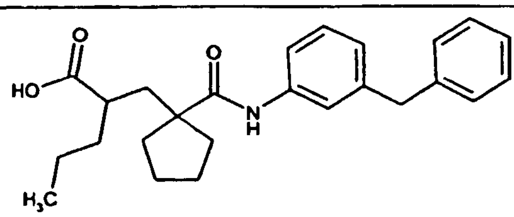 |
| F65 | 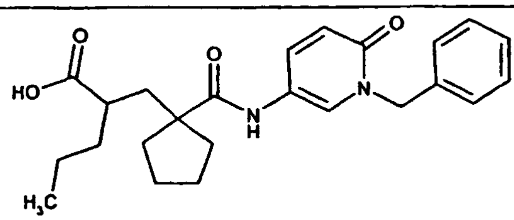 |

| F66 | 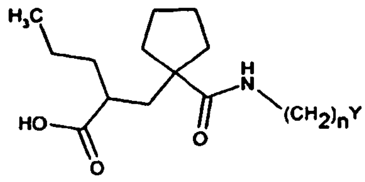 (Ic) |
|---|---|

**BIOAVAILABILITY**

Preferably, the compounds of the invention (and combinations) are orally bioavailable. Oral bioavailablity refers to the proportion of an orally administered drug that reaches the systemic circulation. The factors that determine oral bioavailability of a drug are dissolution, membrane permeability and metabolic stability. Typically, a screening cascade of firstly *in vitro* and then *in vivo* techniques is used to determine oral bioavailablity.

Dissolution, the solubilisation of the drug by the aqueous contents of the gastro-intestinal tract (GIT), can be predicted from *in vitro* solubility experiments conducted at appropriate pH to mimic the GIT. Preferably the compounds of the invention have a minimum solubility of 50 mcg/ml. Solubility can be determined by standard procedures known in the art such as described in Adv. Drug Deliv. Rev. 23, 3-25, 1997.

Membrane permeability refers to the passage of the compound through the cells of the GIT. Lipophilicity is a key property in predicting this and is defined by *in vitro* Log $D_{7.4}$ measurements using organic solvents and buffer. Preferably the compounds of the invention have a Log $D_{7.4}$ of -2 to +4, more preferably -1 to +2. The log D can be determined by standard procedures known in the art such as described in J. Pharm. Pharmacol. 1990, 42:144.

Cell monolayer assays such as $CaCo_2$ add substantially to prediction of favourable membrane permeability in the presence of efflux transporters such as p-glycoprotein, so-called caco-2 flux. Preferably, compounds of the invention have a caco-2 flux of greater than $2x10^{-6}cms^{-1}$, more preferably greater than $5x10^{-6}cms^{-1}$. The caco flux value can be determined by standard procedures known in the art such as described in J. Pharm. Sci, 1990, 79, 595-600

Metabolic stability addresses the ability of the GIT or the liver to metabolise compounds during the absorption process: the first pass effect. Assay systems such as microsomes, hepatocytes etc are predictive of metabolic liability. Preferably the compounds of the Examples show metabolic stablity in the assay system that is commensurate with an hepatic extraction of less then 0.5. Examples of assay systems and data manipulation are described in Curr. Opin. Drug Disc. Devel., 201, 4, 36-44, Drug Met. Disp.,2000, 28, 1518-1523

Because of the interplay of the above processes further support that a drug will be orally bioavailable in humans can be gained by *in vivo* experiments in animals. Absolute bioavailability is determined in these studies by administering the compound separately or in mixtures by the oral route. For absolute determinations (% absorbed) the intravenous route is also employed. Examples of the assessment of oral bioavailability in animals can be found in Drug Met. Disp.,2001, 29, 82-87; J. Med Chem , 1997, 40, 827-829, Drug Met. Disp.,1999, 27, 221-226.

By cross reference herein to compounds contained in patents which can be used in accordance with invention, we mean the therapeutically active compounds as defined in the claims (in particular of claim 1) and the specific examples (all of which is incorporated herein by reference).

How the invention may be put into effect will now be described, by way of example only, with reference to the following examples, some of which are preparative and others of which describe results of biological tests.

**Example 1**

**Effect of (S) 3-(1H-Indol-3-yl)-N-[1-(5-methoxy-pyridin-2-yl)-cyclohexyl-methyl]-2-methyl-2-[3-(4-nitro-phenyl)-ureido]-propionamide (Compound (1)) on female rat sexual proceptivity**

Compound (1)

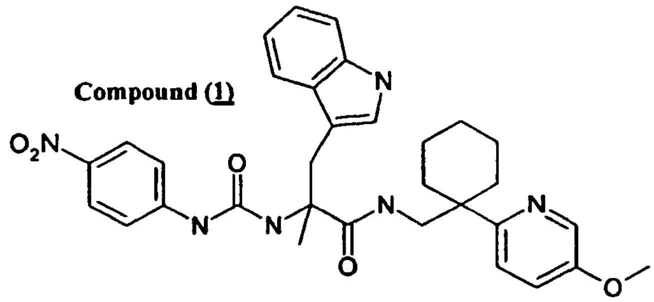

Ovariectomised adult female Sprague Dawley rats (180-200g, from Charles River) were housed in groups of 6 in a reversed lighting system of 12h light:dark (lights off 7.00-19.00h). Two weeks after ovariectomy they were used for sexual activity tests. The experiments started at least 5h into the dark period.

Tests were carried out in a circular arena of 90cm diameter, surrounded by a 30cm high wall. Two small cages with wire-mesh front (15x15cm) are fixed into the wall such that the front of the cage is «flush» with the wall and the 2 cages are opposite each other. They contained two stimuli animals: an intact sexually experienced male and a receptive female (ovariectomised, primed with 5μg oestradiol benzoate dissolved in corn oil and injected subcutaneously 48 hours before the test and with 0.5 mg of progesterone four hours before the test). Sexually naive test and control animals were used. Forty eight hours before the tests, both the test and control animals were primed with 5μg oestradiol benzoate. For animals used as positive controls, progesterone (0.5mg/0.1ml) was dissolved in corn oil and administered subcutaneously (s.c.), 4h before the test. Test and control animals were introduced one at a time for 10 minute periods into the arena. During the 10min test, the time that the test or positive control animal spent investigating each stimulus animal was noted. The arena was thoroughly cleaned between animals. The position of the male/female stimuli boxes was randomised between animals, in order to avoid place preference. The difference in the percentage of time spent investigating the male minus the female stimuli was calculated, out of the total time spent investigating stimuli animals.

Compound (1) was dissolved in 100% β-cyclodextrin and then diluted with saline to a final solution of 50% 2-hydroxypropyl-β-cyclodextrin. It was administered intraperitoneally (i.p.) at doses of 3 and 10mg/kg, in a dosing volume of 1ml/kg, 1h before tests. Progesterone (0.5mg/0.1ml) was dissolved in corn oil and administered subcutaneously (s.c.), 4h before test, as a positive control.

Compound (1) dose-dependently (3mg/kg-10mg/kg) increased the percentage of time spent investigating the male stimulus, with a MED of 10mg/kg (see **Figure 1).** The effect of this dose was similar to the effect of progesterone (prog). (*P<0.05, **P<0.01 Kruskal-Wallis followed by Mann-Whitney test, *vs* vehicle).

**Example 2**

**Effect of Compound (1) on female rat sexual receptivity**

Ovariectomised adult female Sprague Dawley rats (180-200g, from Charles River) were housed in groups of 6 in a reversed lighting system of 12h light:dark (lights off 7.00-19.00h). Two weeks after ovariectomy they were used for sexual activity tests. The experiments started at least 5h into the dark period.

Compound (1) was dissolved in 100% β-cyclodextrin and then diluted with saline to a final solution of 50% 2-hydroxypropyl-β-cyclodextrin. It was administered intraperitoneally (i.p.) at a dose of 10mg/kg, in a dosing volume of 1ml/kg. Quinelorane (6.25μg/kg) was dissolved in water and administered s.c. as positive control. Forty eight hours before testing, ovariectomised female rats (as described above), were primed with 5μg oestradiol benzoate dissolved in corn oil and injected subcutaneously. This is a low dose of oestrogen that does not re-establish sexual behaviour in an ovariectomised female but provides a minimum hormonal background for pharmacological agents to stimulate sexual behaviour. The females were placed with a series of vigorous male rats and subjected to 10 mounts.

The lordotic response of the animal was recorded and expressed as a percentage of the mounts (i.e. lordosis quotient, LQ), as previously described. Animals showing LQ<20 were considered non-receptive and were included in

the study. Each rat was tested prior to administration of the compound and then tested similarly post-injection. The pre-treatment times were 1h for Compound (1) and vehicle (50% β-cyclodextrin, i.p.) or 90min for quinelorane.

As shown in **Figure 2,** a single administration of quinelorane (6.25µg/kg, s.c.) significantly (P<0.01) increased the LQ, 90min after administration, compared to the LQ shown before administration (paired t test). A single administration of Compound (1) (10mg/kg, i.p.) also had a significant (P<0.05) stimulatory effect on the LQ, 1h after administration, compared to the LQ shown before administration (paired t test).

**Example 3**

**The effect of repeated administration of Compound (1) on female rat proceptivity**

In the present study we have investigated whether the repeated administration of a higher dose of Compound (1) (15mg/kg) still results in stimulation of proceptivity.

Ovariectomised adult female Sprague Dawley rats (180-200g) were housed in groups of 5 in a reversed lighting system of 12h light:dark (lights off 5.00-17.00h). They were used for the experiments at least two weeks after ovariectomy. Forty eight hours before tests, the animals were primed with oestradiol benzoate (5µg/0.1ml in corn oil, s.c.). On day 1, progesterone (0.5mg/0.1ml, in corn oil, s.c.) was administered to one of the groups 4h before tests, as a positive control. Compound (1) (15mg/kg, i.p.) was administered in 50% 2-hydroxypropyl-β-cyclodextrin, 1h before tests. The test lasted 10 minutes and was carried out as described before. The difference in the percentage of time spent investigating the male minus the female stimuli was calculated, out of the total time spent investigating stimuli animals. Animals were submitted to a test on day 1 and on day 15. From day 2 to 14 the Compound (1) group received a daily injection of the compound (15mg/kg, i.p.), while both the vehicle and the progesterone groups received an injection of vehicle. On day 15 the test took place again, as described for day 1.

On day 1, both progesterone and Compound (1) had a stimulatory effect on proceptivity, compared to the vehicle group (**P<0.01, ANOVA followed by Dunnett's test). On day 15, a similar stimulatory effect was observed (**P<0.01, ANOVA followed by Dunnett's test) (see **Figure 3**). No significant difference was observed between the effects on day 1 and day 15 for each treatment group (paired t test). The effects of progesterone and Compound (1) were statistically similar. There were no changes in body weight or general behaviour between groups along the experiment.

From this study we can conclude that Compound (1) (15mg/kg, i.p.) has a stimulatory effect on proceptivity in the female rat, comparable to progesterone, and that such effect is unaffected by the repeated administration of the compound, which seems to be well tolerated.

**Example 4**

**Effect of intracerebroventricular administration of Compound (1) on female rat sexual proceptivity**

In order to elucidate the site of action for this effect we have administered the Compound (1) intracerebroventricularly (i.c.v.).

Ovariectomised female rats (Sprague Dawley, obtained from Charles River, UK) were stereotaxically implanted (coordinates 0.89mm behind Bregma, 1.3mm lateral and 2.5mm vertical) with stainless steel cannulae (6 mm long, O.D. 0.75mm), held in place with dental cement. Animals were housed in groups of three and returned to a reversed lighting system of 12h light:dark (lights off 5.00-17.00h). Correct placement of the cannulae was assessed post-mortem. Rats were used for tests two weeks after ovariectomy (one week after cannulation). The experiments started at least 5h into the dark period. Forty eight hours before tests, the animals were primed with 5µg oestradiol benzoate (s.c, in corn oil) and adapted to the apparatus (in the absence of stimuli animals) for 10min on 2 consecutive days prior testing. The 10min test was carried out as previously described. The difference in the percentage of time spent investigating the male minus the female stimuli was calculated, out of the total time spent investigating stimuli.

Compound (1) was dissolved in 50% 2-hydroxypropyl-β-cyclodextrin in saline. It was administered i.c.v. over a 30sec period, with the aid of a pump set to deliver a flow of 10µl/min. The dosing volume was 5µl/rat. The compounds were administered 10min before tests. Progesterone (0.5mg/0.1ml) was dissolved in corn oil and administered subcutaneously (s.c.), 4h before test, as a positive control. As shown in **Figure 4,** Compound (1) dose-dependently (3-30µg/rat) increased the percentage of time spent investigating the male stimulus, with a MED of 10µg. The effect of this dose was similar to the effect of progesterone.

From this study we can conclude that the effect of Compound (1) on female sexual proceptivity is centrally mediated.

In Figure 4 bars represent percentage of time spent investigating male, minus the percentage of time spent investigating the female stimuli±SEM, (n=7-8 per group). *P<0.05, **P<0.01 *vs* vehicle (Kruskal-Wallis ANOVA test

followed by Mann-Whitney's test).

**Example 5**

**Inhibitory effect of NMB on female rat sexual proceptivity and antagonism of this effect by Compound (1)**

We have investigated the potentially inhibitory effect of the $BB_1$ agonist neuromedin B (NMB) on female rat sexual proceptivity.

Ovariectomised female rats (Sprague Dawley, obtained from Charles River, UK) were stereotaxically implanted (coordinates 0.89mm behind Bregma, 1.3mm lateral and 2.5mm vertical) with stainless steel cannulae (6 mm long, O.D. 0.75mm), held in place with dental cement. Animals were housed in groups of three and returned to a reversed lighting system of 12h light:dark (lights off 5.00-17.00h). Correct placement of the cannulae was assessed post-mortem. Rats were used for tests two weeks after ovariectomy (one week after cannulation). The experiments started at least 5h into the dark period. Forty eight hours before tests, the animals were primed with 5µg oestradiol benzoate (OB) (s. c, in corn oil) and adapted to the apparatus (in the absence of stimuli animals) for 10min on 2 consecutive days prior testing. The 10min test was carried out as previously described. The difference in the percentage of time spent investigating male minus female was calculated, out of the total time spent investigating stimuli.

Progesterone (Prog, 0.5mg/0.1ml) was dissolved in corn oil and administered subcutaneously (s.c.), 4h before test, to induce proceptive behaviour. Compound (**1**) (15mg/kg, i.p.) was dissolved in 50% 2-hydroxypropyl-β-cyclodextrin in saline and administered 1h before the i.c.v. administration. Neuromedin B was obtained from Bachem, UK. It was dissolved in isotonic saline and administered i.c.v. over a 30sec period, with the aid of a pump set to deliver a flow of 10µl/min, 10min before tests. The dosing volume was 5µl/rat. Each rat received a total amount of 100ng.

As shown in **Figure 5**, progesterone (Prog) increased the percentage of time spent investigating the male stimulus, compared to the vehicle group, thus showing stimulation of proceptive behaviour. NMB (100ng, i.c.v.) significantly reduced proceptivity in progesterone-treated rats. Moreover, pre-treatment with Compound (**1**) which acts as an antagonist (15mg/kg, i.p.) prevented the inhibitory effect of NMB. However, the blockade obtained with the dose of Compound (**1**) used was not total.

From the present study we can conclude that stimulation of BB1 receptors with an agonist results in inhibition of proceptive behaviour. This inhibitory effect may be prevented by the presence of an antagonist. e.g. Compound (**1**) In figure 5 the bars represent percentage of time spent investigating male, minus the percentage of time spent investigating the female stimuli±SEM, (n=8-12 per group). ***P<0.001 *vs* progesterone (One-way ANOVA followed by Dunnett's test).

**Example 6**

**Demonstration that the effect of Compound (1) on female sexual behaviour is not mediated through sexual hormones**

Previous examples have shown that Compound (1) (nanomolar affinity "mixed" $BB_1/BB_2$ receptor antagonist) has a dose-dependent stimulatory effect on sexual activity in the female rat, both on proceptivity and receptivity. Although the animals used in that study were ovariectomised, and therefore steroid hormones release can not be expected to occur in response to the compound, there is a possibility that the adrenal glands might secrete steroid hormones in response to Compound (**1**). If that was the case, the mediation of the stimulatory effects by progesterone would be relevant for rodents, but it would not be the case for primates. In the present study, we have investigated the potential effect of the bombesin receptor antagonist Compound (**1)** on secretion of progesterone. Oestradiol and pituitary hormones (Luteinising hormone (LH), follicle stimulating hormone (FSH) and prolactin) have also been analysed in the same animals.

Ovariectomised adult female Sprague Dawley rats (180-200g) were housed in groups of 6 in a reversed lighting system of 12h light:dark (lights off 7.00-19.00h). They were used for the experiments at least two weeks after ovariectomy. Forty eight hours before tests, the animals were primed with oestradiol benzoate (5µg/0.1ml in corn oil, s.c.). Progesterone (0.5mg/0.1ml, in corn oil, s.c.) was administered 4h before blood collection, as a positive control. Compound (**1**) (3-10mg/kg, i.p.) was administered in 50% 2-hydroxypropyl-β-cyclodextrin, 1h prior to blood collection. Blood was collected from the trunk, after decapitation. It was immediately centrifuged (3500r.p.m., 4°C, 5min) and the plasmas frozen until assayed for hormonal content, using commercially available radioimmunoassay kits ($^{125}$I-labelled hormones) for oestradiol, progesterone, LH, FSH and prolactin.

A single administration of progesterone resulted in a significant increase in the progesterone plasma levels (P<0.05), and a significant decrease in LH plasma levels (P<0.01), compared to animals injected with vehicle (Kruskal-Wallis followed by Mann-Whitney test). However, Compound (**1**) (3-10mg/kg, i.p.) had no effect on the plasma levels

of progesterone **(Figure 6,** where animals were pre-treated with 5μg oestradiol benzoate, s.c., 48h before the test. They were tested 1h or 4h post-injection of Compound (1) (3-10mg/kg, p.o.) or progesterone (0.5mg/0.1ml, s.c.) respectively. Values represent mean±SEM, (n=9 per group). *P<0.05, *vs* vehicle (Kruskal-Wallis followed by Mann-Whitney test, *vs* vehicle)), oestradiol (**Figure 7**, where animals were pre-treated with 5μg oestradiol benzoate, s.c., 48h before the test. They were tested 1h or 4h post-injection of Compound (1) (3-10mg/kg, p.o.) or progesterone (0.5mg/0.1ml, s.c.) respectively. Values represent mean±SEM, (n=6-7 per group)), prolactin (**Figure 8**, where animals were pre-treated with 5μg oestradiol benzoate, s.c., 48h before the test. They were tested 1h or 4h post-injection of Compound (1) (3-10mg/kg, p.o.) or progesterone (0.5mg/0.1ml, s.c.) respectively. Values represent mean±SEM, (n=10 per group)) LH (**Figure 9**, where animals were pre-treated with 5μg oestradiol benzoate, s.c., 48h before the test. They were tested 1h or 4h post-injection of Compound (1) (3-10mg/kg, p.o.) or progesterone (0.5mg/0.1ml, s.c.) respectively. Values represent mean±SEM, (n=10 per group). **P<0.01, *vs* vehicle (Kruskal-Wallis followed by Mann-Whitney test, *vs* vehicle)) or FSH (**Figure 10**, where animals were pre-treated with 5μg oestradiol benzoate, s.c., 48h before the test. They were tested 1h or 4h post-injection of Compound (1) (3-10mg/kg, p.o.) or progesterone (0.5mg/0.1ml, s.c.) respectively. Values represent mean±SEM, (n=10 per group).

From this experiment we can conclude that Compound (**1**) did not have an effect on the secretion of sexual hormones, thus suggesting that the effects of the compound on female sexual activity must be mediated by different mechanisms, maybe involving neurotransmitters.

**Example 7**

**Effect of Compound (1) on the sexual behaviour of normal male rats**

The potentially stimulatory effect of Compound **(1)** on male sexual behaviour has been tested on sexually vigorous rats. Sprague Dawley male rats (Charles River, UK) were kept, 4 rats per cage, in a reversed lighting regime (12:12 hours, lights off at 5.00 h), with free access to food and water. The rats were pre-selected by being presented with a receptive female at 4 days intervals, i.e. every third day (having 2 clear days between presentations) until completing 6-7 days of baseline determination. The animals showing consistently vigorous behaviour (ejaculatory latencies <300s) were chosen for further experiments (n=24). Animals were randomised into three groups. All animals received all three treatments following a latin-square design. Treatments were administered once a week, with a baseline test in between treatments (4 days intervals between baseline and test day). Treatments were Compound (**1**) (15mg/kg, dissolved in 50% 2-hydroxypropyl-β-cyclodextrin in saline), vehicle, or Fluoxetine (20mg/kg dissolved in 100% DMSO). All treatments were administered i.p. in a 1ml/kg volume, 1h before tests.

For all the sexual behaviour tests, the males were placed in an observation arena (50-60 cm diameter), starting 5 hours into the dark cycle and observed under red illumination. Three to 4 minutes after placing the male in the arena, a receptive female (ovariectomised, bearing a 7 mm Silastic implant of oestradiol benzoate) was introduced to the arena and the following parameters noted: Mount Latency: time (in seconds) taken between introduction of female and first mount. A maximum time of 15 minutes (900 seconds) was allowed, and the test terminated if no mounts were recorded within that time **(Figure 11),** Intromission Latency: time (in seconds) taken between introduction of female and first intromission (**Figure 12**), Number of Mounts: to reach ejaculation. When ejaculation was not reached, the number of mounts was not analyzed, Number of Intromissions: to reach ejaculation. When ejaculation was not reached, the number of intromissions was not analyzed (Figure **13** is number of mounts + intromissions), Ejaculation Latency: time (in seconds) taken from first intromission to ejaculation. A maximum time of 30 minutes (1800 seconds) was given, and the test terminated if ejaculation was not achieved in that time (**Figure 14**), and Refractory Period: time (in seconds) taken from ejaculation to the first mount of the next series of sexual activity. In those animals reaching ejaculation the test was terminated at the end of the refractory period, as indicated by the first mount of the next sexual cycle (**Figure 15**).

A one-way ANOVA followed by Dunnett's t test was used to compare treated vs vehicle groups each day of testing, for all the sexual behaviour parameters. (*P<0.05, **P<0.01; n=15-16).

Mount latency and intromission latency were significantly increased in the fluoxetine-treated group compared to the vehicle group. Ejaculation latency and refractory period were also increased in this group, showing a decrease in sexual performance as well as the decreased arousal. No changes were seen in the number of mounts and intromissions required to achieve ejaculation. Unlike Fluoxetine, Compound (**1**) had no effect on any of the parameters studied, at a dose shown to be stimulatory in sexually dysfunctional males (see example 9). From the present study we can conclude that Compound (**1**) has no effect on sexual behaviour in sexually vigorous males.

**Example 8**

**Effect of Compound (1) on the sexual behaviour of sexually dysfunctional male rats**

Fluoxetine induces ejaculation delay, anorgasmy and loss of sexual desire in humans (Crenshaw and Goldberg, 1996). A model of male sexual dysfunction in the rat, induced by daily administration of fluoxetine until a significant detrimental effect on sexual behaviour (arousal and ejaculation) was established. The potentially stimulatory effect of Compound (**1**) on male sexual behaviour in these sexually dysfunctional male rats was examined. The effects of Compound (**1**) were compared to those of yohimbine. Preclinical and clinical studies suggest that yohimbine may be an effective treatment for sexual side-effects caused SSRI (Hollander, E., McCarley, A. (1993) *J. Clin. Psychiatry* 53: 207-209. and Jacobsen).

Sprague Dawley male rats (Charles River, UK) were kept, 4 rats per cage, in a reversed lighting regime (12: 12 hours, lights off at 5.00 h), with free access to food and water. The rats were pre-selected by being presented with a receptive female at 4 days intervals, i.e. every third day (having 2 clear days between presentations) until completing 6-7 trials of baseline determination. The animals showing consistently vigorous behaviour (ejaculatory latencies <300s) were chosen for further experiments. Animals were treated for 3 consecutive days with either vehicle (water) or fluoxetine (20mg/kg, i.p., in a 2ml/kg dosing volume). On the fourth day, the animals treated with water received vehicle (veh+veh) and the animals treated with fluoxetine received one of the three following treatments: Compound (**1**) (15mg/kg, dissolved in 50% 2-hydroxypropyl-β-cyclodextrin in saline), vehicle (cyclodextrine), or yohimbine (2mg/kg dissolved in water). All treatments were administered i.p. in a 1ml/kg volume, 1h before tests.

For all the sexual behaviour tests, the males were placed in an observation arena (50-60 cm diameter), starting 5 hours into the dark cycle and observed under red illumination. Three to 4 minutes after placing the male in the arena, a receptive female (ovariectomised, bearing a 7 mm Silastic implant of oestradiol benzoate) was introduced to the arena and the following parameters noted: Mount Latency: time (in seconds) taken between introduction of female and first mount. A maximum time of 15 minutes (900 seconds) was allowed, and the test terminated if no mounts were recorded within that time **(Figure 16),** Ejaculation Latency: time (in seconds) taken from first intromission to ejaculation. A maximum time of 30 minutes (1800 seconds) was given, and the test terminated if ejaculation was not achieved in that time **(Figure 17**), Percentage of males achieving ejaculation within 30 minutes was calculated **(Figure 18).**

A one-way ANOVA followed by Dunnett's t test was used to compare the fluoxetine+vehicle group and other groups for mount and ejaculatory latencies. Percentage of animals ejaculating was analysed using a Chi-square test followed by Fisher's test. (*: $P<0.05$, **: $P<0.01$, ***: $P<0.001$; n=15-19).

Mount latency and ejaculation latency were significantly increased in the fluoxetine-treated groups compared to the vehicle+vehicle group, indicating a decrease in sexual desire as well as sexual performance in these groups. The number of animals ejaculating was significantly lower in the fluoxetine-treated groups, indicating anorgasmy. Compound (**1**) significantly decreased the mount and ejaculatory latencies at the same time as increasing the percentage of animals ejaculating in the animals rendered sexually dysfunctional by the fluoxetine treatment, to levels comparable to normal animals (veh+veh). Yohimbine followed a similar trend, although this did not reach significance.

From the present study we can conclude a stimulatory effect of Compound (**1**) on sexual behaviour in males suffering from sexual dysfunction, at the level of sexual desire, sexual performance and anorgasmy.

**Example 9**

**(S)-3-(1*H*-Indol-3-yl)-*N*-[1-(5-methoxy-pyridin-2-yl)-cyclohexylmethyl]-2-methyl-2-[4-(4-nitro-phenyl)-oxazol-2-ylamino]-propionamide**

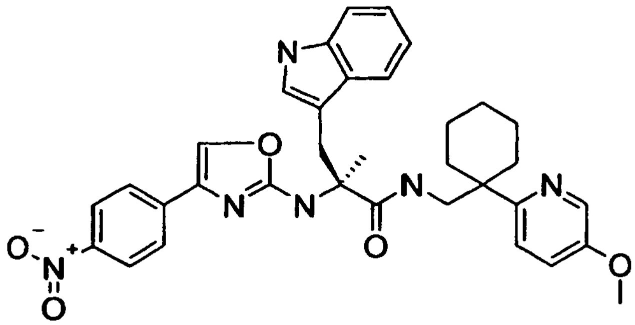

1. To a stirred solution of p-nitrophenylchloroformate (9.27g, 46mmol) in THF (200ml) at 0°C was added dropwise a solution of H-(S)-αMeTrp-OMe (**1a**) (10.7g, 46 mmol) and triethylamine (6.4ml, 46mmol) in THF (100ml) over 1 hour. Stirring was continued for a further 30 minutes at room temperature, after which aqueous ammonia (15ml) was added. IR after 10 minutes indicated bands at 1732 and 1660 $cm^{-1}$. The THF was removed under reduced pressure, and the residue was taken up in EtOAc and washed with 1N HCl (x2), $Na_2CO_3$ solution (until intense yellow colour subsided, ~x8), brine, and dried ($MgSO_4$). The solvent was removed under reduced pressure to give **2a** as a foam (10.3g, 82%):MS m/e (AP+): 276.16 ($M^+$ + H, 100%);

MS m/e (AP-): 274.11 ($M^-$ - H, 100%);

IR (film): 3383, 1724, 1657, 1600, 1539, 1456, 1374, 1256, 1108, 743 $cm^{-1}$;

$^1H$ NMR ($CDCl_3$): δ= 1.70 (3H, s), 3.38 (1H, d, J=14.7 Hz), 3.59 (1H, d, J=14.7 Hz), 3.71 (3H, s), 4.22 (2H, s), 5.16 (1H, s), 6.99 (1H, d, J=2.2 Hz), 7.08-7.20 (2H, m), 7.34 (1H, d, J=8.1 Hz), 7.59 (1H, d, J=7.8 Hz), 8.09 (1H, s).

2. The urea **(2a)** (6.4g, 23mmol) and 2-bromo-1-(4-nitro-phenyl)-ethanone (6.0g, 23mmol) were stirred in toluene (500ml)/dioxan (100ml) and maintained under reflux for 30 hours, after which solvent was removed under reduced pressure and the residue was purified by chromatography using a 90g Biotage cartridge. 10% EtOAc in heptane eluted the bromide starting material. 20% EtOAc eluted the desired product. Removal of solvent under reduced pressure gave **3a** as a foam (840mg, 9%):

MS m/e (ES+): 420.56 ($M^+$, 100%);

IR (film): 3394, 1732, 1632, 1605, 1574, 1515, 1456, 1334, 1253, 1210, 1108, 1072, 940, 854, 734 $cm^{-1}$;

$^1H$ NMR ($CDCl_3$): δ= 1.91 (3H, s), 3.46 (1H, d, J=14.6 Hz), 3.69 (3H, s), 3.78 (1H, d, J=14.6 Hz), 5.57 (1H, s), 6.89 (1H, d, J=2.2 Hz), 7.03-7.08 (1H, m), 7.14-7.18 (1H, m), 7.34 (1H, d, J=8.1 Hz), 7.41 (1H, d, J=8.1 Hz), 7.63 (1H, s), 7.85 (2H, d, J=9.0 Hz), 8.05 (1H, s), 8.24 (2H, d, J=8.6 Hz).

3. The ester (**3a**) (840mg, 2mmol) was dissolved in dioxan (50ml) and $LiOH.H_2O$ (336mg, 8mmol) in $H_2O$ (25ml) was added. The mixture was stirred vigorously overnight, and then neutralised with 1M HCl (8ml, 8mmol). The majority of the dioxan was removed under reduced pressure and the product was crystallised, filtered off, washed with water and dried under reduced pressure to give pure **4a** (668mg, 82%):

MS m/e (ES+): 407 ($M^+$ + H);

IR (film): 1633 $cm^{-1}$;

$^1H$ NMR (DMSO-$d_6$) δ= 1.49 (3H, s), 3.30-3.35 (1H, m, masked by $H_2O$), 3.59 (1H, d, J=14.7 Hz), 6.86-6.90 (1H, m), 6.99-7.03 (2H, m), 7.30-7.36 (2H, m), 7.48 (1H, s), 7.94 (2H, d, J=9.0 Hz), 8.27-8.30 (3H, m), 10.88 (1H, s), (*$CO_2H$ not seen).*

4. The acid (**4a**) (1.148g, 2.8mmol), *O*-benzotriazol-1-yl-*N,N,N',N'*-tetramethyluronium hexafluorophosphate (HBTU, 1.06g, 2.8mmol), and *N,N*-diisopropylethylamine (DIPEA, 490μl, 2.8mmol) were stirred in DMF (10ml) for 5 minutes before adding DIPEA (490μl, 2.8mmol) and [1-(5-methoxy-2-pyridyl)cyclohexyl]-methanamine (see WO 98/07718, 678mg, 3.1mmol). HPLC indicated that reaction was complete within 1 hour. Solvent was removed under reduced pressure and the residue was taken up in EtOAc. The organic layer was washed with brine, saturated

$NaHCO_3$ (x3), brine and dried ($MgSO_4$), after which solvent was removed under reduced pressure. The residue was purified by chromatography using RP silica with 65% MeOH in $H_2O$. Pure fractions were evaporated to give the desired product as an amorphous solid (1.12g, 66%):

MPt: 100-105°C;

MS m/e (ES+): 609.63 ($M^+$+ H, 100%);

IR (film): 3359, 3272, 3054, 2932, 2857, 1628, 1606, 1573, 1515, 1488, 1393, 1336, 1268, 1232, 1181, 1150, 1131, 1097, 1028, 1012, 962, 939, 900, 853, 831, 737 $cm^{-1}$;

$^1H$ NMR ($CDCl_3$): δ= 1.10-1.60 (8H, m), 1.72 (3H, s), 1.95-2.02 (2H, m), 3.31-3.42 (2H, m), 3.41 (1H, d, J=14.6 Hz), 3.50 (1H, d, J=14.6 Hz), 3.69 (3H, s), 5.34 (1H, s), 6.90-6.97 (2H, m), 7.04-7.09 (2H, m,) 7.14-7.19 (1H, m), 7.33 (1H, d, J=8.1 Hz), 7.46 (1H, d, J=7.8 Hz), 7.54 (1 H, s), 7.77 (2H, d, J=8.8 Hz), 8.00 (1H, d, J=2.9 Hz), 8.04 (1H, s), 8.21 (2H, d, J=8.8 Hz); *(amide masked by $CHCl_3$)*

HPLC A: Rt. 11.86 min, 99.8/100% purity, 20-100% $CH_3CN$ in $H_2O$ (+0.1%TFA) over 15 min at 1ml $min^{-1}$, Prodigy ODSIII 250x4.6mm 5μM, 215 and 254 nm;

HPLC B: Rt. 14.32 min, 100/100% purity, 80:20 methanol/Tris buffer at pH9, 1ml$min^{-1}$, Prodigy ODSIII 250x4.6mm 5μM, 215 and 254 nm.

**Example 10**

**(S)-3-(1*H*-Indol-3-yl)-*N*-(1-methoxymethyl-cyclohexylmethyl)-2-methyl-2-[4-(4-nitro-phenyl)-oxazol-2-ylamino]-propionamide**

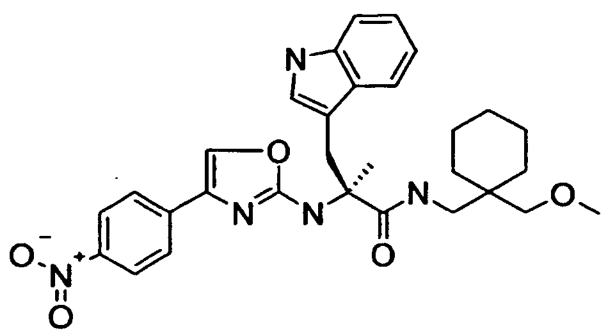

The above compound was synthesized from Intermediate 4a and Intermediate 13 using the same method as used for **Example 9.** The acid **(4a)** (203mg, 0.5mmol), HBTU (190mg, 0.5mmol), and DIPEA (87μl, 0.5mmol) were stirred in DMF (10ml) for 5 minutes before adding DIPEA (87μl x 2, 1.0mmol) and **Intermediate 13** (94mg, 0.5mmol, Scheme 6). After 4 hours the solvent was removed under reduced pressure and residue taken up in EtOAc. The organic layer was washed with brine, saturated $NaHCO_3$ (x3), brine, dried ($MgSO_4$) and solvent removed under reduced pressure. The residue was heated to 60°C in methanol and product filtered off. Drying under reduced pressure gave the desired product as a yellow crystalline solid (214mg, 78%):

MPt: 189-192°C;

MS m/e (ES+): 546.49 ($M^+$ + H, 100%);

IR (film): 3285, 2928, 2849, 1637, 1604, 1516, 1453, 1334, 1260, 1108, 1077, 860, 743, 729 $cm^{-1}$;

$^1H$ NMR (DMSO-$d_6$): δ= 1.10-1.35 (10H, m), 1.44 (3H, s), 2.91-3.01 (3H, m), 3.06-3.12 (1H, m), 3.07 (3H, s), 3.26-3.31 (1H, m), 3.64 (1H, d, J=14.4 Hz), 6.87-6.93 (2H, m), 7.01 (1H, t, J=7.4 Hz), 7.29-7.37 (3H, m), 7.44 (1H, s), 7.94 (2H, d, J=9.0 Hz), 8.26 (2H, d, J=8.8 Hz), 8.34 (1H, s), 10.84 (1H, s);

HPLC A: Rt. 17.07 min, 100/100% purity, 20-100% $CH_3CN$ in $H_2O$ (+0.1%TFA) over 15 min at 1ml$min^{-1}$, Prodigy ODSIII 250x4.6mm 5μM, 215 and 254 nm;

HPLC B: Rt. 14.35 min, 100/100% purity, 80:20 methanol/Tris buffer at pH9, 1ml$min^{-1}$, Prodigy ODSIII 250x4.6mm 5μM, 215 and 254 nm.

**Example 11**

**(S)-3-(1*H*-Indol-3-yl)-2-methyl-2-[4-(4-nitro-phenyl)-oxazol-2-ylamino]-*N*-(2-oxo-2-phenyl-ethyl)-propionamide.**

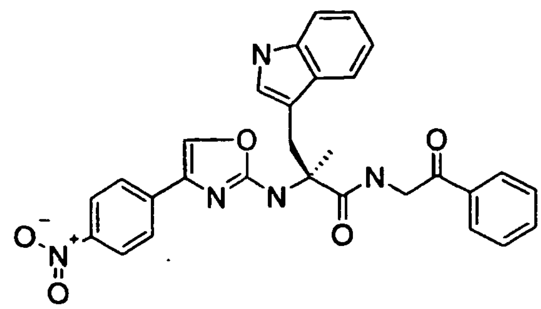

The above compound was synthesised from **Intermediate 4a** using the same method as used for **Example 9.** The acid (**4a**) (203mg, 0.5mmol), HBTU (190mg, 0.5mmol), and DIPEA (87μl, 0.5mmol) were stirred in DMF (10ml) for 5 minutes before adding DIPEA (87μl, 0.5mmol) and 2-amino-1-phenyl-ethanone (103mg, 0.6mmol). After 4 hours the solvent was removed under reduced pressure and residue taken up in EtOAc, washed with brine, saturated $NaHCO_3$ (x3), brine, dried ($MgSO_4$) and solvent removed under reduced pressure. The residue was purified by chromatography using NP 20g Mega Bond Elut cartridge and 40% ethyl acetate in heptane as eluent. Evaporation of pure fractions gave the desired product as a yellow amorphous solid (170mg, 65%):

MPt: 80-90°C;

MS m/e (AP+): 525.83 (16%), 524.44 ($M^+$ + H, 100%);

IR (film): 3396, 3059, 2983, 2932, 1694, 1628, 1605, 1575, 1514, 1449, 1336, 1284, 1264, 1225, 1181, 1154, 1096, 1072, 1010, 1001, 940, 853, 737 $cm^{-1}$;

$^1H$ NMR (DMSO-$d_6$): δ= 1.50 (3H, s), 3.39 (1H, d, J=14.7 Hz), 3.64 (1H, d, J=14.6 Hz), 4.53 (1H, d.d, J=18.1 and 5.4 Hz), 4.66 (1H, d.d, J=18.1 and 5.5 Hz), 6.87 (1H, t, J=7.4 Hz), 6.95 (1H, d, J=2.2 Hz), 7.00 (1H, t, J=7.4 Hz), 7.30 (1H, d, J=8.1 Hz), 7.34 (1H, d, J=8.1 Hz), 7.41 (1H, s), 7.50-7.55 (2H, m), 7.62-7.67 (1H,m), 7.94-7.99 (4H, m), 8.24 (1H, t, J=5.4 Hz), 8.27 (2H, d, J=9.0 Hz), 8.31 (1H, s), 10.86 (1H, s);

HPLC A: Rt. 20.83 min, 98.3/99.6% purity, 20-100% $CH_3CN$ in $H_2O$ (+0.1%TFA) over 25 min at 1mlmim$^{-1}$, Prodigy ODSIII 250x4.6mm 5μM, 215 and 254 nm;

HPLC B: Rt. 6.82 min, 100/100% purity, 80:20 methanol/Tris buffer at pH9, 1mlmin$^{-1}$, Prodigy ODSIII 250x4.6mm 5μM, 215 and 254 nm.

**Example 12**

**(S)-*N*-[1-(5-Methoxy-pyridin-2-yl)-cyclohexylmethyl]-2-methyl-2-[4-(4-nitrophenyl)-oxazol-2-ylamino]-3-phenyl-propionamide**

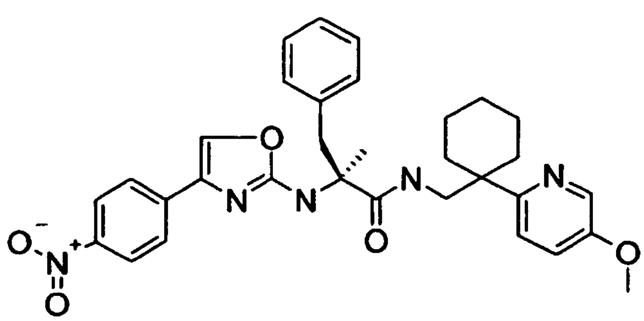

The above compound was synthesised from **1b** and **4b** using the same methods as used for **Example 9.** The acid (**4b**) (120mg, 0.33mmol), HBTU (124mg, 0.33mmol), and DIPEA (114μl, 0.66mmol), and [1-(5-methoxy-2-pyridyl) cyclohexyl]-methanamine[1] (86mg, 0.4mmol) were stirred in DMF (4ml) for 18 hours. Solvent removed under reduced pressure and residue taken up in EtOAc. The organic layer was washed with brine, saturated $NaHCO_3$ (x3), brine, dried ($MgSO_4$) and solvent removed under reduced pressure. The residue was purified by chromatography using NP silica with 10-80% ethyl acetate in heptane. Pure fractions were evaporated to give the desired compound as a yellow amorphous solid (90mg, 49%):

MS m/e (AP+): 570.23 ($M^+$ + H, 100%);

IR (film): 3363, 2930, 2856, 1658, 1651, 1628, 1574, 1515, 1488, 1334, 1268, 1232, 1073, 1030, 938, 852 $cm^{-1}$;

$^1H$ NMR (DMSO-$d_6$): δ= 0.94-1.46 (11H, m), 1.98-2.10 (2H, m), 3.04-3.14 (2H, m), 3.25-3.32 (1H, m), 3.57 (1H, d, J=13.6 Hz), 3.73 (3H, s), 6.95-7.00 (3H, m), 7.10-7.24 (5H, m), 7.44 (1H, s), 7.93 (2H, d, J=8.8 Hz), 8.14 (1H, d, J=2.8 Hz), 8.27 (2H, d, J=9.2 Hz), 8.36 (1H, s);

HPLC A: Rt. 5.49 min, 99.76% purity, 20-100% $CH_3CN$ in $H_2O$ (+0.1%TFA) over 7 min at 1.5 $mlmin^{-1}$, Prodigy ODSIII 150x4.6mm 3μM at 40°C, 200-300 nm;

HPLC B: Rt. 5.72 min, 99.46% purity, 20-90% $CH_3CN$/Tris (1mM) over 7 min at 2$mlmin^{-1}$, Prodigy Phenyl-Ethyl, 100x4.6mm 5μM at 30°C, 200-300 nm.

**Example 13**

**(S)-2-[4-(4-Cyano-phenyl)-oxazol-2-ylamino]-3-(1*H*-indol-3-yl)-*N*-[1-(5-methoxy-pyridin-2-yl)-cyclohexylmethyl]-2-methyl-propionamide**

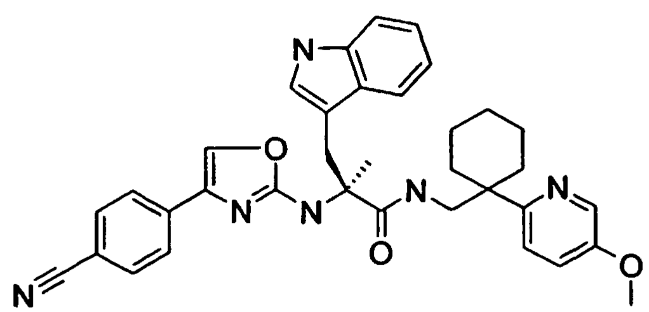

The above compound was synthesized from **2a** via **6a** as outlined in Scheme 2 using methods analogous to those used for **Example 9**. The acid (**6a**) (309mg, 0.8mmol), HBTU (303mg, 0.8mmol), DIPEA (140μl, 0.8mmol) were stirred in DMF (5ml) for 5 minutes before adding DIPEA (140μl, 0.8mmol) and [1-(5-methoxy-2-pyridyl)cyclohexyl]-methanamine (WO 98/07718) (185mg, 0.84mmol). HPLC indicated reaction complete within 1 hour. Solvent removed under reduced pressure and residue taken up in EtOAc. Washed with brine, saturated $NaHCO_3$ (x3), brine, dried ($MgSO_4$) and solvent removed under reduced pressure. Residue purified by chromatography using RP silica with 65% MeOH in $H_2O$. Pure fractions were evaporated to give **Example 13** as a white amorphous solid (320mg, 68%):

MPt: 105-108°C;

MS m/e (ES+): 589.32 ($M^+$+ H, 100%), 590.18 (62%);

IR (film): 3355, 2932, 2857, 2225, 1628, 1572, 1521, 1489, 1456, 1328, 1269, 1232, 1096, 1072, 1029, 938, 844, 741 $cm^{-1}$;

$^1H$ NMR ($CDCl_3$): δ= 1.20-1.60 (8H, m), 1.70 (3H, s), 1.93-2.03 (2H, m), 3.30-3.52 (4H, m), 3.68 (3H, s), 5.30 (1H, s), 6.89 (1H, d, J=2.4 Hz), 6.94 (1H, d.d, J=8.8 and 2.9 Hz), 7.03-7.09 (2H, m,) 7.14-7.19 (1H, m), 7.20-7.25 (1H, m), 7.33 (1H, d, J=8.1 Hz), 7.46 (1H, d, J=7.8 Hz), 7.50 (1H, s), 7.63 (2H, d, J=8.5 Hz), 7.72 (2H, d, J=8.3 Hz); 8.00 (1H, d, J=2.9 Hz), 8.05 (1H, s);

HPLC A: Rt. 11.63 min, 97.7/100% purity, 20-100% $CH_3CN$ in $H_2O$ (+0.1%TFA) over 15 min at 1$mlmin^{-1}$, Prodigy ODSIII 250x4.6mm 5μM, 215 and 254 nm;

HPLC B: Rt. 9.20 min, 100/100% purity, 80:20 methanol/Tris buffer at pH9, 1$mlmin^{-1}$, Prodigy ODSIII 250x4.6mm 5μM, 215 and 254 nm.

**Example 14**

**(S)-3-(1*H*-Indol-3-yl)-*N*-[1-(5-methoxy-pyridin-2-yl)-cyclohexylmethyl]-2-methyl-2-(4-phenyl-oxazol-2-ylamino)-propionamide**

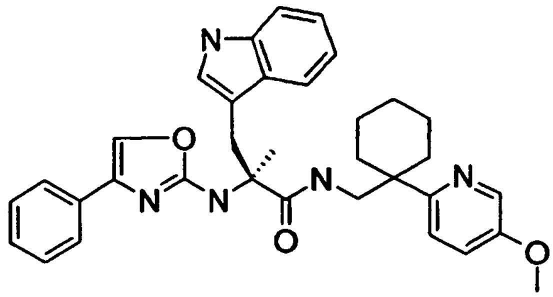

The above compound was synthesised from **2a** via **6b** as outlined in Scheme 2 using methods analogous to those used for **Example 9.** The acid (**6b**) (57mg, 0.148mmol), HBTU (56mg, 0.148mmol), DIPEA (26µl, 0.148mmol) were stirred in DMF (5ml) for 5 minutes before adding DIPEA (26µl, 0.148mmol) and [1-(5-methoxy-2-pyridyl)cyclohexyl]-methanamine (see WO 98/07718, 34mg, 0.148mmol). HPLC indicated that the reaction was complete within 2 hours. Solvent was removed under reduced pressure and the residue was taken up in EtOAc, washed with brine, sat. $NaHCO_3$ (x3), brine, dried ($MgSO_4$) and solvent removed under reduced pressure. The residue was purified by chromatography using RP silica with 70% MeOH in $H_2O$ as eluent. Repurification using NP 8g Biotage cartridge with 45% ethyl acetate in heptane as eluent gave the desired product as a glass (20mg, 24%):

MPt: 85-90°C;

MS m/e (ES+): 564.06 ($M^+$, 87%), 564.96 ($M^+$+ H, 100%);

IR (film): 3289, 2931, 2857, 1627, 1569, 1520, 1488, 1456, 1337, 1267, 1233, 1072, 1072, 1030, 939, 739 $cm^{-1}$;

$^1$H NMR (DMSO-$d_6$): δ= 0.95-1.45 (11H, m), 2.00-2.10 (2H, m), 3.10-3.25 (2H, m), 3.21 (1H, d, J=14.6 Hz), 3.59 (1H, d, J=14.6 Hz), 3.71 (3H, s), 6.84-7.14 (7H, m), 7.24-7.40 (5H, m,), 7.70 (2H, d, J=7.6 Hz), 8.05 (1H, s), 8.15 (1H, d, J=2.9 Hz), 10.82 (1H, s);

HPLC A: Rt. 12.01 min, 96.8/95.3% purity, 20-100% $CH_3CN$ in $H_2O$ (+0.1%TFA) over 15 min at 1mlmin$^{-1}$, Prodigy ODSIII 250x4.6mm 5µM, 215 and 254 nm;

HPLC B: Rt. 17.27 min, 100/100% purity, 80:20 methanol/Tris buffer at pH9, 1mlmin$^{-1}$, Prodigy ODSIII 250x4.6mm 5µM, 215 and 254 nm.

**Example 15**

**(S)-2-(4-Ethyl-oxazol-2-ylamino)-3-(1*H*-indol-3-yl)-*N*-[1-(5-methoxy-pyridin-2-yl)-cyclohexylmethyl]-2-methyl-propionamide**

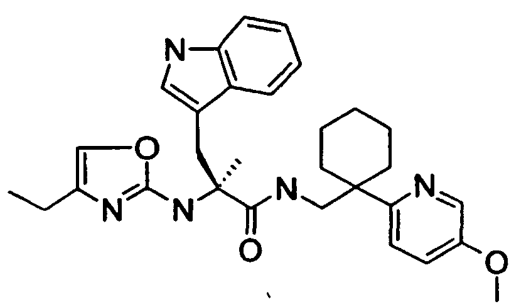

The above compound was synthesized from **2a** via **6c** as outlined in Scheme 2 using methods analogous to

those used for **Example 9.** The acid (**6c**) (188mg, 0.6mmol), HBTU (228mg, 0.6mmol), and DIPEA (105µl, 0.6mmol) were stirred in DMF (10ml) for 5 minutes before adding DIPEA (105µl, 0.6mmol) and [1-(5-methoxy-2-pyridyl)cyclohexyl]-methanamine (see WO 98/07718, 150mg, 0.65mmol). HPLC indicated that the reaction was complete within 4 hours. Solvent was removed under reduced pressure and residue was taken up in EtOAc, washed with brine, sat. $NaHCO_3$ (x3), brine, dried ($MgSO_4$) and solvent removed under reduced pressure. The residue was purified by chromatography using RP silica with 65% MeOH in $H_2O$. The product was repurified using 20g Mega Bond Elut silica cartridge with 45% ethyl acetate in heptane as eluent. Pure fractions were evaporated to give the above compound as a glass (30mg, 10%):

MPt: 60-65°C;

MS m/e (ES+): 516.24 ($M^+$ + H, 47%), 517.01 (100%), 538.10 ($M^+$ + Na, 25%);

IR (film): 3272, 3054, 2931, 2856, 1651, 1622, 1596, 1573, 1520, 1489, 1457, 1358, 1268, 1232, 1206, 1131, 1083, 1028, 949, 830, 740 $cm^{-1}$;

$^1H$ NMR (DMSO-$d_6$): δ= 1.10-1.50 (8H, m), 1.11 (3H, t, J=7.4 Hz), 1.29 (3H, s), 2.05-2.15 (2H, m), 2.28-2.34 (2H, m), 3.08-3.18 (3H, m), 3.48 (1H, d, J=14.4 Hz), 3.79 (3H, s), 6.80-6.90 (3H, m), 6.97-7.04 (2H, m,), 7.10-7.20 (3H, m), 7.27-7.30 (2H, m), 8.17 (1H, d, J=2.9 Hz), 10.80 (1H, s);

LCMS: Rt. 1.36 min, 100% purity, 5-100% $CH_3CN$ in $H_2O$ (+0.1% Formic acid) over 2 min at 4 $mlmin^{-1}$, Prodigy ODSIII 50x4.6mm 5µM, 215 nm, MS m/e (ES+) 515.95 (100%);

HPLC B: Rt. 12.29 min, 100/100% purity, 80:20 methanol/Tris buffer at pH9, 1 $mlmin^{-1}$, Prodigy ODSIII 250x4.6mm 5µM, 215 and 254 nm;

**Example 16**

**(S)-3-(1*H*-Indol-3-yl)-*N*-[1-(5-methoxy-pyridin-2-yl)-cyclohexylmethyl]-2-methyl-2-[4-(4-nitro-phenyl)-thiazol-2-ylamino]-propionamide**

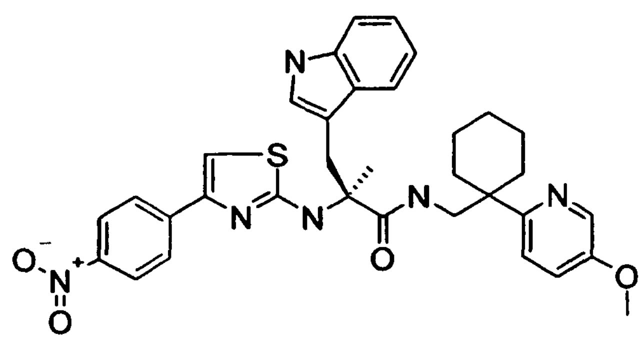

The above compound was synthesized using a one-pot procedure as outlined in Scheme 3. A suspension of H-S-αMeTrp-OH (**Intermediate 7**) (437mg, 2mmol), 2-chloro-4-(4-nitro-phenyl)-thiazole (see Peet, Norton P.; Sunder, Shyam. Reinvestigation of the reported preparation of 3-(4-nitrophenyl)thiazolo[2,3-c][1,2,4]triazepines, *J. Heterocycl. Chem.* (1986), 23(2), 593-5; 481mg, 2mmol), copper (I) iodide (38mg, 0.2mmol), and $K_2CO_3$ (415mg, 3mmol) in DMF (12ml) under nitrogen was heated to 130°C for 12 hours. The reaction mixture was cooled to ambient temperature before adding HBTU (759mg, 2mmol) and [1-(5-methoxy-2-pyridyl)cyclohexyl]-methanamine (see WO 98/07718; 441 mg, 2mmol). The mixture was stirred overnight, then concentrated in vacuo, after which the residue was partitioned between water (20ml) and dichloromethane (30ml). The organic phase was separated and filtered through silica (3x12cm) using 500ml of dichloromethane and then 500ml of dichloromethane-ether (1:1). Fractions containing product were concentrated under reduced pressure. The residue was absorbed onto 3.5g silica and purified by chromatography (3x11cm) using heptane-ethyl acetate (1:1.1). The product was repurified using RP chromatography (Biotage KP-C18-HS Flash 12M, 15ml.$min^{-1}$, 60-100% methanol in water). Concentration under reduced pressure gave the desired compound as a pale yellow amorphous solid (27mg, 2%):

MPt: 110-114°C;

MS m/e (AP+): 624.88 ($M^+$, 100%), 625.70 ($M^+$+ H, 52%);

IR (film): 3385, 3279, 2931, 2855, 1654, 1595, 1542, 1509, 1456, 1341, 1268, 1231, 1108, 1058, 908, 844, 731 $cm^{-1}$;

$^1H$ NMR ($CDCl_3$): δ= 1.15-1.55 (8H, m), 1.71 (3H, s), 1.90-2.00 (2H, m), 3.16-3.42 (2H, m), 3.46 (1H, d, J=14.9

Hz), 3.60 (1H, d, J=14.6 Hz), 3.70 (3H, s), 5.51 (1H, s), 6.89-6.93 (3H, m), 6.98 (1H, d, J=8.8 Hz), 7.05-7.10 (1H, m), 7.15-7.25 (2H, m), 7.34 (1H, d, J=8.3 Hz), 7.47 (1H, d, J=7.8 Hz), 7.90 (2H, d, J=9.0 Hz), 7.98 (1H, d, J=2.9 Hz), 9.05 (1H, s), 8.21 (2H, d, J=8.8 Hz);

HPLC A: Rt. 12.30min, 99.4% purity, 20-100% $CH_3CN$ in $H_2O$ (+0.1%TFA) over 15 min at 1mlmin$^{-1}$, Prodigy ODSIII 250x4.6mm 5μM, 200-300 nm;

HPLC B: Rt. 15.38min, 99.5% purity, 80:20 methanol/Tris buffer at pH9, 1mlmin$^{-1}$, Prodigy ODSIII 250x4.6mm 5μM, 200-300 nm.

**Example 17**

**(S)-2-(Benzooxazol-2-ylamino)-3-(1*H*-indol-3-yl)-2-methyl-*N*-(1-pyridin-2-yl-cyclohexylmethyl)-propionamide**

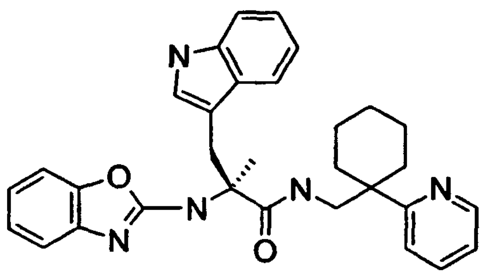

1. The following reagents were combined in the order that they are listed: **Intermediate 7** (545mg, 2.5mmol), 2-chlorobenzoxazole (384mg, 2.5mmol), potassium carbonate (346mg, 2.5mmol), benzyltriethylammonium chloride (TEBA, 114mg, 0.5mmol), triethylamine (1.04ml, 7.5mmol), DMF (12.5ml), deoxygenated water (1.25ml), copper (I) iodide (24mg, 0.125mmol), trans-dichlorobis(tri-*o*-tolylphosphine)palladium(II) (99mg, 0.125mmol). After heating at 100°C under nitrogen for 24 hours the DMF was removed under reduced pressure. The residue was taken up in ethyl acetate/water and the aqueous phase was acidified to pH 6-6.5 using citric acid. The aqueous phase was extracted with three further portions of ethyl acetate. The combined organic layers were dried ($MgSO_4$) and solvent was removed under reduced pressure. The residue was purified by chromatography using 10g NP silica with 0-100% ethyl acetate in heptane. Crystallisation from dichloromethane gave (S)-2-(benzooxazol-2-ylamino)-3-(1*H*-indol-3-yl)-2-methyl-propionic acid (245mg, 29%). MS m/e (ES+) 335.97 ($M^+$ + H, 100%), 336.69 (85%).

2. The propionic acid (234mg, 0.7mmol), HBTU (265mg, 0.7mmol), and DIPEA (122μl, 0.7mmol) were stirred in DMF (10ml) for 5 minutes before adding DIPEA (122μl, 0.7mmol) and [1-(2- pyridyl)cyclohexyl]methylamine (WO 98/07718; 140mg, 0.74mmol). After 4 hours at ambient temperature the solvent was removed under reduced pressure. The residue was purified by chromatography using NP silica with 50% ethyl acetate in heptane as eluent. Pure fractions were evaporated to give the desired compound as fine needles (44mg, 3%):

MPt: 198-200°C;

MS m/e ($ES^+$): 508.59 (100%, $M^+$ + H), 509.92 (10%);

IR (film): 3381, 3222, 3048, 2929, 2856, 1635, 1581, 1552, 1519, 1458, 1353, 1241, 1096, 742 cm$^{-1}$;

$^1H$ NMR ($CDCl_3$): δ = 1.20-1.60 (8H, m), 1.76 (3H, s), 1.95-2.05 (2H, m), 3.34 (1H, d.d, J=13.2 and 4.9 Hz), 3.45 (1H, d.d, J=13.2 and 5.6 Hz), 3.50 (2H, s), 5.67 (1H, s), 6.78-6.82 (1H, m), 6.89 (1H, d, J=2.2 Hz), 6.99-7.35 (10H, m), 7.43 (1H, d, J=8.1 Hz), 8.01 (1H, s), 8.24 (1H, d, J=4.6 Hz);

HPLC A: Rt. 10.54 min, 100/100% purity, 20-100% $CH_3CN$ in $H_2O$ (+0.1%TFA) over 15 min at 1mlmin$^{-1}$, Prodigy ODSIII 250x4.6mm 5μM, 215 and 254 nm;

HPLC B: Rt. 10.67 min, 100/100% purity, 80:20 methanol/Tris buffer at pH9, 1mlmin$^{-1}$, Prodigy ODSIII 250x4.6mm 5μM, 215 and 254 nm;

**Example 18**

**(S)-3-(1*H*-Indol-3-yl)-2-methyl-2-(pyridin-4-ylamino)-*N*-(1-pyridin-2-yl-cyclohexylmethyl)-propionamide**

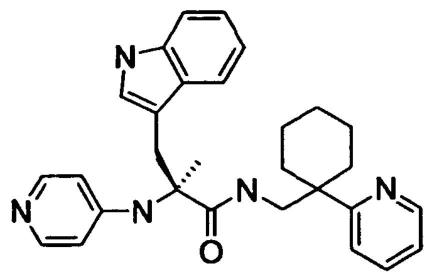

The above compound was prepared on the same scale and using an analogous method as used for **Example 17.**

1. The method of **Example 17** was repeated except that 4-bromopyridine hydrochloride (486mg, 2.5mmol) was used.

2. The acid from step 1 (30mg, 0.1 mmol), HBTU (38mg, 0.1mmol), and DIPEA (18µl, 0.1mmol) were stirred in DMF (10ml) for 5 minutes before adding DIPEA (18µl, 0.1mmol) and [1-(2- pyridyl)cyclohexyl]methylamine (WO 98/07718; 19mg, 0.1mmol). After 2 hours at ambient temperature the solvent was removed under reduced pressure. The residue was taken up in ethyl acetate and washed with sodium bicarbonate solution (x2), brine, and dried ($MgSO_4$). The solvent was removed under reduced pressure. The crude product was purified by chromatography using 10g ISCO Redisep cartridge with ethyl acetate as eluent. Repurification using 20g RP-C18 with 70% methanol in water and subsequent evaporation gave the desired product in crystalline form (6mg, 13%):

MPt: 180-195°C;

MS m/e ($AP^+$): 468.12 ($M^+$ + H, 100%), 469.59 ($M^+$ + 2H, 20%);

MS m/e ($AP^-$): 467.56 ($M^-$, 45%), 466.60 ($M^-$ - H, 100%), 465.64 ($M^-$ - 2H, 88%);

IR (film): 3316, 2930,1651, 1602, 1515, 1430, 1106, 997, 816, 741 $cm^{-1}$;

NMR ($CDCl_3$):$\delta$ = 1.25-1.70 (8H, m), 1.46 (3H, s), 2.00-2.10 (2H, m), 3.27 (1H, d, J=14.9 Hz), 3.30-3.48 (2H, m), 3.36 (1H, d, J=14.9 Hz), 4.43 (1H, s), 6.22 (2H, d, J=5.6 Hz), 6.85 (1H, d, J=2.0 Hz), 6.89-6.93 (1H, m), 7.11-7.37 (5H, m), 7.46-7.54 (2H, m), 8.08-8.13 (4H, m);

HPLC A: Rt. 7.21 min, 96.1/96.5% purity, 20-100% $CH_3CN$ in $H_2O$ (+0.1%TFA) over 15 min at 1mlmin$^{-1}$, Prodigy ODSIII 250x4.6mm 5µM, 215 and 254 nm;

HPLC B: Rt. 6.02 min, 99.1/100% purity, 80:20 methanol/Tris buffer at pH9, 1mlmin$^{-1}$, Prodigy ODSIII 250x4.6mm 5µM, 215 and 254 nm.

**Example 19**

**(S)-3-(1*H*-Indol-3-yl)-2-(isoquinolin-4-ylamino)-2-methyl-*N*-(1-pyridin-2-yl-cyclohexylmethyl)-propionamide**

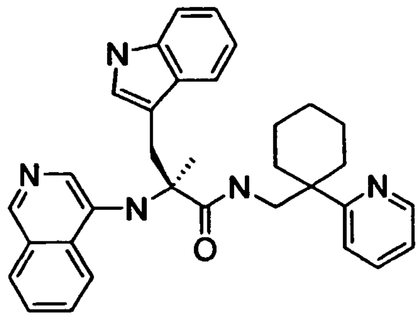

**Example 19** was prepared on the same scale and using an analogous method as used for **Example 17**.

1. The method of **Example 17** was followed except that 4-bromoisoquinoline (520mg, 2.5mmol) was used.

2. The acid from step 1 (40mg, 0.12mmol), HBTU (46mg, 0.12mmol), and DIPEA (21μl, 0.12mmol) were stirred in DMF (10ml) for 5 minutes before adding DIPEA (21μl, 0.12mmol) and [1-(2- pyridyl)cyclohexyl]methylamine (WO 98/07718; 23mg, 0.12mmol). After 2 hours at ambient temperature the solvent was removed under reduced pressure. The residue was taken up in ethyl acetate and washed with sodium bicarbonate solution (x2) and brine and dried ($MgSO_4$). The solvent was removed under reduced pressure. The crude product was purified by chromatography using 10g ISCO Redisep cartridge with 80% ethyl acetate in heptane as eluent. Repurification using 20g RP-C18 with 70% methanol in water and subsequent evaporation gave the desired product as a glass (9mg, 14%):

MPt: 98-101°C;

MS m/e ($AP^+$): 518.28 (100%, $M^+$ + H), 517.40 ($M^+$, 50%);

MS m/e ($AP^-$): 516.53 (75%, $M^-$), 515.63 (100%, $M^-$ - H);

IR (film): 3385, 3278, 3052, 2927, 2849, 1651, 1585, 1520, 1455, 1403, 1343, 781,740 $cm^{-1}$;

NMR ($CDCl_3$): δ= 1.20-1.65 (11H, m), 1.93-2.10 (2H, m), 3.35 (1H, d, J=14.6Hz), 3.39-3.52 (2H, m), 3.48 (1H, d, J=14.9 Hz), 4.62 (1H, s), 6.55-6.59 (1H, m), 6.90 (1H, d, J=2.0 Hz), 7.00 (1H, d, J=8.1 Hz), 7.17-7.28 (4H, m), 7.37-7.55 (4H, m), 7.62 (1H, s), 7.70 (1H, d, J=7.6 Hz), 7.74-7.76 (1H, m), 7.87 (1H, d, J=8.1 Hz), 8.15 (1H, s), 8.63 (1H, s)

HPLC A: Rt. 7.52 min, 100/100% purity, 20-100% $CH_3CN$ in $H_2O$ (+0.1%TFA) over 15 min at 1mlmin$^{-1}$, Prodigy ODSIII 250x4.6mm 5μM, 215 and 254 nm;

HPLC B: Rt. 8.33 min, 99.7/100% purity, 80:20 methanol/Tris buffer at pH9, 1mlmin$^{-1}$, Prodigy ODSIII 250x4.6mm 5μM, 215 and 254 nm;

**Example 20**

**(S)-3-(1*H*-Indol-3-yl)-2-methyl-*N*-(1-pyridin-2-yl-cyclohexylmethyl)-2-(pyrimidin-5-ylamino)-propionamide**

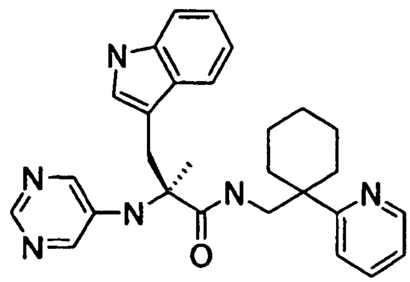

The above compound was prepared on the same scale and using an analogous method as used for **Example 17**.

1. The method of **Example 17** was followed except that 5-bromopyrimidine (397mg, 2.5mmol) was used.

2. The acid from step 1 (150mg, 0.5mmol), HBTU (190mg, 0.5mmol), and DIPEA (87µl, 0.5mmol) were stirred in DMF (10ml) for 5 minutes before adding DIPEA (87µl, 0.5mmol) and [1-(2-pyridyl)cyclohexyl]methylamine (WO 98/07718; 95mg, 0.5mmol). After 2 hours at ambient temperature the solvent was removed under reduced pressure. The residue was taken up in ethyl acetate and washed with sodium bicarbonate solution (x2) and brine and dried ($MgSO_4$). The solvent was removed under reduced pressure. The crude product was purified by chromatography using 10g ISCO Redisep cartridge with 90% ethyl acetate in heptane as eluent. Removal of the solvent under reduced pressure gave the desired product as a foam (135mg, 58%):

MPt: 95-98°C;

MS m/e ($AP^+$): 470.60 (25%), 469.58 ($M^+$ +H, 100%), 468.77 ($M^+$, 92%);

MS m/e ($AP^-$): 467.60 ($M^-$ - H, 70%), 466.85 (100%);

IR (film): 3291, 3052, 2931, 2857, 1651, 1575, 1519, 1470, 1455, 1427, 1357, 1306, 1265, 1237, 1194, 1156, 1106, 1010, 848, 788, 739 $cm^{-1}$;

NMR ($CDCl_3$): δ= 1.20-1.65 (8H, m), 1.48 (3H, s), 2.00-2.10 (2H, m), 3.24-3.48 (4H, m), 4.14 (1H, s), 6.88-6.92 (2H, m), 7.13-7.24 (3H, m), 7.37 (1H, d, J=8.1 Hz), 7.48-7.55 (3H, m), 7.86 (2H, s), 8.08-8.10 (1H, m), 8.16 (1H, s), 8.57 (1H, s);

HPLC A: Rt. 8.94 min, 99.3/99.4% purity, 20-100% $CH_3CN$ in $H_2O$ (+0.1%TFA) over 15 min at 1mlmin$^{-1}$, Prodigy ODSIII 250x4.6mm 5µM, 215 and 254 nm;

HPLC B: Rt. 5.76 min, 95.1/98.7% purity, 80:20 methanol/Tris buffer at pH9, 1mlmin$^{-1}$, Prodigy ODSIII 250x4.6mm 5µM, 215 and 254 nm.

**Example 21**

**(S)-2-(Biphenyl-2-ylamino)-3-(1*H*-indol-3-yl)-2-methyl-*N*-(1-pyridin-2-yl-cyclohexylmethyl)-propionamide**

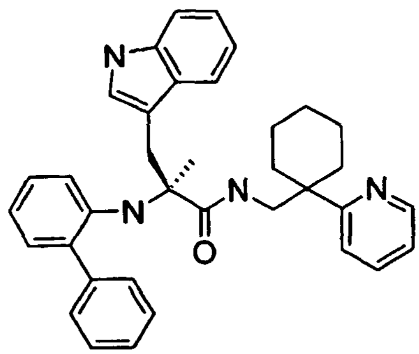

The above compound was prepared on the same scale and using an analogous method as used for **Example 17.**

1. The method of **Example 18** except for the use of 2-bromo biphenyl (583mg, 2.5mmols).

2. The acid from step 1 (350mg, 0.95mmol), HBTU (400mg, 1mmol), $NEt_3$ (0.5ml, 3.5mmol), and 1-(2- pyridyl) cyclohexyl]methylamine (WO 98/07718; 200mg, 1 mmol) were stirred in DMF (15ml). After 1 hour at ambient temperature the reaction mixture was diluted with ethyl acetate (100ml), washed with sodium bicarbonate solution (x2) and dried ($MgSO_4$). The solvent was removed under reduced pressure. The crude product was purified by chromatography using 0-50% ethyl acetate in heptane and then 0-30% dichloromethane in ether as eluent. Removal of the solvent under reduced pressure gave the desired product as a foam (98mg, 19% for step 2):

MS m/e ($AP^+$): 565 ($M^+$ + Na, 100%), 564 (80%), 542 ($M^+$, 30%)

IR (KBr disc): 3404, 2928, 2855, 1650, 1584, 1508, 1489, 1458, 1432 $cm^{-1}$;

NMR (DMSO-$d_6$): $\delta$ = 1.10-1.52 (8H, m), 1.27 (3H, s), 1.95-2.05 (2H, m), 2.95 (1H, d, J=14.4 Hz), 3.02-3.08 (1H, m), 3.08 (1H, d, J=14.6 Hz), 3.28-3.34 (1H, m), 4.36 (1H, s), 6.37 (1H, d, J=8 Hz), 6.49 (1H, d, J=2.2 Hz), 6.71-6.75 (1H, m), 6.82-6.86 (1H, m), 6.95-7.43 (13H, m), 7.52-7.57 (1H, m), 8.33 (1H, d, J=3.7 Hz), 10.81 (1H, s);

HPLC A: Rt. 12.65min, 99.65% purity, 20-100% $CH_3CN$ in $H_2O$ (+0.1%TFA) over 15 min at 1mlmin$^{-1}$, Prodigy ODSIII 250x4.6mm 5μM, 200-300 nm;

HPLC B: Rt. 33.05min, 99.89% purity, 80:20 methanol/Tris buffer at pH9, 1mlmin$^{-1}$, Prodigy ODSIII 250x4.6mm 5μM, 200-300 nm.

**Example 22**

**(S)-3-(1*H*-Indol-3-yl)-2-methyl-*N*-(1-pyridin-2-yl-cyclohexylmethyl)-2-*m*-tolylamino-propionamide**

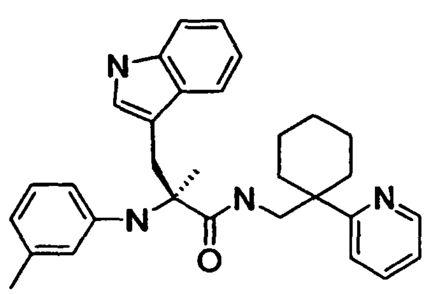

The above compound was prepared using a one-pot procedure analogous to the method used for **Example 16.** The synthesis was carried out on 1mmol scale using 1-bromo-3-methyl-benzene (171mg, 1mmol). The crude product was purified by chromatography using 25g NP silica with 25% ethyl acetate in heptane as eluent. Removal of the solvent under reduced pressure gave the desired compound as a glass (260mg, 54%):

MPt: 70-75°C;

MS m/e ($AP^+$): 481.33 (100%, $M^+$ + H), 482.37 (40%);

IR (film): 3385, 3291, 3049, 2929, 2857, 1652, 1607, 1590, 1513, 1456, 1431, 1341, 1302, 1264, 1237, 1177, 1155, 1104, 1010, 774, 741 $cm^{-1}$;

NMR (DMSO-$d_6$): δ= 1.08-1.50 (8H, m), 1.19 (3H, s), 2.00-2.10 (2H, m), 2.16 (3H, s), 3.03 (1H, d.d, J=12.9 and 5.1 Hz), 3.10 (1H, d, J=14.7Hz), 3.22 (1H, d, J=14.6Hz), 3.24-3.30 (1H, m), 5.43 (1H, s), 6.29 (1H, s), 6.30 and 6.44 (each 1H, each d, J=7.6 Hz), 6.87-7.07 (6H, m), 7.15-7.19 (1H, m), 7.29 (1H, d, J=8.0 Hz), 7.33 (1H, d, J=7.8 Hz), 7.48-7.54 (1H, m), 8.31-8.33 (1H, m), 10.81 (1H, s);

HPLC A: Rt. 11.04 min, 98.3% purity, 20-100% $CH_3CN$ in $H_2O$ (+0.1%TFA) over 15 min at 1mlmin$^{-1}$, Prodigy ODSIII 250x4.6mm 5μM, 200-300 nm;

HPLC B: Rt. 16.87 min, 99.5% purity, 80:20 methanol/Tris buffer at pH9, 1mlmin$^{-1}$, Prodigy ODSIII 250x4.6mm 5μM, 200-300 nm.

**Example 23**

**(S)-3-(1*H*-Indol-3-yl)-2-methyl-2-(6-phenyl-pyridin-2-ylamino)-*N*-(1-pyridin-2-yl-cyclohexylmethyl)-propionamide**

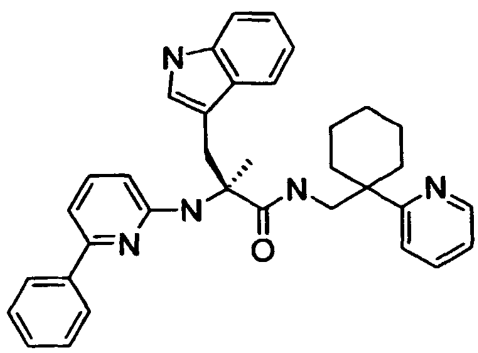

The above compound was prepared using a one-pot procedure analogous to the method used for **Example 16.** The synthesis was carried out on 0.4mmol scale using 2-bromo-6-phenyl-pyridine (95mg, 0.4 mmol). The crude product was purified by chromatography using 25g NP silica with 55% ethyl acetate in heptane as eluent. Removal of the solvent under reduced pressure gave the desired product as a foam (260mg, 54%):

MS m/e ($AP^+$) 544.31 (100%, $M^+$ + H), 545.35 (35%);

MS m/e ($AP^-$) 542.29 (100%, $M^-$ - H), 543.31 ($M^-$, 40%);

IR (film): 3407, 3276, 3056, 2930, 2857, 1651, 1595, 1576, 1519, 1486, 1467, 1455, 1439, 1339, 1264, 1180, 1157, 1105, 1028, 1009, 991, 804, 763, 739 $cm^{-1}$;

NMR ($CDCl_3$) δ= 1.03-1.60 (8H, m), 1.53 (3H, s), 1.90-2.03 (2H, m), 3.32-3.45 (3H, m), 3.65 (1H, d, J=14.6Hz), 4.67 (1H, s), 6.13 (1H, d, J=8.3 Hz), 6.77-7.50 (14H, m), 7.97 (2H, d, J=7.1 Hz), 8.02 (1H, s), 8.23-8.25 (1H, m);

HPLC A: Rt. 4.21 min, 96.8% purity, 20-100% $CH_3CN$ in $H_2O$ (+0.1%TFA) over 7 min at 1.5mlmin$^{-1}$, Prodigy ODSIII 150x4.6mm 5μM, 200-300 nm.

**Example 24**

**(R)-3-Phenyl-2-phenylamino-*N*-[1-pyridin-2-yl-cyclohexylmethyl)-propionamide**

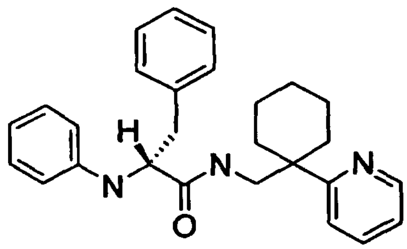

The above compound was synthesised as a two step process from **Intermediate 8** as shown in Scheme 4.

1. To a solution of **Intermediate 8** (0.5g, 3mmol) and bromobenzene (0.35ml, 3.3mmol) in DMA (5ml) under nitrogen was added potassium carbonate (0.6g, 4.3mmol) and copper (I) iodide (50mg, 0.26mmol) after which the mixture was heated to 90°C for 1.5 hours. Solvent was removed under reduced pressure and the residue was purified by flash chromatography eluting with 5% methanol in dichloromethane. Removal of solvent under reduced pressure gave (R)-3-phenyl-2-phenylamino-propionic acid as an oil (0.41g, 56%):
MS m/e ($AP^+$): 242 ($M^+$ + H, 100%).

2. The acid from step 1 (0.40g, 1.66mmol), HBTU (0.6g, 1.8mmol), and $NEt_3$ (0.5ml, 3.5mmol), and 1-(2-pyridyl) cyclohexyl]methylamine (WO 98/07718; 0.35mg, 1.8mmol) were stirred in DMF (15ml). After 1 hour at ambient temperature the reaction mixture was diluted with ethyl acetate (100ml), washed with sodium bicarbonate solution (x2) and dried ($MgSO_4$). The solvent was removed under reduced pressure. The crude product was purified by chromatography using 50% ethyl acetate in heptane and then RP C18 silica with 70% methanol in water as eluent. Removal of the solvent under reduced pressure gave the desired product as a white amorphous solid (0.15g, 22%):
MPt: 113-115°C;
MS m/e ($AP^+$): 414.22 ($M^+$ + H, 100%);
IR (KBr disc): 3300, 2931, 2858, 1649, 1605, 1589, 1523, 1498, 1432, 1318, 748 $cm^{-1}$;
NMR ($CDCl_3$): $\delta$ = 1.20-1.70 (8H, m), 1. 90-2.15 (2H, m), 2.91 (1H, d.d, J=14.2 and 8.8 Hz), 3.27 (1H, d.d, J=14.2 and 4.4 Hz), 3.38 (1H, d.d, J=13.2 and 5.5 Hz), 3.48 (1H, d.d, J=13.2 and 6.1 Hz), 3.80 (1H, d, J=3.4 Hz), 3.88-3.93 (1H, m), 6.44 (2H, d, J=7.8 Hz), 6.74 (1H, t, J=11.3 Hz), 6.90-7.45 (11H,m), 8.28 (1H, d, J=3.6 Hz);
HPLC A: Rt. 4.51 min, 100% purity, 20-100% $CH_3CN$ in $H_2O$ (+0.1%TFA) over 10 min at 1.5 $mlmin^{-1}$, Prodigy ODSIII 250x4.6mm 5μM, 200-300 nm;
HPLC B: Rt. 13.15 min, 99.14% purity, 80:20 methanol/Tris buffer at pH9, 1$mlmin^{-1}$, Prodigy ODSIII 250x4.6mm 5μM, 200-300 nm.

**Example 25**

**(S)-3-(1*H*-Indol-3-yl)-2-methyl-2-phenylethylamino-*N*-(1-pyridin-2-yl-cyclohexylmethyl)-propionamide**

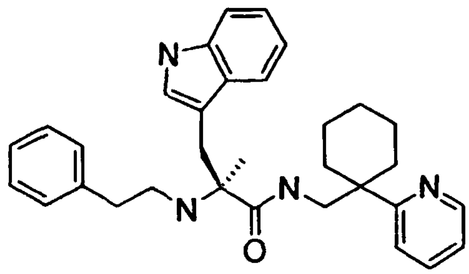

The above compound was prepared as shown in Scheme 5 via **Intermediate 10**.

1. To a stirred solution of H-(S)-αMeTrp-OH (7) (10g, 46mmol) and di-t-butyldicarbonate (10g, 46mmol) in dioxan (100ml) was added water (20ml) and potassium carbonate (10g, 74mmol). After 4 hours the reaction mixture was acidified with 2N hydrochloric acid (150ml) and product was extracted with ethyl acetate (2 x 200ml). The combined organic phases were dried ($MgSO_4$) and evaporated under reduced pressure. The residue was purified by flash chromatography using ethyl acetate as eluent. Removal of solvent under reduced pressure gave Boc-(S)-αMeTrp-OH as an orange oil (14.5g, 99%). To a stirred solution of Boc-(S)-αMeTrp-OH (7g, 22mmol) in DMF (100ml) was added HBTU (8.0g, 22mmol), triethylamine (5ml, 35mmol), and [1-(2-pyridyl)cyclohexyl]methylamine (WO 98/07718; 4.2g, 22mmol). After 1 hour the reaction mixture was diluted with ethyl acetate (300ml), washed with 2N hydrochloric acid (2 x 200ml), dried ($MgSO_4$) and evaporated under reduced pressure at 60°C. The residue was purified by flash chromatography. Elution with 5% methanol in dichloromethane and subsequent removal of solvent under reduced pressure gave intermediate **9** as yellow oil (8.3g, 77%):

MS m/e (AP+): 491 ($M^+$ + H, 100%), 513 ($M^+$ + Na, 20%);

IR (film): 3339, 2929, 2858, 1704, 1659, 1651, 1589, 1519, 1487, 1366, 1249, 1164, 1070, 908, 737 $cm^{-1}$;

NMR ($CDCl_3$): δ = 1.20-1.70 (20H, m), 2.00-2.12 (2H, m), 3.25-3.50 (4H, m), 5.05-5.20 (1H, br.s), 6.92 (1H, d, J=2.0 Hz), 7.02-7.32 (6H, m), 7.51 (1H, d, J=8.0 Hz), 7.59-7.64 (1H, m), 8.03 (1H, s), 8.48 (1H, d, J=4 Hz).

2. To a stirred solution of **Intermediate 9** (8.2g, 16.5mmol) in dichloromethane (100ml) was added trifluoroacetic acid (3.0ml, 39mmol). After 18 hours the solvent was removed under reduced pressure at 60°C. The residue was treated cautiously with saturated sodium carbonate solution (200ml) before extracting with ethyl acetate (3 x 200ml). The combined organic phases were dried ($MgSO_4$) and evaporated under reduced pressure at 60°C. The residue was purified by flash chromatography. Elution with 0-5% methanol in dichloromethane and subsequent removal of solvent under reduced pressure gave **Intermediate 10** as white foam (4.85g, 75%):

MPt: 65-68°C;

MS m/e (AP+): 391 ($M^+$ + H, 100%);

IR (KBr disc): 3367, 2926, 2855, 1648, 1589, 1569, 1522, 1455, 1430, 1366, 1341, 1234, 842, 784, 742 $cm^{-1}$;

NMR ($CDCl_3$): δ = 1.20-1.80 (13H, m), 1.98-2.20 (2H, m), 2.83 (1H, d, J=14.2 Hz), 3.33 (1H, d, J=14.2 Hz), 3.38 (2H, d, J=5.6 Hz), 6.98-7.20 (6H, m), 7.50-7.75 (3H, m), 8.05-8.15 (1H, s), 8.49-8.51 (1H, m);

3. To a stirred solution of **Intermediate 10** (293mg, 0.75mmol) and phenacetaldehyde (90mg, 0.75mmol) in 1,2-dichloroethane (20ml) was added solid sodium triacetoxyborohydride (316mg, 1.5mmol). After stirring overnight, saturated sodium bicarbonate solution was added - effervescence was observed. The aqueous phase was extracted with dichloromethane. The combined organic phases were dried ($MgSO_4$) and solvent was removed under reduced pressure. The residue was purified by chromatography using 20g RP-C18 with 0-50% methanol in water followed by 20g NP silica with 45% ethyl acetate in heptane. Removal of solvent under reduced pressure gave the desired compound as a glass (60mg, 16%):

MS m/e ($ES^+$): 496.56 (28%), 495.5 (52%, $M^+$ + H), 364.43 (22%), 269.34 (51%), 268.90 (88%), 248.37 (100%);

IR (film): 3274, 3058, 2928, 2856, 1651, 1588, 1568, 1519, 1469, 1454, 1431, 1355, 1263, 1236, 1155, 1117,

1053, 1030, 1009, 992, 930, 782, 742 $cm^{-1}$;

$^1H$ NMR ($CDCl_3$): δ = 1.20-1.65 (11H, m), 2.00-2.20 (2H, m), 2.40-2.75 (4H, m), 2.94 and 3.05 (each 1H, each d, J=14.4 Hz), 3.41 (2H, d, J=6.1 Hz), 6.74 (1H, d, J=2.2 Hz), 7.04-7.25 (9H, m), 7.32 (1H, d, J=7.8 Hz), 7.55-7.60 (3H, m), 7.90 (1H, s), 8.55-8.58 (1H, m);

HPLC A: Rt. 8.52 min, 99.0/98.6% purity, 20-100% $CH_3CN$ in $H_2O$ (+0.1%TFA) over 15 min at 1mlmin$^{-1}$, Prodigy ODSIII 250x4.6mm 5µM, 215 and 254 nm;

HPLC B: Rt. 23.84 min, 99.6/100% purity, 80:20 methanol/Tris buffer at pH9, 1mlmin$^{-1}$, Prodigy ODSIII 250x4.6mm 5µM, 215 and 254 nm.

**Example 26**

**(S)-2-[(Benzofuran-2-ylmethyl)-amino]-3-(1*H*-indol-3-yl)-2-methyl-*N*-(1-pyridin-2-yl-cyclohexylmethyl)-propionamide**

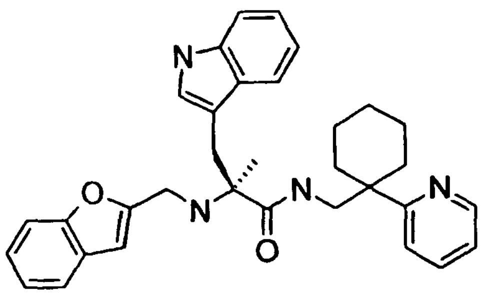

The above compound was prepared as shown in Scheme 5 via **Intermediate 10**.
To a stirred solution of **Intermediate 10** (150mg, 0.38mmol) and benzofuran-2-carbaldehyde (56mg, 0.38mmol) in 1,2-dichloroethane (5ml) was added solid sodium triacetoxyborohydride (162mg, 0.77mmol). After stirring at room temperature for 48 hours saturated sodium bicarbonate solution was added - effervescence was observed. The aqueous phase was extracted with ethyl acetate. The combined organic phases were dried ($MgSO_4$) and solvent removed under reduced pressure. The residue was purified by chromatography using 60% ethyl acetate in heptane. Removal of solvent under reduced pressure gave the desired product as an amorphous white solid (29mg, 15%):

MS m/e ($ES^+$): 521.08 ($M^+$ + H, 100%), 391.06 (50%);

IR (film): 3268, 3056, 2930, 2856, 1656, 1588, 1569, 1519, 1469, 1454, 1431, 1355, 1342, 1255, 1171, 1105, 1052, 1009, 909, 788, 740 $cm^{-1}$;

$^1H$ NMR ($CDCl_3$): δ = 1.20-2.20 (14H, m), 3.08 (1H, d, J=14.4 Hz), 3.14 (1H, d, J=14.8 Hz), 3.45-3.49 (2H, m), 3.66 (1H, d, J=14.4 Hz), 3.76 (1H, d, J=14.8 Hz), 6.33 (1H, s), 6.84-6.88 (1H, m), 7.00-7.65 (12H, m), 8.32 (1H, s), 8.39 (1H, d, J=4.0 Hz);

HPLC A: Rt. 8.86 min, 99.7/99.1% purity, 20-100% $CH_3CN$ in $H_2O$ (+0.1%TFA) over 15 min at 1mlmin$^{-1}$, Prodigy ODSIII 250x4.6mm 5µM, 215 and 254 nm.

**Example 27**

**(S)-3-(1*H*-Indol-3-yl)-2-methyl-2-(4-nitro-benzylamino)-*N*-(1-pyridin-2-yl-cyclohexylmethyl)-propionamide**

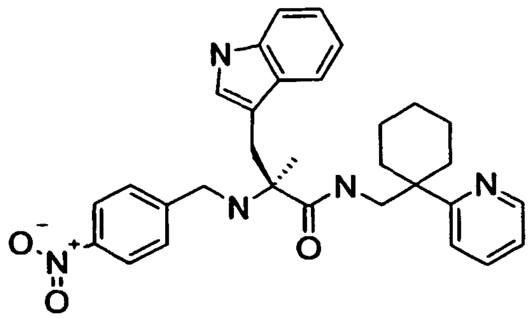

The above compound was prepared as shown in Scheme 5 via **Intermediate 10**. To a stirred solution of **Intermediate 10** (150mg, 0.38mmol) and 4-nitrobenzaldehyde (58mg, 0.38mmol) in 1,2-dichloroethane (5ml) was added solid sodium triacetoxyborohydride (114mg, 0.54mmol). After stirring at room temperature for 24 hours saturated sodium bicarbonate solution was added - effervescence was observed. The aqueous phase was extracted with ethyl acetate. The combined organic phases were dried ($MgSO_4$) and solvent removed under reduced pressure. The residue was purified by chromatography using 60% ethyl acetate in heptane. Repurifcation using RP silica with 45% methanol in water (+ 1% acetic acid) gave pure product. The pure fractions were combined, basified (sodium carbonate), and extracted with ethyl acetate. Removal of solvent under reduced pressure gave the desired compound as a glass (10.5mg, 5%):

MPt: 58-60°C;

MS m/e ($ES^+$): 526.15 ($M^+$ + H, 100%), 527.14 (33%);

IR (film): 3365, 2924, 2856, 1652, 1513, 1429, 1346, 1257, 1048 $cm^{-1}$; $^1H$ NMR (DMSO-$d_6$): $\delta$ = 1.10-1.55 (8H, m), 1.19 (3H, s), 1.88-2.08 (2H, m), 2.25-2.30 (1H, m), 2.95-3.02 (2H, m), 3.10-3.20 (1H, m), 3.17-3.27 (1H, m), 3.50-3.80 (2H, m), 6.93-7.63 (11H, m), 8.12 (2H, d, J=8.8 Hz), 8.42 (1H, d, J=3.6 Hz), 10.86 (1H, s).

**Example 28**

**$BB_1$ and $BB_2$ Binding Assays**

In the following experiments, measurement of $BB_1$ and $BB_2$ binding was as follows. CHO-K1 cells stably expressing cloned human NMB (for ($BB_1$ assay) and GRP receptors (for $BB_2$ assay) were routinely grown in Ham's F12 culture medium supplemented with 10% foetal calf serum and 2 mM glutamine. For binding experiments, cells were harvested by trypsinization, and stored frozen at -70°C in Ham's F12 culture medium containing 5% DMSO until required. On the day of use, cells were thawed rapidly, diluted with an excess of culture medium, and centrifuged for 5 minutes at 2000 g. Cells were resuspended in 50 mM Tris-HCl assay buffer (pH 7.4 at 21°C, containing 0.02% BSA, 40μg/mL bacitracin, 2μg/mL chymostatin, 4μg/mL leupeptin, and 2μM phosphoramidon), counted, and polytronned (setting 5, 10 sec) before centrifuging for 10 minutes at 28,000 g. The final pellet was resuspended in assay buffer to a final cell concentration of $1.5 \times 10^5$/mL. For binding assays, 200μL aliquots of membranes were incubated with [$^{125}I$][$Tyr^4$]bombesin (<0.1 nM) in the presence and absence of test compounds (final assay volume 250μL) for 60 minutes and 90 minutes for NMB and GRP receptors, respectively. Nonspecific binding was defined by 1μM bombesin. Assays were terminated by rapid filtration under vacuum onto Whatman GF/C filters presoaked in 0.2% PEI for >2 hours, and washed 50 mM Tris-HCl (pH 6.9 at 21°C; 6 × 1 mL). Radioactivity bound was determined using a gamma counter.

All competition data was analysed using nonlinear regression utilizing iterative curve-plotting procedures in Prism® (GraphPad Software Inc., San Diego, USA). $IC_{50}$ values were corrected to $K_i$ values using the Cheng-Prusoff equation (Cheng Y., Prusoff W. H., *Biochem. Pharmacol.* 22: 3099-3108, 1973).

The results obtained are listed in Table 1.

Table 1 :

| Human NMB and GRP receptor binding affinities | | |
|---|---|---|
| Example No. | NMB $K_i$ (nM) | GRP $K_i$ (nM) |
| 9 | 4 | 24 |
| 10 | 469 | |
| 11 | 5580 | |
| 12 | 16 | 2820 |
| 13 | 19 | 1385 |
| 14 | 106 | 1190 |
| 15 | 213 | 1770 |
| 16 | 15 | |
| 17 | 2080 | |
| 18 | 303 | |
| 19 | 1249 | |
| 20 | 3163 | |
| 21 | 824 | |
| 22 | 653 | |
| 23 | 3371 | |
| 24 | 137 | |
| 25 | 616 | 2620 |
| 26 | 2400 | |
| 27 | 652 | |

**Example 29**

**Effect of (S)-3-(1*H*-Indol-3-yl)-*N*-[1-(5-methoxy-pyridin-2-yl)-cyclohexylmethyl]-2-methyl-2-[4-(4-nitro-phenyl)-oxazol-2-ylamino]-propionamide (Compound (2)) in PEG200 on female rat sexual proceptivity**

Ovariectomised adult female Sprague Dawley rats (180-200g, from Charles River) were housed in groups of 6 in a reversed lighting system of 12h light:dark (lights off 7.00-19.00h). Two weeks after ovariectomy they were used for sexual activity tests. Animals were adapted to the apparatus (in the absence of stimuli animals) for 10min on 2 consecutive days prior to testing. The experiments started at least 5h into the dark period.

Tests were carried out in a circular arena of 90cm diameter, surrounded by a 30cm high wall. Two small cages with wire-mesh front (15x15cm) are fixed into the wall such that the front of the cage is "flush" with the wall and the 2 cages are opposite each other. They contain two stimuli animals: an intact sexually experienced male and a receptive female (ovariectomised, primed with 5µg oestradiol benzoate dissolved in corn oil and injected subcutaneously 48 hours before the test and with 0.5 mg of progesterone four hours before the test). Sexually naive test and control animals were used. Forty eight hours before the tests, both the test and control animals were primed with 5µg oestradiol benzoate. Test animals were treated with the above compound (30-100mg/kg) which was dissolved in PEG 200 vehicle and administered orally in a 1ml/kg volume 1h before each test. For animals used as positive controls, progesterone (0.5mg/0.1ml) was dissolved in corn oil and administered subcutaneously (s.c.), 4h before the test. Test and control animals were introduced one at a time for 10 minute periods into the arena. During the 10min test, the time that the test or positive control animal spent investigating each stimulus animal was noted. The arena was thoroughly cleaned between animals. The position of the male/female stimuli boxes was randomised between animals, in order to avoid place preference. The difference in the percentage of time spent investigating male minus female was calculated, out of the total time spent investigating stimuli animals.

It was found (see **Figure 19)** that the above compound dose-dependently (30-100) increased the percentage of time spent investigating the male stimulus, with a MED of 100mg/kg (see below). The effect of this dose was similar to the effect of progesterone (maximal). (*P<0.05, **P<0.01 Kruskal-Wallis followed by Mann-Whitney test, *vs* vehicle).

**Example 30**

**Effect of Compound (2) in methyl cellulose on female rat sexual proceptivity.**

Example 29 was repeated except that the above compound (3-30 mg/kg) was dissolved in 0.5% methyl cellulose and was administered p.o. in a dosing volume of 3ml/kg 1h before tests. Progesterone, (0.5mg/0.1ml) was dissolved in corn oil and administered s.c., 4h before test, as a positive control.

The above compound dose-dependently (3-30mg/kg) increased the percentage of time spent investigating the male stimulus, with a MED of 10mg/kg. This represents a 10-fold increase in potency compared to the oral results obtained in the PEG200 vehicle (MED=100mg/kg). The results are shown in **Figure 20** in which bars represent percentage of time spent investigating male, minus the percentage of time spent investigating the female stimuli±SEM, (n=6-9 per group). *P<0.05, **P<0.01 *vs* vehicle (One-way ANOVA followed by Dunnett's test *vs* vehicle group).

**Example 31**

**Effect of Compound (2) in PEG 200 on female rat sexual receptivity.**

Ovariectomised adult female Sprague Dawley rats (180-200g, from Charles River) were housed in groups of 6 in a reversed lighting system of 12h light:dark (lights off 7.00-19.00h). Two weeks after ovariectomy they were used for sexual activity tests. The experiments started at least 5h into the dark period.

The above compound was dissolved in PEG200 vehicle and administered orally. Quinelorane dihydrochloride (LY 163,502, 6.25µg/kg) was dissolved in water and administered subcutaneously (s.c.), as a positive control. Both compounds were administered in a 1ml/kg volume.

Forty eight hours before tests, the animals were primed with 5µg oestradiol benzoate (Sigma Chemical. Co. Ltd., UK) dissolved in corn oil and injected subcutaneously. The females were placed with a series of vigorous male rats and subjected to 10 mounts. The lordotic response of the animal was recorded and expressed as a percentage of the mounts (i.e. lordosis quotient, LQ). Treatment induced LQ=0-10% in most of the animals, which were considered non-receptive (NR). Animals showing higher LQ were not included in the study. Each rat was tested prior to administration of the compound and then tested similarly at 1h and 90min post-injection of the above compound or quinelorane respectively.

A single administration of quinelorane (6.25µg/kg) significantly (P<0.01) increased the LQ, 90min after administration, compared to the LQ shown before administration (paired t test). A single oral administration of the above compound dose-dependently (10-100mg/kg) increased the LQ 1h after administration, with a MED of 100mg/kg (P<0.01) compared to the LQ shown before administration (paired t test). The effect of the above compound (100mg/kg) was similar to the effect of quinelorane (6.25µg/kg) as is shown in **Figure 21.**

**Synthesis Example (compounds of formula (III))**

**(S)-2-Amino-3-(1 *H*-indol-3-yl)-2-methyl-*N*-(1-pyridin-2-yl-cyclohexylmethyl)-propionamide (Intermediate III-7) and (S)-2-Amino-3-(1 *H*-indol-3-yl)-2-methyl-*N*-(1-(5-methoxy-pyridin-2-yl)-cyclohexylmethyl)-propionamide (Intermediate III-6)**

In reaction scheme 7 below, Intermediates III-6 and III-7 are made by (i) protecting the amino group of the starting amino acid **a** with di-*t*-butyl carbonate and potassium carbonate in dioxane/water, (ii) forming an amide by reaction of the *N*-protected amino acid with an amine **b1** or **b2** in dimethylformamide in the presence of *O*-benzotriazol-1-yl-*N,N,N',N'*-tetramethyluronium hexafluorophosphate (HBTU) and *N,N*-diisopropyl-ethylamine (DIPEA), and (iii) deprotecting the amino group of the product **c1** or **c2** by reaction with trifluoroacetic acid in dichloromethane.

**Scheme 7**

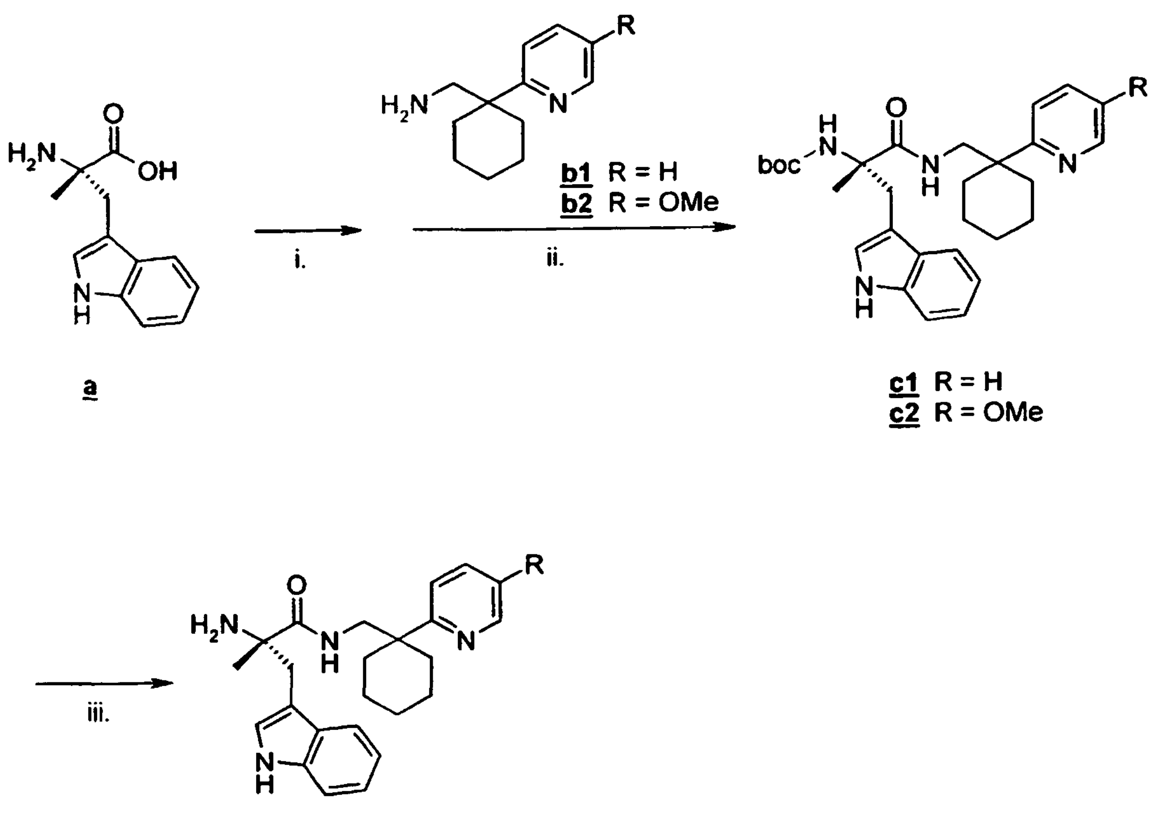


a

c1 R = H
c2 R = OMe

**Intermediate III-7** R = H
**Intermediate III-6** R = OMe

i. $BOC_2O$, $K_2CO_3$, dioxane, water
ii. HBTU, DIPEA, DMF
iii. TFA, $CH_2Cl_2$

**{(S)-2-(1-*H*-Indol-3-yl)-1-methyl-1-[(1-pyridin-2-yl-cyclohexylmethyl)-carbamoyl]-ethyl}-carbamic acid *tert*-butyl ester (c1)**

(1) To a stirred solution of H-(S)-aMeTrp-OH (a) (10g, 46mmol) and di-t-butyldicarbonate (10g, 46mmol) in dioxane (100ml) was added water (20ml) and potassium carbonate (10g, 74mmol). After 4 hours the reaction mixture was acidified with 2N hydrochloric acid (150ml) and product extracted with ethyl acetate (2 x 200ml). The combined organic phases were dried ($MgSO_4$) and evaporated under reduced pressure. The residue was purified by flash chromatography, eluting with ethyl acetate. Removal of solvent under reduced pressure gave Boc-(S)-αMeTrp-OH as orange oil (14.5g, 99%).

(2) To a stirred solution of Boc-(S)-αMeTrp-OH (7g, 22mmol) in DMF (100ml) was added HBTU (8.0g, 22mmol), triethylamine (5ml, 35mmol), and [1-(2-pyridyl)cyclohexyl]methylamine (**b1**, 4.2g, 22mmol, described in WO 98/07718). After 1 hour the reaction mixture was diluted with ethyl acetate (300ml) and washed with 2N hydrochloric acid (2 x 200ml), dried ($MgSO_4$) and evaporated under reduced pressure at 60°C. The residue was purified by flash chromatography. Elution with 5% methanol in dichloromethane and subsequent removal of solvent under reduced pressure gave **c1** as yellow oil (8.3g, 77%):

IR (film): 3339, 2929, 2858, 1704, 1659, 1651, 1589, 1519, 1487, 1366, 1249, 1164, 1070, 908, 737 $cm^{-1}$;

NMR ($CDCl_3$): δ = 1.20-1.70 (20H, m), 2.00-2.12 (2H, m), 3.25-3.50 (4H, m), 5.05-5.20 (1H, br.s), 6.92 (1H, d, J=2.0 Hz), 7.02-7.32 (6H, m), 7.51 (1H, d, J=8.0 Hz), 7.59-7.64 (1H, m), 8.03 (1H, s), 8.48 (1H, d, J=4 Hz);

MS m/e (AP+): 491 ($M^+$ + H, 100%), 513 ($M^+$ + Na, 20%).

(3) **(S)-2-Amino-3-(1*H*-indol-3-yl)-2-methyl-*N*-(1-pyridin-2-yl-cyclohexyl methyl)-propionamide (Intermediate III-7)**

To a stirred solution of **c1** (8.2g, 16.5mmol) in dichloromethane (100ml) was added trifluoroacetic acid (3.0ml, 39mmol). After 18 hours the solvent was removed under reduced pressure at 60°C. The residue was treated cautiously with saturated sodium carbonate solution (200ml) before extracting with ethyl acetate (3 x 200ml). The combined organic phases were dried ($MgSO_4$) and evaporated under reduced pressure at 60°C. The residue was purified by flash chromatography. Elution with 0-5% methanol in dichloromethane and subsequent removal of solvent under reduced pressure gave **Intermediate III-7** as white foam (4.85g, 75%).

MPt: 65-68°C;

IR (KBr disc): 3367, 2926, 2855, 1648, 1589, 1569, 1522, 1455, 1430, 1366, 1341, 1234, 842, 784, 742 $cm^{-1}$;

NMR ($CDCl_3$): δ = 1.20-1.80 (13H, m), 1.98-2.20 (2H, m), 2.83 (1H, d, J=14.2 Hz), 3.33 (1H, d, J=14.2 Hz), 3.38 (2H, d, J=5.6 Hz), 6.98-7.20 (6H, m), 7.50-7.75 (3H, m), 8.05-8.15 (1H, s), 8.49-8.51 (1H, m);

MS m/e (AP+): 391 ($M^+$ + H, 100%).

**{(S)-2-(1-*H*-Indol-3-yl)-1-methyl-1-[(1-(5-methoxy-pyridin-2-yl)-cyclohexylmethyl)-carbamoyl]-ethyl}-carbamic acid *tert*-butyl ester (c2)**

To a stirred solution of Boc-(S)-αMeTrp-OH (1.44g, 4.5mmol) in DMF (50ml) was added HBTU (1.72g, 4.5mmol), DIPEA (2.38ml, 13.6mmol), and [1-(5-methoxy-2-pyridyl)cyclohexyl]methanamine (1g, 4.5mmol). After over night the reaction mixture was diluted with ethyl acetate (300ml) and water, dried ($MgSO_4$) and evaporated under reduced pressure. The residue was purified by flash chromatography. Elution with ethylacetate/heptane (1:1) and subsequent removal of solvent under reduced pressure gave **c2** as an oil (2.207g, 94%).

NMR ($CDCl_3$): δ = 1.24-1.60 (8H, m), 1.39 (9H, s), 1.52 (3H, s), 2.00-2.18 (2H, m), 3.20-3.43 (4H, m), 3.82 (3H, s), 6.92 (1H, d, J=2.4 Hz), 7.02-7.20 (6H, m), 7.30 (1H, d, J=6.0 Hz), 7.51 (1H, d, J=8Hz), 8.00 (1H, s), 8.17 (1H, d, J=2.8Hz).

MS m/e (ES+): 521.36 ($M^+$ + H, 100%), 543.25 ($M^+$ + Na).

**Intermediate III-6**

To a stirred solution of **c2** (2.2g, 4.2mmol) in dichloromethane (10ml) was added trifluoroacetic acid (5ml, excess). After stirring over night the reaction mixture was taken up in 1N HCl and extracted with diethylether. Organic phase discarded. The aqueous phase was basified cautiously with saturated sodium carbonate solution before extracting with ethyl acetate (3 x 50ml). The combined organic phases were dried ($MgSO_4$) and evaporated under reduced pressure at 60°C to give **Intermediate III-6** as a glass (1.253g, 71%).

IR (film): 3272, 2930, 2857, 1651, 1595, 1573, 1520, 1489, 1478, 1455, 1393, 1358, 1291, 1268, 1232, 1181, 1150, 1131, 1030, 1012, 831, 741 $cm^{-1}$;

NMR (DMSO): δ = 1.10-1.65 (13H, m), 1.80-1.90 (1H, m), 2.00-2.10 (1H, m), 2.70 (1H, d, J=13.9 Hz), 3.10 (1H, d, J=13.9 Hz), 3.10-3.22 (2H, m), 3.77 (3H,s), 6.93-7.07 (4H, m), 7.16-7.19 (1H, m), 7.32 (1H, d, J=8.1 Hz), 7.48-7.55 (2H, m), 8.21 (1H, d, J=3.2 Hz), 10.88 (1H, s);

MS m/e (ES+): 421.27 ($M^+$ + H, 100%), 443.26 ($M^+$ + Na).

**Examples 32-86**

***N*-acyl derivatives of Intermediate III-6 and III-7**

Scheme 8 describes the synthesis of *N*-acyl derivatives of **Intermediates III-7** and **III-6.**

## Scheme 8

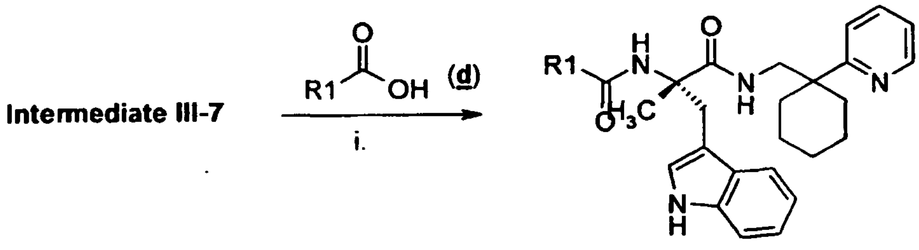


**Examples 32-85**

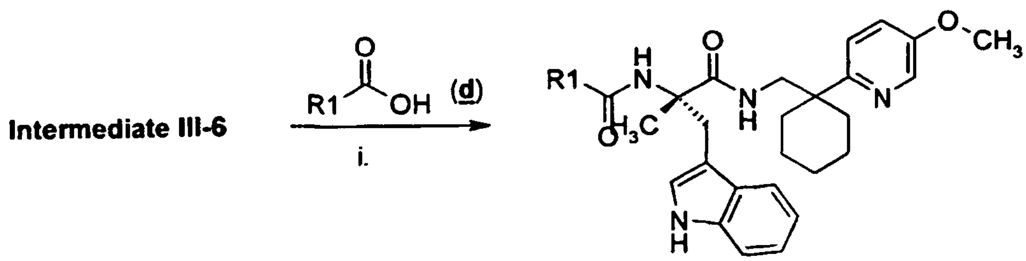


**Example 86**

i. HBTU, DIPEA, DMF

In scheme 8, R1 represents the rest of the carboxylic acid d molecule. These intermediates d are listed in table 2.

**N-acyl derivatives of Intermediate III-7**

To acid **d** (0.18 mmol) was added 0.50 M HBTU in DMF (300 µL, 0.15 mmol), 1.0 M diisopropylethylamine in DMF (300 µL, 0.30 mmol) and 0.40 M **Intermediate III-7** in DMF (375 µL, 0.15 mmol). The solution was shaken on an orbital shaker at room temperature for 18 h. Water (1.0 mL) was added and the mixture was loaded onto a LC-18 SPE cartridge (0.5 g sorbent) and the cartridge was eluted with water (3 mL), 25% methanol/water (3 mL), 50% methanol/water (4 mL) and methanol (4.5 mL)). The methanol fraction was concentrated and analysed by LCMS. When the purity was <90% the product was further purified by prep. HPLC (column: Phenomenex primesphere 10 µ C18-HC 110A, 100x21.20 mm; mobile phase: methanol / water 10 to 100% gradient). The products were characterised and analysed by LCMS (column: 50x4.6 mm Prodigy ODSIII (5µ) column; mobile phase: acetonitrile / water (0.1% formic acid) 5 to 100% gradient over 2 min, held at 100% acetonitrile for 1 min; flow rate 4 mL/min; UV detection at 215 nm; mass spec: 150-900 Da full scan APCI+ centroid data)

The following products were made by the above method, with the starting material listed in Table 2 and gave the test results indicated in Table 3:

TABLE 2

| Example | Intermediate d |
|---|---|
| 32 | Benzoic acid |
| 33 | 4-Methyl-benzoic acid |
| 34 | 4-Chloro-benzoic acid |
| 35 | 4-Methoxy-benzoic acid |
| 36 | 4-Nitro-benzoic acid |

TABLE 2 (continued)

| Example | Intermediate d |
|---|---|
| 37 | 4-Methanesulfonyl-benzoic acid |
| 38 | 3-Cyano-benzoic acid |
| 39 | 3-Chloro-benzoic acid |
| 40 | 3-Methoxy-benzoic acid |
| 41 | 3-Methanesulfonyl-benzoic acid |
| 42 | 3-Dimethylamino-benzoic acid |
| 43 | 3-Methyl-benzoic acid |
| 44 | 2-Chloro-benzoic acid |
| 45 | 2-Nitro-benzoic acid |
| 46 | 2-Methoxy-benzoic acid |
| 47 | 2-Methyl-benzoic acid |
| 48 | 2-Dimethylamino-benzoic acid |
| 49 | 2-Fluoro-benzoic acid |
| 50 | *p*-Tolyl-acetic acid |
| 51 | *o*-Tolyl-acetic acid |
| 52 | (4-Hydroxy-phenyl)-acetic acid |
| 53 | (3-Hydroxy-phenyl)-acetic acid |
| 54 | *m*-Tolyl-acetic acid |
| 55 | (2-Fluoro-phenyl)-acetic acid |
| 56 | Thiophen-3-yl-acetic acid |
| 57 | Pyridine-2-carboxylic acid |
| 58 | Isonicotinic acid |
| 59 | Furan-3-carboxylic acid |
| 60 | Furan-2-carboxylic acid |
| 61 | 1*H*-Indole-2-carboxylic acid |
| 62 | 5-Methyl-isoxazole-3-carboxylic acid |
| 63 | 1-Methyl-1*H*-pyrrole-2-carboxylic acid |
| 64 | Thiophene-2-carboxylic acid |
| 65 | Thiophene-3-carboxylic acid |
| 66 | 1*H*-Indole-6-carboxylic acid |
| 67 | 1*H*-Indole-5-carboxylic acid |
| 68 | 1*H*-Indole-4-carboxylic acid |
| 69 | 1*H*-Indole-7-carboxylic acid |
| 70 | 1-Methyl-1*H*-indole-2-carboxylic acid |
| 71 | Benzo[*b*]thiophene-2-carboxylic acid |
| 72 | Benzothiazole-6-carboxylic acid |
| 73 | 1*H*-Benzotriazole-5-carboxylic acid |
| 74 | 3-Methyl-thiophene-2-carboxylic acid |

TABLE 2 (continued)

| Example | Intermediate d |
|---|---|
| 75 | 5-Methyl-thiophene-2-carboxylic acid |
| 76 | 6-Methyl-pyridine-2-carboxylic acid |
| 77 | Isoquinoline-3-carboxylic acid |
| 78 | Quinoxaline-2-carboxylic acid |
| 79 | Quinoline-8-carboxylic acid |
| 80 | 5-Phenyl-oxazole-4-carboxylic acid |
| 81 | 2-Pyrrol-1-yl-benzoic acid |
| 82 | (4-Methoxy-phenyl)-acetic acid |
| 83 | (4-Dimethylamino-phenyl)-acetic acid |
| 84 | (2-Nitro-phenyl)-acetic acid |
| 85 | (2-Methoxy-phenyl)-acetic acid |
| 86 | 1*H*-Indole-2-carboxylic acid |

TABLE 3

| Example No | Product | $MH^+$ | Purity % | LCMS Ret time (min) | BB1 IC50 (nM) | BB2 IC50 (nM) |
|---|---|---|---|---|---|---|
| 32 | N-{(S)-2-(1H-Indol-3-yl)-1-methyl-1-[(1-pyridin-2-yl-cyclohexylmethyl)-carbamoyl]-ethyl}-benzamide | 494,64 | 100 | 1.71 | 2499 | IA |
| 33 | N-{(S)-2-(1H-Indol-3-yl)-1-methyl-1-[(1-pyridin-2-yl-cyclohexylmethyl)-carbamoyl]-ethyl}-4-methyl-benzamide | 508,67 | 95 | 1.76 | 2499 | IA |
| 34 | 4-Chloro-N-{(S)-2-(1H-indol-3-yl)-1-methyl-1-[(1-pyridin-2-yl-cyclohexyl-methyl)-carbamoyl]-ethyl}-benzamide | 529,09 | 94 | 1.84 | 1349 | IA |
| 35 | N-{(S)-2-(1H-Indol-3-yl)-1-methyl-1-[(1-pyridin-2-yl-cyclohexylmethyl)-carbamoyl]-ethyl}-4-methoxy-benzamide | 524,67 | 94 | 1.68 | 2879 | IA |
| 36 | N-{(S)-2-(1H-Indol-3-yl)-1-methyl-1-[(1-pyridin-2-yl-cyclohexylmethyl)-carbamoyl]-ethyl}-4-nitro-benzamide | 539,64 | 80 | 1.79 | 343 | IA |

TABLE 3 (continued)

| Example No | Product | MH+ | Purity % | LCMS Ret time (min) | BB1 IC50 (nM) | BB2 IC50 (nM) |
|---|---|---|---|---|---|---|
| 37 | N-{(S)-2-(1H-Indol-3-yl)-1-methyl-1-[(1-pyridin-2-yl-cyclohexylmethyl)-carbamoyl]-ethyl}-4-methanesulfonyl-benzamide | 572,73 | 95 | 1.60 | 2272 | IA |
| 38 | 3-Cyano-N-{(S)-2-(1H-indol-3-yl)-1-methyl-1-[(1-pyridin-2-yl-cyclohexylmethyl)-carbamoyl]-ethyl}-benzamide | 519,65 | 91 | 1.71 | 2042 | IA |
| 39 | 3-Chloro-N-{(S)-2-(1H-indol-3-yl)-1-methyl-1-[(1-pyridin-2-yl-cyclohexyl-methyl)-carbamoyl]-ethyl}-benzamide | 529,09 | 97 | 1.84 | 1269 | IA |
| 40 | N-{(S)-2-(1H-Indol-3-yl)-1-methyl-1-[(1-pyridin-2-yl-cyclohexylmethyl)-carbamoyl]-ethyl}-3-methoxy-benzamide | 524,67 | 98 | 1.73 | 2859 | IA |
| 41 | N-{(S)-2-(1H-Indol-3-yl)-1-methyl-1-[(1-pyridin-2-yl-cyclohexylmethyl)-carbamoyl]-ethyl}-3-methanesulfonyl-benzamide | 572,73 | 95 | 1.60 | 3051 | IA |
| 42 | Dimethylamino-N-{(S)-2-(1H-indol-3-yl)-1-methyl-1-[(1-pyridin-2-yl-cyclohexyl-methyl)-carbamoyl]-ethyl}-benzamide | 537,71 | 91 | 1.74 | 2518 | IA |
| 43 | N-{(S)-2-(1H-Indol-3-yl)-1-methyl-1-[(1-pyridin-2-yl-cyclohexylmethyl)-carbamoyl]-ethyl}-3-methyl-benzamide | 508,67 | 100 | 1.79 | 2351 | IA |
| 44 | 2-Chloro-N-{(S)-2-(1H-indol-3-yl)-1-methyl-1-[(1-pyridin-2-yl-cyclohexyl-methyl)-carbamoyl]-ethyl}-benzamide | 529,09 | 98 | 1.79 | 3229 | IA |

TABLE 3 (continued)

| Example No | Product | MH+ | Purity % | LCMS Ret time (min) | BB1 IC50 (nM) | BB2 IC50 (nM) |
|---|---|---|---|---|---|---|
| 45 | N-{(S)-2-(1H-Indol-3-yl)-1-methyl-1-[(1-pyridin-2-yl-cyclohexylmethyl)-carbamoyl]-ethyl}-2-nitro-benzamide | 539,64 | 91 | 1.71 | 4581 | IA |
| 46 | N-{(S)-2-(1H-Indol-3-yl)-1-methyl-1-[(1-pyridin-2-yl-cyclohexylmethyl)-carbamoyl]-ethyl}-2-methoxy-benzamide | 524,67 | 100 | 1.73 | 2559 | IA |
| 47 | N-{(S)-2-(1H-Indol-3-yl)-1-methyl-1-[(1-pyridin-2-yl-cyclohexylmethyl)-carbamoyl]-ethyl} -2-methyl-benzamide | 508,67 | 100 | 1.79 | 3283 | IA |
| 48 | C-Dimethylamino-N-{(S)-2-(1H-indol-3-yl)-1-methyl-1-[(1-pyridin-2-yl-cyclohexyl-methyl)-carbamoyl]-ethyl}-benzamide | 537,71 | 93 | 1.79 | 716 | IA |
| 49 | 2-Fluoro-N-{(S)-2-(1H-indol-3-yl)-1-methyl-1-[(1-pyridin-2-yl-cyclohexylmethyl)-carbamoyl]-ethyl}-benzamide | 512,63 | 98 | 1.76 | 3949 | IA |
| 50 | (S)-3-(1H-Indol-3-yl)-2-methyl-N-(1-pyridin-2-yl-cyclohexylmethyl)-2-(2-p-tolyl-ethanoylamino)-propionamide | 522,70 | 94 | 1.76 | 944 | IA |
| 51 | (S)-3-(1H-Indol-3-yl)-2-methyl-N-(1 -pyridin-2-yl-cyclohexylmethyl)-2-(2-o-tolyl-ethanoylamino)-propionamide | 522,70 | 98 | 1.76 | 944 | IA |
| 52 | (S)-2-[2-(4-Hydroxy-phenyl)-ethanoylamino]-3-(1H-indol-3-yl)-2-methyl-N-(1-pyridin-2-yl-cyclohexylmethyl)-propionamide | 524,67 | 96 | 1.50 | 3135 | IA |

TABLE 3 (continued)

| Example No | Product | MH+ | Purity % | LCMS Ret time (min) | BB1 IC50 (nM) | BB2 IC50 (nM) |
|---|---|---|---|---|---|---|
| 53 | (S)-2-[2-(3-Hydroxy-phenyl)-ethanoylamino]-3-(1H-indol-3-yl)-2-methyl-N-(1-pyridin-2-yl-cyclohexylmethyl)-propionamide | 524,67 | 90 | 1.52 | 1437 | IA |
| 54 | (S)-3-(1H-Indol-3-yl)-2-methyl-N-(1-pyridin-2-yl-cyclohexylmethyl)-2-(2-m-tolyl-ethanoylamino)-propionamide | 522,70 | 95 | 1.76 | 817 | IA |
| 55 | (S)-2-[2-(2-Fluoro-phenyl)-ethanoylamino]-3-(1H-indol-3-yl)-2-methyl-N-(1-pyridin-2-yl-cyclohexylmethyl)-propionamide | 526,66 | 94 | 1.71 | 878 | 1546 |
| 56 | (S)-3-(1H-Indol-3-yl)-2-methyl-N-(1-pyridin-2-yl-cyclohexylmethyl)-2-(2-thiophen-3-yl-ethanoylamino)-propionamide | 514,70 | 93 | 1.65 | 1437 | IA |
| 57 | Pyridine-2-carboxylic acid {(S)-2-(1H-indol-3-yl)-1-methyl-1-[(1-pyridin-2-yl-cyclohexylmethyl)-carbamoyl]-ethyl}-amide | 495,63 | 98 | 1.68 | 3709 | IA |
| 58 | N-{(S)-2-(1H-Indol-3-yl)-1-methyl-1-[(1-pyridin-2-yl-cyclohexylmethyl)-carbamoyl]-ethyl}-isonicotinamide | 495,63 | 98 | 1.47 | 1365 | IA |
| 59 | Furan-3-carboxylic acid {(S)-2-(1H-indol-3-yl)-1-methyl-1-[(1-pyridin-2-yl-cyclohexylmethyl)-carbamoyl]-ethyl}-amide | 484,60 | 97 | 1.60 | 1204 | IA |

TABLE 3 (continued)

| Example No | Product | MH+ | Purity % | LCMS Ret time (min) | BB1 IC50 (nM) | BB2 IC50 (nM) |
|---|---|---|---|---|---|---|
| 60 | Furan-2-carboxylic acid {(S)-2-(1H-indol-3-yl)-1-methyl-1-[(1-pyridin-2-yl-cyclohexylmethyl)-carbamoyl]-ethyl}-amide | 484,60 | 100 | 1.60 | 1204 | IA |
| 61 | 1H-Indole-2-carboxylic acid {(S)-2-(1H-indol-3-yl)-1-methyl-1-[(1-pyridin-2-yl-cyclohexylmethyl)-carbamoyl]-ethyl}-amide | 533,68 | 100 | 1.79 | 289 | 527 |
| 62 | 5-Methyl-isoxazole-3-carboxylic acid {(S)-2-(1H-indol-3-yl)-1-methyl-1-[(1-pyridin-2-yl-cyclohexylmethyl)-carbamoyl]-ethyl}-amide | 499,62 | 94 | 1.46 | 4127 | IA |
| 63 | 1-Methyl-1H-pyrrole-2-carboxylic acid {(S)-2-(1H-indol-3-yl)-1-methyl-1-[(1-pyridin-2-yl-cyclohexylmethyl)-carbamoyl]-ethyl}-amide | 497,65 | 96 | 1.46 | 4819 | - |
| 64 | Thiophene-2-carboxylic acid {(S)-2-(1H-indol-3-yl)-1-methyl-1-[(1-pyridin-2-yl-cyclohexylmethyl)-carbamoyl]-ethyl}-amide | 500,67 | 100 | 1.42 | 1437 | IA |
| 65 | Thiophene-3-carboxylic acid {(S)-2-(1H-indol-3-yl)-1-methyl-1-[(1-pyridin-2-yl-cyclohexylmethyl)-carbamoyl]-ethyl}-amide | 500,67 | 100 | 1.39 | 2201 | IA |
| 66 | 1H-Indole-6-carboxylic acid {(S)-2-(1H-indol-3-yl)-1-methyl-1-[(1-pyridin-2-yl-cyclohexylmethyl)-carbamoyl]-ethyl}-amide | 533,68 | 100 | 1.42 | 1604 | IA |

TABLE 3 (continued)

| Example No | Product | MH+ | Purity % | LCMS Ret time (min) | BB1 IC50 (nM) | BB2 IC50 (nM) |
|---|---|---|---|---|---|---|
| 67 | 1H-Indole-5-carboxylic acid {(S)-2-(1H-indol-3-yl)-1-methyl-1-[(1-pyridin-2-yl-cyclohexylmethyl)-carbamoyl]-ethyl}-amide | 533,68 | 100 | 1.35 | 1881 | IA |
| 68 | 1H-Indole-4-carboxylic acid {(S)-2-(1H-indol-3-yl)-1-methyl-1-[(1-pyridin-2-yl-cyclohexylmethyl)-carbamoyl]-ethyl}-amide | 533,68 | 99 | 1.35 | 4503 | IA |
| 69 | 1H-Indole-7-carboxylic acid {(S)-2-(1H-indol-3-yl)-1-methyl-1-[(1-pyridin-2-yl-cyclohexylmethyl)-carbamoyl]-ethyl}-amide | 533,68 | 100 | 1.60 | 1369 | IA |
| 70 | 1-Methyl-1H-indole-2-carboxylic acid {(S)-2-(1H-indol-3-yl)-1-methyl-1-[(1-pyridin-2-yl-cyclohexylmethyl)-carbamoyl]-ethyl}-amide | 547,71 | 100 | 1.70 | 1233 | IA |
| 71 | Benzo[b]thiophene-2-carboxylic acid {(S)-2-(1H-indol-3-yl)-1-methyl-1-[(1-pyridin-2-yl-cyclohexylmethyl)-carbamoyl]-ethyl}-amide | 550,73 | 100 | 1.63 | 611 | IA |
| 72 | Benzothiazole-6-carboxylic acid {(S)-2-(1H-indol-3-yl)-1-methyl-1-[(1-pyridin-2-yl-cyclohexylmethyl)-carbamoyl]-ethyl}-amide | 551,72 | 95 | 1.35 | 897 | 1495 |
| 73 | 1H-Benzotriazole-5-carboxylic acid {(S)-2-(1H-indol-3-yl)-1-methyl-1-[(1-pyridin-2-yl-cyclohexylmethyl)-carbamoyl]-ethyl}-amide | 535,65 | 95 | 1.25 | 3167 | - |

TABLE 3 (continued)

| Example No | Product | MH+ | Purity % | LCMS Ret time (min) | BB1 IC50 (nM) | BB2 IC50 (nM) |
|---|---|---|---|---|---|---|
| 74 | 3-Methyl-thiophene-2-carboxylic acid {(S)-2-(1H-indol-3-yl)-1-methyl-1-[(1-pyridin-2-yl-cyclohexylmethyl)-carbamoyl]-ethyl}-amide | 514,70 | 100 | 1.53 | 744 | IA |
| 75 | 5-Methyl-thiophene-2-carboxylic acid {(S)-2-(1H-indol-3-yl)-1-methyl-1-[(1-pyridin-2-yl-cyclohexylmethyl)-carbamoyl]-ethyl}-amide | 514,70 | 100 | 1.60 | 1663 | IA |
| 76 | 6-Methyl-pyridine-2-carboxylic acid {(S)-2-(1H-indol-3-yl)-1-methyl-1-[(1-pyridin-2-yl-cyclohexylmethyl)-carbamoyl]-ethyl}-amide | 509,66 | 98 | 1.6 | 2816 | IA |
| 77 | Isoquinoline-3-carboxylic acid {(S)-2-(1H-indol-3-yl)-1-methyl-1-[(1-pyridin-2-yl-cyclohexylmethyl)-carbamoyl]-ethyl}-amide | 545,69 | 100 | 1.71 | 1363 | - |
| 78 | Quinoxaline-2-carboxylic acid {(S)-2-(1H-indol-3-yl)-1-methyl-1-[(1-pyridin-2-yl-cyclohexylmethyl)-carbamoyl]-ethyl}-amide | 546,68 | 94 | 1.67 | 1425 | IA |
| 79 | Quinoline-8-carboxylic acid {(S)-2-(1H-indol-3-yl)-1-methyl-1-[(1-pyridin-2-yl-cyclohexylmethyl)-carbamoyl]-ethyl}-amide | 545,69 | 96 | 1.57 | 4479 | IA |
| 80 | 5-Phenyl-oxazole-4-carboxylic acid {(S)-2-(1H-indol-3-yl)-1-methyl-1-[(1-pyridin-2-yl-cyclohexylmethyl)-carbamoyl]-ethyl}-amide | 561,69 | 95 | 1.81 | 2660 | IA |

TABLE 3 (continued)

| Example No | Product | MH+ | Purity % | LCMS Ret time (min) | BB1 IC50 (nM) | BB2 IC50 (nM) |
|---|---|---|---|---|---|---|
| 81 | N-{(S)-2-(1H-Indol-3-yl)-1-methyl-1-[(1-pyridin-2-yl-cyclohexylmethyl)-carbamoyl]-ethyl}-2-pyrrol-1-yl-benzamide | 559,72 | 98 | 1.71 | 361 | IA |
| 82 | (S)-3-(1H-Indol-3-yl)-2-[2-(4-methoxy-phenyl)-ethanoylamino]-2-methyl-N-(1-pyridin-2-yl-cyclohexylmethyl)-propionamide | 538,70 | 98 | 1.71 | 1694 | IA |
| 83 | (S)-2-[2-(4-Dimethylamino-phenyl)-ethanoylamino]-3-(1H-indol-3-yl)-2-methyl-N-(1-pyridin-2-yl-cyclohexylmethyl)-propionamide | 551,74 | 100 | 1.36 | 2708 | IA |
| 84 | (S)-3-(1H-Indol-3-yl)-2-methyl-2-[2-(2-nitro-phenyl)-ethanoylamino]-N-(1-pyridin-2-yl-cyclohexylmethyl)-propionamide | 553,67 | 95 | 1.5 | 1979 | IA |
| 85 | (S)-3-(1H-Indol-3-yl)-2-[2-(2-methoxy-phenyl)-ethanoylamino]-2-methyl-N-(1-pyridin-2-yl-cyclohexylmethyl)-propionamide | 538,70 | 100 | 1.57 | 1326 | 2479 |
| IA: IC50 > 10000 nM | | | | | | |

**N-acyl derivative of Intermediate III-6**

**Example 86**

**1H-Indole-2-carboxylic acid ((S)-2-(1H-indol-3-yl)-1-{[1-(5-methoxy-pyridin-2-yl)-cyclohexylmethyl]-carbamoyl}-1-methyl-ethyl)-amide**

To a solution of 1-*H*-Indole-2-carboxylic acid (38 mg, 0.24 mmol), **Intermediate III-6** (100 mg, 0.19 mmol) and diisopropylethylamine (61 mg, 0.47 mmol) in DMF (5 mL) was added HBTU (90 mg, 0.24 mmol). The reaction mixture was stirred at room temperature for 16 h. The reaction mixture was concentrated under reduced pressure and the residue was diluted with ethyl acetate, washed with brine, dried ($MgSO_4$) and concentrated under reduced pressure. The residue was purified by column chromatography (60% ethyl acetate/heptane) to give **Example 86** as an amorphous white solid (65 mg, 61%).

IR (film): 3285, 2931, 2855, 1651, 1537, 1489, 1456, 1420, 1342, 1310, 1267, 1028, 908, 744 $cm^{-1}$;

NMR ($CDCl_3$): δ = 1.10-1.61 (11H, m), 1.95-2.04 (2H, m), 3.29-3.52 (4H, m), 3.43 (3H, s), 6.47 (1H,s), 6.86-6.90 (1H, m), 6.98-6.99 (2H, m), 7.09-7.42 (8H, m), 7.52-7.58 (2H, m), 7.73-7.74 (1 H, m) 8.05 (1H, s), 9.11 (1 H, s);

MS m/e (ES+): 564 ($M^+$ + H, 100%).

Binding studies of **Example 86** to the bombesin receptors gave the following results ($IC_{50}$): BB1: 11 nM, BB2: 119 nM.

**Examples 87-110**

***N*-terminal urethane derivatives of Intermediate III-7**

Scheme 9 describes the synthesis of urethane derivatives of **Intermediate III-7:**

- Conversion of alcohol into 4-nitrophenyl carbonates
- *N*-terminal urethane formation

**Scheme 9**

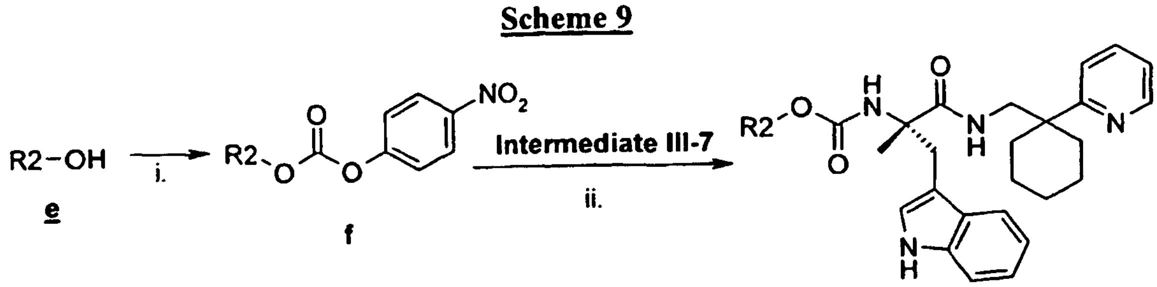


**Examples 87-110**

i. 4-nitrophenyl chloroformate, pyridine, THF

ii. DMAP, DMF

In scheme 9, R2 represents the rest of the intermediate e. These intermediates e are listed in table 4.

To a stirred solution of alcohol **e** (10 mmol) and 4-nitrophenyl chloroformate (2.01 g, 10 mmol) in dichloromethane (50 mL) at 0°C was added dropwise a solution of pyridine (0.81 mL, 10 mmol) in dichloromethane (10 mL). The reaction mixture was allowed to slowly warm to room temperature and was stirred at room temperature for 16 h. The solvent was removed under reduced pressure and the residue was taken up in ethyl acetate (50 mL) and was washed successively with 10% citric acid (2x30 mL), water (30 mL), sat. $NaHCO_3$ solution (2x50 mL) and brine (50 mL). The organic phase was dried ($MgSO_4$) and was concentrated under reduced pressure. The crude product was recrystallised from typically ethyl acetate, diethyl ether or heptane to give pure carbonate **f**. The product was characterised by IR (see Table 4 for carbonate signals).

To carbonate **f** (0.21 mmol) was added DMF (0.4 mL) followed by 0.50 M DMAP in DMF (400 μL, 0.20 mmol) and 0.50 M **Intermediate III-7** in DMF (200 μL, 0.10 mmol). The solution was shaken on an orbital shaker at room temperature for 42 h. Water (1.0 mL) was added and the mixture was loaded onto a LC-18 SPE cartridge (0.5 g sorbent) and the cartridge was eluted with 25% methanol/water (3.4 mL) and methanol (4 mL). The methanol fraction was concentrated and purified by prep. HPLC (column: Phenomenex primesphere 10 μ C18-HC 110A, 100x21.20 mm; mobile phase: methanol/water 10 to 100% gradient). The products were characterised and analysed by LCMS (column: 50x4.6 mm Prodigy ODSIII (5μ) column; mobile phase: acetonitrile/water (0.1% formic acid) 5 to 100% gradient over 2 min, held at 100% acetonitrile for 1 min; flow rate 4 mL/min; UV detection at 215 nm; mass spec: 150-900 Da full scan APCI+ centroid data).

The following products were made by the above method, with the starting material listed in Table 4 and gave the test results indicated in Table 5:

TABLE 4

| Example | intermediate e | intermediate f: IR ($cm^{-1}$) |
|---|---|---|
| 87 | Naphthalen-1-yl-methanol | 1754 |
| 88 | (3,4-Dimethoxy-phenyl)-methanol | 1754 |
| 89 | Naphthalen-2-yl-methanol | 1752 |
| 90 | Indan-2-ol | 1765 |
| 91 | (3,4-Dichloro-phenyl)-methanol | 1754 |
| 92 | (4-Methoxy-phenyl)-methanol | 1748 |
| 93 | (4-Chloro-phenyl)-methanol | 1761 |
| 94 | (2-Fluoro-phenyl)-methanol | 1752 |
| 95 | (2-Chloro-phenyl)-methanol | 1764 |
| 96 | (4-Nitro-phenyl)-methanol | 1761 |
| 97 | *o*-Tolyl-methanol | 1757 |
| 98 | (4-*tert*-Butyl-phenyl)-methanol | 1766 |
| 99 | (3-Nitro-phenyl)-methanol | 1769 |
| 100 | (2-Methoxy-phenyl)-methanol | 1766 |
| 101 | (4-Trifluoromethyl-phenyl)-methanol | 1763 |
| 102 | (3-Ethoxy-phenyl)-methanol | 1767 |
| 103 | 3-Hydroxymethyl-benzonitrile | 1769 |
| 104 | (2,4-Dichloro-phenyl)-methanol | 1768 |
| 105 | *m*-Tolyl-methanol | 1757 |
| 106 | (3-Phenoxy-phenyl)-methanol | 1766 |
| 107 | (3-Trifluoromethyl-phenyl)-methanol | 1770 |
| 108 | p-Tolyl-methanol | 1759 |
| 109 | (2,3-Dichloro-phenyl)-methanol | 1758 |
| 110 | Quinolin-6-yl-methanol | 1761 |

TABLE 5

| Example No | Product | $MH^+$ | Purity % | LCMS Ret time (min) | BB1 IC50 (nm) | BB2 IC50 (nm) |
|---|---|---|---|---|---|---|
| 87 | {(S)-2-(1H-Indol-3-yl)-1-methyl-1[(1-pyridin-2-yl-cyclohexylmethyl) carbamoyl]-ethyl}-carbamic acid naphthalen-1-ylmethyl ester | 574,73 | 100 | 1.67 | 239 | IA |

TABLE 5 (continued)

| Example No | Product | MH+ | Purity % | LCMS Ret time (min) | BB1 IC50 (nm) | BB2 IC50 (nm) |
|---|---|---|---|---|---|---|
| 88 | {(S)-2-(1H-Indol-3-yl)-1-methyl-1[(1-pyridin-2-yl-cyclohexylmethyl)-carbamoyl]-ethyl}-carbamic acid 3,4-dimethoxy-benzyl ester | 584,72 | 95 | 1.41 | 1758 | IA |
| 89 | {(S)-2-(1H-Indol-3-yl)-1-methyl-1-[(1-pyridin-2-yl-cyclohexylmethyl)-carbamoyl]-ethyl}-carbamic acid naphthalen-2-ylmethyl ester | 574,73 | 100 | 1.67 | 1001 | IA |
| 90 | {(S)-2-(1H-Indol-3-yl)-1-methyl-1-[(1-pyridin-2-yl-cyclohexylmethyl)-carbamoyl]-ethyl}-carbamic acid indan-2-yl ester | 550,71 | 91 | 1.59 | 955 | IA |
| 91 | {(S)-2-(1H-Indol-3-yl)-1-methyl-1-[(1-pyridin-2-yl-cyclohexylmethyl)-carbamoyl]-ethyl}-carbamic acid 3,4-dichloro-benzyl ester | 593,56 | 93 | 1.73 | 202 | IA |
| 92 | {(S)-2-(1H-Indol-3-yl)-1-methyl-1-[(1-pyridin-2-yl-cyclohexylmethyl)-carbamoyl]-ethyl}-carbamic acid 4-methoxy-benzyl ester | 554,70 | 93 | 1.49 | 1610 | IA |
| 93 | {(S)-2-(1H-Indol-3-yl)-1-methyl-1-[(1-pyridin-2-yl-cyclohexylmethyl)-carbamoyl]-ethyl}-carbamic acid 4-chloro-benzyl ester | 559,11 | 98 | 1.62 | 681 | IA |

TABLE 5 (continued)

| Example No | Product | MH+ | Purity % | LCMS Ret time (min) | BB1 IC50 (nm) | BB2 IC50 (nm) |
|---|---|---|---|---|---|---|
| 94 | {(S)-2-(1H-Indol-3-yl)-1-methyl-1-[(1-pyridin-2-yl-cyclohexylmethyl)-carbamoyl]-ethyl}-carbamic acid 2-fluoro-benzyl ester | 542,66 | 91 | 1.52 | 923 | IA |
| 95 | {(S)-2-(1H-Indol-3-yl)-1-methyl-1-[(1-pyridin-2-yl-cyclohexylmethyl)-carbamoyl]-ethyl}-carbamic acid 2-chloro-benzyl ester | 559,11 | 89 | 1.62 | 624 | IA |
| 96 | {(S)-2-(1H-Indol-3-yl)-1-methyl-1-[(1-pyridin-2-yl-cyclohexylmethyl)-carbamoyl]-ethyl}-carbamic acid 4-nitro-benzyl ester | 569,67 | 97 | 1.51 | 41 | 463 |
| 97 | {(S)-2-(1H-Indol-3-yl)-1-methyl-1-[(1-pyridin-2-yl-cyclohexylmethyl)-carbamoyl]-ethyl}-carbamic acid 2-methyl-benzyl ester | 538,70 | 94 | 11.60 | 751 | IA |
| 98 | {(S)-2-(1H-Indol-3-yl)-1-methyl-1-[(1-pyridin-2-yl-cyclohexylmethyl)-carbamoyl]-ethyl}-carbamic acid 4-tert-butyl-benzyl ester | 580,78 | 100 | 1.86 | 1986 | IA |
| 99 | {(S)-2-(1H-Indol-3-yl)-1-methyl-1-[(1-pyridin-2-yl-cyclohexylmethyl)-carbamoyl]-ethyl}-carbamic acid 3-nitro-benzyl ester | 569,67 | 97 | 1.51 | 17 | 612 |

TABLE 5 (continued)

| Example No | Product | MH+ | Purity % | LCMS Ret time (min) | BB1 IC50 (nm) | BB2 IC50 (nm) |
|---|---|---|---|---|---|---|
| 100 | {(S)-2-(1H-Indol-3-yl)-1-methyl-1-[(1-pyridin-2-yl-cyclohexylmethyl)-carbamoyl]-ethyl}-carbamic acid 2-methoxy-benzyl ester | 554,70 | 96 | 1.52 | 818 | IA |
| 101 | {(S)-2-(1H-Indol-3-yl)-1-methyl-1-[(1-pyridin-2-yl-cyclohexylmethyl)-carbamoyl]-ethyl}-carbamic acid 4-trifluoromethyl-benzyl ester | 592,67 | 97 | 1.7 | 1102 | IA |
| 102 | {(S)-2-(1H-Indol-3-yl)-1-methyl-1-[(1-pyridin-2-yl-cyclohexylmethyl)-carbamoyl]-ethyl}-carbamic acid 3-ethoxy-benzyl ester | 568,72 | 89 | 1.60 | 1065 | IA |
| 103 | {(S)-2-(1H-Indol-3-yl)-1-methyl-1-[(1-pyridin-2-yl-cyclohexylmethyl)-carbamoyl]-ethyl}-carbamic acid 3-cyano-benzyl ester | 549,68 | 99 | 1.43 | 85 | IA |
| 104 | {(S)-2-(1H-Indol-3-yl)-1-methyl-1-[(1-pyridin-2-yl-cyclohexylmethyl)-carbamoyl]-ethyl}-carbamic acid 2,4-dichloro-benzyl ester | 593,56 | 95 | 1.78 | 450 | IA |
| 105 | {(S)-2-(1H-Indol-3-yl)-1-methyl-1-[(1-pyridin-2-yl-cyclohexylmethyl)-carbamoyl]-ethyl}-carbamic acid 3-methyl-benzyl ester | 538,70 | 96 | 1.59 | 841 | IA |

TABLE 5 (continued)

| Example No | Product | MH+ | Purity % | LCMS Ret time (min) | BB1 IC50 (nm) | BB2 IC50 (nm) |
|---|---|---|---|---|---|---|
| 106 | {(S)-2-(1H-Indol-3-yl)-1-methyl-1-[(1-pyridin-2-yl-cyclohexylmethyl)-carbamoyl]-ethyl}-carbamic acid 3-phenoxy-benzyl ester | 616,77 | 96 | 1.78 | 1350 | IA |
| 107 | {(S)-2-(1H-Indol-3-yl)-1-methyl-1-[(1-pyridin-2-yl-cyclohexylmethyl)-carbamoyl]-ethyl}-carbamic acid 3-trifluoromethyl-benzyl ester | 592,67 | 96 | 1.67 | 182 | IA |
| 108 | {(S)-2-(1H-Indol-3-yl)-1-methyl-1-[(1-pyridin-2-yl-cyclohexylmethyl)-carbamoyl]-ethyl}-carbamic acid 4-methyl-benzyl ester | 538,70 | 97 | 1.60 | 1084 | IA |
| 109 | {(S)-2-(1H-Indol-3-yl)-1-methyl-1-[(1-pyridin-2-yl-cyclohexylmethyl)-carbamoyl]-ethyl}-carbamic acid 2,3-dichloro-benzyl ester | 593,56 | 94 | 1.73 | 152 | IA |
| 110 | {(S)-2-(1H-Indol-3-yl)-1-methyl-1-[(1-pyridin-2-yl-cyclohexylmethyl)-carbamoyl]-ethyl}-carbamic acid quinolin-6-ylmethyl ester | 575,72 | 97 | 1.22 | 171 | IA |

**Examples 111-168**

***N*-terminal sulfonamide derivatives of Intermediate III-7**

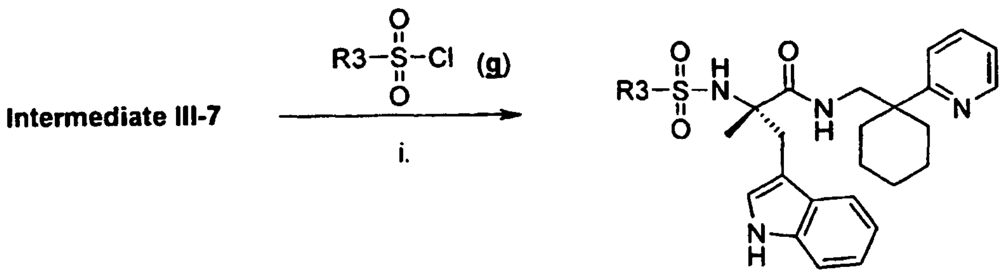


Scheme 10

Examples 111-168

In scheme 10, R3 represents the rest of the intermediate g. These intermediates g are listed in table 6.

To sulfonyl chloride **g** (0.14 mmol) was added 0.143 M **Intermediate III-7** in DMF (700 µL, 0.10 mmol) followed by 300 µL of a solution containing a mixture of diisopropylethylamine (0.667 M in DMF, 0.20 mmol) and 4-dimethylaminopyridine (0.033 M in DMF, 0.01 mmol). The reaction mixture was shaken in an orbital shaker at 70°C for 16 h. The crude reaction mixture was loaded onto a 5 g silica cartridge and the cartridge was eluted with ethyl acetate in heptane (30 to 100% gradient). Removal of the solvent under reduced pressure gave the sulfonamides (**Examples 111-168**). The purity of the sulfonamide was checked by LCMS. Those samples that were less than 95% pure were further purified by prep HPLC (column: YMC-Pack ODS-AM, 5µm, 150x20 mm; mobile phase: acetonitrile / water 40 to 100% gradient). The products were characterised and analysed by LCMS (column: 150x4.6 mm Prodigy ODS3 (3µ) column; mobile phase: acetonitrile (0.085% TFA) / water (0.1% TFA) 20 to 100% gradient over 7 min, held at 100% acetonitrile (0.085% TFA) for 1 min; flow rate 1.5 mL/min; detection: diode array 200-300 nm; mass spec: 150-900 Da full scan APCI+ centroid data) (see Table 7).

The following examples were made by the above method, with the starting material listed in Table 6 and gave the test results indicated in Table 7:

TABLE 6

| Example | intermediate g |
|---|---|
| 111 | Phenyl-methanesulfonyl chloride |
| 112 | 4-Methyl-benzenesulfonyl chloride |
| 113 | 2-Chloro-benzenesulfonyl chloride |
| 114 | 2-Fluoro-benzenesulfonyl chloride |
| 115 | Naphthalene-1-sulfonyl chloride |
| 116 | 4-Chloro-benzenesulfonyl chloride |
| 117 | 5-Dimethylamino-naphthalene-1-sulfonyl chloride |
| 118 | Naphthalene-2-sulfonyl chloride |
| 119 | Thiophene-2-sulfonyl chloride |
| 120 | Quinoline-8-sulfonyl chloride |
| 121 | 3-Nitro-benzenesulfonyl chloride |
| 122 | 4-Fluoro-benzenesulfonyl chloride |
| 123 | 4-Nitro-benzenesulfonyl chloride |
| 124 | 3-Trifluoromethyl-benzenesulfonyl chloride |

TABLE 6 (continued)

| Example | intermediate g |
|---|---|
| 125 | 3,4-Dichloro-benzenesulfonyl chloride |
| 126 | 3-Fluoro-benzenesulfonyl chloride |
| 127 | 4-Trifluoromethyl-benzenesulfonyl chloride |
| 128 | 5-Chloro-thiophene-2-sulfonyl chloride |
| 129 | 2-Trifluoromethyl-benzenesulfonyl chloride |
| 130 | 3-Chloro-benzenesulfonyl chloride |
| 131 | 3-Methyl-benzenesulfonyl chloride |
| 132 | 3,4-Dimethoxy-benzenesulfonyl chloride |
| 133 | 4-Cyano-benzenesulfonyl chloride |
| 134 | 2-Cyano-benzenesulfonyl chloride |
| 135 | 5-Chloro-1,3-dimethyl-1*H*-pyrazole-4-sulfonyl chloride |
| 136 | 3,5-Dimethyl-isoxazole-4-sulfonyl chloride |
| 137 | Benzo[1,2,5]thiadiazole-4-sulfonyl chloride |
| 138 | 1-Methyl-1*H*-imidazole-4-sulfonyl chloride |
| 139 | Benzo[1,2,5]oxadiazole-4-sulfonyl chloride |
| 140 | 3-Chlorosulfonyl-thiophene-2-carboxylic acid methyl ester |
| 141 | 5-Isoxazol-3-yl-thiophene-2-sulfonyl chloride |
| 142 | (2-Nitro-phenyl)-methanesulfonyl chloride |
| 143 | 3-Cyano-benzenesulfonyl chloride |
| 144 | 1,2-Dimethyl-1*H*-imidazole-4-sulfonyl chloride |
| 145 | 3-Methoxy-benzenesulfonyl chloride |
| 146 | 8-Nitro-naphthalene-1-sulfonyl chloride |
| 147 | 2-Chloro-5-nitro-benzenesulfonyl chloride |
| 148 | 2,4,6-Trichloro-benzenesulfonyl chloride |
| 149 | 4-Chloro-2-nitro-benzenesulfonyl chloride |
| 150 | 5-Benzenesulfonyl-thiophene-2-sulfonyl chloride |
| 151 | 4-Trifluoromethoxy-benzenesulfonyl chloride |
| 152 | 5-Methyl-2-phenoxy-benzenesulfonyl chloride |
| 153 | 2-*p*-Tolyloxy-benzenesulfonyl chloride |
| 154 | Biphenyl-2-sulfonyl chloride |
| 155 | 2-Chlorosulfonyl-benzoic acid methyl ester |
| 156 | 3-Chloro-4-fluoro-benzenesulfonyl chloride |
| 157 | 2,5-Dichloro-thiophene-3-sulfonyl chloride |
| 158 | 3-Chloro-4-methyl-benzenesulfonyl chloride |
| 159 | 2-Methoxy-4-methyl-benzenesulfonyl chloride |
| 160 | 5-Pyridin-2-yl-thiophene-2-sulfonyl chloride |
| 161 | 5-Bromo-6-chloro-pyridine-3-sulfonyl chloride |
| 162 | 2,4-Dinitro-benzenesulfonyl chloride |

TABLE 6 (continued)

| Example | intermediate g |
|---|---|
| 163 | 4-Methanesulfonyl-benzenesulfonyl chloride |
| 164 | 4-*tert*-Butyl-benzenesulfonyl chloride |
| 165 | 2,4-Dichloro-5-methyl-benzenesulfonyl chloride |
| 166 | Chloro-trifluoromethyl-benzenesulfonyl chloride |
| 167 | Nitro-trifluoromethyl-benzenesulfonyl chloride |
| 168 | 4-Butyl-benzenesulfonyl chloride |

TABLE 7

| Example No | Product | $MH^+$ | Purity % | LCMS Ret time (min) | BB1 IC50 (nm) | BB2 IC50 (nm) |
|---|---|---|---|---|---|---|
| 111 | (S)-3-(1H-Indol-3-yl)-2-methyl-2-phenylmethanesulfonylamino-N-(1-pyridin-2-yl-cyclohexylmethyl)-propionamide | 544,72 | 100 | 4.64 | 186 | IA |
| 112 | (S)-3-(1H-Indol-3-yl)-2-methyl-N-(1-pyridin-2-yl-cyclohexylmethyl)-2-(toluene-4-sulfonylamino)-propionamide | 544,72 | 100 | 4.74 | 557 | IA |
| 113 | (S)-2-(2-Chlorobenzenesulfonylamino)-3-(1H-indol-3-yl)-2-methyl-N-(1-pyridin-2-yl-cyclohexylmethyl)-propionamide | 565,14 | 100 | 4.71 | 257 | IA |
| 114 | (S)-2-(2-Fluorobenzenesulfonylamino)-3-(1H-indol-3-yl)-2-methyl-N-(1-pyridin-2-yl-cyclohexylmethyl)-propionamide | 548,68 | 100 | 4.54 | 267 | IA |
| 115 | (S)-3-(1H-Indol-3-yl)-2-methyl-2-(naphthalene-1-sulfonylamino) -N-(1-pyridin-2-yl-cyclohexylmethyl)-propionamide | 580,76 | 99 | 4.98 | 185 | 1576 |
| 116 | (S)-2-(4-Chlorobenzenesulfonylamino)-3-(1H-indol-3-yl)-2-methyl-N-(1-pyridin-2-yl-cyclohexylmethyl)-propionamide | 565,14 | 97 | 4.89 | 373 | 4386 |
| 117 | (S)-2-(5-Dimethylamino-naphthalene-1-sulfonylamino)-3-(1H-indol-3-yl)-2-methyl-N-(1-pyridin-2-yl-cyclohexylmethyl)-propionamide | 623,82 | 100 | 4.39 | 1302 | IA |
| 118 | (S)-3-(1H-Indol-3-yl)-2-methyl-2-(naphthalene-2-sulfonylamino) -N-(1-pyridin-2-yl-cyclohexylmethyl)-propionamide | 580,76 | 100 | 5.01 | 322 | IA |
| 119 | (S)-3-(1H-Indol-3-yl)-2-methyl-N-(1-pyridin-2-yl-cyclohexylmethyl)-2-(thiophene-2-sulfonylamino)-propionamide | 536,72 | 99 | 4.39 | 232 | Ia |
| 120 | (S)-3-(1H-Indol-3-yl)-2-methyl-N-(1-pyridin-2-yl-cyclohexylmethyl)-2-(quinoline-8-sulfonylamino)-propionamide | 581,74 | 99 | 4.53 | 108 | IA |

TABLE 7 (continued)

| Example No | Product | MH+ | Purity % | LCMS Ret time (min) | BB1 IC50 (nm) | BB2 IC50 (nm) |
|---|---|---|---|---|---|---|
| 121 | (S)-3-(1H-Indol-3-yl)-2-methyl-2-(3-nitro-benzenesulfonylamino)-N-(1-pyridin-2-yl-cyclohexylmethyl)-propionamide | 575,69 | 99 | 4.58 | 208 | 1960 |
| 122 | (S)-2-(4-Fluoro-benzenesulfonylamino)-3-(1H-indol-3-yl)-2-methyl-N-(1-pyridin-2-yl-cyclohexylmethyl)-propionamide | 548,68 | 100 | 4.60 | 560 | 4165 |
| 123 | (S)-3-(1H-Indol-3-yl)-2-methyl-2-(4-nitro-benzenesulfonylamino)-N-(1-pyridin-2-yl-cyclohexylmethyl)-propionamide | 575,69 | 98 | 4.65 | 515 | IA |
| 124 | (S)-3-(1H-Indol-3-yl)-2-methyl-N-(1-pyridin-2-yl-cyclohexylmethyl)-2-(3-trifluoromethyl-benzenesulfonylamino)-propionamide | 599,58 | 100 | 5.03 | 440 | 2246 |
| 125 | (S)-2-(3,4-Dichloro-benzenesulfonylamino)-3-(1H-indol-3-yl)-2-methyl-N-(1-pyridin-2-yl-cyclohexylmethyl)-propionamide | 599,58 | 99 | 5.47 | 216 | IA |
| 126 | (S)-2-(3-Fluoro-benzenesulfonylamino)-3-(1H-indol-3-yl)-2-methyl-N-(1-pyridin-2-yl-cyclohexylmethyl)-propionamide | 548,68 | 100 | 4.65 | 407 | 2761 |
| 127 | (S)-3-(1H-Indol-3-yl)-2-methyl-N-(1-pyridin-2-yl-cyclohexylmethyl)-2-(4-trifluoromethyl-benzenesulfonylamino)-propionamide | 598,69 | 95 | 5.31 | 553 | IA |
| 128 | (S)-2-(5-Chloro-thiophene-2-sulfonylamino)-3-(1H-indol-3-yl)-2-methyl-N-(1-pyridin-2-yl-cyclohexylmethyl)-propionamide | 571,17 | 99 | 4.94 | 404 | IA |
| 129 | (S)-3-(1H-Indol-3-yl)-2-methyl-N-(1-pyridin-2-yl-cyclohexylmethyl)-2-(2-trifluoromethyl-benzenesulfonylamino)-propionamide | 598,69 | 99 | 5.11 | 134 | - |
| 130 | (S)-2-(3-Chloro-benzenesulfonylamino)-3-(1 H-indol-3-yl)-2-methyl-N-(1-pyridin-2-yl-cyclohexylmethyl)-propionamide | 565,14 | 99 | 5.05 | 331 | 2687 |
| 131 | (S)-3-(1H-Indol-3-yl)-2-methyl-N-(1-pyridin-2-yl-cyclohexylmethyl)-2-(toluene-3-sulfonylamino)-propionamide | 544,72 | 99 | 4.93 | 393 | 1019 |
| 132 | (S)-2-(3,4-Dimethoxy-benzenesulfonylamino)-3-(1H-indol-3-yl)-2-methyl-N-(1-pyridin-2-yl-cyclohexylmethyl)-propionamide | 590,75 | 98 | 4.50 | 608 | IA |
| 133 | (S)-2-(4-Cyano-benzenesulfonylamino)-3-(1H-indol-3-yl)-2-methyl-N-(1-pyridin-2-yl-cyclohexylmethyl)-propionamide | 555,70 | 99 | 4.61 | 766 | IA |
| 134 | (S)-2-(2-Cyano-benzenesulfonylamino)-3-(1H-indol-3-yl)-2-methyl-N-(1-pyridin-2-yl-cyclohexylmethyl)-propionamide | 555,70 | 97 | 4.62 | 408 | IA |

TABLE 7 (continued)

| Example No | Product | MH+ | Purity % | LCMS Ret time (min) | BB1 IC50 (nm) | BB2 IC50 (nm) |
|---|---|---|---|---|---|---|
| 135 | (S)-2-(5-Chloro-1,3-dimethyl-1H-pyrazole-4-sulfonylamino)-3-(1H-indol-3-yl)-2-methyl-N-(1-pyridin-2-yl-cyclohexylmethyl)-propionamide | 583,16 | 98 | 4.38 | 1252 | IA |
| 136 | (S)-2-(3,5-Dimethyl-isoxazole-4-sulfonylamino)-3-(1H-indol-3-yl)-2-methyl-N-(1-pyridin-2-yl-cyclohexylmethyl)-propionamide | 549,70 | 96 | 4.54 | 515 | IA |
| 137 | (S)-2-(Benzo[1,2,5]thiadiazole-4-sulfonylamino)-3-(1H-indol-3-yl)-2-methyl-N-(1-pyridin-2-yl-cyclohexylmethyl)-propionamide | 588,76 | 97 | 4.67 | 256 | IA |
| 138 | (S)-3-(1H-Indol-3-yl)-2-methyl-2-(1-methyl-1H-imidazole-4-sulfonylamino)-N-(1-pyridin-2-yl-cyclohexylmethyl)-propionamide | 534,69 | 100 | 3.60 | 3667 | IA |
| 139 | (S)-2-(Benzo[1,2,5]oxadiazole-4-sulfonylamino)-3-(1H-indol-3-yl)-2-methyl-N-(1-pyridin-2-yl-cyclohexylmethyl)-propionamide | 572,69 | 100 | 4.70 | 507 | IA |
| 140 | 3-{(S)-2-(1H-Indol-3-yl)-1-methyl-1-[(1-pyridin-2-yl-cyclohexylmethyl)-carbamoyl]-ethylsulfamoyl}-thiophene-2-carboxylic acid methyl ester | 594,76 | 100 | 4.79 | 167 | IA |
| 141 | (S)-3-(1H-Indol-3-yl)-2-(5-isoxazol-3-yl-thiophene-2-sulfonylamino)-2-methyl-N-(1-pyridin-2-yl-cyclohexylmethyl)-propionamide | 603,77 | 98 | 4.60 | 534 | IA |
| 142 | (S)-3-(1H-Indol-3-yl)-2-methyl-2-(2-nitro-phenylmethanesulfonylamino)-N-(1-pyridin-2-yl-cyclohexylmethyl)-propionamide | 589,72 | 100 | 4.65 | 430 | IA |
| 143 | (S)-2-(3-Cyano-benzenesulfonylamino)-3-(1H-indol-3-yl)-2-methyl-N-(1-pyridin-2-yl-cyclohexylmethyl)-propionamide | 555,70 | 99 | 4.55 | 460 | IA |
| 144 | (S)-2-(1,2-Dimethyl-1H-imidazole-4-sulfonylamino)-3-(1H-indol-3-yl)-2-methyl-N-(1-pyridin-2-yl-cyclohexylmethyl)-propionamide | 548,71 | 96 | 3.55 | 2482 | IA |
| 145 | (S)-3-(1H-Indol-3-yl)-2-(3-methoxy-benzenesulfonylamino)-2-methyl-N-(1-pyridin-2-yl-cyclohexylmethyl)-propionamide | 560,72 | 99 | 4.75 | 295 | 3686 |
| 146 | (S)-3-(1H-Indol-3-yl)-2-methyl-2-(8-nitro-naphthalene-1-sulfonylamino)-N-(1-pyridin-2-yl-cyclohexylmethyl)-propionamide | 625,75 | 99 | 4.89 | 177 | IA |

TABLE 7 (continued)

| Example No | Product | $MH^+$ | Purity % | LCMS Ret time (min) | BB1 IC50 (nm) | BB2 IC50 (nm) |
|---|---|---|---|---|---|---|
| 147 | (S)-2-(2-Chloro-5-nitro-benzenesulfonylamino)-3-(1H-indol-3-yl)-2-methyl-N-(1-pyridin-2-yl-cyclohexylmethyl)-propionamide | 610,14 | 96 | 5.00 | 374 | Ia |
| 148 | (S)-3-(1H-Indol-3-yl)-2-methyl-N-(1-pyridin-2-yl-cyclohexylmethyl)-2-(2,4,6-trichloro-benzenesulfonylamino)-propionamide | 634,03 | 100 | 5.45 | 215 | Ia |
| 149 | (S)-2-(4-Chloro-2-nitro-benzenesulfonylamino)-3-(1H-indol-3-yl)-2-methyl-N-(1-pyridin-2-yl-cyclohexylmethyl)-propionamide | 610,14 | 100 | 5.13 | 513 | IA |
| 150 | (S)-2-(5-Benzenesulfonyl-thiophene-2-sulfonylamino)-3-(1H-indol-3-yl)-2-methyl-N-(1-pyridin-2-yl-cyclohexylmethyl)-propionamide | 676,88 | 100 | 5.03 | 297 | IA |
| 151 | (S)-3-(1H-Indol-3-yl)-2-methyl-N-(1-pyridin-2-yl-cyclohexylmethyl)-2-(4-trifluoromethoxy-benzenesulfonylamino)-propion amide | 614,69 | 99 | 5.35 | 635 | IA |
| 152 | (S)-3-(1H-Indol-3-yl)-2-methyl-2-(5-methyl-2-phenoxy-benzenesulfonylamino) -N-(1-pyridin-2-yl-cyclohexylmethyl)-propionamide | 636,82 | 97 | 5.79 | 76 | IA |
| 153 | (S)-3-(1H-Indol-3-yl)-2-methyl-N-(1-pyridin-2-yl-cyclohexylmethyl)-2-(2-p-tolyloxy-benzenesulfonylamino)-propionamide | 636,82 | 97 | 5.79 | 90 | IA |
| 154 | (S)-2-(Biphenyl-2-sulfonylamino)-3-(1H-indol-3-yl)-2-methyl-N-(1-pyridin-2-yl-cyclohexylmethyl)-propionamide | 606,79 | 97 | 5.52 | 166 | IA |
| 155 | 2-{(S)-2-(1H-Indol-3-yl)-1-methyl-1-[(1-pyridin-2-yl-cyclohexylmethyl)-carbamoyl]-ethylsulfamoyl} -benzoic acid methyl ester | 588,73 | 99 | 4.84 | 242 | IA |
| 156 | (S)-2-(3-Chloro-4-fluoro-benzenesulfonylamino)-3-(1H-indol-3-yl)-2-methyl-N-(1-pyridin-2-yl-cyclohexylmethyl)-propionamide | 583,13 | 95 | 5.12 | 284 | 1216 |
| 157 | (S)-2-(2,5-Dichloro-thiophene-3-sulfonylamino)-3-(1H-indol-3-yl)-2-methyl-N-(1-pyridin-2-yl-cyclohexylmethyl)-propionamide | 605,61 | 99 | 5.23 | 214 | IA |
| 158 | (S)-2-(3-Chloro-4-methyl-benzenesulfonylamino)-3-(1H-indol-3-yl)-2-methyl-N-(1-pyridin-2-yl-cyclohexylmethyl)-propionamide | 579,17 | 97 | 5.28 | 299 | 3939 |

TABLE 7 (continued)

| Example No | Product | MH+ | Purity % | LCMS Ret time (min) | BB1 IC50 (nm) | BB2 IC50 (nm) |
|---|---|---|---|---|---|---|
| 159 | (S)-3-(1H-Indol-3-yl)-2-(2-methoxy-4-methyl-benzenesulfonylamino)-2-methyl-N-(1-pyridin-2-yl-cyclohexylmethyl)-propionamide | 574,75 | 96 | 4.92 | 445 | IA |
| 160 | (S)-3-(1H-Indol-3-yl)-2-methyl-N-(1-pyridin-2-yl-cyclohexylmethyl)-2-(5-pyridin-2-yl-thiophene-2-sulfonylamino)-propionamide | 613,81 | 100 | 4.79 | 344 | IA |
| 161 | (S)-2-(5-Bromo-6-chloro-pyridine-3-sulfonylamino)-3-(1H-indol-3-yl)-2-methyl-N-(1-pyridin-2-yl-cyclohexylmethyl)-propionamide | 645,02 | 95 | 5.09 | 187 | IA |
| 162 | (S)-2-(2,4-Dinitro-benzenesulfonylamino)-3-(1H-indol-3-yl)-2-methyl-N-(1-pyridin-2-yl-cyclohexylmethyl)-propionamide | 620,69 | 100 | 4.97 | 475 | IA |
| 163 | (S)-3-(1H-Indol-3-yl)-2-(4-methanesulfonyl-benzenesulfonylamino)-2-methyl-N-(1-pyridin-2-yl-cyclohexylmethyl)-propionamide | 608,78 | 98 | 4.20 | 1043 | IA |
| 164 | (S)-2-(4-tert-Butyl-benzenesulfonylamino)-3-(1H-indol-3-yl)-2-methyl-N-(1-pyridin-2-yl-cyclohexylmethyl)-propionamide | 586,80 | 96 | 5.65 | 406 | IA |
| 165 | (S)-2-(2,4-Dichloro-5-methyl-benzenesulfonylamino)-3-(1H-indol-3-yl)-2-methyl-N-(1-pyridin-2-yl-cyclohexylmethyl)-propionamide | 613,61 | 97 | 5.64 | 172 | IA |
| 166 | (S)-2-(Chloro-trifluoromethyl-benzenesulfonylamino)-3-(1H-indol-3-yl)-2-methyl-N-(1-pyridin-2-yl-cyclohexylmethyl)-propionamide | 633,14 | 100 | 5.33 | 627 | IA |
| 167 | (S)-3-(1H-Indol-3-yl)-2-methyl-2-(nitro-trifluoromethyl-benzenesulfonylamino) -N-(1-pyridin-2-yl-cyclohexylmethyl)-propionamide | 643,69 | 100 | 5.34 | 758 | IA |
| 168 | (S)-2-(4-Butyl-benzenesulfonylamino)-3-(1H-indol-3-yl)-2-methyl-N-(1-pyridin-2-yl-cyclohexylmethyl)-propionamide | 586,80 | 96 | 5.84 | 492 | IA |

**Example 169**

**Bombesin antagonists potentiate pelvic nerve-stimulated increases in female genital blood flow in the anaesthetised rabbit model of sexual arousal.**

**Bombesin anatgonists used = Compound 1 and (2*S*)-*N*-{[1-(4-aminophenyl) cyclohexyl] methyl}-3-(1*H*-indol-3-yl)-2-methyl-2-{[(4-nitroanilino) carbonyl] amino} propanamide (Compound 3).**

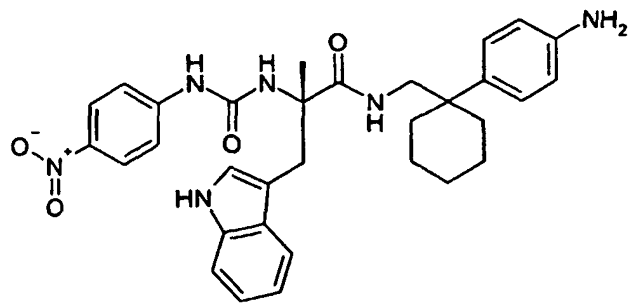

$hBB_1$ Ki 0.25 nM

$hBB_2$ Ki 46 nM

Female New Zealand rabbits (~2.5kg) were pre-medicated with a combination of Medetomidine (Domitor®) 0.5ml/kg *i.m.*, and Ketamine (Vetalar®) 0.25ml/kg *i.m.* whilst maintaining oxygen intake via a face mask. The rabbits were tracheotomised using a Portex™ uncuffed endotracheal tube 3 ID., connected to ventilator and maintained at a ventilation rate of 30-40 breaths per minute, with an approximate tidal volume of 18-20 ml, and a maximum airway pressure of 10 cm $H_2O$. Anaesthesia was then switched to Isoflurane and ventilation continued with $O_2$ at 21/min. The right marginal ear vein was cannulated using a 23G or 24G catheter, and Lactated Ringer solution perfused at 0.5ml/min. The rabbit was maintained at 3% Isoflurane during invasive surgery, dropping to 2% for maintenance anaesthesia.

The left groin area of the rabbit was shaved and a vertical incision was made approximately 5cm in length along the thigh. The femoral vein and artery were exposed, isolated and then cannulated with a PVC catheter (17G) for the infusion of drugs and compounds. Cannulation was repeated for the femoral artery, inserting the catheter to a depth of 10cm to ensure that the catheter reached the abdominal aorta. This arterial catheter was linked to a Gould system to record blood pressure. Samples for blood gas analysis were also taken via the arterial catheter. Systolic and diastolic pressures were measured, and the mean arterial pressure calculated using the formula (diastolic x2 + systolic) ÷3. Heart rate was measured via the pulse oxymeter and *Po-ne-mah* data acquisition software system (Ponemah Physiology Platform, Gould Instrument Systems Inc). A ventral midline incision was made into the abdominal cavity. The incision was about 5cm in length just above the pubis. The fat and muscle was *bluntly* dissected away to reveal the hypogastric nerve which runs down the body cavity. It was essential to keep close to the side curve of the pubis wall in order to avoid damaging the femoral vein and artery which lie above the pubis. The sciatic and pelvic nerves lie deeper and were located after further dissection on the dorsal side of the rabbit. Once the sciatic nerve is identified, the pelvic nerve was easily located. The term *pelvic nerve* is loosely applied; anatomy books on the subject fail to identify the nerves in sufficient detail. However, stimulation of the nerve causes an increase in vaginal and clitoral blood flow, and innervation of the pelvic region. The pelvic nerve was freed away from surrounding tissue and a *Harvard* bipolar stimulating electrode was placed around the nerve. The nerve was slightly lifted to give some tension, then the electrode was secured in position. Approximately 1ml of light paraffin oil was placed around the nerve and electrode. This acts as a protective lubricant to the nerve and prevents blood contamination of the electrode. The electrode was connected to a *Grass* S88 Stimulator. The pelvic nerve was stimulated using the following parameters:- 5V, pulse width 0.5ms, duration of stimulus 10 seconds and a frequency range of 2 to 16Hz. Reproducible responses were obtained when the nerve was stimulated every 15-20 minutes. A frequency response curve was determined at the start of each experiment in order to determine the optimum frequency to use as a sub-maximal response, normally 4Hz. A ventral

midline incision was made, at the caudal end of the pubis, to expose the pubic area. Connective tissue was removed to expose the tunica of the clitoris, ensuring that the wall was free from small blood vessels. The external vaginal wall was also exposed by removing any connective tissue. One laser Doppler flow probe was inserted 3cm into the vagina, so that half the probe shaft was still visible. A second probe was positioned so that it lay just above the external clitoral wall. The position of these probes was then adjusted until a signal was obtained. A second probe was placed just above the surface of a blood vessel on the external vaginal wall. Both probes were clamped in position.

Compound **1** and compound **3** were dissolved in 50% β-cyclodextrin in saline. They were administered at a dose of 15mg/kg subcutaneously (sc). Vaginal and clitoral blood flow was recorded either as numbers directly from the Flowmeter using *Po-ne-mah* data acquisition software (Ponemah Physiology Platform, Gould Instrument Systems Inc), or indirectly from Gould chart recorder trace. Calibration was set at the beginning of the experiment (0-125ml/min/100g tissue). All data are reported as mean ± s.e.m.. Significant changes were identified using Student's t-tests.

The non-selective BB1/BB2 bombesin receptor antagonist (Compound **1;** 15mg/kg sc) acts as a potent enhancer of pelvic-nerve stimulated (PNS) increases in genital blood flow in the anaesthetised rabbit (Figure 22). Compound **1** had no effect on basal genital blood flow in the absence of PNS (Figure 22). This reinforces our view that antagonising/blocking BB1/BB2 receptors will enhance the arousal response by potentiating the central mechanism (s) that control sexual arousal/genital blood flow, and will not induce arousal in the absence of sexual stimulation.

The selective BB1 receptor antagonist (Compound **3;** 15mg/kg sc) acts as a potent enhancer of pelvic-nerve stimulated (PNS) increases in vaginal and clitoral blood flow in the anaesthetised rabbit (Figure 23). The potentiation was significant 45mins after sc dosing and remained elevated for circa 1hr. Compound **3** had no effect on basal genital blood flow in the absence of PNS (Figure 23). This reinforces our view that a selective BB1 receptor antagonist will enhance the arousal response by potentiating the central mechanism(s) that control sexual arousal/genital blood flow, thereby treating FSAD, and will not induce arousal in the absence of sexual stimulation. Since these agents also enhance clitoral blood flow it is likely that they will be effective in the treatment of orgasmic disorders.

At the level of the genitalia, the enhancement observed is similar to the beneficial effects observed with apomorphine, and consequently we believe that a centrally mediated potentiation of the descending neuronal pathways that control genital blood flow is responsible.

**Example 170**

**Bombesin antagonists (compounds 1 and 3) induce increases in penile intracavernosal pressure in the conscious male rat.**

**Bombesin anatgonists used = Compound 1 and (2*S*)-*N*-{[1-(4-aminophenyl) cyclohexyl] methyl}-3-(1*H*-indol-3-yl)-2-methyl-2-{[(4-nitroanilino) carbonyl] amino} propanamide (Compound 3).**

In addition to treating women with FSD, bombesin antagonists will be useful in treating male erectile dysfunction (MED. Both the non-selective BB1/2 antagonists (Compound 1; 10mg/kg sc) and the selective BB1 receptor antagonist (Compound 3; 15mg/kg sc) is pro-erectile in a conscious rat model of penile erection (Figures 24 and 25). Erectile responses were recorded by measuring intracavernosal pressure using surgically implanted telemetric device. The specific details the surgical procedures, data acquisition and analysis can be found in detail in Bemabe 1999.

Compound **1** and compound **3** were dissolved in 50% β-cyclodextrin in saline. They were administered at a dose of 15mg/kg subcutaneously (sc). One hour after 10mg/kg subcutaneous administration of Compound **1** and 45 minutes after a subcutaneous administration of 15mg/kg compound **3** one observes significant increases in intracavernosal pressure. These increases equate to penile erection. Both Compounds **1** and **3** induced a number of erections in a similar manner to those observed with apomorphine or melanotan-II - both of which are clinically proven agents that are effective in the treatment of MED. Moreover the amplitude of the increases observed were similar to those observed with apomorphine or melanotan-II. The mechanism of action is thought to be similar to the effects on female genital flow ie CNS potentiation of the descending neuronal pathways that control penile erection.

**Example 171**

**Concomitant administration of a Bombesin antagonists with a PDE5 inhibitor enhance pelvic nerve stimulated increases in penile intracavernosal pressure in an anaesthetised rabbit model of erection**

In addition to treating women with FSD, bombesin antagonists will be useful in treating male erectile dysfunction (MED) either alone or in combination with a selective PDE5 inhibitor.

Male New Zealand rabbits (~2.5kg) were pre-medicated with a combination of Medetomidine (Domitor®) 0.5ml/kg *i.m.,* and Ketamine (Vetalar®) 0.25ml/kg *i.m*. whilst maintaining oxygen intake via a face mask. The rabbits

were tracheotomised using a Portex™ uncuffed endotracheal tube 3 ID., connected to ventilator and maintained at a ventilation rate of 30-40 breaths per minute, with an approximate tidal volume of 18-20 ml, and a maximum airway pressure of 10 cm $H_2O$. Anaesthesia was then switched to Isoflurane and ventilation continued with $O_2$ at 2l/min. The right marginal ear vein was cannulated using a 23G or 24G catheter, and Lactated Ringer solution perfused at 0.5ml/min. The rabbit was maintained at 3% Isoflurane during invasive surgery, dropping to 2% for maintenance anaesthesia. The left jugular vein was exposed, isolated and then cannulated with a PVC catheter (17G) for the infusion of drugs and compounds.

The left groin area of the rabbit was shaved and a vertical incision was made approximately 5cm in length along the thigh. The femoral vein and artery were exposed, isolated and then cannulated with a PVC catheter (17G) for the infusion of drugs and compounds. Cannulation was repeated for the femoral artery, inserting the catheter to a depth of 10cm to ensure that the catheter reached the abdominal aorta. This arterial catheter was linked to a Gould system to record blood pressure. Samples for blood gas analysis were also taken via the arterial catheter. Systolic and diastolic pressures were measured, and the mean arterial pressure calculated using the formula (diastolic x2 + systolic) ÷3. Heart rate was measured via the pulse oxymeter and *Po-ne-mah* data acquisition software system (Ponemah Physiology Platform, Gould Instrument Systems Inc).

A ventral midline incision was made into the abdominal cavity. The incision was about 5cm in length just above the pubis. The fat and muscle was *bluntly* dissected away to reveal the hypogastric nerve which runs down the body cavity. It was essential to keep close to the side curve of the pubis wall in order to avoid damaging the femoral vein and artery which lie above the pubis. The sciatic and pelvic nerves lie deeper and were located after further dissection on the dorsal side of the rabbit. Once the sciatic nerve is identified, the pelvic nerve was easily located. The term *pelvic nerve* is loosely applied; anatomy books on the subject fail to identify the nerves in sufficient detail. However, stimulation of the nerve causes an increase in intracavernosal pressure and cavernosal blood flow, and innervation of the pelvic region. The pelvic nerve was freed away from surrounding tissue and a *Harvard* bipolar stimulating electrode was placed around the nerve. The nerve was slightly lifted to give some tension, then the electrode was secured in position. Approximately 1ml of light paraffin oil was placed around the nerve and electrode. This acts as a protective lubricant to the nerve and prevents blood contamination of the electrode. The electrode was connected to a *Grass* S88 Stimulator. The pelvic nerve was stimulated using the following parameters:- 5V, pulse width 0.5ms, duration of stimulus 20 seconds with a frequency of 16Hz. Reproducible responses were obtained when the nerve was stimulated every 15-20 minutes. Several stimulations using the above parameters were performed to establish a mean control response. The compound (s) to be tested were infused, via the jugular vein, using a *Harvard* 22 infusion pump allowing a continuous 15 minute stimulation cycle. The skin and connective tissue around the penis was removed to expose the penis. A catheter set (*Insyte-W,* Becton-Dickinson 20 Gauge 1.1 x 48mm) was inserted through the tunica albica into the left *corpus cavernosal* space and the needle removed, leaving a flexible catheter. This catheter was linked via a pressure transducer (Ohmeda 5299-04) to a Gould system to record intracavernosal pressure. Once an intracavemosal pressure was established, the catheter was sealed in place using *Vetbond* (tissue adhesive, 3M). Heart rate was measured via the pulse oxymeter and *Po-ne-mah* data acquisition software system (Ponemah Physiology Platform, Gould Instrument Systems Inc).

Intracavemosal blood flow was recorded either as numbers directly from the Flowmeter using *Po-ne-mah* data acquisition software (Ponemah Physiology Platform, Gould Instrument Systems Inc), or indirectly from Gould chart recorder trace. Calibration was set at the beginning of the experiment (0-125ml/min/100g tissue). All data are reported as mean ± s.e.m.. Significant changes were identified using Student's t-tests.

Compound **1** and compound **3** were dissolved in 50% β-cyclodextrin in saline. They were administered at a dose of 15mg/kg subcutaneously (sc). Concomitant inhibition of BB1 receptors with compound 3 and PDE5 enzyme with a PDE5 inhibitor produced a marked enhancement of intracavemosal pressure, or the erectile process.

Figure 26 demonstrate that concomitant inhibition of Compound 3 (10mg/kg sc) and a selective inhibitor of PDE5 (3-ethyl-5-{5-[4-ethylpiperzino)sulphonyl-2-propoxyphenyl}-2-(2-pyridylmethyl)-6,7-dihydro-2H-pyrazolo[4,3-*d*]pyrimidin-7-one also known as 3-ethyl-5-{S-[4-ethylpiperzin-1-ylsulphonyl)-2-n-propoxyphenyl}-2-(2-pyridyl)methyl-2,6-dihydro-7H-pyrazolo[4,3-*d*]pyrimidin-7-one; See WO98/491066; 1mg/kg iv) produced a marked enhancement of the ICP, or the erectile process, than was achievable with the same dose of the same Compound 3 inhibitor alone. BB1 antagonists and PDE5 inhibitors or combinations of the two, have no significant effect on un-stimulated intracavemosal pressure ie they do not induce an increase in the absence of sexual drive/arousal. This data illustrates that there are a number of clinical benefits of concomitant administration of a PDE5 inhibitor and a bombesin antagonist over PDE5 inhibitor therapy alone. These include increased efficacy and opportunities to treat MED subgroups that do not respond to PDE5 inhibitor therapy.

**Test Assays: Auxiliary Compounds**

**NEP ENZYME ASSAY**

THE PREPARATION AND ASSAY OF SOLUBLE (NEP) NEUTRAL ENDOPEPTIDASE FROM CANINE, RAT, RABBIT AND HUMAN KIDNEY CORTEX.

**Soluble NEP is obtained from the kidney cortex and activity is assayed by measuring the rate of cleavage of the NEP substrate Abz-D-Arg-Arg-Leu-EDDnp to generate its fluorescent product, Abz-D-Arg-Arg.**

**EXPERIMENTAL PROCEDURE:**

**1. MATERIALS**

**All water is double de ionised.**

1.1 Tissues

Human Kidney IIAM (Pennsylvania. U.S.A.)
Rat Kidney
Rabbit Kidney
Canine Kidney

1.2 Homogenisation medium

100mM Mannitol and 20mM Tris @ pH 7.1
2.42g Tris (Fisher T/P630/60) is diluted in 1 litre of water and the pH adjusted to 7.1 using 6M HCl at room temperature. To this 18.22g Mannitol (Sigma M-9546) is added.

1.3 Tris buffer (NEP buffer).
50ml of 50mM Tris pH 7.4 (Sigma T2663) is diluted in 950ml of water.
1.4 Substrate (Abz-D-Arg-Arg-Leu-EDDnp)
Made to order from SNPE, and is stored as a powder at -20°C. A 2mM stock is made by gently re-suspending the substrate in Tris buffer, this should not be vortexed or sonicated. 600μl aliquots of the 2mM stock are stored at -20 for up to one month. (Medeiros, M.A.S., Franca, M.S.F. et al., (1997), Brazilian Journal of Medical and Biological Research, 30, 1157-1162).
1.5 Total product
Samples corresponding to 100% substrate to product conversion are included on the plate to enable the % substrate turnover to be determined. The total product is generated by incubating 1ml of 2mM substrate with 20μl of enzyme stock for 24 hours at 37°C.
1.6 Stop solution.
A 300μM stock of Phosphoramidon (Sigma R7385) is made up in NEP buffer and stored in 50μl aliquots at -20.
1.7 Dimethyl sulphoxide (DMSO).
1.8 Magnesium Chloride -$MgCl_2.6H_2O$ (Fisher M0600/53).
1.9 Black 96 well flat bottom assay plates (Costar 3915).
1.10 Topseal A (Packard 6005185).
1.11 Centrifuge tubes

**2. SPECIFIC EQUIPTMENT**

2.1 Sorvall RC-5B centrifuge (SS34 GSA rotor, pre-cooled to 4°C).
2.2 Braun miniprimer mixer.
2.3 Beckman CS-6R centrifuge.
2.4 Fluostar galaxy.
2.5 Wesbart 1589 shaking incubator.

**3. METHODS**

3.1 TISSUE PREPARATION

3.2 Dog, rat, rabbit, and human NEP is obtained from the kidney cortex using a method adapted from Booth, A. G. & Kenny, A.J. (1974) *Biochem. J.* 142, 575-581.

3.3 Frozen kidneys are allowed to thaw at room temperature and the cortex is dissected away from the medulla.

3.4 The cortex is finely chopped and homogenised in approximately 10 volumes of homogenisation buffer (1.2) using a Braun miniprimer (2.2).

3.5 Magnesium chloride (1.8) (20.3mg/gm tissue) is added to the homogenate and stirred in an ice-water bath for 15 minutes.

3.6 The homogenate is centrifuged at 1,500g (3,820rpm) for 12 minutes in a Beckman centrifuge (2.3) before removing the supernatant to a fresh centrifuge tube and discarding the pellet.

3.7 The supernatant is centrifuged at 15,000g (12,100rpm) for 12 minutes in a Sovall centrifuge (2.1) and the supernatant is discarded.

3.8 The pale pink layer on the top of the remaining pellet is removed and resuspended in homogenisation buffer containing magnesium chloride (9mg MgCl in 5ml buffer per 1g tissue).

3.9 The suspension is centrifuged at 2,200g (4,630rpm) for 12 minutes in a Beckman centrifuge (2.3) before discarding the pellet.

3.10 The supernatant is centrifuged at 15,000g (12,100rpm) for 12 minutes using the Sorvall centrifuge (2.1) and the supernatant is discarded.

3.11 The final pellet is resuspended in homogenisation buffer containing magnesium chloride (0.9mg MgCl in 0.5ml buffer per 1g tissue). A homogenous suspension is obtained using a Braun miniprimer (2.2). This is then frozen down in 100µl aliquots to be assayed for NEP activity.

**4.0 DETERMINATION OF NEP ACTIVITY**

The activity of the previously aliquoted NEP is measured by its ability to cleave the NEP specific peptide substrate.

4.1 A 4% DMSO/NEP buffer solution is made (4mls DMSO in 96mls NEP buffer).

4.2 Substrate, total product, enzyme, and Phosphoramidon stocks are left on ice to thaw.

4.3 50µl of 4% DMSO/NEP buffer solution is added to each well.

4.4 The 2mM substrate stock is diluted 1:40 to make a 50µM solution. 100µl of 50µM substrate is added to each well (final concentration 25µM).

4.5 50µl of a range of enzyme dilutions is added to initiate the reaction (usually 1:100, 1:200, 1:400, 1:800, 1:1600, and 1:3200 are used). 50µl of NEP buffer is added to blank wells.

4.6 The 2mM total product is diluted 1:80 to make a 25µM solution. 200µl of 25µM product is added to the first four wells of a new plate.

4.7 Plates are incubated at 37oC in a shaking incubator for 60 minutes.

4.8 The 300µM Phosphoramidon stock is diluted 1:100 to 300nM. The reaction is stopped by the addition of 100µl 300nM Phosphoramidon and incubated at 37°C in a shaking incubator for 20 minutes before being read on the Fluostar (ex320/em420).

**5. NEP INHIBITION ASSAYS**

5.1 Substrate, total product, enzyme and Phoshoramidon stocks are left on ice to thaw.

5.2 Compound stocks are made up in 100% DMSO and diluted 1:25 in NEP buffer to give a 4% DMSO solution. All further dilutions are carried out in a 4% DMSO solution (4mls DMSO in 96mls NEP buffer).

5.3 50µl of compound in duplicate is added to the 96 well plate and 50µl of 4% DMSO/NEP buffer is added to control and blank wells.

5.4 The 2mM substrate stock is diluted 1:40 in NEP buffer to make a 50µM solution (275µl 2mM substrate to 10.73ml buffer is enough for 1 plate).

5.5 The enzyme stock diluted in NEP buffer (determined from activity checks).

5.6 The 2mM total product stock is diluted 1:80 in NEP buffer to make a 25µM solution. 200µl is added to the first

four wells of a separate plate.

5.7 The 300µM Phosphoramidon stock is diluted 1:1000 to make a 300nM stock (11µl Phosphoramidon to 10.99ml NEP buffer.

5.8 To each well in the 96 well plate the following is added:

Table Reagents to be added to 96 well plate.

| | Compound/ DMSO | Tris Buffer | Substrate | NEP enzyme | Total product |
|---|---|---|---|---|---|
| Samples | 2µl compound | 50µl | 100µl | 50µl | None |
| Controls | 2µl DMSO | 50µl | 100µl | 50µl | None |
| Blanks | 2µl DMSO | 100µl | 100µl | None | None |
| Totals | 2µl DMSO | None | None | None | 200µl |

5.9 The reaction is initiated by the addition of the NEP enzyme before incubating at 37°C for 1 hour in a shaking incubator.

5.10 The reaction is stopped with 100µl 300nM Phosphoramidon and incubated at 37°C for 20 minutes in a shaking incubator before being read on the Fluostar (ex320/em420).

**6. CALCULATIONS**

The activity of the NEP enzyme is determined in the presence and absence of compound and expressed as a percentage.

% Control activity (turnover of enzyme):

$$\frac{\text{Mean FU of controls - Mean FU of blanks}}{\text{Mean FU of totals - Mean FU of blanks}} \times 100$$

% Activity with inhibitor:

$$\frac{\text{Mean FU of compound - Mean FU of blanks}}{\text{Mean FU of totals - Mean FU of blanks}} \times 100$$

Activity expressed as % of control:

$$\frac{\text{\% Activity with inhibitor}}{\text{\% Control activity}} \times 100$$

A sigmoidal dose-response curve is fitted to the % activities (% of control) vs compound concentration and IC50 values calculated using LabStats fit-curve in Excel.

**ACE ASSAY**

THE PREPARATION AND ASSAY OF SOLUBLE ANGIOTENSIN CONVERTING ENZYME (ACE), FROM PORCINE AND HUMAN KIDNEY CORTEX.

**Soluble ACE activity is obtained from the kidney cortex and assayed by measuring the rate of cleavage of the ACE substrate Abz-Gly-p-nitro-Phe-Pro-OH to generate its fluorescent product, Abz-Gly.**

1. MATERIALS

**All water is double de ionised.**

1.1 Human Kidney IIAM (Pennsylvania. U.S.A.) or UK Human Tissue Bank (UK HTB)

1.2 Porcine kidney ACE Sigma (A2580)

1.3 Homogenisation buffer-1

100mM Mannitol and 20mM Tris @ pH 7.1

2.42g Tris (Fisher T/P630/60) is diluted in 1 litre of water and the pH adjusted to 7.1 using 6M HCl at room temperature. To this 18.22g Mannitol (Sigma M-9546) is added.
1.4 Homogenisation buffer-2
100mM Mannitol, 20mM Tris @ pH7.1 and 10mM $MgCl_2.6H_2O$ (Fisher M0600/53) To 500ml of the homogenisation buffer 1 (1.4) 1.017g of $MgCl_2$ is added.
1.5 Tris buffer (ACE buffer).
50mM Tris and 300mM NaCl @ pH 7.4
50ml of 50mM Tris pH 7.4 (Sigma T2663) and 17.52g NaCl (Fisher S/3160/60) are made up to 1000ml in water.
1.6 Substrate (Abz-D-Gly-p-nitro-Phe-Pro-OH) (Bachem M-1100)
ACE substrate is stored as a powder at -20°C. A 2mM stock is made by gently re-suspending the substrate in ACE buffer, this must not be vortexed or sonicated. 400μl aliquots of the 2mM stock are stored at -20°C for up to one month.
1.7 Total product
Samples corresponding to 100% substrate to product conversion are included on the plate to enable the % substrate turnover to be determined (see calculations). The total product is generated by incubating 1ml of 2mM substrate with 20μl of enzyme stock for 24 hours at 37°C.
1.8 Stop solution.
0.5M EDTA (Promega CAS[6081/92/6]) is diluted 1:250 in ACE buffer to make a 2mM solution.
1.9 Dimethyl sulphoxide (DMSO).
1.10 Magnesium Chloride -$MgCl_2.6H_2O$ (Fisher M0600/53).
1.11 Black 96 well flat bottom assay plates (Costar 3915 or Packard).
1.12 Topseal A (Packard 6005185).
1.13 Centrifuge tubes

**2. SPECIFIC EQUIPTMENT**

2.1 Sorvall RC-5B centrifuge (SS34 GSA rotor, pre-cooled to 4°C).
2.2 Braun miniprimer mixer.
2.3 Beckman CS-6R centrifuge.
2.4 BMG Fluostar Galaxy.
2.5 Wesbart 1589 shaking incubator.

**3. METHODS**

**3.1 TISSUE PREPARATION**
3.3 Human ACE is obtained from the kidney cortex using a method adapted from Booth, A.G. & Kenny, A.J. (1974) *Biochem. J.* 142, 575-581.
3.3 Frozen kidneys are allowed to thaw at room temperature and the cortex is dissected away from the medulla.
3.4 The cortex is finely chopped and homogenised in approximately 10 volumes of homogenisation buffer-1 (1.4) using a Braun miniprimer (2.2).
3.5 Magnesium chloride (1.11) (20.3mg/gm tissue) is added to the homogenate and stirred in an ice-water bath for 15 minutes.
3.6 The homogenate is centrifuged at 1,500g (3,820rpm) for 12 minutes in a Beckman centrifuge (2.3) before removing the supernatant to a fresh centrifuge tube and discarding the pellet.
3.7 The supernatant is centrifuged at 15,000g (12,100rpm) for 12 minutes in a Sovall centrifuge (2.1) and the supernatant is discarded.
3.8 The pale pink layer on the top of the remaining pellet is removed and resuspended in homogenisation buffer-2 (1.5) (5ml buffer per 1g tissue).
3.9 The suspension is centrifuged at 2,200g (4,630rpm) for 12 minutes in a Beckman centrifuge before discarding the pellet.
3.10 The supernatant is centrifuged at 15,000g (12,100rpm) for 12 minutes using the Sorvall centrifuge and the supernatant is discarded.
3.11 The final pellet is resuspended in homogenisation buffer-2 (0.5ml buffer per 1g tissue). A homogenous suspension is obtained using a Braun miniprimer. This is then frozen down in 100μl aliquots to be assayed for NEP

activity.

**4.0 DETERMINATION OF ACE ACTIVITY**

The activity of the previously aliquoted ACE is measured by its ability to cleave the ACE specific peptide substrate.

Porcine ACE (1.2) is defrosted and resuspended in ACE buffer (1.6) at 0.004U/µl, this is frozen down in 50µl aliquots.

4.1 A 4% DMSO/ACE buffer solution is made (4mls DMSO in 96mls ACE buffer).
4.2 Substrate (1.7), total product (1.8) and enzyme (1.1, 1.2, 1.3), are left on ice to thaw.
4.3 50µl of 4% DMSO/ACE buffer solution is added to each well.
4.4 The 2mM substrate stock is diluted 1:100 to make a 20µM solution. 100µl of 20µM substrate is added to each well (final concentration in the assay 10µM).
4.5 50µl of a range of enzyme dilutions is added to initiate the reaction (usually 1:100, 1:200, 1:400, 1:800, 1:1600, and 1:3200 are used). 50µl of ACE buffer is added to blank wells.
4.6 The 2mM total product is diluted 1:200 to make 10µM solution. 200µl 10µM product is added to the first four wells of a new plate.
4.7 Plates are incubated at 37°C in a shaking incubator for 60 minutes.
4.8 The enzyme reaction is stopped by the addition of 100µl 2mM EDTA in ACE buffer and incubated at 37°C in a shaking incubator for 20 minutes before being read on the BMG Fluostar Galaxy (ex320/em420).

**5. ACE INHIBITION ASSAYS**

5.1 Substrate, total product, and enzyme stocks are left on ice to thaw.
5.2 Compound stocks are made up in 100% DMSO and diluted 1:25 in ACE buffer to give a 4% DMSO solution. All further dilutions are carried out in a 4% DMSO/ACE buffer solution (4mls DMSO in 96mls ACE buffer).
5.3 50µl of compound, in duplicate, is added to the 96 well plate and 50µl of 4% DMSO/ACE buffer is added to control and blank wells.
5.4 Steps 5.2 and 5.3 can be carried out either by hand or using the Packard multiprobe robots
5.5 The 2mM substrate stock is diluted 1:100 in ACE buffer to make a 20µM solution (10µM final concentration in the assay) (110µl of 2mM substrate added to 10.89ml buffer is enough for 1 plate).
5.6 The enzyme stock is diluted in ACE buffer, as determined from activity checks (4.0).
5.7 The 2mM total product stock is diluted 1:200 in ACE buffer to make a 10µM solution. 200µl is added to the first four wells of a separate plate.
5.8 The 0.5mM EDTA stock is diluted 1:250 to make a 2mM stock (44µl EDTA to 10.96ml ACE buffer).
5.9 To each well of the 96 well plate the following reagents are added:

Table 1:

| Reagents added to 96 well plate. | | | | | |
|---|---|---|---|---|---|
| | Compound/ DMSO | Tris Buffer | Substrate | ACE enzyme | Total product |
| Samples | 2µl compound | 50µl | 100µl | 50µl | None |
| Controls | 2µl DMSO | 50µl | 100µl | 50µl | None |
| Blanks | 2µl DMSO | 100µl | 100µl | None | None |
| Totals | 2µl DMSO | None | None | None | 200µl |

5.10 50µl of the highest concentration of each compound used in the assay is added in duplicate to the same 96 well plate as the totals (5.7). 150µl of ACE buffer is added to determine any compound fluorescence.
5.11 The reaction is initiated by the addition of the ACE enzyme before incubating at 37°C for 1 hour in a shaking incubator.
5.12 The reaction is stopped by the addition of 100µl 2mM EDTA and incubated at 37°C for 20 minutes in a shaking incubator, before being read on the BMG Fluostar Galaxy (ex320/em420).

## 6. CALCULATIONS

The activity of the ACE enzyme is determined in the presence and absence of compound and expressed as a percentage.
FU = Fluorescence units

(i) % Control activity (turnover of enzyme):

$$\frac{\text{Mean FU of controls - Mean FU of blanks}}{\text{Mean FU of totals - Mean FU of blanks}} \text{ X } 100$$

(ii) % Activity with inhibitor:

$$\frac{\text{Mean FU of compound - Mean FU of blanks}}{\text{Mean FU of totals - Mean FU of blanks}} \text{ X } 100$$

(iii) Activity expressed as % of control:

$$\frac{\text{\% Activity with inhibitor}}{\text{\% Control activity}} \text{ X } 100$$

$$\text{OR } \frac{\text{Mean FU of compound - Mean FU of blanks}}{\text{Mean FU of controls - Mean FU of blanks}} \text{ X } 100$$

(iv) % Inhibition = 100 - % control

(v) For fluorescent compounds the mean FU of blanks containing compound (5.10) is deducted from the mean FU of compound values used to calculate the % Activity.

A sigmoidal dose-response curve is fitted to the % activities (% of control) vs compound concentration and $IC_{50}$ values calculated using LabStats fit-curve in Excel.

## PDE5 inhibitor - TEST METHODS

### Phosphodiesterase (PDE) inhibitory activity

In vitro PDE inhibitory activities against cyclic guanosine 3',5'-monophosphate (cGMP) and cyclic adenosine 3',5'-monophosphate (cAMP) phosphodiesterases were determined by measurement of their $IC_{50}$ values (the concentration of compound required for 50% inhibition of enzyme activity).

The required PDE enzymes were isolated from a variety of sources, including human corpus cavernosum, human and rabbit platelets, human cardiac ventricle, human skeletal muscle and human and canine retina, essentially by the method of W.J. Thompson and M.M. Appleman (Biochem., 1971, 10, 311). In particular, the cGMP-specific PDE (PDE5) and the cGMP-inhibited cAMP PDE (PDE3) were obtained from human corpus cavernosum or human platelets; the cGMP-stimulated PDE (PDE2) was obtained from human corpus cavernosum and human platelets; the calcium/calmodulin (Ca/CAM)-dependent PDE (PDE1) from human cardiac ventricle; the cAMP-specific PDE (PDE4) from human skeletal muscle and human recombinant, expressed in SF9 cells; and the photoreceptor PDE (PDE6) from human or canine retina. Phosphodiesterases 7-11 were generated from full length human recombinant clones transfected into SF9 cells.

Assays can be performed either using a modification of the "batch" method of W.J. Thompson et al. (Biochem., 1979, 18, 5228) or using a scintillation proximity assay for the direct detection of AMP/GMP using a modification of the protocol described by Amersham plc under product code TRKQ7090/7100. In summary, the effect of PDE inhibitors was investigated by assaying a fixed amount of enzyme in the presence of varying inhibitor concentrations and low substrate, (cGMP or cAMP in a 3:1 ratio unlabelled to [$^3$H]-labeled at a conc ~1/3 $K_m$) such that $IC_{50} \cong K_i$. The final assay volume was made up to 100µl with assay buffer [20 mM Tris-HCl pH 7.4, 5 mM $MgCl_2$, 1 mg/ml bovine serum albumin]. Reactions were initiated with enzyme, incubated for 30-60 min at 30°C to give <30% substrate turnover and terminated with 50 µl yttrium silicate SPA beads (containing 3 mM of the respective unlabelled cyclic nucleotide for PDEs 9 and 11). Plates were re-sealed and shaken for 20 min, after which the beads were allowed to settle for 30 min in the dark and then counted on a TopCount plate reader (Packard, Meriden, CT) Radioactivity units were converted

to % activity of an uninhibited control (100%), plotted against inhibitor concentration and inhibitor $IC_{50}$ values obtained using the 'Fit Curve' Microsoft Excel extension (or in-house equivalent). Results from these tests show that the compounds of the present invention are inhibitors of cGMP-specific PDE5.

Functional activity

This can be assessed in vitro by determining the capacity of a compound of the invention to enhance sodium nitroprusside or electrical field stimulation-induced relaxation of pre-contracted rabbit corpus cavernosum tissue strips, using methods based on that described by S.A. Ballard et al. (Brit. J. Pharmacol., 1996, 118 (suppl.), abstract 153P) or S.A. Ballard et al. (J. Urology, 1998, 159, 2164-2171).

In vivo activity

Compounds can be screened in anaesthetised dogs to determine their capacity, after i.v. administration, to enhance the pressure rises in the corpora cavernosa of the penis induced by intracavemosal injection of sodium nitroprusside, using a method based on that described by Trigo-Rocha et al. (Neurourol. and Urodyn., 1994, 13, 71).

**NPY assay:**

An assay for identifying NPY inhibitors is presented in WO-A-98/52890 (see page 96, lines 2 to 28).

**Other References**

Anastasi A. et al., *Experientia*, 1971; 27: 166
S A Ballard *et al*, Journal of Urology, 1998, vol. 159, pages 2164-2171
Barker, P. E.; Shipp, M. A.; D'Adamio, L.; Masteller, E. L.; Reinherz, E. L. The common acute lymphoblastic leukemia antigen gene maps to chromosomal region 3(q21-q27). J. Immun. 142: 283-287, 1989.
Battcy J., et al., *TINS,* 1991;14:524
Baum M.J., *J. Biosci.,* 1983; **33**:578-582
Benet A.E and Melman A, 1995, *Urol. Clin. N. Amer.* 22: 699-709
Berman et al., 1999, hypoactive sexual desire disorders, sexual arousal disorders, anorgasmy and sexual pain disorders, *Urology* 54: 385-391.
Berman, J., Goldstein, I., Werbin, T. *et al*. (1999a). Double blind placebo controlled study with crossover to assess effect of sildenafil on physiological parameters of the female sexual response. *J. **Urol.,*** 161, 805.
Berman J.R. et al. *Int. J. Impot. Res.,* 1999, 11: S31-38
Bernabe J, Rampin O, Sachs BD, Giuliano F. Intracavernous pressure during erection in rats: an integrative approach based on telemetric recording. Am J Physiol. 1999 Feb;276(2 Pt 2):R441-9.
Bonney R.C et al., *Scrip's Complete Guide to Women's Healthcare*, PJB Publications Ltd, London, 2000
Braun M., et al., *Life. Sci.,* 1978; 23: 2721
Bundgaard et al., *Acta Pharm. Suec.*, 1987; 24: 233-246
D'Adamio, L.; Shipp, M. A.; Masteller, E. L.; Reinherz, E. L. : Organization of the gene encoding common acute lymphoblastic leukemia antigen (neutral endopeptidase 24.11): multiple miniexons and separate 5-prime untranslated regions. Proc. Nat. Acad. Sci. 86: 7103-7107, 1989.
Dutta A.S., *Small Peptides; Chemistry, Biology, and Clinical Studies,* Chapter 2, pp 66-82
J. Frohlich *et al., Heterocycles* 1994, 37, 1879-91
Goldstein, I. & Berman, J.R. (1998). Vasculogenic female sexual dysfunction: vaginal engorgement and clitoral erectile insufficiency syndromes. *Int. J. Impot. Res.,* 10, S84-S90.
Johnson M and Everitt B., *Essential Reproduction (3rd edn),* Blackwell, Oxford, 1988.
Ladenheim E.E et al, *Brain Res.,* 1990; 537: 233-240
Laumann et al., 1999 JAMA 281:537-544)
Letarte, M.; Vera, S.; Tran, R.; Addis, J. B. L.; Onizuka, R. J.; Quackenbush, E. J.; Jongeneel, C. V.; McInnes, R. R. : Common acute lymphocytic leukemia antigen is identical to neutral endopeptidase. J. Exp. Med. 168: 1247-1253, 1988.
Meyerson B.J, Lindstrom L.H., *Acta Physiol. Scand.,* 1973; 389 (Suppl.): 1-80.
O'Donohue W, et al, *Clin. Psychol. Rev.* 1997; 17: 537-566.
Park, K., Goldstein, I., Andry, C., *et al*. (1997). Vasculogenic female sexual dysfunction: The hemodynamic basis for vaginal engorgement insufficiency and clitoral erectile insufficiency. *Int. J. Impotence Res.,* 9, 27-37.
Sachs B. D., *Neuroscience and Biobehavioral Review,* 2000, 24 541-560.

Shipp, M. A.; Vijayaraghavan, J.; Schmidt, E. V.; Masteller, E. L.; D'Adamio, L.; Hersh, L. B.; Reinherz, E. L. : Common acute lymphoblastic leukemia antigen (CALLA) is active neutral endopeptidase 24.11 ('enkephalinase'): direct evidence by cDNA transfection analysis. Proc. Nat. Acad. Sci. 86: 297-301, 1989.
Tran-Paterson, R.; Willard, H. F.; Letarte, M. : The common acute lymphoblastic leukemia antigen (neutral endopeptidase--3.4.24.11) gene is located on human chromosome 3. Cancer Genet. Cytogenet. 42: 129-134, 1989.
Werbin, T., Salimpour, P., Berman, L., et al. (1999). Effect of sexual stimulation and age on genital blood flow in women with sexual stimulation. *J. Urol.,* 161, 688.
Wilson C.A., *Pharmacological targets for the control of male and female sexual behaviour,* In: *Sexual Pharmacology,* Riley A.J. et al, (Eds), Clarendon Press, Oxford, 1993: 1-58

## Claims

1. Use in the preparation of a medicament for the treatment or prophylaxis of drug induced sexual dysfunction in the male, male erectile dysfunction, drug induced sexual dysfunction in the female, female hypoactive sexual desire disorder, female sexual arousal disorder, female anorgasmy or female sexual pain disorders of a pharmaceutical combination (for simultaneous, separate or sequential administration) of a bombesin receptor antagonist and one or more materials selected from (1) to (32) below:

(1) naturally occurring or synthetic prostaglandins or esters thereof;
(2) α - adrenergic receptor antagonist compounds also known as α - adrenoceptor antagonists or α-receptor antagonists or α-blockers;
(3) NO-donor (NO-agonist) compounds;
(4) potassium channel openers or modulators;
(5) dopaminergic agents;
(6) nimodepine, pinacidil, cyclandelate, isoxsuprine, chloropromazine, halo peridol, Rec 15/2739, trazodone, alprostadil or phentolamine;
(7) thromboxane A2 agonists;
(8) ergot alkaloids;
(9) angiotensin receptor antagonists such as losartan;
(10) substrates for NO-synthase;
(11) calcium channel blockers;
(12) cholesterol lowering agents;
(13) antiplatelet and antithrombotic agents;
(14) insulin sensitising agents and hypoglycaemic agents;
(15) L-DOPA or carbidopa;
(16) acetylcholinesterase inhibitors;
(17) steroidal or non-steroidal anti-inflammatory agents;
(18) estrogen receptor modulators and/or estrogen agonists and/or estrogen antagonists, and pharmaceutically acceptable salts thereof;
(19) PDE inhibitors;
(20) NPY (neuropeptide Y) inhibitors;
(21) NEP inhibitors;
(22) vasoactive intestinal proteins (VIP), VIP mimetics, VIP analogues acting through one or more of the VIP receptor subtypes VPAC1, VPAC2 or PACAP (pituitory adenylate cyclase activating peptide), VIP analogue Ro-125-1553, VIP receptor agonists or VIP fragments, or α-adrenoceptor antagonists with VIP combinations;
(23) melanocortin receptor agonists or modulators or melanocortin enhancers;
(24) serotonin receptor agonists, antagonists or modulators;
(25) testosterone replacement agents, testosternone, dihydrotestosterone or a testosterone implant;
(26) estrogen, estrogen and medroxyprogesterone or medroxyprogesterone acetate (MPA) (i.e. as a combination), or estrogen and methyl testosterone hormone replacement therapy agents;
(27) monoamine metabolism or uptake modifiers that inhibit catecholamine metabolism or reuptake;
(28) purinergic receptor agonists and/or modulators;
(29) neurokinin (NK) receptor antagonists;
(30) opioid receptor agonists, antagonists or modulators;
(31) agonists or modulators for oxytocin/vasopressin receptors; and
(32) modulators of cannabinoid receptors.

**2.** Use according to claim 1, wherein the medicament is for treating antidepressant-induced sexual dysfunction in a male.

**3.** Use according to claim 1, wherein the medicament is for treating antidepressant-induced sexual dysfunction in a female.

**4.** Use according to claim 1, 2 or 3, wherein the bombesin receptor antagonist is a selective bombesin BB1 antagonist.

**5.** Use according to claim 4, wherein the bombesin BB1 antagonist has a selectivity for $BB_1$ over the other bombesin receptor subtypes greater than 10.

**6.** Use according to claim 4, wherein the bombesin BB1 antagonist has a selectivity for $BB_1$ over the other bombesin receptor subtypes greater than 30.

**7.** Use according to claim 4, wherein the bombesin BB1 antagonist has a selectivity for $BB_1$ over the other bombesin receptor subtypes greater than 100.

**8.** Use according to any of claims 4-7, wherein the bombesin receptor antagonist has a Ki against BB1 of less than 1000nM.

**9.** Use according to any of claims 4-7, wherein the bombesin receptor antagonist has a Ki against BB1 of less than 500nM.

**10.** Use according to any of claims 4-7, wherein the bombesin receptor antagonist has a Ki against BB1 of less than 100nM.

**11.** Use according to any of claims 4-7, wherein the bombesin receptor antagonist has a Ki against BB1 of less than 50nM.

**12.** Use according to any of claims 4-7, wherein the bombesin receptor antagonist has a Ki against BB1 of less than 10nM.

**13.** Use according to any of claims 1-3, wherein the bombesin receptor antagonist is a mixed BB1/BB2 antagonist.

**14.** Use according to any preceding claim, wherein the medicament is adapted for oral administration.

**15.** Use according to any preceding claim, wherein the medicament comprises an effective amount of a non-peptide bombesin receptor antagonist.

**16.** Use according to claim 15, wherein the non-peptide bombesin receptor antagonist is a compound that is absorbable when administered orally.

**17.** Use according to any of claims 1-14, wherein the medicament comprises an effective amount of a bombesin receptor antagonist which is a peptide.

**18.** Use according to any of claims 1-3, wherein the bombesin receptor antagonist is a compound of the formula (I)

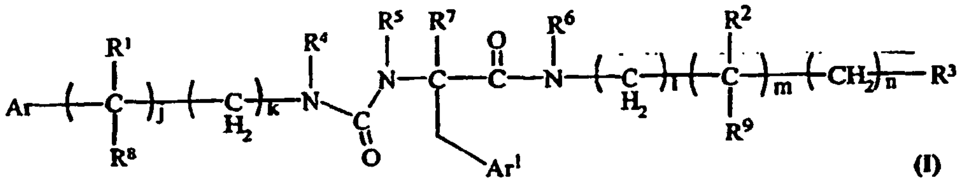

(I)

or a pharmaceutically acceptable salt thereof, wherein:

- j is 0 or 1;

- k is 0 or 1;
- l is 0, 1, 2, or 3;
- m is 0 or 1;
- n is 0, 1 or 2;
- Ar is phenyl, pyridyl or pyrimidyl, each unsubstituted or substituted by from 1 to 3 substituents selected from alkyl, halogen, alkoxy, acetyl, nitro, amino, $-CH_2NR^{10}R^{11}$, cyano, $-CF_3$, $-NHCONH_2$, and $-CO_2R^{12}$;
- $R^1$ is hydrogen or straight, branched, or cyclic alkyl of from 1 to 7 carbon atoms;
- $R^8$ is hydrogen or forms a ring with $R^1$ of from 3 to 7 carbon atoms;
- $R^2$ is hydrogen or straight, branched, or cyclic alkyl of from 1 to 8 carbon atoms which can also contain 1 to 2 oxygen or nitrogen atoms;
- $R^9$ is hydrogen or forms with $R^2$ a ring of from 3 to 7 carbon atoms which can contain an oxygen or nitrogen atom; or $R^2$ and $R^9$ can together be a carbonyl;
- $Ar^1$ can be independently selected from Ar and can also include pyridyl-N-oxide, indolyl, imidazolyl, and pyridyl;
- $R^4$, $R^5$, $R^6$, and $R^7$ are each independently selected from hydrogen and lower alkyl; $R^4$ can also form with $R^5$ a covalent link of 2 to 3 atoms which may include an oxygen or a nitrogen atom;
- $R^3$ can be independently selected from Ar or is hydrogen, hydroxy, - $NMe_2$, N-methyl-pyrroiyl, imidazolyl, N-methyl-imidazolyl, tetrazolyl, N-methyl-tetrazolyl, thiazolyl, $-CONR^{13}R^{14}$, alkoxy,

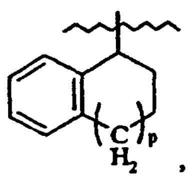 , 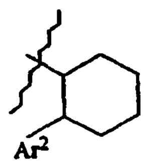 , 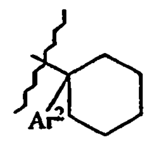 ,

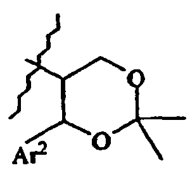 , or 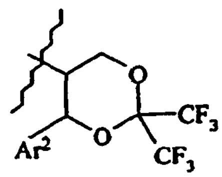 ,

wherein p is 0, 1 or 2 and $Ar^2$ is phenyl or pyridyl;

- $R^{10}$, $R^{11}$, $R^{12}$, $R^{13}$ and $R^{14}$ are each independently selected from hydrogen or straight, branched, or cyclic alkyl of from 1 to 7 carbon atoms.

**19.** Use according to any of claims 1-3, wherein the bombesin receptor antagonist is a compound of Formula (Ia)

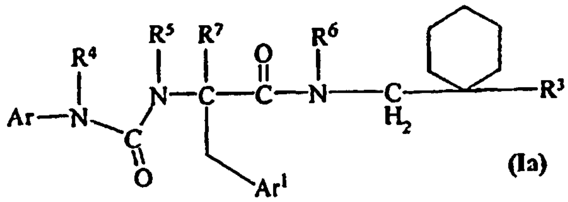

(Ia)

wherein

- Ar is phenyl unsubstituted or substituted with 1 or 2 substituents selected from isopropyl, halo, nitro, and cyano;
- $R^4$, $R^5$, and $R^6$ are hydrogen;
- $R^7$ is methyl or hydrogen;
- $R^3$ is 2-pyridyl or hydroxy; and
- $Ar^1$ is indolyl, pyridyl, pyridyl-N-oxide, or imidazolyl.

**20.** Use according to claim 18, wherein the bombesin receptor antagonist is a compound of Formula I wherein

- Ar is unsubstituted phenyl;
- $R^1$ is cyclopentyl or *tert*-butyl;
- $R^4$ and $R^5$ are hydrogen;
- $R^7$ is methyl;
- $R^6$ is hydrogen;
- $R^3$ is phenyl with two isopropyl substituents, unsubstituted phenyl, or

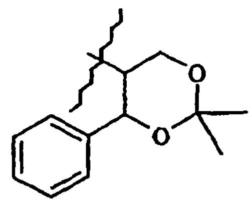
;

and
$Ar^1$ is indolyl.

**21.** Use according to claim 18, wherein the bombesin receptor antagonist is a compound of Formula I wherein

- Ar is 2,6-diisopropyl-phenyl, 4-nitro-phenyl, and 4-cyano-phenyl;
- $R^4$, $R^5$, and $R^6$ are hydrogen;
- $R^7$ is methyl;
- $R^2$ is hydrogen or cyclohexyl; and
- $R^3$ is hydroxyl, pyridyl,

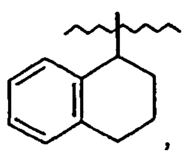
,
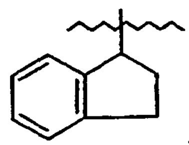
, or
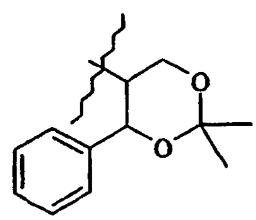
.

**22.** Use according to any of claims 1-3, wherein the bombesin receptor antagonist is (S)3-(1H-Indol-3-yl)-N-[1-(5-methoxy-pyridin-2-yl)-cyclohexylmethyl]-2-methyl-2-[3-(4-nitro-phenyl)-ureido]-propionamide (also referred to as Compound 1) or one of its pharmaceutically acceptable salts or is

(2*S*)-*N*-{[1-(4-aminophenyl) cyclohexyl] methyl}-3-(1*H*-indol-3-yl)-2-methyl-2-{[(4-nitroanilino) carbonyl] amino} propanamide (also knowm as Compound 3) or one of its pharmaceutically acceptable salts.

**23.** Use according to any of claims 1-3, wherein the bombesin receptor antagonist is a compound set out below or a pharmaceutically acceptable salt thereof:

(S) *N*-cyclohexylmethyl-2-[3-(2,6-diisopropyl-phenyl)-ureido]-3-(1*H*-indol-3-yl)-2-methyl-propionamide;
*N*-cyclohexylmethyl-2-[3-(2,6-diisopropyl-phenyl)-ureido]-3-(1*H*-indol-3-yl)-*N*-methyl-propionamide;
*N*-cyclohexylmethyl-2-[3-(2,6-diisopropyl-phenyl)-1-methyl-ureido]-3-(1*H*-indol-3-yl)-propionamide;
2-[3-(2,6-diisopropyl-phenyl)-ureido]-2-methyl-3-(1-oxy-pyridin-2-yl)-*N*-(1-pyridin-2-yl-cyclohexylmethyl)-propionamide;
2-[3-(2,6-diisopropyl-phenyl)-ureido]-2-methyl-3-pyridin-2-yl-*N*-(1-pyridin-2-yl-cyclohexylmethyl)-propionamide;
2-[3-(2-*tert*-butyl-phenyl)-ureido]-*N*-cyclohexylmethyl-3-(1*H*-indol-3-yl)-2-methyl-propionamide;
*N*-cyclohexylmethyl-2-[3-(2,6-dichloro-phenyl)ureido]-3-(1*H*-indol-3-yl)-2-methyl-propionamide;

*N*-cyclohexylmethyl-2-[3-(2,6-dimethoxy-phenyl)ureido]-3-(1*H*-indol-3-yl)-2-methyl-propionamide;
*N*-cyclohexylmethyl-2-[3-(2,6-dimethylamino-phenyl)-ureido]-3-(1*H*indol-3-yl)-2-methyl-propionamide;
(S) *N*-cyclohexylmethyl-3-(1*H*-indol-3-yl)-2-methyl-2-[3-(4-nitro-phenyl)-ureido]-propionannide;
*N*-cyclohexylmethyl-2-[3-(2,2-dimethyl- 1 -phenyl)propyl)-ureido]-3-(1*H*-indol-3-yl)-2-methyl-propionamide;
[S-(R*, R*)] 3-(1*H*-indol-3-yl)-2-methyl-2-{3-[1-(4-nitro-phenyl)-ethyl]-ureido}-*N*-(1-pyridin-2-yl-cyclohexylmethyl)-propionamide;
*N*-(2,2-dimethyl-4-phenyl-[1,3]dioxan-5-yl)-3-(1*H*-indol-3-yl)-2-methyl-2-[3-(1-phenyl-cyclopentylmethyl)ureido]-propionamide;
(S)-*N*-(2,6-diisopropyl-phenyl)-2-[3-(2,2-dimethyl-1-phenyl-propyl)-ureido]-3-(1*H*-indol-3-yl)-propionamide;
(R)-*N*-(2,6-diisopropyl-phenyl)-2-[3-(2,2-dimethyl-1-phenyl-propyl)-ureido]-3-(1*H*-indol-3-yl)-propionainide;
2-[3-(2,6-diisopmpyl-phenyl)-ureido]-*N*-(2,2-dimethyl-4-phenyl-[1,3]dioxan-5-yl)-3-(1*H*-indol-3-yl)-2-methyl-propionamide;
*N*-cyclohexyl-2-[3-(2,6-diisopropyl-phenyl)-ureido]-3-(1*H*-indol-3-yl)-2-methyl-propionamide;
*N*-(2-cyclohexyl-ethyl)-2-[3-(2,6-diisopropyl-phenyl)-ureido]-3-(1*H*-indol-3-yl)-2-methyl-propionamide;
2-[3-(2,6-diisopropyl-phenyl)-ureido]-3-(1*H*-indol-3-yl)-2-methyl-propionamide;
2-[3-(2,6-diisopropyl-phenyl)-ureido]-3-(1*H*-indol-3-yl)-2-methyl-*N*-(3-methyl-butyl)-propionamide;
2-[3-(2,6-diisopropyl-phenyl)-ureido]-3-(1*H*-indol-3-yl)-2-methyl-*N*-(3-phenyl-propyl)-propionamide;
2-[3-(2,6-diisopropyl-phenyl)-ureido]-3-(1*H*-indol-3-yl)-2-methyl-*N*-(1,2,3,4-tetrahydro-naphthalen-1-yl)-propionamide;
2-[3-(2,6-diisopropyl-phenyl)-ureido]-3-(1*H*-indol-3-yl)-2-methyl-*N*-(2-phenyl-cyclohexyl)-propionamide;
2-[3-(2,6-diisopropyl-phenyl)-ureido]-*N*-indan-1-yl-3-(1*H*-indol-3-yl)-2-methyl-propionamide;
2-[3-(2,6-diisopropyl-phenyl)-ureido]-*N*-(1-hydroxy-cyclohexylmethyl)-3-(1*H*-indol-3-yl)-2-methyl-propionamide;
2-[3-(2,6-diisopropyl-phenyl)-ureido]-3-(1*H*-indol-3-yl)-2-methyl-*N*-(1-pyridin-2-yl-cyclohexylmethyl)-propionamide;
2-[3-(2,6-diisopropyl-phenyl)-ureido]-3-(1*H*-indol-3-yl)-2-methyl-*N*-(6,7,8,9-tetrahydro-5*H*-benzocyclohepten-5-yl)-propionamide;
2-[3-(2,6-diisopropyl-phenyl)-ureido]-3-(1*H*-indol-3-yl)-2-methyl-*N*-phenyl-propionamide;
*N*-(1-hydroxy-cyclohexylmethyl)-3-(1*H*-indol-3-yl)-2-methyl-2-[3-(4-nitro-phenyl)-ureido]-propionamide;
2-[3-(4-cyano-phenyl)-ureido]-3-(1*H*-indol-3-yl)-2-methyl-*N*-(1-pyridin-2-yl-cyclohexylmethyl)-propionamide;
(S) 3-(1*H*-indol-3-yl)-2-methyl-2-[3-(4-nitro-phenyl)-ureido]-*N*-(1-pyridin-2-yl-cyclohexylmethyl)-propionamide;
(S) 3-(1*H*-indol-3-yl)-2-methyl-*N*-(1-pyridin-2-yl-cyclohexylmethyl)-2-[3-(4-trifluoromethyl-phenyl)-ureido]-propionamide;
(S) 4-(3-{2-(1*H*-indol-3-yl)-1-methyl-1-[(1-pyridin-2-yl-cyclohexylmethyl)-carbamoyl]-ethyl}-ureido)-benzoic acid ethyl ester;
2-[3-(2,6-diisopropyl-phenyl)-ureido]-3-(1*H*-imidazol-4-yl)-*N*-(1-pyridin-2-yl-cyclohexylmethyl)-propionamide;
2-[3-(2,6-diisopropyl-phenyl)-ureido]-2-methyl-*N*-(1-pyridin-2-yl-cyclohexylmethyl)-3-(2-trifluoromethyl-phenyl)-propionamide;
2-[3-(2,6-diisopropyl-phenyl)-ureido]-2-methyl-3-(2-nitro-phenyl)-*N*-(1-pyridin-2-yl-cyclohexylmethyl)-propionamide;
(S) 3-(1*H*-indol-3-yl)-*N*-[1-(5-methoxy-pyridin-2-yl)-cyclohexylmethyl]-2-methyl-2-[3-(4-nitro-phenyl)-ureido]-propionamide; and
*N*-cyclohexylmethyl-2-[3-(2,6-diisopropyl-phenyl)-ureido]-2-methyl-3-pyridin-2-yl-propionamide.

**24.** The use of any of claims 1-3, wherein the bombesin receptor antagonist is a compound of formula (II) or a pharmaceutically acceptable salt thereof:

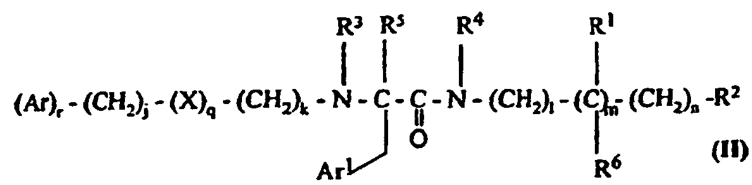

(II)

wherein:

- j is 0, 1 or 2;
- k is 0 or 1;
- l is 0, 1, 2, or 3;
- m is 0 or 1;
- n is 0, 1 or 2;
- q is 0 or 1;
- r is 0 or 1; when r is 0, Ar is replaced by hydrogen;
- Ar is phenyl, pyridyl, pyrimidyl, thienyl, furyl, imidazolyl, pyrrolyl or thiazolyl each unsubstituted or substituted by from 1 to 3 substituents selected from acetyl, alkoxy, alkyl, amino, cyano, halo, hydroxy, nitro, sulfonamido, sulfonyl, $-CF_3$, $-OCF_3$, $-CO_2H$, $-CH_2CN$, $-SO_2CF_3$, - $CH_2CO_2H$ and $-(CH_2)_SNR^7R^8$ wherein s is 0, 1, 2 or 3 and $R^7$ and $R^8$ are each independently selected from H, straight or branched alkyl of up to 6 carbon atoms, or $R^7$ and $R^8$ together with the nitrogen atom to which they are linked can form a 5- to 7-membered aliphatic ring which may contain 1 or 2 oxygen atoms;
- $R^1$ is hydrogen, straight or branched alkyl of up to 6 carbon atoms or cycloalkyl of between 5 and 7 carbon atoms which may contain 1 or 2 nitrogen or oxygen atoms;
- $R^6$ is hydrogen, methyl, or forms with $R^1$ an aliphatic ring of from 3 to 7 atoms which can contain an oxygen or nitrogen atom, or together with $R^1$ is a carbonyl group;
- $Ar^1$ is independently selected from Ar or is indolyl or pyridyl-*N*-oxide;
- $R^3$, $R^4$, and $R^5$ are each independently selected from hydrogen and lower alkyl;
- $R^2$ is independently selected from Ar or is hydrogen, hydroxy, alkoxy, $-NMe_2$, $-CONR^9R^{10}$ wherein $R^9$ and $R^{10}$ are each independently selected from hydrogen, straight or branched alkyl of up to 6 carbon atoms, or $R^9$ and $R^{10}$ together with the nitrogen atom to which they are linked can form a 5-to 7-membered aliphatic ring which may contain 1 or 2 oxygen or nitrogen atoms, or $R^2$ is

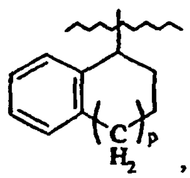 , 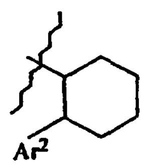 , 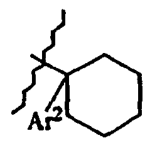 ,

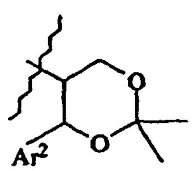 , or 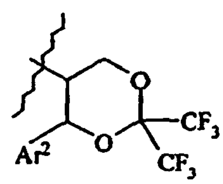

wherein p is 0, 1 or 2 and $Ar^2$ is phenyl or pyridyl;

- X is a divalent radical derived from any of the following

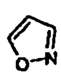 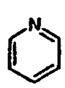 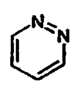 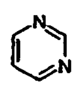 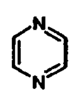 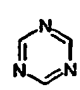

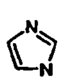 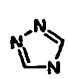 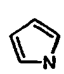 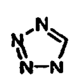 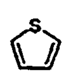 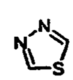

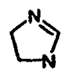
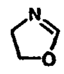
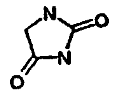
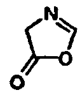
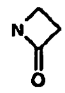
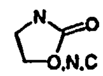

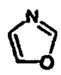
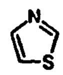
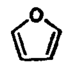
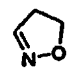
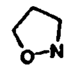
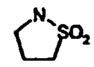

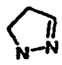
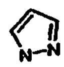
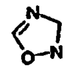
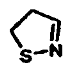
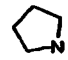
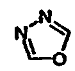

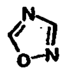
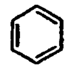
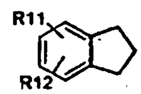
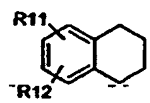
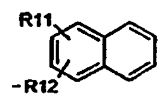

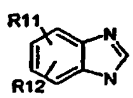
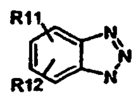
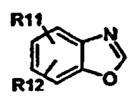
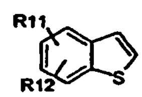
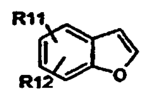

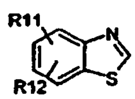
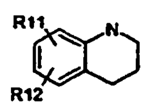
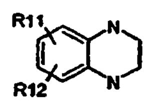
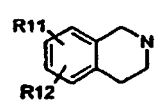

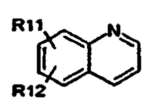
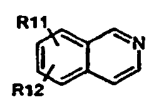

where the ring nitrogen atoms may have lower alkyl groups attached thereto, $R^{11}$ and $R^{12}$ are independently selected from H, halogen, hydroxy, alkoxy, acetyl, nitro, cyano, amino, $CF_3$ and $-(CH_2)_tNR^{13}R^{14}$ where t can be 0 or 1, $R^{13}$ and $R^{14}$ are each independently selected from hydrogen, straight or branched alkyl of up to 6 carbon atoms or cycloalkyl of 5 to 7 carbon atoms, containing up to 2 oxygen or nitrogen atoms.

**25.** The use of any of claims 1-3, wherein the bombesin receptor antagonist is a compound of the formula (IIa), or a pharmaceutically acceptable salt thereof:

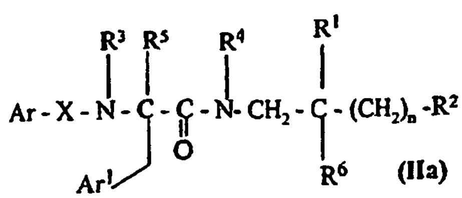

(IIa)

wherein:

- n is 0 or 1;

- Ar is phenyl or pyridyl which may be unsubstituted or substituted with from 1 to 3 substituents selected from halogen, alkoxy, nitro and cyano;
- $Ar^1$ is independently selected from Ar or is pyridyl-*N*-oxide or indolyl;
- $R^6$ forms with $R^1$ an aliphatic ring of from 3 to 7 atoms which can contain an oxygen or nitrogen atom, or together with $R^1$ is a carbonyl group;
- $R^2$ is independently selected from Ar or is hydrogen, hydroxy, alkoxy, dimethylamino, tetrazolyl or $-CONR^9R^{10}$ wherein $R^9$ and $R^{10}$ are each independently selected from hydrogen or methyl or $R^2$ is any of

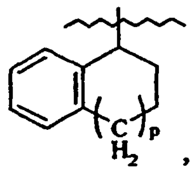 , 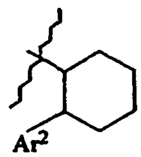 , 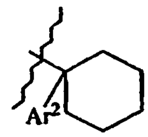 ,

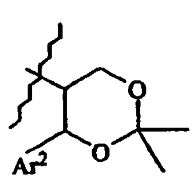 , or 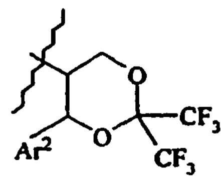

wherein p is 0, 1 or 2 and $Ar^2$ is phenyl or pyridyl;
- $R^3$, $R^4$ and $R^5$ are each independently selected from hydrogen and methyl; and
- X is selected from:

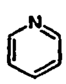 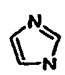 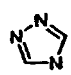 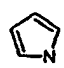 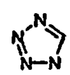 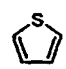

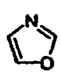 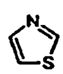 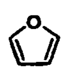 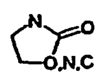 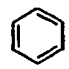 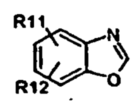

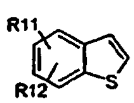 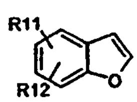 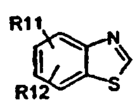 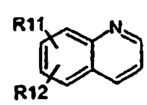 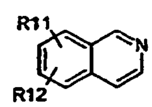

$R^{11}$ and $R^{12}$ being independently selected from H, halogen, hydroxy, allcoxy, acetyl, nitro, cyano, amino, $CF_3$ and $(CH_2)_tNR^{13}R^{14}$ wherein t is 0 or 1 and $R^{13}$ and $R^{14}$ are independently selected from hydrogen and methyl.

**26.** The use of any of claims 1-3, wherein the bombesin receptor antagonist is a compound has the formula (IIb) or (IIc) or is a pharmaceutically acceptable salt thereof:

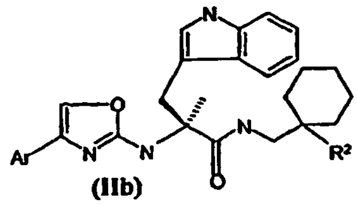 (IIb) 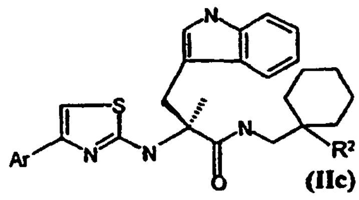 (IIc)

wherein Ar and $R^2$ independently represent phenyl or pyridyl which may be unsubstituted or substituted with from 1 to 3 substituents selected from halogen, alkoxy, nitro and cyano, and pharmaceutically acceptable salts thereof.

**27.** The use of any of claims 1-3, wherein the bombesin receptor antagonist is (S)-3-(1*H*-indol-3-yl)-*N*-[1-(5-methoxy-pyridin-2-yl)-cyclohexylmethyl]-2-methyl-2-[4-(4-nitro-phenyl)-oxazol-2-ylamino]-propionamide (also referred to as Compound 2) or a pharmaceutically acceptable salt.

**28.** The use of any of claims 1-3, wherein the bombesin receptor antagonist is one of the following compounds or a pharmaceutically acceptable salt thereof:

(S)-3-(1*H*-indol-3-yl)-*N*-(1-methoxymethyl-cyclohexylmethyl)-2-methyl-2-[4-(4-nitro-phenyl)-oxazol-2-ylamino]-propionamide;
(S)-3-(1*H*-indol-3-yl)-2-methyl-2-[4-(4-nitro-phenyl)-oxazol-2-ylamino]-*N*-(2-oxo-2-phenyl-ethyl)-propionamide;
(S)-*N*-[1-(5-methoxy-pyridin-2-yl)-cyclohexylmethyl]-2-methyl-2-[4-(4-nitro-phenyl)-oxazol-2-ylamino]-3-phenyl-propionamide;
(S)-2-[4-(4-cyano-phenyl)-oxazol-2-ylamino]-3-(1*H*-indol-3-yl)-*N*-[1-(5-methoxy-pyridin-2-yl)-cyclohexylmethyl]-2-methyl-propionamide;
(S)-3-(1*H*-indol-3-yl)-*N*-[1-(5-methoxy-pyridin-2-yl)-cyclohexylmethyl]-2-methyl-2-(4-phenyl-oxazol-2-ylamino)-propionamide;
(S)-2-(4-ethyl-oxazol-2-ylamino)-3-(1*H*-indol-3-yl)-*N*-[1-(5-methoxy-pyridin-2-yl)-cyclohexylmethyl]-2-methyl-propionamide;
(S)-3-(1*H*-indol-3-yl)-*N*-[1-(5-methoxy-pyridin-2-yl)-cyclohexylmethyl]-2-methyl-2-[4-(4-nitro-phenyl)-thiazol-2-ylamino]-propionamide;
(S)-2-(benzooxazol-2-ylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(pyridin-4-ylamino)-*N*-(1-pyridin-2-yl-cyclohexylmethyl)-propionamide;
(S)-3-(1*H*-indol-3-yl)-2-(isoquinol-4-ylamino)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(pyrimidin-5-ylamino)-propionamide;
(S)-2-(biphenyl-2-ylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-*m*-tolylamino-propionamide;
(S)-3-(1*H*-indol-3-yl)-2-methyl-2-(6-phenyl-pyridin-2-ylamino)-*N*-(1-pyridin-2-yl-cyclohexylmethyl)-propionamide;
(R)-3-phenyl-2-phenylamino-*N*-(1-pyridin-2-yl-cyclohexylmethyl)-propionamide;
(S)-3-(1*H*-indol-3-yl)-2-methyl-2-phenylethylamino-*N*-(1-pyridin-2-yl-cyclohexylmethyl)-propionamide;
(S)-2-[(benzofuran-2-ylmethyl)-amino]-3-(1*H*-indol-3-yl)-2-methyl-*N*-(1-pyridin-2-yl-cyclohexylmethyl)-propionamide, and
(S)-3-(1*H*-indol-3-yl)-2-methyl-2-(4-nitro-benzylamino)-*N*-(1-pyridin-2-yl-cyclohexylmethyl)-propionamide.

**29.** The use of any of claims 1-3, wherein the bombesin receptor antagonist is a compound of formula (III) or a pharmaceutically acceptable salt thereof:

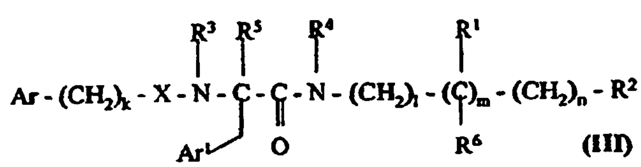 (III)

wherein:

- k is 0, 1 or 2;
- l is 0, 1, 2 or 3;
- m is 0 or 1;
- n is 0, 1 or 2;
- X is -CO-, -OCO, -SO- and $-SO_2-$;
- Ar is benzimidazolyl, benzofuryl, benzothiadiazolyl, benzothiazolyl, benzothienyl, benzopyrazinyl, benzotriazolyl, benzoxadiazolyl, furyl, imidazolyl, indanyl, indolyl, isoquinolyl, isoxazolyl, naphthyl, oxazolyl, phenyl, pyrazinyl, pyrazolyl, pyridyl, pyridazinyl, pyrimidyl, pyrrolyl, quinolinyl, tetralinyl, tetrazolyl, thiazolyl, thienyl or triazolyl each unsubstituted or substituted with from 1 to 3 substituents selected from amino, acetyl, alkyl (straight chain or branched with from 1 to 6 carbon atoms), alkoxy, cyano, halogen, hydroxy, nitro, phenyl, pyridyl, pyrrolyl, isoxazolyl, phenoxy, tolyloxy, $-CF_3$, $-OCF_3$, $-SO_2CF_3$, $-NHCONH_2$, $-CO_2H$, $-CH_2CO_2H$, $-CH_2CN$, $SO_2Me$, $SO_2NH_2$, $SO_2Ph$, $-(CH_2)_qNR^7R^8$, $-CONR^9R^{10}$, and $CO_2R^{11}$, wherein q is 0, 1 or 2 and $R^7$, $R^8$, $R^9$, $R^{10}$, $R^{11}$ are each independently selected from hydrogen or straight or branched alkyl of up to 6 carbon atoms or cyclic alkyl of between 5 to 7 atoms which may contain 1 or 2 oxygen or nitrogen atoms or $R^7$ and $R^8$ or $R^9$ and $R^{10}$ together with the nitrogen atom to which they are linked can form a 5- to 7-membered aliphatic ring which may contain 1 or 2 oxygen or nitrogen atoms;
- $Ar^1$ is independently selected from Ar and can also be pyridyl-*N*-oxide;
- $R^1$ is hydrogen or straight or branched alkyl of up to 6 carbon atoms or cyclic alkyl of between 5 and 7 atoms which may contain I or 2 oxygen or nitrogen atoms;
- $R^2$ is independently selected from Ar or is hydrogen, hydroxy, alkoxy, $-NMe_2$, $-CONR^{12}R^{13}$,

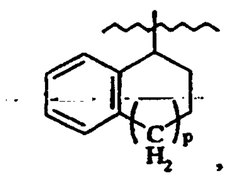 , 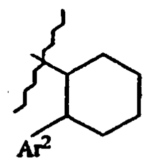 , 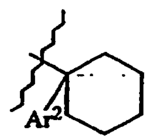 ,

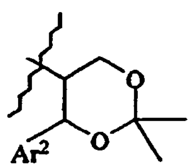 , or 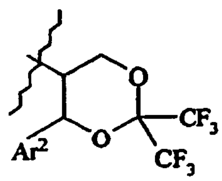 ,

  wherein p is 0, 1 or 2, $Ar^2$ is phenyl or pyridyl; and, $R^{12}$ and $R^{13}$ are each independently selected from hydrogen, straight or branched alkyl of up to 6 carbon atoms or cyclic alkyl of between 5 and 7 carbon atoms;
- $R^3$, $R^4$ and $R^5$ are each independently selected from hydrogen and lower alkyl; and
- $R^6$ is hydrogen, methyl or forms with $R^1$ a ring of from 3 to 7 carbon atoms which can contain an oxygen or nitrogen atom, or $R^1$ and $R^6$ can together be carbonyl.

**30.** The use of any of claims 1-3, wherein the bombesin receptor antagonist is a compound formula (III) in which:

- k is 0 or 1;
- l is 1;
- m is 0 or 1;
- n is 0 or 1;
- X is -C(O)-, -OC(O)-, or $-SO_2-$;
- Ar is benzofuryl, furyl, indolyl, isoquinolyl, naphthyl, phenyl, pyridyl, quinolyl or thienyl each unsubstituted or substituted with 1 or 2 substituents selected from alkoxy, cyano, halogen, nitro, phenyl, phenoxy, $-CF_3$, $-(CH_2)_qNR^7R^8$, wherein $R^7$ and $R^8$ can form a ring of between 5 to 7 atoms which may contain 1 or 2 oxygen or nitrogen atoms, or $R^7$ and $R^8$ can be independently selected from hydrogen, straight or branched alkyl of up to 4 carbon atoms or cyclic alkyl of 5 carbon atoms;
- $Ar^1$ is independently selected from Ar, preferably indolyl, and can also be pyridyl-*N*-oxide;

- $R^1$ and $R^6$ can form a cyclic alkyl of from 5 to 7 carbon atoms or $R^1$ and $R^6$ together are carbonyl;
- $R^2$ is independently selected from unsubstituted or substituted pyridyl or is hydrogen, hydroxy, alkoxy, $-NMe_2$, $-CONR^{12}R^{13}$ wherein $R^{12}$ and $R^{13}$ are each independently selected from H and $CH_3$;
- $R^3$, $R^4$ and $R^5$ are each independently selected from hydrogen and methyl.

**31.** The use of any of claims 1-3, wherein the bombesin receptor antagonist is a compound of Formula (III) in which,

- l is 1;
- m is 1;
- n is 0;
- $R^2$ is 2-pyridyl;
- $R^6$ forms a cyclohexyl with $R^1$.

**32.** The use of any of claims 1-3, wherein the bombesin receptor antagonist is a compound of formula (IIIa) or a salt thereof:

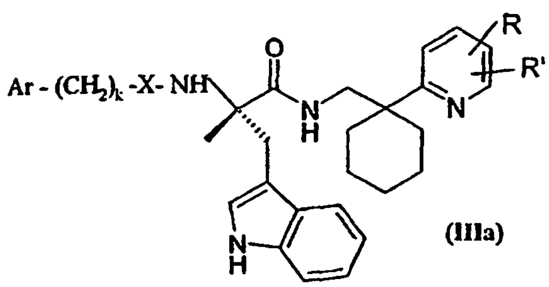

(IIIa)

wherein Ar, k and X have the meanings given above in first, and the pyridine ring is optionally substituted by with 1 or 2 substituents, R and R', independently selected from alkoxy, cyano, halogen, nitro, phenyl, phenoxy, $-CF_3$, $-(CH_2)_qNR^7R^8$, wherein $R^7$ and $R^8$ together with the nitrogen atom to which they are linked can form a 5- to 7-membered aliphatic ring which may contain 1 or 2 oxygen or nitrogen atoms, or $R^7$ and $R^8$ can be independently selected from hydrogen or cyclic alkyl of between 5 to 7 carbon atoms, and their pharmaceutically acceptable salts thereof.

**33.** The use of any of claims 1-3, wherein the bombesin receptor antagonist is one of the following compounds or a salt thereof:

*N*-{(S)-2-(1*H*-indol-3-yl)-1-methyl-1-[(1-pyridin-2-yl-cyclohexylmethyl)-carbamoyl]-ethyl}-4-nitro-benzamide;
*C*-dimethylamino-*N*-{(S)-2-(1*H*-indol-3-yl)-1-methyl-1-[(1-pyridin-2-yl-cyclohexylmethyl)-carbamoyl]-ethyl}-benzamide;
1*H*-indole-2-carboxylic acid {(S)-2-(1*H*-indol-3-yl)-1-methyl-1-[(1-pyridin-2-yl-cyclohexylmethyl)-carbamoyl]-ethyl}-amide;
benzo[b]thiophene-2-carboxylic acid {(S)-2-(1*H*-indol-3-yl)-1-methyl-1-[(1-pyridin-2-yl-cyclohexylmethyl)-carbamoyl]-ethyl}-amide;
N-{(S)-2-(1H-Indol-3-yl)-1-methyl-1-[(1-pyridin-2-yl-cyclohexylmethyl)-carbamoyl]-ethyl}-2-pyrrol-1-yl-benzamide
1*H*-indole-5-carboxylic acid {(S)-2-(1*H*-indol-3-yl)-1-methyl-1-[(1-pyridin-2-yl-cyclohexylmethyl)-carbamoyl]-ethyl}-amide; and
1*H*-indole-2-carboxylic acid ((S)-2-(1*H*-indol-3-yl)-1-{[1-(5-methoxy-pyridin-2-yl)-cyclohexylmethyl]-carbamoyl}-1-methyl-ethyl)-amide.

**34.** The use of any of claims 1-3, wherein the bombesin receptor antagonist is one of the following compounds or a salt thereof

*N*-{(S)-2-(1*H*-indol-3-yl)-1-methyl-1-[(1-pyridin-2-yl-cyclohexylmethyl)-carbamoyl]-ethyl}-benzamide;
*N*-{(S)-2-(1*H*-indol-3-yl)-1-methyl-1-[(1-pyridin-2-yl-cyclohexylmethyl)-carbamoyl]-ethyl}-4-methyl-benzamide;

4-chloro-*N*-{(S)-2-(1*H*-indol-3-yl)-1-methyl-1-[(1-pyridin-2-yl-cyclohexylmethyl)-carbamoyl]-ethyl}-benzamide;
*N*-{(S)-2-(1*H*-indol-3-yl)-1-methyl-1-[(1-pyridin-2-yl-cyclohexylmethyl)-carbamoyl]-ethyl}-4-methoxy-benzamide;
*N*-{(S)-2-(1*H*-indol-3-yl)-1-methyl-1-[(1-pyridin-2-yl-cyclohexylmethyl)-carbamoyl]-ethyl}-4-methanesulfonyl-benzamide;
3-cyano-*N*-{(S)-2-(1*H*-indol-3-yl)-1-methyl-1-[(1-pyridin-2-yl-cyclohexylmethyl)-carbamoyl]-ethyl}-benzamide;
3-chloro-*N*-{(S)-2-(1*H*-indol-3-yl)-1-methyl-1-[(1-pyridin-2-yl-cyclohexylmethyl)-carbamoyl]-ethyl}-benzamide;
*N*-{(S)-2-(1*H*-indol-3-yl)-1-methyl-1-[(1-pyridin-2-yl-cyclohexylmethyl)-carbamoyl]-ethyl}-3-methoxy-benzamide;
*N*-{(S)-2-(1*H*-indol-3-yl)-1-methyl-1-[(1-pyridin-2-yl-cyclohexylmethyl)-carbamoyl]-ethyl}-3-methanesulfonyl-benzamide;
dimethylamino-*N*-{(S)-2-(1*H*-indol-3-yl)-1-methyl-1-[(1-pyridin-2-yl-cyclobexylmethyl)-carbamoyl]-ethyl}-benzamide;
*N*-{(S)-2-(1*H*-indol-3-yl)-1-methyl-1-[(1-pyridin-2-yl-cyclohexylmethyl)-carbamoyl]-ethyl}-3-methyl-benzamide;
2-chloro-*N*-{(S)-2-(1*H*-indol-3-yl)-1-methyl-1-[(1-pyridin-2-yl-cyclohexylmethyl)-carbamoyl]-ethyl}-benzamide;
*N*-{(S)-2-(1*H*-indol-3-yl)-1-methyl-1-[(1-pyridin-2-yl-cyclohexylmethyl)-carbamoyl]-ethyl}-2-nitro-benzamide;
*N*-{(S)-2-(1*H*-indol-3-yl)-1-methyl-1-[(1-pyridin-2-yl-cyclohexylmethyl)-carbamoyl]-ethyl}-2-methoxy-benzamide;
*N*-{(S)-2-(1*H*-indol-3-yl)-1-methyl-1-[(1-pyridin-2-yl-cyclohexylmethyl)-carbamoyl]-ethyl}-2-methyl-benzamide;
2-fluoro-*N*-{(S)-2-(1*H*-indol-3-yl)-1-methyl-1-[(1-pyridin-2-yl-cyclohexylmethyl)-carbamoyl]-ethyl}-benzamide;
(S)-3-(1*H*-indol-3-yl)-2-methyl-*N*-(1-pyridin-2-yl-cyclohexylmethyl)-2-(2-p-tolyl-ethanoylamino)-propionamide;
(S)-3-(1*H*-indol-3-yl)-2-methyl-*N*-(1-pyridin-2-yl-cyclohexylmethyl)-2-(2-o-tolyl-ethanoylamino)-propionamide;
(S)-2-[2-(4-hydroxy-phenyl)-ethanoylamino]-3-(1*H*-indol-3-yl)-2-methyl-*N*-(1-pyridin-2-yl-cyclohexylmethyl)-propionamide;
(S)-2-[2-(3-hydroxy-phenyl)-ethanoylamino]-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(2-m-tolyl-ethanoylamino)-propionamide;
(S)-2-[2-(2-fluoro-phenyl)-ethanoylamino]-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(2-thiophen-3-yl-ethanoylamino)-propionamide;
pyridine-2-carboxylic acid {(S)-2-(1H-indol-3-yl)-1-methyl-1-[(1-pyridin-2-yl-cyclohexylmethyl)-carbamoyl]-ethyl}-amide
*N*-{(S)-2-(1*H*-indol-3-yl)-1-methyl-1-[(1-pyridin-2-yl-cyclohexylmethyl)-carbamoyl]-ethyl}-isonicotinamide;
furan-3-carboxylic acid {(S)-2-(1*H*-indol-3-yl)-1-methyl-1-[(1-pyridin-2-yl-cyclohexylmethyl)-carbamoyl]-ethyl}-amide;
furan-2-carboxylic acid {(S)-2-(1*H*-indol-3-yl)-1-methyl-1-[(1-pyridin-2-yl-cyclohexylmethyl)-carbamoyl]-ethyl}-amide;
5-methyl-isoxazole-3-carboxylic acid {(S)-2-(1*H*-indol-3-yl)-1-methyl-1-[(1-pyridin-2-yl-cyclohexylmethyl)-carbamoyl]-ethyl}-amide;
1-methyl-1*H*-pyrrole-2-carboxylic acid {(S)-2-(1*H*-indol-3-yl)-1-methyl-1-[(1-pyridin-2-yl-cyclohexylmethyl)-carbamoyl]-ethyl}-amide;
thiophene-2-carboxylic acid {(S)-2-(1*H*-indol-3-yl)-1-methyl-1-[(1-pyridin-2-yl-cyclohexylmethyl)-carbamoyl]-ethyl}-amide;
thiophene-3-carboxylic acid {(S)-2-(1*H*-indol-3-yl)-1-methyl-1-[(1-pyridin-2-yl-cyclohexylmethyl)-carbamoyl]-ethyl}-amide;
1*H*-indole-6-carboxylic acid {(S)-2-(1*H*-indol-3-yl)-1-methyl-1-[(1-pyridin-2-yl-cyclohexylmethyl)-carbamoyl]-ethyl}-amide;
1*H*-indole-5-carboxylic acid {(S)-2-(1*H*-indol-3-yl)-1-methyl-1-[(1-pyridin-2-yl-cyclohexylmethyl)-carbamoyl]-ethyl}-amide;
1*H*-indole-4-carboxylic acid {(S)-2-(1*H*-indol-3-yl)-1-methyl-1-[(1-pyridin-2-yl-cyclohexylmethyl)-carbamoyl]-ethyl}-amide;
1*H*-indole-7-carboxylic acid {(S)-2-(1*H*-indol-3-yl)-1-methyl-1-[(1-pyridin-2-yl-cyclohexylmethyl)-carbamoyl]-ethyl}-amide;
1-methyl-1*H*-indole-2-carboxylic acid {(S)-2-(1*H*-indol-3-yl)-1-methyl-1-[(1-pyridin-2-yl-cyclohexylmethyl)-carbamoyl]-ethyl}-amide;
benzothiazole-6-carboxylic acid {(S)-2-(1*H*-indol-3-yl)-1-methyl-1-[(1-pyridin-2-yl-cyclohexylmethyl)-carbamoyl]-ethyl}-amide;
1*H*-benzotriazole-5-carboxylic acid {(S)-2-(1*H*-indol-3-yl)-1-methyl-1-[(1-pyridin-2-yl-cyclohexylmethyl)-carbamoyl]-ethyl}-amide;
3-methyl-thiophene-2-carboxylic acid {(S)-2-(1*H*-indol-3-yl)-1-methyl-1-[(1-pyridin-2-yl-cyclohexylmethyl)-carbamoyl]-ethyl}-amide;
5-methyl-thiophene-2-carboxylic acid {(S)-2-(1*H*-indol-3-yl)-1-methyl-1-[(1-pyridin-2-yl-cyclohexylmethyl)-carbamoyl]-ethyl}-amide;
6-methyl-pyridine-2-carboxylic acid {(S)-2-(1*H*-indol-3-yl)-1-methyl-1-[(1-pyridin-2-yl-cyclohexylmethyl)-carbamoyl]-ethyl}-amide;
isoquinoline-3-carboxylic acid {(S)-2-(1*H*-indol-3-yl)-1-methyl-1-[(1-pyridin-2-yl-cyclohexylmethyl)-carbamoyl]-ethyl}-amide;
quinoxaline-2-carboxylic acid {(S)-2-(1*H*-indol-3-yl)-1-methyl-1-[(1-pyridin-2-yl-cyclohexylmethyl)-carbamoyl]-ethyl}-amide;
quinoline-8-carboxylic acid {(S)-2-(1*H*-indol-3-yl)-1-methyl-1-[(1-pyridin-2-yl-cyclohexylmethyl)-carbamoyl]-ethyl}-amide;
5-phenyl-oxazole-4-carboxylic acid {(S)-2-(1*H*-indol-3-yl)-1-methyl-1-[(1-pyridin-2-yl-cyclohexylmethyl)-carbamoyl]-ethyl}-amide;
(S)-3-(1*H*-indol-3-yl)-2-[2-(4-methoxy-phenyl)-ethanoylamino]-2-methyl-*N*-(1-pyridin-2-yl-cyclohexylmethyl)-propionamide;
(S)-2-[2-(4-dimethylamino-phenyl)-ethanoylamino]-3-(1*H*-indol-3-yl)-2-methyl-*N*-(1-pyridin-2-yl-cyclohexylmethyl)-propionamide;
(S)-3-(1*H*-indol-3-yl)-2-methyl-2-[2-(2-nitro-phenyl)-ethanoylamino]-*N*-(1-pyridin-2-yl-cyclohexylmethyl)-propionamide;
(S)-3-(1*H*-indol-3-yl)-2-[2-(2-methoxy-phenyl)-ethanoylamino]-2-methyl-*N*-(1-pyridin-2-yl-cyclohexylmethyl)-propionamide; and
*N*-{(S)-2-(1*H*-indol-3-yl)-1-methyl-1-[(1-pyridin-2-yl-cyclohexylmethyl)-carbamoyl]-ethyl}-2-pyrrol-1-yl-benzamide.

**35.** The use of any of claims 1-3, wherein the bombesin receptor antagonist is one of the following compounds or a salt thereof

{(S)-2-(1*H*-indol-3-yl)-1-methyl-1-[(1-pyridin-2-yl-cyclohexylmethyl)-carbamoyl]-ethyl}-carbamic acid naphthalen-1-ylmethyl ester,
{(S)-2-(1*H*-indol-3-yl)-1-methyl-1-[(1-pyridin-2-yl-cyclohexylmethyl)-carbamoyl]-ethyl}-carbamic acid 3,4-dichloro-benzyl ester;
{(S)-2-(1*H*-indol-3-yl)-1-methyl-1-[(1-pyridin-2-yl-cyclohexylmethyl)-carbamoyl]-ethyl}-carbamic acid 3-nitrobenzyl ester;
{(S)-2-(1*H*-indol-3-yl)-1-methyl-1-[(1-pyridin-2-yl-cyclohexylmethyl)-carbamoyl]-ethyl}-carbamic acid 3-trifluoromethyl-benzyl ester;
{(S)-2-(1*H*-indol-3-yl)-1-methyl-1-[(1-pyridin-2-yl-cyclohexylmethyl)-carbamoyl]-ethyl}-carbamic acid quinolin-6-ylmethyl ester;
{(S)-2-(1*H*-indol-3-yl)-1-methyl-1-[(1-pyridin-2-yl-cyclohexylmethyl)-carbamoyl]-ethyl}-carbamic acid 4-nitrobenzyl ester; and
{(S)-2-(1*H*-indol-3-yl)-1-methyl-1-[(1-pyridin-2-yl-cyclohexylmethyl)-carbamoyl]-ethyl}-carbamic acid 3-cyano-benzyl ester.

**36.** The use of any of claims 1-3, wherein the bombesin receptor antagonist is one of the following compounds or a salt thereof:

{(S)-2-(1*H*-indol-3-yl)-1-methyl-1-[(1-pyridin-2-yl-cyclohexylmethyl)-carbamoyl]-ethyl}-carbamic acid

3,4-dimethoxy-benzyl ester;
{(S)-2-(1*H*-indol-3-yl)-1-methyl-1-[(1-pyridin-2-yl-cyclohexylmethyl)-carbamoyl]-ethyl}-carbamic acid naphthalen-2-ylmethyl ester;
{(S)-2-(1*H*-indol-3-yl)-1-methyl-1-[(1-pyridin-2-yl-cyclohexylmethyl)-carbamoyl]-ethyl}-carbamic acid indan-2-yl ester;
{(S)-2-(1*H*-indol-3-yl)-1-methyl-1-[(1-pyridin-2-yl-cyclohexylmethyl)-carbamoyl]-ethyl}-carbamic acid 4-methoxy-benzyl ester;
{(S)-2-(1*H*-indol-3-yl)-1-methyl-1-[(1-pyridin-2-yl-cyclohexylmethyl)-carbamoyl]-ethyl}-carbamic acid 4-chloro-benzyl ester;
{(S)-2-(1*H*-indol-3-yl)-1-methyl-1-[(1-pyridin-2-yl-cyclohexylmethyl)-carbamoyl]-ethyl}-carbamic acid 2-fluorobenzyl ester;
{(S)-2-(1*H*-indol-3-yl)-1-methyl-1-[(1-pyridin-2-yl-cyclohexylmethyl)-carbamoyl]-ethyl}-carbamic acid 2-chloro-benzyl ester;
{(S)-2-(1*H*-indol-3-yl)-1-methyl-1-[(1-pyridin-2-yl-cyclohexylmethyl)-carbamoyl]-ethyl}-carbamic acid 2-methyl-benzyl ester;
{(S)-2-(1*H*-indol-3-yl)-1-methyl-1-[(1-pyridin-2-yl-cyclohexylmethyl)-carbamoyl]-ethyl}-carbamic acid 4-tert-butyl-benzyl ester;
{(S)-2-(1*H*-indol-3-yl)-1-methyl-1-[(1-pyridin-2-yl-cyclohexylmethyl)-carbamoyl]-ethyl}-carbamic acid 2-methoay-benzyl ester;
{(S)-2-(1*H*-indol-3-yl)-1-methyl-1-[(1-pyridin-2-yl-cyclohexylmethyl)-carbamoyl]-ethyl}-carbamic acid 4-trifluoromethyl-benzyl ester;
{(S)-2-(1*H*-indol-3-yl)-1-methyl-1-[(1-pyridin-2-yl-cyclohexylmethyl)-carbamoyl]-ethyl}-carbamic acid 3-ethoxy-benzyl ester;
{(S)-2-(1*H*-indol-3-yl)-1-methyl-1-[(1-pyridin-2-yl-cyclohexylmethyl)-carbamoyl]-ethyl}-carbamic acid 2,4-dichloro-benzyl ester;
{(S)-2-(1*H*-indol-3-yl)-1-methyl-1-[(1-pyridin-2-yl-cyclohexylmethyl)-carbamoyl]-ethyl}-carbamic acid 3-methyl-benzyl ester;
{(S)-2-(1*H*-indol-3-yl)-1-methyl-1-[(1-pyridin-2-yl-cyclohexylmethyl)-carbamoyl]-ethyl}-carbamic acid 3-phenoxy-benzyl ester; and
{(S)-2-(1*H*-indol-3-yl)-1-methyl-1-[(1-pyridin-2-yl-cyclohexylmethyl)-carbamoyl]-ethyl}-carbamic acid 4-methyl-benzyl ester.

**37.** The use of any of claims 1-3, wherein the bombesin receptor antagonist is one of the following compounds or a salt thereof:

(S)-3-(1*H*-indol-3-yl)-2-methyl-2-phenylmethanesulfonylamino-*N*-(1-pyridin-2-yl-cyclohexylmetbyl)-propionamide;
(S)-2-(2-chloro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(naphthalene-1-sulfonylamino)-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(quinoline-8-sulfonylamino)-propionamide;
(S)-3-(1*H*-indol-3-yl)-2-methyl-*N*-(1-pyridin-2-yl-cyclohexylmethyl)-2-(2-trifluoromethyl-benzenesulfonylamino)-propionamide;
(S)-2-(biphenyl-2-sulfonylamino)-3-(1*H*-indol-3-yl)-2-methyl-*N*-(1-pyridm-2-yl-cyclohexylmethyl)-propionamide;
(S)-3-(1*H*-indol-3-yl)-2-methyl-2-(5-methyl-2-phenoxy-benzenesulfonyl-amino)-*N*-(1-pyridin-2-yl-cyclohexylmethyl)-propionamide; and
(S)-3-(1*H*-indol-3-yl)-2-methyl-*N*-(1-pyridin-2-yl-cyclohexylmethyl)-2-(2-p-tolyloxy-benzenesulfonylamino)-propionamide.

**38.** The use of any of claims 1-3, wherein the bombesin receptor antagonist is one of the following compounds or a salt thereof:

(S)-3-(1*H*-indol-3-yl)-2-methyl-*N*-(1-pyridin-2-yl-cyclohexylmethyl)-2-(toluene-4-sulfonylamino)-propionamide;
(S)-3-(1*H*-indol-3-yl)-2-methanesulfonylamino-2-methyl-*N*-(1-pyridin-2-yl-cyclohexylmethyl)-propionamide;

(S)-2-(2-fluoro-benzenesulfonylamino)-3-(1*H*-indol-3-yl)-2-methyl-*N*-(1-pyridin-2-yl-cyclohexylmethyl)-propionamide;
(S)-2-(4-chloro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(2,2,2-trifluoro-ethanesulfonylamino)-propionamide;
(S)-2-(5-dimethylamino-naphthalene-1-sulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(naphthalene-2-sulfonylamino)-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(thiophene-2-sulfonylamino)-propionamide;
(S)-3-(1*H*-indol-3-yl)-2-methyl-2-(3-nitro-benzenesulfonylamino)-*N*-(1-pyridin-2-yl-cyclohexylmethyl)-propionamide;
(S)-2-(4-fluoro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(4-nitro-benzenesulfonylamino)-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(3-trifluoromethyl-benzenesulfonylamino)-propionamide;
(S)-2-(3,4-dichloro-benzenesulfonylamino)-3-(1*H*-indol-3-yl)-2-methyl-*N*-(1-pyridin-2-yl-cyclohexylmethyl)-propionamide;
(S)-2-(3-fluoro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyzidin-2-yl-cyclohexylmethyl)-2-(4-trifluoromethyl-benzenesulfonylamino)-propionamide;
(S)-2-(5-chloro-thiophene-2-sulfonylamino)-3-(1*H*-indol-3-yl)-2-methyl-*N*-(1-pyridin-2-yl-cyclohexylmethyl)-propionamide;
(S)-2-(3-chloro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(toluene-3-sulfonylamino)-propionamide;
(S)-2-(3,4-dimethoxy-benzenesulfonylamino)-3-(1*H*-indol-3-yl)-2-methyl-*N*-(1-pyridin-2-yl-cyclohexylmethyl)-propionamide;
(S)-2-(4-cyano-benzenesulfonylamino)-3-(1*H*-indol-3-yl)-2-methyl-*N*-(1-pyridin-2-yl-cyclohexylmethyl)-propionamide;
(S)-2-(2-cyano-benzenesulfonylamino)-3-(1*H*-indol-3-yl)-2-methyl-*N*-(1-pyridin-2-yl-cyclohexylmethyl)-propionamide;
(S)-2-(5-chloro-1,3-dimethyl-1*H*-pyrazole-4-sulfonylamino)-3-(1*H*-indol-3-yl)-2-methyl-*N*-(1-pyridin-2-yl-cyclohexylmethyl)-propionamide;
(S)-2-(3,5-dimethyl-isoxazole-4-sulfonylamino)-3-(1*H*-indol-3-yl)-2-methyl-*N*-(1-pyridin-2-yl-cyclohexylmethyl)-propionamide;
(S)-2-(benzo[1,2,5]thiadiazole-4-sulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(1-methyl-1*H*-imidazole-4-sulfonylamino)-*N*-(1-pyridin-2-yl-cyclohexylmethyl)-propionamide;
(S)-2-(benzo[1,2,5]oxadiazole-4-sulfonylamino)-3-(1*H*-indol-3-yl)-2-methyl-*N*-(1-pyridin-2-yl-cyclohexylmethyl)-propionamide;
3-{(S)-2-(1*H*-indol-3-yl)-1-methyl-1-[(1-pyridin-2-yl-cyclohexylmethyl)-carbamoyl]-ethylsulfamoyl}-thiophene-2-carboxylic acid methyl ester;
(S)-3-(1*H*-indol-3-yl)-2-(5-isoxazol-3-yl-thiophene-2-sulfonylamino)-2-methyl-*N*-(1-pyridin-2-yl-cyclohexylmethyl)-propionamide;
(S)-3-(1*H*-indol-3-yl)-2-methyl-2-(2-nitro-phenylmethanesulfonylamino)-*N*-(1-pyridin-2-yl-cyclohexylmethyl)-propionamide;
(S)-2-(3-cyano-benzenesulfonylamino)-3-(1*H*-indol-3-yl)-2-methyl-*N*-(1-pyridin-2-yl-cyclohexylmethyl)-propionamide;
(S)-2-(1,2-dimethyl-1*H*-imidazole-4-sulfonylamino)-3-(1*H*-indol-3-yl)-2-methyl-*N*-(1-pyridin-2-yl-cyclohexylmethyl)-propionamide;

(S)-3-(1*H*-indol-3-yl)-2-(3-methoxy-benzenesulfonylamino)-2-methyl-*N*-(1-pyridin-2-yl-cyclohexylmethyl)-propionamide;
(S)-3-(1*H*-indol-3-yl)-2-methyl-2-(8-nitro-naphthalene-1-sulfonylamino)-*N*-(1-pyridin-2-yl-cyclohexylmethyl)-propionamide;
(S)-2-(2-chloro-5-nitro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cycloheaylmethyl)-2-(2,4,6-trichloro-benzenesulfonylamino)-propionamide;
(S)-2-(4-chloro-2-nitro-benzenesulfonylamino)-3-(1*H*-indol-3-yl)-2-methyl-*N*-(1-pyridin-2-yl-cyclohexylmethyl)-propionamide;
(S)-2-(5-benzenesulfonyl-thiophene-2-sulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(4-trifluoromethoxy-benzenesulfonylamino)-propionamide;
2-{(S)-2-(1*H*-indol-3-yl)-1-methyl-1-[(1-pyridin-2-yl-cyclohexylmethyl)-carbamoyl]-ethylsulfamoyl}-benzaic acid methyl ester;
(S)-2-(3-chloro-4-fluoro-benzenesulfonylamino)-3-(1*H*-indol-3-yl)-2-methyl-*N*-(1-pyridin-2-yl-cyclohexylmethyl)-propionamide;
(S)-2-(2,5-dichloro-thiophene-3-sulfonylamino)-3-(1*H*-indol-3-yl)-2-methyl-*N*-(1-pyridin-2-yl-cyclohexylmethyl)-propionamide;
(S)-2-(3-chloro-4-methyl-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-(2-methoxy-4-methyl-benzenesulfonylamino)-2-methyl-*N*-(1-pyridin-2-yl-cyclohexylmethyl)-propionamide;
(S)-3-(1*H*-indol-3-yl)-2-methyl-*N*-(1-pyridin-2-yl-cyclohexylmethyl)-2-(5-pyridin-2-yl-thiophene-2-sulfonylamino)-propionamide;
(S)-2-(5-bromo-6-chloro-pyridine-3-sulfonylamino)-3-(1*H*-indol-3-yl)-2-methyl-*N*-(1-pyridin-2-yl-cyclohexylmethyl)-propionamide;
(S)-2-(2,4-dinitro-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-(4-methanesulfonyl-benzenesulfonylamino)-2-methyl-*N*-(1-pyridin-2-yl-cyclohexylmethyl)-propionamide;
(S)-2-(4-*tert*-butyl-benzenesulfonylamino)-3-(1*H*-indol-3-yl)-2-methyl-*N*-(1-pyridin-2-yl-cyclohexylmethyl)-propionamide;
(S)-2-(2,4-dichloro-5-methyl-benzenesulfonylamino)-3-(1*H*-indol-3-yl)-2-methyl-*N*-(1-pyridin-2-yl-cyclohexylmethyl)-propionamide;
(S)-2-(2-chloro-5-trifluoromethyl-benzenesulfonylamino)-3-(1*H*-indol-3-yl)-2-methyl-*N*-(1-pyridin-2-yl-cyclohexylmethyl)-propionamide;
(S)-3-(1*H*-indol-3-yl)-2-methyl-2-(2-nitro-4-trifluoromethyl-benzenesulfonylamino)-*N*-(1-pyridin-2-yl-cyclohexylmethyl)-propionamide; and
(S)-2-(4-butyl-benzenesulfonylamino)-3-(1*H*-indol-3-yl)-2-methyl-*N*-(1-pyridin-2-yl-cyclohexylmethyl)-propionamide.

**39.** Use in the preparation of a medicament for the treatment or prophylaxis of drug induced sexual dysfunction in the male, male erectile dysfunction, drug induced sexual dysfunction in the female, female hypoactive sexual desire disorder, female sexual arousal disorder, female anorgasmy or female sexual pain disorders of a pharmaceutical combination (for simultaneous, separate or sequential administration) of a bombesin receptor antagonist and a PDE 5 inhibitor.

**40.** Use in the preparation of a medicament for the treatment or prophylaxis of drug induced sexual dysfunction in the male, male erectile dysfunction, drug induced sexual dysfunction in the female, female hypoactive sexual desire disorder, female sexual arousal disorder, female anorgasmy or female sexual pain disorders of a pharmaceutical combination (for simultaneous, separate or sequential administration) of a bombesin receptor antagonist and a NEP inhibitor.

**41.** Use in the preparation of a medicament for the treatment or prophylaxis of drug induced sexual dysfunction in the male, male erectile dysfunction, drug induced sexual dysfunction in the female, female hypoactive sexual desire disorder, female sexual arousal disorder, female anorgasmy or female sexual pain disorders of a pharmaceutical

combination (for simultaneous, separate or sequential administration) of a bombesin receptor antagonist and one or more estrogen receptor modulators (SERM) and/or estrogen agonists and/or estrogen antagonists.

**42.** Use in the preparation of a medicament for the treatment or prophylaxis of drug induced sexual dysfunction in the male, male erectile dysfunction, drug induced sexual dysfunction in the female, female hypoactive sexual desire disorder, female sexual arousal disorder, female anorgasmy or female sexual pain disorders of a pharmaceutical combination (for simultaneous, separate or sequential administration) of a bombesin receptor antagonist and and lasofoxifene.

## Patentansprüche

**1.** Verwendung einer pharmazeutischen Kombination (zur gleichzeitigen, getrennten oder aufeinanderfolgenden Verabreichung) von einem Bombesin-Rezeptorantagonisten und einem oder mehreren Materialien, die aus den folgenden (1) bis (32) ausgewählt sind,

(1) natürlich vorkommende oder synthetische Prostaglandine oder Ester derselben,
(2) α-Adrenorezeptorantagonistverbindungen, die auch als α-Adrenorezeptorantagonisten oder α-Rezeptorantagonisten oder α-Blocker bekannt sind,
(3) NO-Donor(NO-Agonist)verbindungen,
(4) Kaliumkanalöffner oder -modulatoren,
(5) dopaminerge Mittel,
(6) Nimodepin, Pinacidil, Cyclandelat, Isoxsuprin, Chlorpromazin, Haloperidol, Rec 15/2739, Trazodon, Alprostadil oder Phentolamin,
(7) Thromboxan-A2-Agonisten,
(8) Ergot-Alkaloide,
(9) Angiotensin-Rezeptorantagonisten, wie Losartan,
(10) Substrate für NO-Synthase,
(11) Calciumkanalblocker,
(12) Cholesterinsenker,
(13) Antithrombocyten- und Antithrombosemittel,
(14) Insulinsensibilisierungsmittel und Hypoglykämika,
(15) L-DOPA oder Carbidopa,
(16) Acetylcholinesteraseinhibitoren,
(17) steroidale oder nichtsteroidale entzündungshemmende Mittel,
(18) Östrogenrezeptormodulatoren und/oder Östrogenagonisten und/oder Östrogenantagonisten und pharmazeutisch akzeptable Salze derselben,
(19) PDE-Inhibitoren,
(20) NPY(Neuropeptid Y)-Inhibitoren,
(21) NEP-Inhibitoren,
(22) vasoaktive intestinale Peptide (VIP), VIP-Mimetika, VIP-Analoga, die über einen oder mehrere der VIP-Rezeptorsubtypen VPAC1, VPAC2, oder PACAP (Pituitory Adenylate Cyclase Activating Peptide) wirken, das VIP-Analogon Ro-125-1553, VIP-Rezeptoragonisten oder VIP-Fragmente oder α-Adrenorezeptorantagonisten mit VIP-Kombinationen,
(23) Melanocortin-Rezeptoragonisten oder -modulatoren oder Melanocortin-Verstärker,
(24) Serotonin-Rezeptoragonisten, -antagonisten oder -modulatoren,
(25) Testosteron-Ersatzmittel, Testosternon, Dihydrotestosteron oder ein Testosteronimplantat,
(26) Östrogen, Östrogen und Medroxyprogesteron oder Medroxyprogesteronacetat (MPA) (d.h. als Kombination) oder Östrogen und Methyltestosteron-Hormonersatztherapiemittel,
(27) Monoaminstoffwechsel- oder -wiederaufnahmemodifizierungsmittel, die den Stoffwechsel oder die Wiederaufnahme von Catecholamin hemmen,
(28) Agonisten und/oder Modulatoren purinerger Rezeptoren,
(29) Neurokinin(NK)-Rezeptorantagonisten,
(30) Opioid-Rezeptoragonisten, -antagonisten oder -modulatoren,
(31) Agonisten oder Modulatoren für Oxytocin/Vasopressin-Rezeptoren und
(32) Modulatoren von Cannabinoidrezeptoren, bei der Herstellung eines Medikaments zur Behandlung oder Prophylaxe von arzneimittelinduzierter sexueller Dysfunktion beim Mann, männlicher erektiler Dysfunktion, arzneimittelinduzierter sexueller Dysfunktion bei der Frau, der weiblichen Störung von hypoaktivem sexuellem

Begehren, einer weiblichen Störung der sexuellen Vigilanz, weiblicher Anorgasmie oder weiblichen sexuellen Schmerzstörungen.

**2.** Verwendung nach Anspruch 1, wobei das Medikament zur Behandlung einer Antidepressivum-induzierten sexuellen Dysfunktion bei einem Mann dient.

**3.** Verwendung nach Anspruch 1, wobei das Medikament zur Behandlung einer Antidepressivum-induzierten sexuellen Dysfunktion bei einer Frau dient.

**4.** Verwendung nach Anspruch 1, 2 oder 3, wobei der Bombesin-Rezeptorantagonist ein selektiver Bombesin-BB1-Antagonist ist.

**5.** Verwendung nach Anspruch 4, wobei der Bombesin-BB1-Antagonist eine Selektivität für $BB_1$ gegenüber den anderen Bombesin-Rezeptorsubtypen von größer als 10 aufweist.

**6.** Verwendung nach Anspruch 4, wobei der Bombesin-BB1-Antagonist eine Selektivität für $BB_1$ gegenüber den anderen Bombesin-Rezeptorsubtypen von größer als 30 aufweist.

**7.** Verwendung nach Anspruch 4, wobei der Bombesin-BB1-Antagonist eine Selektivität für $BB_1$ gegenüber den anderen Bombesin-Rezeptorsubtypen von größer als 100 aufweist.

**8.** Verwendung nach einem der Ansprüche 4-7, wobei der Bombesin-Rezeptorantagonist einen Ki-Wert gegenüber BB1 von weniger als 1000 nM aufweist.

**9.** Verwendung nach einem der Ansprüche 4-7, wobei der Bombesin-Rezeptorantagonist einen Ki-Wert gegenüber BB1 von weniger als 500 nM aufweist.

**10.** Verwendung nach einem der Ansprüche 4-7, wobei der Bombesin-Rezeptorantagonist einen Ki-Wert gegenüber BB1 von weniger als 100 nM aufweist.

**11.** Verwendung nach einem der Ansprüche 4-7, wobei der Bombesin-Rezeptorantagonist einen Ki-Wert gegenüber BB1 von weniger als 50 nM aufweist.

**12.** Verwendung nach einem der Ansprüche 4 bis 7, wobei der Bombesin-Rezeptorantagonist einen Ki-Wert gegenüber BB1 von weniger als 10 nM aufweist.

**13.** Verwendung nach einem der Ansprüche 1-3, wobei der Bombesin-Rezeptorantagonist ein gemischter BB1/BB2-Antagonist ist.

**14.** Verwendung nach einem der vorhergehenden Ansprüche, wobei das Medikament zur oralen Verabreichung angepasst ist.

**15.** Verwendung nach einem der vorhergehenden Ansprüche, wobei das Medikament eine wirksame Menge eines Nichtpeptid-Bombesin-Rezeptorantagonisten umfasst.

**16.** Verwendung nach Anspruch 15, wobei der Nichtpeptid-Bombesin-Rezeptorantagonist eine bei oraler Verabreichung absorbierbare Verbindung ist.

**17.** Verwendung nach einem der Ansprüche 1-14, wobei das Medikament eine wirksame Menge eines Bombesin-Rezeptorantagonisten, der ein Peptid ist, umfasst.

**18.** Verwendung nach einem der Ansprüche 1-3, wobei der Bombesin-Rezeptorantagonist eine Verbindung der Formel (I)

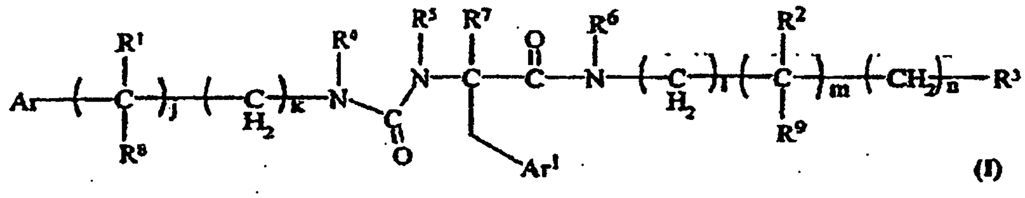

(I)

oder ein pharmazeutisch akzeptables Salz derselben ist, worin:

j 0 oder 1 ist;
k 0 oder 1 ist;
l 0, 1, 2 oder 3 ist;
m 0 oder 1 ist;
n 0, 1 oder 2 ist;
Ar Phenyl, Pyridyl oder Pyrimidyl, die jeweils unsubstituiert oder mit ein bis drei Substituenten substituiert sind, die aus Alkyl, Halogen, Alkoxy, Acetyl, Nitro, Amino, $-CH_2NR^{10}R^{11}$, Cyano, $-CF_3$, $-NHCONH_2$ und $-CO_2R^{12}$ ausgewählt sind, bedeutet;
$R^1$ Wasserstoff oder gerades, verzweigtes oder cyclisches Alkyl mit 1 bis 7 Kohlenstoffatomen bedeutet; $R^8$ Wasserstoff bedeutet oder mit $R^1$ einen Ring mit 3 bis 7 Kohlenstoffatomen bildet;
$R^2$ Wasserstoff oder gerades, verzweigtes oder cyclisches Alkyl mit 1 bis 8 Kohlenstoffatomen, das auch 1 bis 2 Sauerstoff- oder Stickstoffatome enthalten kann, bedeutet;
$R^9$ Wasserstoff bedeutet oder mit $R^2$ einen Ring mit 3 bis 7 Kohlenstoffatomen, der ein Sauerstoff- oder Stickstoffatom enthalten kann, bildet; oder $R^2$ und $R^9$ zusammen ein Carbonyl sein können;
$Ar^1$ in unabhängiger Weise aus Ar ausgewählt sein kann und auch Pyridyl-N-oxid, Indolyl, Imidazolyl und Pyridyl umfassen kann;
$R^4$, $R^5$, $R^6$ und $R^7$ jeweils unabhängig voneinander aus Wasserstoff und Niederalkyl ausgewählt sind; wobei $R^4$ auch mit $R^5$ eine kovalente Verknüpfung aus 2 bis 3 Atomen, die ein Sauerstoff- oder Stickstoffatom umfassen kann, bilden kann;
$R^3$ in unabhängiger Weise aus Ar ausgewählt sein kann oder Wasserstoff, Hydroxy, $-NMe_2$, N-Methyl-pyrrolyl, Imidazolyl, N-Methyl-imidazolyl, Tetrazolyl, N-Methyl-tetrazolyl, Thiazolyl, $-CONR^{13}R^{14}$, Alkoxy,

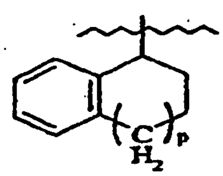 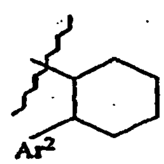, 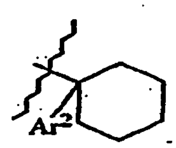,

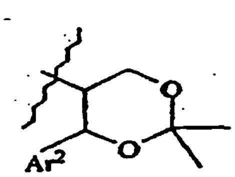 oder 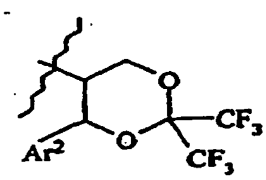

worin p 0, 1 oder 2 ist und $Ar^2$ Phenyl oder Pyridyl ist, bedeutet;
$R^{10}$, $R^{11}$, $R^{12}$, $R^{13}$ und $R^{14}$ jeweils unabhängig voneinander aus Wasserstoff oder geradem, verzweigtem oder cyclischem Alkyl mit 1 bis 7 Kohlenstoffatomen ausgewählt sind.

**19.** Verwendung nach einem der Ansprüche 1-3, wobei der Bombesin-Rezeptorantagonist eine Verbindung der Formel (Ia)

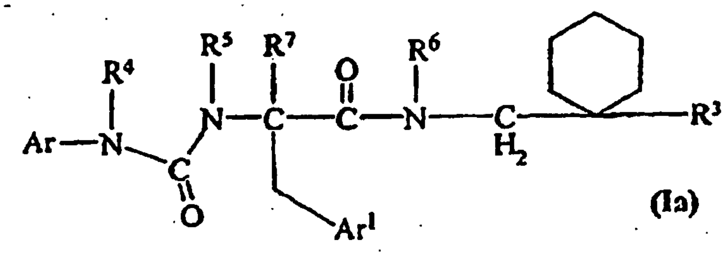

(Ia)

ist, worin

Ar Phenyl bedeutet, das unsubstituiert oder mit einem oder zwei Substituenten, die aus Isopropyl, Halogen, Nitro und Cyano ausgewählt sind, substituiert ist;
$R^4$, $R^5$ und $R^6$ Wasserstoff bedeuten;
$R^7$ Methyl oder Wasserstoff bedeutet;
$R^3$ 2-Pyridyl oder Hydroxy bedeutet; und
$Ar^1$ Indolyl, Pyridyl, Pyridyl-N-oxid oder Imidazolyl bedeutet.

**20.** Verwendung nach Anspruch 18, wobei der Bombesin-Rezeptorantagonist eine Verbindung der Formel I ist, worin Ar unsubstituiertes Phenyl bedeutet;

$R^1$ Cyclopentyl oder tert-Butyl bedeutet;
$R^4$ und $R^5$ Wasserstoff bedeuten;
$R^7$ Methyl bedeutet;
$R^6$ Wasserstoff bedeutet;
$R^3$ Phenyl mit zwei Isopropylsubstituenten, unsubstituiertes Phenyl oder

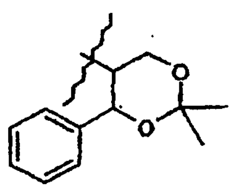

bedeutet; und
$Ar^1$ Indolyl bedeutet.

**21.** Verwendung nach Anspruch 18, wobei der Bombesin-Rezeptorantagonist eine Verbindung der Formel I ist, worin Ar 2,6-Diisopropyl-phenyl, 4-Nitro-phenyl und 4-Cyano-phenyl bedeutet;

$R^4$, $R^5$ und $R^6$ Wasserstoff bedeuten;
$R^7$ Methyl bedeutet;
$R^2$ Wasserstoff oder Cyclohexyl bedeutet und
$R^3$ Hydroxyl, Pyridyl,

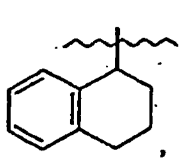
,
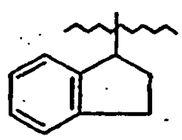
oder
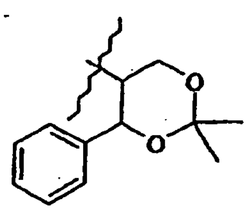

bedeutet.

22. Verwendung nach einem der Ansprüche 1-3, wobei der Bombesin-Rezeptorantagonist (S)-3-(1H-Indol-3-yl)-N-[1-(5-methoxy-pyridin-2-yl)-cyclohexylmethyl]-2-methyl-2-[3-(4-nitro-phenyl)ureido]propionamid (auch als Verbindung 1 bezeichnet) oder eines von dessen pharmazeutisch akzeptablen Salzen ist oder (2S)-N-{[1-(4-Aminophenyl)cyclohexyl]methyl}-3-(1H-indol-3-yl)-2-methyl-2-{[(4-nitroanilino)carbonyl]amino}propanamid (auch als Verbindung 3 bekannt) oder eines von dessen pharmazeutisch akzeptablen Salzen ist.

23. Verwendung nach einem der Ansprüche 1-3, wobei der Bombesin-Rezeptorantagonist eine der im folgenden angegebenen Verbindungen oder ein pharmazeutisch akzeptables Salz derselben ist:

(S)-N-Cyclohexylmethyl-2-[3-(2,6-diisopropyl-phenyl)-ureido]-3-(1H-indol-3-yl)-2-methyl-propionamid;
N-Cyclohexylmethyl-2-[3-(2,6-diisopropyl-phenyl)-ureido]-3-(1H-indol-3-yl)-N-methyl-propionamid;
N-Cyclohexylmethyl-2-[3-(2,6-diisopropyl-phenyl)-1-methyl-ureido]-3-(1H-indol-3-yl)-propionamid;
2-[3-(2,6-Diisopropyl-phenyl)-ureido]-2-methyl-3-(1-oxy-pyridin-2-yl)-N-(1-pyridin-2-yl-cyclohexylmethyl)-propionamid;
2-[3-(2,6-Diisopropyl-phenyl)-ureido]-2-methyl-3-pyridin-2-yl-N-(1-pyridin-2-yl-cyclohexylmethyl)-propionamid;
2-[3-(2-tert-Butyl-phenyl)-ureido]-N-cyclohexylmethyl-3-(1H-indol-3-yl)-2-methyl-propionamid;
N-Cyclohexylmethyl-2-[3-(2,6-dichlor-phenyl)ureido]-3-(1H-indol-3-yl)-2-methyl-propionamid;
N-Cyclohexylmethyl-2-[3-(2,6-dimethoxy-phenyl)ureido]-3-(1H-indol-3-yl)-2-methyl-propionamid;
N-Cyclohexylmethyl-2-[3-(2,6-dimethylamino-phenyl)-ureido]-3-(1H-indol-3-yl)-2-methyl-propionamid;
(S)-N-Cyclohexylmethyl-3-(1H-indol-3-yl)-2-methyl-2-[3-(4-nitro-phenyl)-ureido]-propionamid;
N-Cyclohexylmethyl-2-[3-(2,2-dimethyl-1-phenyl)propyl)-ureido]-3-(1H-indol-3-yl)-2-methyl-propionamid;
[S-(R*,R*)]-3-(1H-Indol-3-yl)-2-methyl-2-{3-[1-(4-nitro-phenyl)-ethyl]-ureido}-N-(1-pyridin-2-yl-cyclohexylmethyl)-propionamid;
N-(2,2-Dimethyl-4-phenyl-[1,3]dioxan-5-yl)-3-(1H-indol-3-yl)-2-methyl-2-[3-(1-phenyl-cyclopentylmethyl)ureido]-propionamid;
(S)-N-(2,6-Diisopropyl-phenyl)-2-[3-(2,2-dimethyl-1-phenyl-propyl)-ureido]-3-(1H-indol-3-yl)-propionamid;
(R)-N-(2,6-Diisopropyl-phenyl)-2-[3-(2,2-dimethyl-1-phenyl-propyl)-ureido]-3-(1H-indol-3-yl)-propionamid;
2-[3-(2,6-Diisopropyl-phenyl)-ureido]-N-(2,2-dimethyl-4-phenyl-[1,3]dioxan-5-yl)-3-(1H-indol-3-yl)-2-methyl-propionamid;
N-Cyclohexyl-2-[3-(2,6-diisopropyl-phenyl)-ureido]-3-(1H-indol-3-yl)-2-methyl-propionamid;
N-(2-Cyclohexyl-ethyl)-2-[3-(2,6-diisopropyl-phenyl)-ureido]-3-(1H-indol-3-yl)-2-methyl-propionamid;
2-[3-(2,6-Diisopropyl-phenyl)-ureido]-3-(1H-indol-3-yl)-2-methyl-propionamid;
2-[3-(2,6-Diisopropyl-phenyl)-ureido]-3-(1H-indol-3-yl)-2-methyl-N-(3-methyl-butyl)-propionamid;
2-[3-(2,6-Diisopropyl-phenyl)-ureido]-3-(1H-indol-3-yl)-2-methyl-N-(3-phenyl-propyl)-propionamid;
2-[3-(2,6-Diisopropyl-phenyl)-ureido]-3-(1H-indol-3-yl)-2-methyl-N-(1,2,3,4-tetrahydro-naphthalin-1-yl)-propionamid;
2-[3-(2,6-Diisopropyl-phenyl)-ureido]-3-(1H-indol-3-yl)-2-methyl-N-(2-phenyl-cyclohexyl)-propionamid;
2-[3-(2,6-Diisopropyl-phenyl)-ureido]-N-indan-1-yl-3-(1H-indol-3-yl)-2-methyl-propionamid;
2-[3-(2,6-Diisopropyl-phenyl)-ureido]-N-(1-hydroxy-cyclohexylmethyl)-3-(1H-indol-3-yl)-2-methyl-propionamid;
2-[3-(2,6-Diisopropyl-phenyl)-ureido]-3-(1H-indol-3-yl)-2-methyl-N-(1-pyridin-2-yl-cyclohexylmethyl)-propionamid;
2-[3-(2,6-Diisopropyl-phenyl)-ureido]-3-(1H-indol-3-yl)-2-methyl-N-(6,7,8,9-tetrahydro-5H-benzocyclohepten-5-yl)-propionamid;
2-[3-(2,6-Diisopropyl-phenyl)-ureido]-3-(1H-indol-3-yl)-2-methyl-N-phenyl-propionamid;
N-(1-Hydroxy-cyclohexylmethyl)-3-(1H-indol-3-yl)-2-methyl-2-[3-(4-nitro-phenyl)-ureido]-propionamid;
2-[3-(4-Cyano-phenyl)-ureido]-3-(1H-indol-3-yl)-2-methyl-N-(1-pyridin-2-yl-cyclohexylmethyl)-propionamid;
(S)-3-(1H-Indol-3-yl)-2-methyl-2-[3-(4-nitro-phenyl)-ureido]-N-(1-pyridin-2-yl-cyclohexylmethyl)-propionamid;
(S)-3-(1H-Indol-3-yl)-2-methyl-N-(1-pyridin-2-yl-cyclohexylmethyl)-2-[3-(4-trifluormethyl-phenyl)-ureido]-propionamid;
(S)-4-(3-{2-(1H-Indol-3-yl)-1-methyl-1-[(1-pyridin-2-yl-cyclohexylmethyl)-carbamoyl]-ethyl}-ureido)-benzoesäureethylester;
2-[3-(2,6-Diisopropyl-phenyl)-ureido]-3-(1H-imidazol-4-yl)-N-(1-pyridin-2-yl-cyclohexylmethyl)-propionamid;
2-[3-(2,6-Diisopropyl-phenyl)-ureido]-2-methyl-N-(1-pyridin-2-yl-cyclohexylmethyl)-3-(2-trifluormethylphenyl)-propionamid;

2-[3-(2,6-Diisopropyl-phenyl)-ureido]-2-methyl-3-(2-nitro-phenyl)-N-(1-pyridin-2-yl-cyclohexylmethyl)-propionamid;
(S)-3-(1H-Indol-3-yl)-N-[1-(5-methoxy-pyridin-2-yl)-cyclohexylmethyl]-2-methyl-2-[3-(4-nitro-phenyl)-ureido]-propionamid und
N-Cyclohexylmethyl-2-[3-(2,6-diisopropyl-phenyl)-ureido]-2-methyl-3-pyridin-2-yl-propionamid.

**24.** Verwendung nach einem der Ansprüche 1-3, wobei der Bombesin-Rezeptorantagonist eine Verbindung der Formel (II) oder ein pharmazeutisch akzeptables Salz derselben ist:

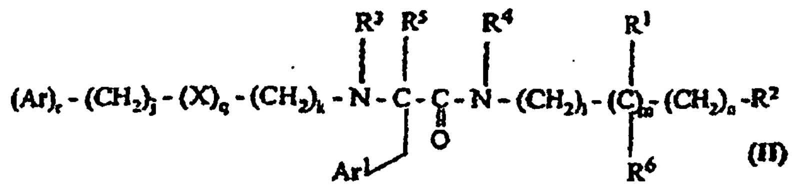

(II)

worin:

j 0, 1 oder 2 ist;
k 0 oder 1 ist;
l 0, 1, 2 oder 3 ist;
m 0 oder 1 ist;
n 0, 1 oder 2 ist;
q 0 oder 1 ist;
r 0 oder 1 ist; wobei, wenn r 0 ist, Ar durch Wasserstoff ersetzt ist;
Ar Phenyl, Pyridyl, Pyrimidyl, Thienyl, Furyl, Imidazolyl, Pyrrolyl oder Thiazolyl bedeutet, die jeweils unsubstituiert oder mit 1 bis 3 Substituenten substituiert sind, die aus Acetyl, Alkoxy, Alkyl, Amino, Cyano, Halogen, Hydroxy, Nitro, Sulfonamido, Sulfonyl, $-CF_3$, $-OCF_3$, $-CO_2H$, $-CH_2CN$, $-SO_2CF_3$, $-CH_2CO_2H$ und $-(CH_2)_sNR^7R^8$, wobei s 0, 1, 2 oder 3 bedeutet und $R^7$ und $R^8$ jeweils unabhängig voneinander aus H, geradem oder verzweigtem Alkyl mit bis zu 6 Kohlenstoffatomen ausgewählt sind oder $R^7$ und $R^8$ zusammen mit dem Stickstoffatom, an das sie gebunden sind, einen 5- bis 7-gliedrigen aliphatischen Ring, der 1 oder 2 Sauerstoffatome enthalten kann, bilden können, ausgewählt sind;
$R^1$ Wasserstoff, gerades oder verzweigtes Alkyl mit bis zu 6 Kohlenstoffatomen oder Cycloalkyl mit zwischen 5 und 7 Kohlenstoffatomen, das 1 oder 2 Stickstoff- oder Sauerstoffatome enthalten kann, bedeutet;
$R^6$ Wasserstoff, Methyl bedeutet oder mit $R^1$ einen aliphatischen Ring mit 3 bis 7 Atomen, der ein Sauerstoff- oder Stickstoffatom enthalten kann, bildet oder zusammen mit $R^1$ eine Carbonylgruppe bedeutet;
$Ar^1$ in unabhängiger Weise aus Ar ausgewählt ist oder Indolyl oder Pyridyl-N-oxid bedeutet;
$R^3$, $R^4$ und $R^5$ jeweils unabhängig voneinander aus Wasserstoff und Niederalkyl ausgewählt sind;
$R^2$ in unabhängiger Weise aus Ar ausgewählt ist oder Wasserstoff, Hydroxy, Alkoxy, $-NMe_2$, $-CONR^9R^{10}$, wobei $R^9$ und $R^{10}$ jeweils unabhängig voneinander aus Wasserstoff, geradem oder verzweigtem Alkyl mit bis zu 6 Kohlenstoffatomen ausgewählt sind oder $R^9$ und $R^{10}$ zusammen mit dem Stickstoffatom, an das sie gebunden sind, einen 5- bis 7-gliedrigen aliphatischen Ring, der 1 oder 2 Sauerstoff- oder Stickstoffatome enthalten kann, bilden können, bedeutet, oder $R^2$

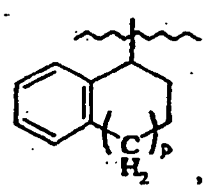 ,
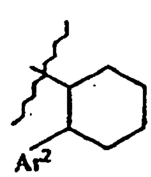 ,
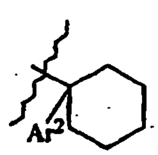 ,

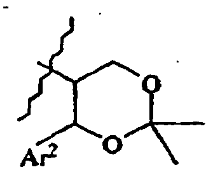 oder 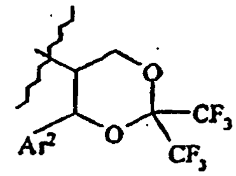

worin p 0, 1 oder 2 ist und $Ar^2$ Phenyl oder Pyridyl ist, bedeutet;
X einen zweiwertigen Rest bedeutet, der von einem der folgenden abgeleitet ist,

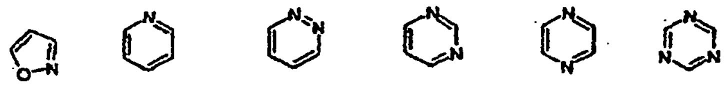

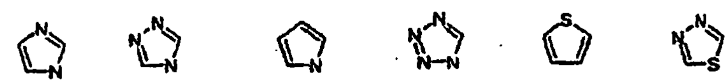

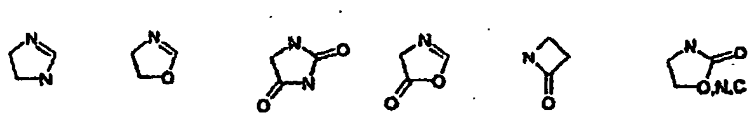

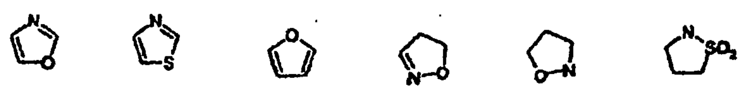

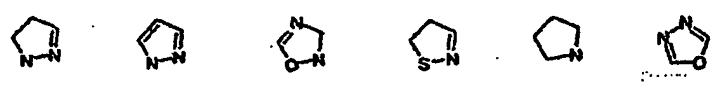

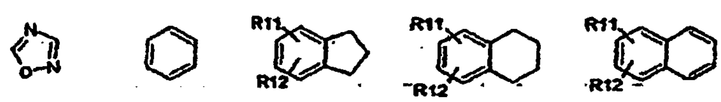

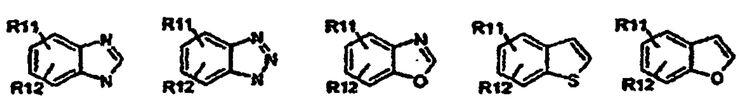

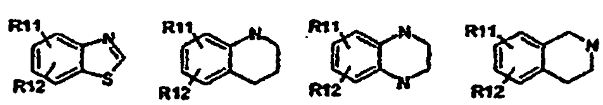

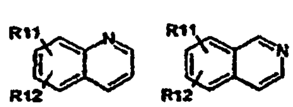

wobei die Ringstickstoffatome daran gebundene Niederalkylgruppen aufweisen können, $R^{11}$ und $R^{12}$ unabhängig voneinander aus H, Halogen, Hydroxy, Alkoxy, Acetyl, Nitro, Cyano, Amino, $CF_3$ und $-(CH_2)_tNR^{13}R^{14}$, wobei t 0 oder 1 sein kann, $R^{13}$ und $R^{14}$ jeweils unabhängig voneinander aus Wasserstoff, geradem oder verzweigtem Alkyl mit bis zu 6 Kohlenstoffatomen oder Cycloalkyl mit 5 bis 7 Kohlenstoffatomen, das bis zu 2 Sauerstoff- oder Stickstoffatome enthält, ausgewählt sind, ausgewählt sind.

**25.** Verwendung nach einem der Ansprüche 1-3, wobei der Bombesin-Rezeptorantagonist eine Verbindung der Formel (IIa) oder ein pharmazeutisch akzeptables Salz derselben ist:

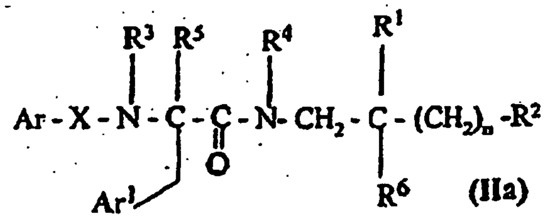

(IIa)

worin:

n 0 oder 1 ist;
Ar Phenyl oder Pyridyl, das unsubstituiert oder mit 1 bis 3 Substituenten, die aus Halogen, Alkoxy, Nitro und Cyano ausgewählt sind, substituiert sein kann, bedeutet;
$Ar^1$ in unabhängiger Weise aus Ar ausgewählt ist oder Pyridyl-N-oxid oder Indolyl bedeutet;
$R^6$ mit $R^1$ einen aliphatischen Ring mit 3 bis 7 Atomen, der ein Sauerstoff- oder Stickstoffatom enthalten kann, bildet oder zusammen mit $R^1$ eine Carbonylgruppe bedeutet;
$R^2$ in unabhängiger Weise aus Ar ausgewählt ist oder Wasserstoff, Hydroxy, Alkoxy, Dimethylamino, Tetrazolyl oder $-CONR^9R^{10}$, wobei $R^9$ und $R^{10}$ jeweils unabhängig voneinander aus Wasserstoff oder Methyl ausgewählt sind, bedeutet, oder $R^2$ einen Rest von

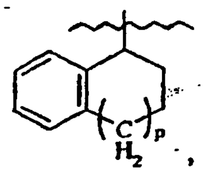 , 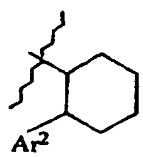 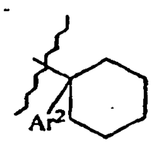 ,

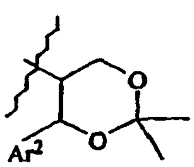 oder 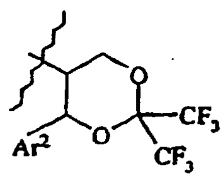

worin p 0, 1 oder 2 ist und $Ar^2$ Phenyl oder Pyridyl ist, bedeutet;
$R^3$, $R^4$ und $R^5$ jeweils unabhängig voneinander aus Wasserstoff und Methyl ausgewählt sind; und X ausgewählt ist aus:

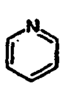 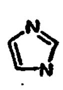 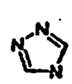 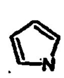 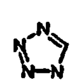 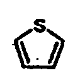

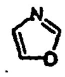 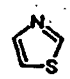 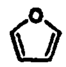 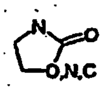 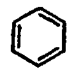 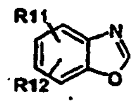

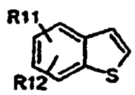 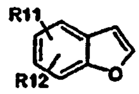 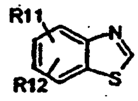 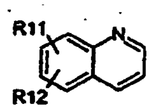 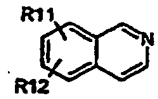

wobei $R^{11}$ und $R^{12}$ unabhängig voneinander aus H, Halogen, Hydroxy, Alkoxy, Acetyl, Nitro, Cyano, Amino, $CF_3$ und $(CH_2)_tNR^{13}R^{14}$, wobei t 0 oder 1 ist und $R^{13}$ und $R^{14}$ unabhängig voneinander aus Wasserstoff und Methyl ausgewählt sind, ausgewählt sind.

**26.** Verwendung nach einem der Ansprüche 1-3, wobei der Bombesin-Rezeptorantagonist eine Verbindung der Formel (IIb) oder (IIc) oder ein pharmazeutisch akzeptables Salz derselben ist:

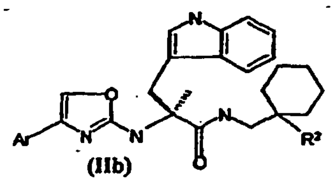
(IIb)
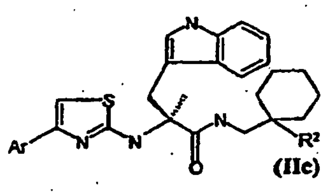
(IIc)

worin Ar und $R^2$ unabhängig voneinander Phenyl oder Pyridyl, die unsubstituiert oder mit 1 bis 3 Substituenten, die aus Halogen, Alkoxy, Nitro und Cyano ausgewählt sind, substituiert sein können, bedeuten, und pharmazeutisch akzeptable Salze derselben.

**27.** Verwendung nach einem der Ansprüche 1-3, wobei der Bombesin-Rezeptorantagonist (S)-3-(1H-Indol-3-yl) -N-[1-(5-methoxy-pyridin-2-yl)-cyclohexylmethyl]-2-methyl-2-[4-(4-nitro-phenyl)-oxazol-2-ylamino]-propionamid (auch als Verbindung 2 bezeichnet) oder ein pharmazeutisch akzeptables Salz ist.

**28.** Verwendung nach einem der Ansprüche 1-3, wobei der Bombesin-Rezeptorantagonist eine der folgenden Verbindungen oder ein pharmazeutisch akzeptables Salz derselben ist:

(S)-3-(1H-Indol-3-yl)-N-[1-methoxymethyl-cyclohexylmethyl)-2-methyl-2-[4-(4-nitro-phenyl)-oxazol-2-ylamino]-propionamid;
(S)-3-(1H-Indol-3-yl)-2-methyl-2-[4-(4-nitro-phenyl)-oxazol-2-ylamino]-N-(2-oxo-2-phenyl-ethyl)-propionamid;
(S)-N-[1-(5-Methoxy-pyridin-2-yl)-cyclohexylmethyl]-2-methyl-2-[4-(4-nitro-phenyl)-oxazol-2-ylamino]-3-phenyl-propionamid;
(S)-2-[4-(4-Cyano-phenyl)-oxazol-2-ylamino]-3-(1H-indol-3-yl)-N-[1-(5-methoxy-pyridin-2-yl)-cyclohexylmethyl]-2-methyl-propionamid;
(S)-3-(1H-Indol-3-yl)-N-[1-(5-methoxy-pyridin-2-yl)-cyclohexylmethyl]-2-methyl-2-(4-phenyl-oxazol-2-ylamino-propionamid;
(S)-2-(4-Ethyl-oxazol-2-ylamino)-3-(1H-indol-3-yl)-N-[1-(5-methoxy-pyridin-2-yl)-cyclohexylmethyl]-2-methyl-propionamid;
(S)-3-(1H-Indol-3-yl)-N-[1-(5-methoxy-pyridin-2-yl)-cyclohexylmethyl]-2-methyl-2-[4-(4-nitro-phenyl)-thiazol-2-ylamino]-propionamid;
(S)-2-(Benzoxazol-2-ylamino)-3-(1H-indol-3-yl)-2-methyl-N-(1-pyridin-2-yl-cyclohexylmethyl)-propionamid;
(S)-3-(1H-Indol-3-yl)-2-methyl-2-(pyridin-4-ylamino)-N-(1-pyridin-2-yl-cyclohexylmethyl)-propionamid;
(S)-3-(1H-Indol-3-yl)-2-(isochinol-4-ylamino)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(pyrimidin-5-ylamino)-propionamid;
(S)-2-(Biphenyl-2-ylamino)-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-m-tolylamino-propionamid;

(S)-3-(1H-Indol-3-yl)-2-methyl-2-(6-phenyl-pyridin-2-ylamino)-N-(1-pyridin-2-yl-cyclohexylmethyl)-propionamid;
(R)-3-Phenyl-2-phenylamino-N-(1-pyridin-2-yl-cyclohexylmethyl)-propionamid;
(S)-3-(1H-Indol-3-yl)-2-methyl-2-phenylethylamino-N-(1-pyridin-2-yl-cyclohexylmethyl)-propionamid;
(S)-2-[(Benzofuran-2-ylmethyl)-amino]-3-(1H-indol-3-yl)-2-methyl-N-(1-pyridin-2-yl-cyclohexylmethyl)-propionamid und
(S)-3-(1H-Indol-3-yl)-2-methyl-2-(4-nitro-benzylamino)-N-(1-pyridin-2-yl-cyclohexylmethyl)-propionamid.

**29.** Verwendung nach einem der Ansprüche 1-3, wobei der Bombesin-Rezeptorantagonist eine Verbindung der Formel (III) oder ein pharmazeutisch akzeptables Salz derselben ist:

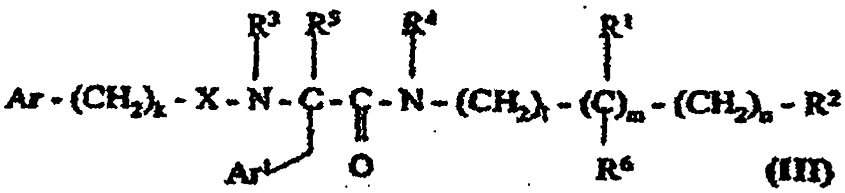

(III)

worin

k 0, 1 oder 2 ist;
l 0, 1, 2, oder 3 ist;
m 0 oder 1 ist;
n 0, 1 oder 2 ist;
X -CO-, -OCO, -SO- und $-SO_2-$ bedeutet;
Ar Benzimidazolyl, Benzofuryl, Benzothiadiazolyl, Benzothiazolyl, Benzothienyl, Benzopyrazinyl, Benzotriazolyl, Benzoxadiazolyl, Furyl, Imidazolyl, Indanyl, Indolyl, Isochinolyl, Isoxazolyl, Naphthyl, Oxazolyl, Phenyl, Pyrazinyl, Pyrazolyl, Pyridyl, Pyridazinyl, Pyrimidyl, Pyrrolyl, Chinolinyl, Tetralinyl, Tetrazolyl, Thiazolyl, Thienyl oder Triazolyl bedeutet, die jeweils unsubstituiert oder mit 1 bis 3 Substituenten substituiert sind, die aus Amino, Acetyl, Alkyl (geradkettiges oder verzweigtes mit 1 bis 6 Kohlenstoffatomen), Alkoxy, Cyano, Halogen, Hydroxy, Nitro, Phenyl, Pyridyl, Pyrrolyl, Isoxazolyl, Phenoxy, Tolyloxy, $-CF_3$, $-OCF_3$, $-SO_2CF_3$, $-NHCONH_2$, $-CO_2H$, $-CH_2CO_2H$, $-CH_2CN$, $SO_2Me$, $SO_2NH_2$, $SO_2Ph$, $-(CH_2)_qNR^7R^8$, $-CONR^9R^{10}$ und $CO_2R^{11}$, worin q 0, 1 oder 2 ist und $R^7$, $R^8$, $R^9$, $R^{10}$, $R^{11}$ jeweils unabhängig voneinander aus Wasserstoff oder geradem oder verzweigtem Alkyl mit bis zu 6 Kohlenstoffatomen oder cyclischem Alkyl mit zwischen 5 und 7 Atomen, das 1 oder 2 Sauerstoff- oder Stickstoffatome enthalten kann, ausgewählt sind oder $R^7$ und $R^8$ oder $R^9$ und $R^{10}$ zusammen mit dem Stickstoffatom, an das sie gebunden sind, einen 5- bis 7-gliedrigen aliphatischen Ring, der 1 oder 2 Sauerstoff-oder Stickstoffatome enthalten kann, bilden können, ausgewählt sind;
$Ar^1$ in unabhängiger Weise aus Ar ausgewählt ist und auch Pyridyl-N-oxid sein kann;
$R^1$ Wasserstoff oder gerades oder verzweigtes Alkyl mit bis zu 6 Kohlenstoffatomen oder cyclisches Alkyl mit zwischen 5 und 7 Atomen, das 1 oder 2 Sauerstoff- oder Stickstoffatome enthalten kann, bedeutet;
$R^2$ in unabhängiger Weise aus Ar ausgewählt ist oder Wasserstoff, Hydroxy, Alkoxy, $-NMe_2$, $-CONR^{12}R^{13}$,

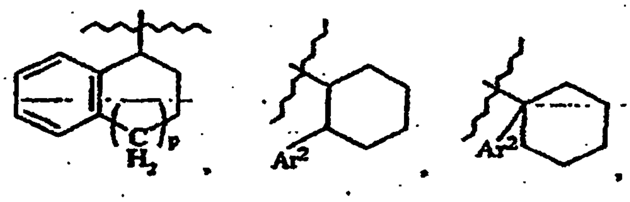

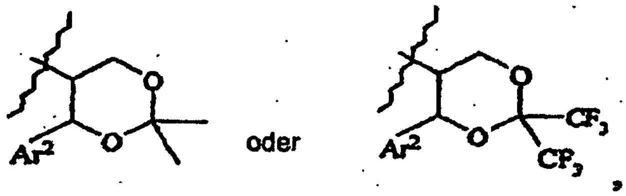

worin p 0, 1 oder 2 ist, $Ar^2$ Phenyl oder Pyridyl bedeutet; und $R^{12}$ und $R^{13}$ jeweils unabhängig voneinander aus Wasserstoff, geradem oder verzweigtem Alkyl mit bis zu 6 Kohlenstoffatomen oder cyclischem Alkyl mit zwischen 5 und 7 Kohlenstoffatomen ausgewählt sind, bedeutet;
$R^3$, $R^4$ und $R^5$ jeweils unabhängig voneinander aus Wasserstoff und Niederalkyl ausgewählt sind; und
$R^6$ Wasserstoff, Methyl bedeutet oder mit $R^1$ einen Ring mit 3 bis 7 Kohlenstoffatomen, der ein Sauerstoff- oder Stickstoffatom enthalten kann, bildet oder $R^1$ und $R^6$ zusammen Carbonyl sein können.

**30.** Verwendung nach einem der Ansprüche 1-3, wobei der Bombesin-Rezeptorantagonist eine Verbindung der Formel (III) ist, worin:

k 0 oder 1 ist;
l 1 ist;
m 0 oder 1 ist;
n 0 oder 1 ist;
X -C(O)-, -OC(O)- oder $-SO_2-$ bedeutet;
Ar Benzofuryl, Furyl, Indolyl, Isochinolyl, Naphthyl, Phenyl, Pyridyl, Chinolyl oder Thienyl bedeutet, die jeweils unsubstituiert oder mit 1 oder 2 Substituenten substituiert sind, die aus Alkoxy, Cyano, Halogen, Nitro, Phenyl, Phenoxy, $-CF_3$, $-(CH_2)_qNR^7R^8$, wobei $R^7$ und $R^8$ einen Ring mit zwischen 5 und 7 Atomen, der 1 oder 2 Sauerstoff- oder Stickstoffatome enthalten kann, bilden können oder $R^7$ und $R^8$ unabhängig voneinander aus Wasserstoff, geradem oder verzweigtem Alkyl mit bis zu 4 Kohlenstoffatomen oder cyclischem Alkyl mit 5 Kohlenstoffatomen ausgewählt sein können, ausgewählt sind;
$Ar^1$ in unabhängiger Weise aus Ar, vorzugsweise Indolyl, ausgewählt ist, und auch Pyridyl-N-oxid sein kann;
$R^1$ und $R^6$ ein cyclisches Alkyl mit 5 bis 7 Kohlenstoffatomen bilden können oder $R^1$ und $R^6$ zusammen Carbonyl sind;
$R^2$ in unabhängiger Weise aus unsubstituiertem oder substituiertem Pyridyl ausgewählt ist oder Wasserstoff, Hydroxy, Alkoxy, $-NMe_2$, $-CONR^{12}R^{13}$, wobei $R^{12}$ und $R^{13}$ jeweils unabhängig voneinander aus H und $CH_3$ ausgewählt sind, bedeutet;
$R^3$, $R^4$ und $R^5$ jeweils unabhängig voneinander aus Wasserstoff und Methyl ausgewählt sind.

**31.** Verwendung nach einem der Ansprüche 1-3, wobei der Bombesin-Rezeptorantagonist eine Verbindung der Formel (III) ist, worin

l 1 ist;
m 1 ist;
n 0 ist;
$R^2$ 2-Pyridyl bedeutet;
$R^6$ mit $R^1$ ein Cyclohexyl bildet.

**32.** Verwendung nach einem der Ansprüche 1-3, wobei der Bombesin-Rezeptorantagonist eine Verbindung der Formel (IIIa) oder ein Salz derselben ist:

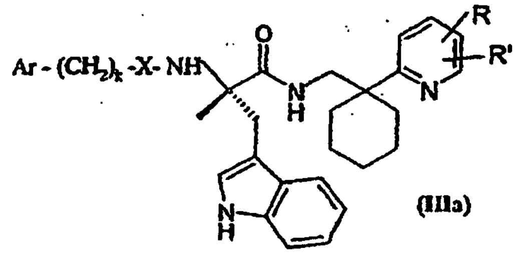

(IIIa)

worin Ar, k und X die oben als erstes angegebenen Bedeutungen besitzen und der Pyridinring optional mit 1 oder 2 Substituenten R und R' substituiert ist, die unabhängig voneinander aus Alkoxy, Cyano, Halogen, Nitro, Phenyl, Phenoxy, $-CF_3$, $-(CH_2)_qNR^7R^8$, wobei $R^7$ und $R^8$ zusammen mit dem Stickstoffatom, an das sie gebunden sind, einen 5- bis 7-gliedrigen aliphatischen Ring, der 1 oder 2 Sauerstoff- oder Stickstoffatome enthalten kann, bilden können, oder $R^7$ und $R^8$ unabhängig voneinander aus Wasserstoff oder cyclischem Alkyl mit zwischen 5 und 7 Kohlenstoffatomen ausgewählt sind, ausgewählt sind, und die pharmazeutisch akzeptablen Salze derselben.

**33.** Verwendung nach einem der Ansprüche 1-3, wobei der Bombesin-Rezeptorantagonist eine der im folgenden angegebenen Verbindungen oder ein Salz derselben ist:

N-{(S)-2-(1H-Indol-3-yl)-1-methyl-1-[(1-pyridin-2-yl-cyclohexylmethyl)-carbamoyl]-ethyl}-4-nitro-benzamid;
C-Dimethylamino-N-{(S)-2-(1H-indol-3-yl)-1-methyl-1-[(1-pyridin-2-yl-cyclohexylmethyl)-carbamoyl]-ethyl}-benzamid;
1H-Indol-2-carbonsäure-{(S)-2-(1H-indol-3-yl)-1-methyl-1-[(1-pyridin-2-yl-cyclohexylmethyl)-carbamoyl]-ethyl}-amid;
Benzo[b]thiophen-2-carbonsäure-{(S)-2-(1H-indol-3-yl)-1-methyl-1-[(1-pyridin-2-yl-cyclohexylmethyl)-carbamoyl]-ethyl}-amid;
N-{(S)-2-(1H-Indol-3-yl)-1-methyl-1-[(1-pyridin-2-yl-cyclohexylmethyl)-carbamoyl]-ethyl}-2-pyrrol-1-yl-benzamid;
1H-Indol-5-carbonsäure-{(S)-2-(1H-indol-3-yl)-1-methyl-1-[(1-pyridin-2-yl-cyclohexylmethyl)-carbamoyl]-ethyl}-amid und
1H-Indol-2-carbonsäure-((S)-2-(1H-indol-3-yl)-1-{[1-(5-methoxy-pyridin-2-yl)-cyclohexylmethyl]-carbamoyl}-1-methyl-ethyl)-amid.

**34.** Verwendung nach einem der Ansprüche 1-3, wobei der Bombesin-Rezeptorantagonist eine der im folgenden angegebenen Verbindungen oder ein Salz derselben ist:

N-{(S)-2-(1H-Indol-3-yl)-1-methyl-1-[(1-pyridin-2-yl-cyclohexylmethyl)-carbamoyl]-ethyl}-benzamid;
N-{(S)-2-(1H-Indol-3-yl)-1-methyl-1-[(1-pyridin-2-yl-cyclohexylmethyl)-carbamoyl]-ethyl}-4-methyl-benzamid;
4-Chlor-N-{(S)-2-(1H-indol-3-yl)-1-methyl-1-[(1-pyridin-2-yl-cyclohexylmethyl)-carbamoyl]-ethyl}-benzamid;
N-{(S)-2-(1H-Indol-3-yl)-1-methyl-1-[(1-pyridin-2-yl-cyclohexylmethyl)-carbamoyl]-ethyl}-4-methoxy-benzamid;
N-{(S)-2-(1H-Indol-3-yl)-1-methyl-1-[(1-pyridin-2-yl-cyclohexylmethyl)-carbamoyl]-ethyl}-4-methansulfonyl-benzamid;
3-Cyano-N-{(S)-2-(1H-indol-3-yl)-1-methyl-1-[(1-pyridin-2-yl-cyclohexylmethyl)-carbamoyl]-ethyl}-benzamid;
3-Chlor-N-{(S)-2-(1H-indol-3-yl)-1-methyl-1-[(1-pyridin-2-yl-cyclohexylmethyl)-carbamoyl]-ethyl}-benzamid;
N-{(S)-2-(1H-Indol-3-yl)-1-methyl-1-[(1-pyridin-2-yl-cyclohexylmethyl)-carbamoyl]-ethyl}-3-methoxy-benzamid;
N-{(S)-2-(1H-Indol-3-yl)-1-methyl-1-[(1-pyridin-2-yl-cyclohexylmethyl)-carbamoyl]-ethyl}-3-methansulfonyl-benzamid;
Dimethylamino-N-{(S)-2-(1H-indol-3-yl)-1-methyl-1-[(1-pyridin-2-yl-cyclohexylmethyl)-carbamoyl]-ethyl}-benzamid;
N-{(S)-2-(1H-Indol-3-yl)-1-methyl-1-[(1-pyridin-2-yl-cyclohexylmethyl)-carbamoyl]-ethyl}-3-methyl-benzamid;
2-Chlor-N-{(S)-2-(1H-indol-3-yl)-1-methyl-1-[(1-pyridin-2-yl-cyclohexylmethyl)-carbamoyl]-ethyl}-benzamid;
N-{(S)-2-(1H-Indol-3-yl)-1-methyl-1-[(1-pyridin-2-yl-cyclohexylmethyl)-carbamoyl]-ethyl}-2-nitro-benzamid;
N-{(S)-2-(1H-Indol-3-yl)-1-methyl-1-[(1-pyridin-2-yl-cyclohexylmethyl)-carbamoyl]-ethyl}-2-methoxy-benzamid;

N-{(S)-2-(1H-Indol-3-yl)-1-methyl-1-[(1-pyridin-2-yl*-cyclohexylmethyl)-carbamoyl]-ethyl}-2-methyl-benzamid;
2-Fluor-N-{(S)-2-(1H-indol-3-yl)-1-methyl-1-[(1-pyridin-2-yl-cyclohexylmethyl)-carbamoyl]-ethyl}-benzamid;
(S)-3-(1H-Indol-3-yl)-2-methyl-N-(1-pyridin-2-yl-cyclohexylmethyl)-2-(2-p-tolyl-ethanoylamino)-propionamid;
(S)-3-(1H-Indol-3-yl)-2-methyl-N-(1-pyridin-2-yl-cyclohexylmethyl)-2-(2-o-tolyl-ethanoylamino)-propionamid;
(S)-2-[2-(4-Hydroxy-phenyl)-ethanoylamino]-3-(1H-indol-3-yl)-2-methyl-N-(1-pyridin-2-yl-cyclohexylmethyl)-propionamid;
(S)-2-[2-(3-Hydroxy-phenyl)-ethanoylamino]-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(2-m-tolyl-ethanoylamino)-propionamid;
(S)-2-[2-(2-Fluor-phenyl)-ethanoylamino]-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(2-thiophen-3-yl-ethanoylamino)-propionamid;
Pyridin-2-carbonsäure-{(S)-2-(1H-indol-3-yl)-1-methyl-1-[(1-pyridin-2-yl-cyclohexylmethyl)-carbamoyl]-ethyl}-amid;
N-{(S)-2-(1H-Indol-3-yl)-1-methyl-1-[(1-pyridin-2-yl-cyclohexylmethyl)-carbamoyl]-ethyl}-isonicotinamid;
Furan-3-carbonsäure-{(S)-2-(1H-indol-3-yl)-1-methyl-1-[(1-pyridin-2-yl-cyclohexylmethyl)-carbamoyl]-ethyl}-amid;
Furan-2-carbonsäure-{(S)-2-(1H-indol-3-yl)-1-methyl-1-[(1-pyridin-2-yl-cyclohexylmethyl)-carbamoyl]-ethyl}-amid;
5-Methyl-isoxazol-3-carbonsäure-{(S)-2-(1H-indol-3-yl)-1-methyl-1-[(1-pyridin-2-yl-cyclohexylmethyl)-carbamoyl]-ethyl}-amid;
1-Methyl-1H-pyrrol-2-carbonsäure-{(S)-2-(1H-indol-3-yl)-1-methyl-1-[(1-pyridin-2-yl-cyclohexylmethyl)-carbamoyl]-ethyl}-amid;
Thiophen-2-carbonsäure-{(S)-2-(1H-indol-3-yl)-1-methyl-1-[(1-pyridin-2-yl-cyclohexylmethyl)-carbamoyl]-ethyl}-amid;
Thiophen-3-carbonsäure-{(S)-2-(1H-indol-3-yl)-1-methyl-1-[(1-pyridin-2-yl-cyclohexylmethyl)-carbamoyl]-ethyl}-amid;
1H-Indol-6-carbonsäure-{(S)-2-(1H-indol-3-yl)-1-methyl-1-[(1-pyridin-2-yl-cyclohexylmethyl)-carbamoyl]-ethyl}-amid;
1H-Indol-5-carbonsäure-{(S)-2-(1H-indol-3-yl)-1-methyl-1-[(1-pyridin-2-yl-cyclohexylmethyl)-carbamoyl]-ethyl}-amid;
1H-Indol-4-carbonsäure-{(S)-2-(1H-indol-3-yl)-1-methyl-1-[(1-pyridin-2-yl-cyclohexylmethyl)-carbamoyl]-ethyl}-amid;
1H-Indol-7-carbonsäure-{(S)-2-(1H-indol-3-yl)-1-methyl-1-[(1-pyridin-2-yl-cyclohexylmethyl)-carbamoyl]-ethyl}-amid;
1-Methyl-1H-indol-2-carbonsäure-{(S)-2-(1H-indol-3-yl)-1-methyl-1-[(1-pyridin-2-yl-cyclohexylmethyl)-carbamoyl]-ethyl}-amid;
Benzothiazol-6-carbonsäure-{(S)-2-(1H-indol-3-yl)-1-methyl-1-[(1-pyridin-2-yl-cyclohexylmethyl)-carbamoyl]-ethyl}-amid;
1H-Benzotriazol-5-carbonsäure-{(S)-2-(1H-indol-3-yl)-1-methyl-1-[(1-pyridin-2-yl-cyclohexylmethyl)-carbamoyl]-ethyl}-amid;
3-Methyl-thiophen-2-carbonsäure-{(S)-2-(1H-indol-3-yl)-1-methyl-1-[(1-pyridin-2-yl-cyclohexylmethyl)-carbamoyl]-ethyl}-amid;
5-Methyl-thiophen-2-carbonsäure-{(S)-2-(1H-indol-3-yl)-1-methyl-1-[(1-pyridin-2-yl-cyclohexylmethyl)-carbamoyl]-ethyl}-amid;
6-Methyl-pyridin-2-carbonsäure-{(S)-2-(1H-indol-3-yl)-1-methyl-1-[(1-pyridin-2-yl-cyclohexylmethyl)-carbamoyl]-ethyl}-amid;
Isochinolin-3-carbonsäure-{(S)-2-(1H-indol-3-yl)-1-methyl-1-[(1-pyridin-2-yl-cyclohexylmethyl)-carbamoyl]-ethyl}-amid;
Chinoxalin-2-carbonsäure-{(S)-2-(1H-indol-3-yl)-1-methyl-1-[(1-pyridin-2-yl-cyclohexylmethyl)-carbamoyl]-ethyl}-amid;
Chinolin-8-carbonsäure-{(S)-2-(1H-indol-3-yl)-1-methyl-1-[(1-pyridin-2-yl-cyclohexylmethyl)-carbamoyl]-ethyl}-amid;
5-Phenyl-oxazol-4-carbonsäure-{(S)-2-(1H-indol-3-yl)-1-methyl-1-[(1-pyridin-2-yl-cyclohexylmethyl)-carbamoyl]-ethyl}-amid;
(S)-3-(1H-Indol-3-yl)-2-[2-(4-methoxy-phenyl)-ethanoylamino]-2-methyl-N-(1-pyridin-2-yl-cyclohexylmethyl)-propionamid;
(S)-2-[2-(4-Dimethylamino-phenyl)-ethanoylamino]-3-(1H-indol-3-yl)-2-methyl-N-(1-pyridin-2-yl-cyclohexylmethyl)-propionamid;
(S)-3-(1H-Indol-3-yl)-2-methyl-2-[2-(2-nitro-phenyl)-ethanoylamino]-N-(1-pyridin-2-yl-cyclohexylmethyl)-propionamid;
(S)-3-(1H-Indol-3-yl)-2-[2-(2-methoxy-phenyl)-ethanoylamino]-2-methyl-N-(1-pyridin-2-yl-cyclohexylmethyl)-propionamid und
N-{(S)-2-(1H-Indol-3-yl)-1-methyl-1-[(1-pyridin-2-yl-cyclohexylmethyl)-carbamoyl]-ethyl}-2-pyrrol-1-yl-benzamid.

**35.** Verwendung nach einem der Ansprüche 1-3, wobei der Bombesin-Rezeptorantagonist eine der im folgenden angegebenen Verbindungen oder ein Salz derselben ist:

{(S)-2-(1H-Indol-3-yl)-1-methyl-1-[(1-pyridin-2-yl-cyclohexylmethyl)-carbamoyl]-ethyl}-carbaminsäure-naphthalin-1-ylmethylester;
{(S)-2-(1H-Indol-3-yl)-1-methyl-1-[(1-pyridin-2-yl-cyclohexylmethyl)-carbamoyl]-ethyl}-carbaminsäure-3,4-dichlorbenzylester;
{(S)-2-(1H-Indol-3-yl)-1-methyl-1-[(1-pyridin-2-yl-cyclohexylmethyl)-carbamoyl]-ethyl}-carbaminsäure-3-nitrobenzylester;
{(S)-2-(1H-Indol-3-yl)-1-methyl-1-[(1-pyridin-2-yl-cyclohexylmethyl)-carbamoyl]-ethyl}-carbaminsäure-3-trifluormethyl-benzylester;
{(S)-2-(1H-Indol-3-yl)-1-methyl-1-[(1-pyridin-2-yl-cyclohexylmethyl)-carbamoyl]-ethyl}-carbaminsäure-chinolin-6-ylmethylester;
{(S)-2-(1H-Indol-3-yl)-1-methyl-1-[(1-pyridin-2-yl-cyclohexylmethyl)-carbamoyl]-ethyl}-carbaminsäure-4-nitro-benzylester und
{(S)-2-(1H-Indol-3-yl)-1-methyl-1-[(1-pyridin-2-yl-cyclohexylmethyl)-carbamoyl]-ethyl}-carbaminsäure-3-cyano-benzylester.

**36.** Verwendung nach einem der Ansprüche 1-3, wobei der Bombesin-Rezeptorantagonist eine der im folgenden angegebenen Verbindungen oder ein Salz derselben ist:

{(S)-2-(1H-Indol-3-yl)-1-methyl-1-[(1-pyridin-2-yl-cyclohexylmethyl)-carbamoyl]-ethyl}-carbaminsäure-3,4-dimethoxy-benzylester;
{(S)-2-(1H-Indol-3-yl)-1-methyl-1-[(1-pyridin-2-yl-cyclohexylmethyl)-carbamoyl]-ethyl}-carbaminsäure-naphthalin-2-ylmethylester;
{(S)-2-(1H-Indol-3-yl)-1-methyl-1-[(1-pyridin-2-yl-cyclohexylmethyl)-carbamoyl]-ethyl}-carbaminsäure-indan-2-ylester;
{(S)-2-(1H-Indol-3-yl)-1-methyl-1-[(1-pyridin-2-yl-cyclohexylmethyl)-carbamoyl]-ethyl}-carbaminsäure-4-methoxy-benzylester;
{(S)-2-(1H-Indol-3-yl)-1-methyl-1-[(1-pyridin-2-yl-cyclohexylmethyl)-carbamoyl]-ethyl}-carbaminsäure-4-chlor-benzylester;
{(S)-2-(1H-Indol-3-yl)-1-methyl-1-[(1-pyridin-2-yl-cyclohexylmethyl)-carbamoyl]-ethyl}-carbaminsäure-2-fluor-benzylester;
{(S)-2-(1H-Indol-3-yl)-1-methyl-1-[(1-pyridin-2-yl-cyclohexylmethyl)-carbamoyl]-ethyl}-carbaminsäure-2-chlor-benzylester;
{(S)-2-(1H-Indol-3-yl)-1-methyl-1-[(1-pyridin-2-yl-cyclohexylmethyl)-carbamoyl]-ethyl}-carbaminsäure-2-methyl-benzylester;
{(S)-2-(1H-Indol-3-yl)-1-methyl-1-[(1-pyridin-2-yl-cyclohexylmethyl)-carbamoyl]-ethyl}-carbaminsäure-4-tert-butyl-benzylester;
{(S)-2-(1H-Indol-3-yl)-1-methyl-1-[(1-pyridin-2-yl-cyclohexylmethyl)-carbamoyl]-ethyl}-carbaminsäure-2-methoxy-benzylester;
{(S)-2-(1H-Indol-3-yl)-1-methyl-1-[(1-pyridin-2-yl-cyclohexylmethyl)-carbamoyl]-ethyl}-carbaminsäure-4-trifluormethyl-benzylester;
{(S)-2-(1H-Indol-3-yl)-1-methyl-1-[(1-pyridin-2-yl-cyclohexylmethyl)-carbamoyl]-ethyl}-carbaminsäure-3-ethoxy-benzylester;
{(S)-2-(1H-Indol-3-yl)-1-methyl-1-[(1-pyridin-2-yl-cyclohexylmethyl)-carbamoyl]-ethyl}-carbaminsäure-2,4-dichlor-benzylester;
{(S)-2-(1H-Indol-3-yl)-1-methyl-1-[(1-pyridin-2-yl-cyclohexylmethyl)-carbamoyl]-ethyl}-carbaminsäure-3-methyl-benzylester;
{(S)-2-(1H-Indol-3-yl)-1-methyl-1-[(1-pyridin-2-yl-cyclohexylmethyl)-carbamoyl]-ethyl}-carbaminsäure-3-phenoxy-benzylester und
{(S)-2-(1H-Indol-3-yl)-1-methyl-1-[(1-pyridin-2-yl-cyclohexylmethyl)-carbamoyl]-ethyl}-carbaminsäure-4-methyl-benzylester.

**37.** Verwendung nach einem der Ansprüche 1-3, wobei der Bombesin-Rezeptorantagonist eine der im folgenden angegebenen Verbindungen oder ein Salz derselben ist:

(S)-3-(1H-Indol-3-yl)-2-methyl-2-phenylmethansulfonylamino-N-(1-pyridin-2-yl-cyclohexylmethyl)-propionamid;
(S)-2-(2-Chlor-benzoylsulfonylamino)-3-(1H-indol-3-yl)-2-methyl-N-(1-pyridin-2-yl-cyclohexylmethyl)-propionamid;
(S)-3-(1H-Indol-3-yl)-2-methyl-2-(naphthalin-1-sulfonylamino)-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(chinolin-8-sulfonylamino)-propionamid;
(S)-3-(1H-Indol-3-yl)-2-methyl-N-(1-pyridin-2-yl-cyclohexylmethyl)-2-(2-trifluormethyl-benzolsulfonylamino)-propionamid;
(S)-2-(Biphenyl-2-sulfonylamino)-3-(1H-indol-3-yl)-2-methyl-N-(1-pyridin-2-yl-cyclohexylmethyl)-propionamid;
(S)-3-(1H-Indol-3-yl)-2-methyl-2-(5-methyl-2-phenoxy-benzolsulfonylamino)-N-(1-pyridin-2-yl-cyclohexylmethyl)-propionamid und
(S)-3-(1H-Indol-3-yl)-2-methyl-N-(1-pyridin-2-yl-cyclohexylmethyl)-2-(2-p-tolyloxy-benzolsulfonylamino)-propionamid.

**38.** Verwendung nach einem der Ansprüche 1-3, wobei der Bombesin-Rezeptorantagonist eine der im folgenden angegebenen Verbindungen oder ein Salz derselben ist:

(S)-3-(1H-Indol-3-yl)-2-methyl-N-(1-pyridin-2-yl-cyclohexylmethyl)-2-(toluol-4-sulfonylamino)-propionamid;
(S)-3-(1H-Indol-3-yl)-2-methansulfonylamino-2-methyl-N-(1-pyridin-2-yl-cyclohexylmethyl)-propionamid;
(S)-2-(2-Fluor-benzolsulfonylamino)-3-(1H-indol-3-yl)-2-methyl-N-(1-pyridin-2-yl-cyclohexylmethyl)-propionamid;
(S)-2-(4-Chlor-benzolsulfonylamino)-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(2,2,2-trifluor-ethansulfonylamino)-propionamid;
(S)-2-(5-Dimethylamino-naphthalin-1-sulfonylamino)-3-(1H-indol-3-yl)-2-methyl-N-(1-pyridin-2-yl-cyclohexylmethyl)-propionamid;
(S)-3-(1H-Indol-3-yl)-2-methyl-2-(naphthalin-2-sulfonylamino)-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(thiophen-2-sulfonylamino)-propionamid;
(S)-3-(1H-Indol-3-yl)-2-methyl-2-(3-nitro-benzolsulfonylamino)-N-(1-pyridin-2-yl-cyclohexylmethyl)-propionamid;
(S)-2-(4-Fluor-benzolsulfonylamino)-3-(1H-indol-3-yl)-2-methyl-N-(1-pyridin-2-yl-cyclohexylmethyl)-propionamid;
(S)-3-(1H-Indol-3-yl)-2-methyl-2-(4-nitro-benzolsulfonylamino)-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(3-trifluormethyl-benzolsulfonylamino)-propionamid;
(S)-2-(3,4-Dichlor-benzolsulfonylamino)-3-(1H-indol-3-yl)-2-methyl-N-(1-pyridin-2-yl-cyclohexylmethyl)-propionamid;
(S)-2-(3-Fluor-benzolsulfonylamino)-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(4-trifluormethyl-benzolsulfonylamino)-propionamid;
(S)-2-(5-Chlor-thiophen-2-sulfonylamino)-3-(1H-indol-3-yl)-2-methyl-N-(1-pyridin-2-yl-cyclohexylmethyl)-propionamid;

(S)-2-(3-Chlor-benzol-sulfonylamino)-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(toluol-3-sulfonylamino)-propionamid;
(S)-2-(3,4-Dimethoxy-benzolsulfonylamino)-3-(1H-indol-3-yl)-2-methyl-N-(1-pyridin-2-yl-cyclohexylmethyl)-propionamid;
(S)-2-(4-Cyano-benzolsulfonylamino)-3-(1H-indol-3-yl)-2-methyl-N-(1-pyridin-2-yl-cyclohexylmethyl)-propionamid;
(S)-2-(2-Cyano-benzolsulfonylamino)-3-(1H-indol-3-yl)-2-methyl-N-(1-pyridin-2-yl-cyclohexylmethyl)-propionamid;
(S)-2-(5-Chlor-1,3-dimethyl-1H-pyrazol-4-sulfonylamino)-3-(1H-indol-3-yl)-2-methyl-N-(1-pyridin-2-yl-cyclohexylmethyl)-propionamid;
(S)-2-(3,5-Dimethyl-isoxazol-4-sulfonylamino)-3-(1H-indol-3-yl)-2-methyl-N-(1-pyridin-2-yl-cyclohexylmethyl)-propionamid;
(S)-2-(Benzo[1,2,5]thiadiazol-4-sulfonylamino)-3-(1H-indol-3-yl)-2-methyl-N-(1-pyridin-2-yl-cyclohexylmethyl)-propionamid;
(S)-3-(1H-Indol-3-yl)-2-methyl-2-(1-methyl-1H-imidazol-4-sulfonylamino)-N-(1-pyridin-2-yl-cyclohexylmethyl)-propionamid;
(S)-2-(Benzo[1,2,5]oxadiazol-4-sulfonylamino)-3-(1H-indol-3-yl)-2-methyl-N-(1-pyridin-2-yl-cyclohexylmethyl)-propionamid;
3-{(S)-2-(1H-Indol-3-yl)-1-methyl-1-[(1-pyridin-2-yl-cyclohexylmethyl)-carbamoyl]-ethylsulfamoyl}-thiophen-2-carbonsäuremethylester;
(S)-3-(1H-Indol-3-yl)-2-(5-isoxazol-3-yl-thiophen-2-sulfonylamino)-2-methyl-N-(1-pyridin-2-yl-cyclohexylmethyl)-propionamid;
(S)-3-(1H-Indol-3-yl)-2-methyl-2-(2-nitro-phenylmethansulfonylamino)-N-(1-pyridin-2-yl-cyclohexylmethyl)-propionamid;
(S)-2-(3-Cyano-benzolsulfonylamino)-3-(1H-indol-3-yl)-2-methyl-N-(1-pyridin-2-yl-cyclohexylmethyl)-propionamid;
(S)-2-(1,2-Dimethyl-1H-imidazol-4-sulfonylamino)-3-(1H-indol-3-yl)-2-methyl-N-(1-pyridin-2-yl-cyclohexylmethyl)-propionamid;
(S)-3-(1H-Indol-3-yl)-2-(3-methoxy-benzolsulfonylamino)-2-methyl-N-(1-pyridin-2-yl-cyclohexylmethyl)-propionamid;
(S)-3-(1H-Indol-3-yl)-2-methyl-2-(8-nitro-naphthalin-1-sulfonylamino)-N-(1-pyridin-2-yl-cyclohexylmethyl)-propionamid;
(S)-2-(2-Chlor-5-nitro-benzolsulfonylamino)-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(2,4,6-trichlor-benzolsulfonylamino)-propionamid;
(S)-2-(4-Chlor-2-nitro-benzolsulfonylamino)-3-(1H-indol-3-yl)-2-methyl-N-(1-pyridin-2-yl-cyclohexylmethyl)-propionamid;
(S)-2-(5-Benzolsulfonyl-thiophen-2-sulfonylamino)-3-(1H-indol-3-yl)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(4-trifluormethoxy-benzolsulfonylamino)-propionamid;
2-{(S)-2-(1H-Indol-3-yl)-1-methyl-1-[(1-pyridin-2-yl-cyclohexylmethyl)-carbamoyl]-ethylsulfamoyl}-benzoesäuremethylester;
(S)-2-(3-Chlor-4-fluor-benzolsulfonylamino)-3-(1H-indol-3-yl)-2-methyl-N-(1-pyridin-2-yl-cyclohexylmethyl)-propionamid;
(S)-2-(2,5-Dichlor-thiophen-3-sulfonylamino)-3-(1H-indol-3-yl)-2-methyl-N-(1-pyridin-2-yl-cyclohexylmethyl)-propionamid;
(S)-2-(3-Chlor-4-methyl-benzolsulfonylamino)-3-(1H-indol-3-yl)-2-methyl-N-(1-pyridin-2-yl-cyclohexylmethyl)-propionamid;
(S)-3-(1H-Indol-3-yl)-2-(2-methoxy-4-methyl-benzolsulfonylamino)-2-methyl-N-(1-pyridin-2-yl-cyclohexylmethyl)-propionamid;
(S)-3-(1H-Indol-3-yl)-2-methyl-N-(1-pyridin-2-yl-cyclohexylmethyl)-2-(5-pyridin-2-yl-thiophen-2-sulfonylamino)-propionamid;
(S)-2-(5-Brom-6-chlor-pyridin-3-sulfonylamino)-3-(1H-indol-3-yl)-2-methyl-N-(1-pyridin-2-yl-cyclohexylmethyl)-propionamid;
(S)-2-(2,4-Dinitro-benzolsulfonylamino)-3-(1H-indol-3-yl)-2-methyl-N-(1-pyridin-2-yl-cyclohexylmethyl)-propionamid;
(S)-3-(1H-Indol-3-yl)-2-(4-methansulfonyl-benzolsulfonylamino)-2-methyl-N-(1-pyridin-2-yl-cyclohexylmethyl)-propionamid;
(S)-2-(4-tert-Butyl-benzolsulfonylamino)-3-(1H-indol-3-yl)-2-methyl-N-(1-pyridin-2-yl-cyclohexylmethyl)-propionamid;
(S)-2-(2,4-Dichlor-5-methyl-benzolsulfonylamino)-3-(1H-indol-3-yl)-2-methyl-N-(1-pyridin-2-yl-cyclohexylmethyl)-propionamid;
(S)-2-(2-Chlor-5-trifluormethyl-benzolsulfonylamino)-3-(1H-indol-3-yl)-2-methyl-N-(1-pyridin-2-yl-cyclohexylmethyl)-propionamid;
(S)-3-(1H-Indol-3-yl)-2-methyl-2-(2-nitro-4-trifluormethyl-benzolsulfonylamino)-N-(1-pyridin-2-yl-cyclohexylmethyl)-propionamid und
(S)-2-(4-Butyl-benzolsulfonylamino)-3-(1H-indol-3-yl)-2-methyl-N-(1-pyridin-2-yl-cyclohexylmethyl)-propionamid.

**39.** Verwendung einer pharmazeutischen Kombination (zur gleichzeitigen, getrennten oder aufeinanderfolgenden Verabreichung) von einem Bombesin-Rezeptorantagonisten und einem PDE-5-Inhibitor bei der Herstellung eines Medikaments zur Behandlung oder Prophylaxe von arzneimittelinduzierter sexueller Dysfunktion beim Mann, männlicher erektiler Dysfunktion, arzneimittelinduzierter sexueller Dysfunktion bei der Frau, der weiblichen Störung von hypoaktivem sexuellem Begehren, einer weiblichen Störung der sexuellen Vigilanz, weiblicher Anorgasmie oder weiblichen sexuellen Schmerzstörungen.

**40.** Verwendung einer pharmazeutischen Kombination (zur gleichzeitigen, getrennten oder aufeinanderfolgenden Verabreichung) von einem Bombesin-Rezeptorantagonisten und einem NEP-Inhibitor bei der Herstellung eines Medikaments zur Behandlung oder Prophylaxe von arzneimittelinduzierter sexueller Dysfunktion beim Mann, männlicher erektiler Dysfunktion, arzneimittelinduzierter sexueller Dysfunktion bei der Frau, der weiblichen Störung von hypoaktivem sexuellem Begehren, einer weiblichen Störung der sexuellen Vigilanz, weiblicher Anorgasmie oder weiblichen sexuellen Schmerzstörungen.

**41.** Verwendung einer pharmazeutischen Kombination (zur gleichzeitigen, getrennten oder aufeinanderfolgenden Verabreichung) von einem Bombesin-Rezeptorantagonisten und einem oder mehreren Östrogen-Rezeptormodulatoren (SERM) und/oder Östrogenagonisten und/oder Östrogenantagonisten bei der Herstellung eines Medikaments zur Behandlung oder Prophylaxe von arzneimittelinduzierter sexueller Dysfunktion beim Mann, männlicher erektiler Dysfunktion, arzneimittelinduzierter sexueller Dysfunktion bei der Frau, der weiblichen Störung von hypoaktivem sexuellem Begehren, einer weiblichen Störung der sexuellen Vigilanz, weiblicher Anorgasmie oder weiblichen sexuellen Schmerzstörungen.

**42.** Verwendung einer pharmazeutischen Kombination (zur gleichzeitigen, getrennten oder aufeinanderfolgenden Verabreichung) von einem Bombesin-Rezeptorantagonisten und Lasofloxifen bei der Herstellung eines Medikaments zur Behandlung oder Prophylaxe von arzneimittelinduzierter sexueller Dysfunktion beim Mann, männlicher erektiler Dysfunktion, arzneimittelinduzierter sexueller Dysfunktion bei der Frau, der weiblichen Störung von hypoaktivem sexuellem Begehren, einer weiblichen Störung der sexuellen Vigilanz, weiblicher Anorgasmie oder weiblichen sexuellen Schmerzstörungen.

## Revendications

**1.** Utilisation, dans la préparation d'un médicament pour le traitement ou la prophylaxie d'une dysfonction sexuelle induite par un médicament chez l'homme, de la dysfonction érectile masculine, d'une dysfonction sexuelle induite par un médicament chez la femme, d'un trouble du désir sexuel hypoactif chez la femme, d'un trouble de l'excitation sexuelle chez la femme, de l'anorgasmie féminine ou de troubles douloureux sexuels féminins, d'une composition pharmaceutique (pour l'administration simultanée, séparée ou successive) d'un antagoniste de récepteurs de la bombésine et d'un ou plusieurs matériaux sélectionnés parmi de (1) à (32) ci-dessous :

1) des prostaglandines d'origine naturelle ou synthétique, ou des esters de celles-ci ;
2) des composés antagonistes de récepteur α-adrénergique, également connus sous le nom d'antagonistes d'α-adrénocepteur ou d'antagonistes d'α-récepteur ou d'α-bloquant ;
3) des composés donneurs de NO (agonistes de NO) ;
4) des ouvreurs ou modulateurs de canaux potassiques ;

5) des agents dopaminergiques ;
6) la nimodépine, le pinacidil, le cyclandélate, l'isoxsuprine, la chloropromazine, l'halopéridol, le Rec 15/2739, la trazodone, l'alprostadil ou la phentolamine ;
7) les agonistes de la thromboxane A2 ;
8) les alcaloïdes de l'ergot ;
9) les antagonistes de récepteur de l'angiotensine tels que le losartan ;
10) les substrats pour la NO-synthase ;
11) les bloqueurs de canaux calciques ;
12) les agents abaissant le taux de cholestérol ;
13) les agents antiplaquettes et antithrombotiques ;
14) les agents sensibilisant à l'insuline et les agents hypoglycémiants ;
15) la L-DOPA ou la carbidopa ;
16) les inhibiteurs de l'acétylcholinestérase ;
17) les agents anti-inflammatoires stéroïdiens ou non-stéroïdiens ;
18) les modulateurs de récepteur d'oestrogènes et/ou les agonistes d'oestrogènes et/ou les antagonistes d'oestrogènes, et leurs sels pharmaceutiquement acceptables ;
19) les inhibiteurs de la PDE ;
20) les inhibiteurs du NPY (neuropeptide Y) ;
21) les inhibiteurs du NEP ;
22) les protéines intestinales vasoactives (VIP), les VIP-mimétiques, les VIP-analogues agissant via un ou plusieurs sous-type(s) de récepteur VIP VPAC1, VPAC2 ou PACAP (peptide activant l'adénylate cyclase pituitaire), le Ro-125-1553 analogue aux VIP, les agonistes de récepteur VIP ou les fragments de VIP, les combinaisons d'antagonistes d'$\alpha$-adrénocepteurs et de VIP ;
23) des agonistes ou modulateurs de récepteur de la mélanocortine ou des stimulants de la mélanocortine ;
24) des agonistes, antagonistes ou modulateurs de récepteur de la sérotonine ;
25) des agents de remplacement de la testostérone, la testostérnone, la déhydrotestéstérone ou un implant de testostérone ;
26) les oestrogènes, oestrogènes et médroxyprogestérone ou acétate de médroxyprogestérone (MPA) (c'est-à-dire sous la forme d'une combinaison), ou les agents de thérapie de remplacement des oestrogènes et de l'hormone méthyltestostérone ;
27) des modificateurs du métabolisme ou de la capture de monoamines qui inhibent le métabolisme ou la recapture de catécholamines ;
28) les agonistes et/ou modulateurs de récepteur purinergique ;
29) les antagonistes de récepteur de neurokinine (NK) ;
30) les agonistes, les antagonistes ou les modulateurs de récepteur d'opiacés ;
31) les agonistes ou modulateurs de récepteur d'oxytocine/vasopressine ; et
32) les modulateurs de récepteur de cannabinoïdes.

**2.** Utilisation selon la revendication 1, dans laquelle le médicament est pour le traitement de la dysfonction sexuelle masculine induite par un antidépresseur.

**3.** Utilisation selon la revendication 1, dans laquelle le médicament est pour le traitement de la dysfonction sexuelle féminine induite par un antidépresseur.

**4.** Utilisation selon la revendication 1, 2 ou 3, dans laquelle l'antagoniste de récepteurs de la bombésine est un antagoniste sélectif de la bombésine BB1.

**5.** Utilisation selon la revendication 4, dans laquelle l'antagoniste de la bombésine BB1 a une sélectivité vis-à-vis de la BB1, par rapport aux autres sous-types de récepteurs de la bombésine, supérieure à 10.

**6.** Utilisation selon la revendication 4, dans laquelle l'antagoniste de la bombésine BB1 a une sélectivité vis-à-vis de la BB1, par rapport aux autres sous-types de récepteurs de la bombésine, supérieure à 30.

**7.** Utilisation selon la revendication 4, dans laquelle l'antagoniste de la bombésine BB1 a une sélectivité vis-à-vis de la BB1, par rapport aux autres sous-types de récepteurs de la bombésine, supérieure à 100.

**8.** Utilisation selon l'une quelconque des revendications 4-7, dans laquelle l'antagoniste de récepteurs de la bombésine a un Ki vis-à-vis de la BB1 qui est inférieur à 1000 nM.

**9.** Utilisation selon l'une quelconque des revendications 4-7, dans laquelle l'antagoniste de récepteurs de la bombésine a un Ki vis-à-vis de la BB1 qui est inférieur à 500 nM.

**10.** Utilisation selon l'une quelconque des revendications 4-7, dans laquelle l'antagoniste de récepteurs de la bombésine a un Ki vis-à-vis de la BB1 qui est inférieur à 100 nM.

**11.** Utilisation selon l'une quelconque des revendications 4-7, dans laquelle l'antagoniste de récepteurs de la bombésine a un Ki vis-à-vis de la BB1 qui est inférieur à 50 nM.

**12.** Utilisation selon l'une quelconque des revendications 4-7, dans laquelle l'antagoniste de récepteurs de la bombésine a un Ki vis-à-vis de la BB1 qui est inférieur à 10 nM.

**13.** Utilisation selon l'une quelconque des revendications 1-3, dans laquelle l'antagoniste de récepteurs de la bombésine est un antagoniste de la BB1/BB2 mixte.

**14.** Utilisation selon l'une quelconque des revendications précédentes, dans laquelle le médicament est adapté à une administration orale.

**15.** Utilisation selon l'une quelconque des revendications précédentes, dans laquelle le médicament comprend une quantité efficace d'un antagoniste de récepteurs de la bombésine non-peptidique.

**16.** Utilisation selon la revendication 15, dans laquelle l'antagoniste de récepteurs de la bombésine non-peptidique est un composé qui est absorbable lorsqu'il est administré par voie orale.

**17.** Utilisation selon l'une quelconque des revendications 1-14, dans laquelle le médicament comprend une quantité efficace d'un antagoniste de récepteurs de la bombésine qui est un peptide.

**18.** Utilisation selon l'une quelconque des revendications 1-3, dans laquelle l'antagoniste de récepteurs de la bombésine est un composé de formule (I)

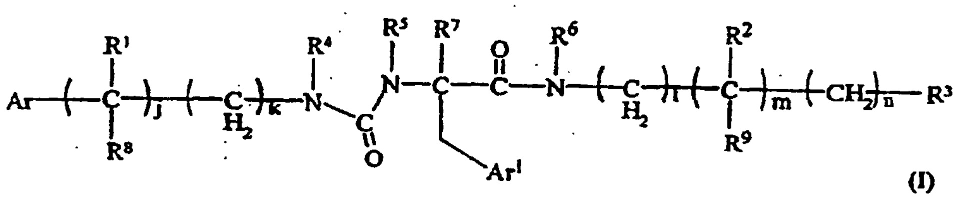

(I)

ou un sel pharmaceutiquement acceptable d'un tel composé, formule dans laquelle :

j représente 0 ou 1 ;
k représente 0 ou 1 ;
l représente 0, 1, 2 ou 3 ;
m représente 0 ou 1 ;
n représente 0, 1 ou 2 ;
Ar représente phényle, pyridyle ou pyrimidyle, dont chacun est non-substitué ou substitué par de 1 à 3 substituants sélectionnés parmi alkyle, halogéno, alcoxy, acétyle, nitro, amino, $-CH_2NR^{10}R^{11}$, cyano, $-CF_3$, $-NHCONH_2$ et - $CO_2R^{12}$ ;
$R^1$ représente l'hydrogène ou un groupe alkyle à chaîne droite, ramifiée ou cyclique ayant de 1 à 7 atomes de carbone ;
$R^8$ représente l'hydrogène ou forme, avec $R^1$, un noyau ayant de 3 à 7 atomes de carbone ;
$R^2$ représente l'hydrogène, ou un groupe alkyle à chaîne droite, ramifiée ou cyclique ayant de 1 à 8 atomes de carbone et qui peut également contenir 1 ou 2 atomes d'oxygène ou d'azote ;
$R^9$ représente l'hydrogène ou forme, avec $R^2$, un noyau ayant de 3 à 7 atomes de carbone, qui peut contenir un atome d'oxygène ou d'azote ; ou $R^2$ et $R^9$ peuvent ensemble représenter un groupe carbonyle ;
$Ar^1$ peut être indépendamment sélectionné parmi Ar et peut également inclure pyridyl-N-oxyde, indolyle, imidazolyle et pyridyle ;

$R^4$, $R^5$, $R^6$ et $R^7$ représentent chacun indépendamment l'hydrogène ou alkyle inférieur ; $R^4$ peut également former avec $R^5$ une liaison covalente ayant 2 ou 3 atomes pouvant inclure un atome d'oxygène ou d'azote ;
$R^3$ peut être indépendamment sélectionné parmi Ar ou représente l'hydrogène, hydroxy, $-NMe_2$, N-méthyl-pyrrolyle, imidazolyle, N-méthyl-imidazolyle, tétrazolyle, N-méthyl-tétrazolyle, thiazolyle, $-CONR^{13}R^{14}$, alcoxy,

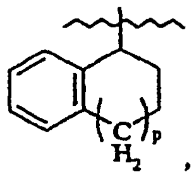,
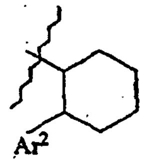,
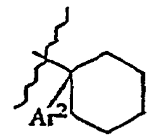,

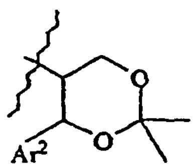, ou
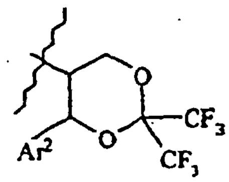,

où p représente 0, 1 ou 2 et $Ar^2$ représente phényle ou pyridyle ;
$R^{10}$, $R^{11}$, $R^{12}$, $R^{13}$ et $R^{14}$ sont chacun indépendamment sélectionnés parmi l'hydrogène ou les groupes alkyles à chaîne droite, ramifiée ou cyclique ayant de 1 à 7 atomes de carbone.

**19.** Utilisation selon l'une quelconque des revendications 1-3, dans laquelle l'antagoniste de récepteurs de la bombésine est un composé de Formule (Ia)

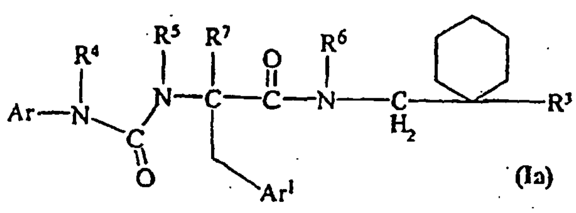
(Ia)

dans laquelle

Ar représente phényle non-substitué ou substitué par 1 ou 2 substituants sélectionnés parmi isopropyle, halogéno, nitro et cyano ;
$R^4$, $R^5$ et $R^6$ représentent l'hydrogène ;
$R^7$ représente méthyle ou hydrogène ;
$R^3$ représente 2-pyridyle ou hydroxy ; et
$Ar^1$ représente indolyle, pyridyle, pyridyl-N-oxyde ou imidazolyle.

**20.** Utilisation selon la revendication 18, dans laquelle l'antagoniste de récepteurs de la bombésine est un composé de Formule I dans laquelle

Ar représente phényle non-substitué ;
$R^1$ représente cyclopentyle ou *tert*-butyle ;
$R^4$ et $R^5$ représentent l'hydrogène ;
$R^7$ représente méthyle ;
$R^6$ représente l'hydrogène ;
$R^3$ est un groupe phényle ayant deux substituants isopropyles, un groupe phényle non-substitué ou

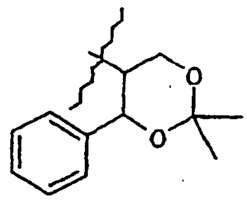 ;

et
$Ar^1$ représente indolyle.

**21.** Utilisation selon la revendication 18, dans laquelle l'antagoniste de récepteurs de la bombésine est un composé de Formule I, dans.laquelle

Ar représente 2,6-diisopropyl-phényle, 4-nitro-phényle et 4-cyano-phényle ;
$R^4$, $R^5$ et $R^6$ représentent l'hydrogène ;
$R^7$ représente méthyle ;
$R^2$ représente l'hydrogène ou cyclohexyle ; et
$R^3$ représente hydroxyle, pyridyle,

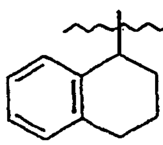 ,
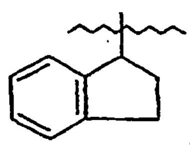 , ou
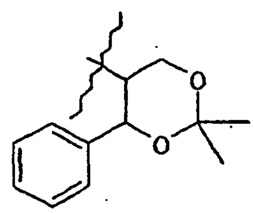

**22.** Utilisation selon l'une quelconque des revendications 1-3, dans laquelle l'antagoniste de récepteurs de la bombésine est le (S)3-(1H-indol-3-yl)-N-[1-(5-méthoxy-pyridin-2-yl)-cyclohexyl-méthyl]-2-méthyl-2-[3-(4-nitro-phényl)-uréido]-propionamide (également appelé Composé 1) ou l'un de ses pharmaceutiquement acceptables, ou est le (25)-*N*-{[1-(4-aminophényl)cyclohexyl]méthyl}-3-(1*H*-indol-3-yl)-2-méthyl-2-{[(4-nitroanilino)carbonyl] amino}propanamide (également appelé Composé 3) ou l'un de ses sels pharmaceutiquement acceptables.

**23.** Utilisation selon l'une quelconque des revendications 1-3, dans laquelle l'antagoniste de récepteurs de la bombésine est un composé indiqué ci-dessous, ou un sel pharmaceutiquement acceptable d'un tel composé :

- le (S)*N*-cyclohexylméthyl-2-[3-(2,6-diisopropyl-phényl)-uréido]-3-(1*H*-indol-3-yl)-2-méthyl-propionamide ;
- le *N*-cyclohexylméthyl-2-[3-(2,6-diisopropyl-phényl)-uréido]-3-(1*H*-indol-3-yl)-*N*-méthyl-propionamide ;
- le *N*-cyclohexylméthyl-2-[3-(2,6-diisopropyl-phényl)-1-méthyl-uréido]-3-(1*H*-indol-3-yl)-propionamide ;
- le 2-[3-(2,6-diisopropyl-phényl)-uréido]-2-méthyl-3-(1-oxy-pyridin-2-yl)-N-(1-pyridin-2-yl-cyclohexylméthyl)-propionamide ;
- le 2-[3-(2,6-diisopropyl-phényl)-uréido]-2-méthyl-3-pyridin-2-yl-*N*-(1-pyridin-2-yl-cyclohexylméthyl)-propionamide ;
- le 2-[3-(2-*tert*-butyl-phényl)-uréido]-*N*-cyclohexylméthyl-3-(1*H*-indol-3-yl)-2-méthyl-propionamide ;
- le *N*-cyclohexylméthyl-2-[3-(2,6-dichloro-phényl)-uréido]-3-(1*H*-indol-3-yl)-2-méthyl-propionamide ;
- le *N*-cyclohexylméthyl-2-[3-(2,6-diméthoxy-phényl)-uréido]-3-(1*H*-indol-3-yl)-2-méthyl-propionamide ;
- le *N*-cyclohexylméthyl-2-[3-(2,6-diméthylamino-phényl)-uréido]-3-(1*H*-indol-3-yl)-2-méthyl-propionamide ;
- le (S)*N*-cyclohexylméthyl-3-(1*H*-indol-3-yl)-2-méthyl-2-[3-(4-nitro-phényl)-uréido]-propionamide ;
- le *N*-cyclohexylméthyl-2-[3-(2,2-diméthyl-1-phényl)propyl)-uréido]-3-(1*H*-indol-3-yl)-2-méthyl-propionamide ;
- le [S-(R*,R*)]3-(1*H*-indol-3-yl)-2-méthyl-2-{3-[1-(4-nitro-phényl)-éthyl]-uréido}-*N*-(1-pyridin-2-yl-cyclohexylméthyl)-propionamide ;
- le *N*-(2,2-diméthyl-4-phényl-[1,3]dioxan-5-yl)-3-(1*H*-indol-3-yl)-2-méthyl-2-[3-(1-phényl-cyclopentylméthyl)-uréido]-propionamide ;
- le (S)-*N*-(2,6-diisopropyl-phényl)-2-[3-(2,2-diméthyl-1-phényl-propyl)-uréido]-3-(1*H*-indol-3-yl)-propionamide ;

- le (R)-*N*-(2,6-diisopropyl-phényl)-2-[3-(2,2-diméthyl-1-phényl-propyl)-uréido]-3-(1*H*-indol-3-yl)-propionamide ;
- le 2-[3-(2,6-diisopropyl-phényl)-uréido]-*N*-(2,2-diméthyl-4-phényl-[1,3]dioxan-5-yl)-3-(1*H*-indol-3-yl)-2-méthyl-propionamide ;
- le *N*-cyclohexyl-2-[3-(2,6-diisopropyl-phényl)-uréido]-3-(1*H*-indol-3-yl)-2-méthyl-propionamide ;
- le *N*-(2-cyclohexyl-éthyl)-2-[3-(2,6-diisopropyl-phényl)-uréido]-3-(1*H*-indol-3-yl)-2-méthyl-propionamide ;
- le 2-[3-(2,6-diisopropyl-phényl)-uréido]-3-(1*H*-indol-3-yl)-2-méthyl-propionamide ;
- le 2-[3-(2,6-diisopropyl-phényl)-uréido]-3-(1*H*-indol-3-yl)-2-méthyl-*N*-(3-méthyl-butyl)-propionamide ;
- le 2-[3-(2,6-diisopropyl-phényl)-uréido]-3-(1*H*-indol-3-*yl)-2-méthyl-N-(3-phényl-propyl)-propionamide ;*
- le 2-[3-(2,6-diisopropyl-phényl)-uréido]-3-(1*H*-indol-3-yl)-2-méthyl-*N*-(1,2,3,4-tétrahydro-naphtalén-1-yl)-propionamide ;
- le 2-[3-(2,6-diisopropyl-phényl)-uréido]-3-(1*H*-indol-3-yl)-2-méthyl-*N*-(2-phényl-cyclohexyl)-propionamide ;
- le 2-[3-(2,6-diisopropyl-phényl)-uréido]-*N*-indan-1-yl-3-(1*H*-indol-3-yl)-2-méthyl-propionamide ;
- le 2-[3-(2,6-diisopropyl-phényl)-uréido]-*N*-(1-hydroxy-cyclohexylméthyl)-3-(1*H*-indol-3-yl)-2-méthyl-propionamide ;
- le 2-[3-(2,6-diisopropyl-phényl)-uréido]-3-(1*H*-indol-3-yl)-2-méthyl-*N*-(1-pyridin-2-yl-cyclohexylméthyl)-propionamide ;
- le 2-[3-(2,6-diisopropyl-phényl)-uréido]-3-(1*H*-indol-3-yl)-2-méthyl-*N*-(6,7,8,9-tétrahydro-5*H*-benzocycloheptén-5-yl)-propionamide ;
- le 2-[3-(2,6-diisopropyl-phényl)-uréido]-3-(1*H*-indol-3-yl)-2-méthyl-*N*-phényl-propionamide ;
- le *N*-(1-hydroxy-cyclohexylméthyl)-3-(1*H*-indol-3-yl)-2-méthyl-2-[3-(4-nitro-phényl)-uréido]-propionamide ;
- le 2-[3-(4-cyano-phényl)-uréido]-3-(1*H*-indol-3-yl)-2-méthyl-*N*-(1-pyridin-2-yl-cyclohexylméthyl)-propionamide ;
- le (S)3-(1*H*-indol-3-yl)-2-méthyl-2-[3-(4-nitro-phényl)-uréido]-*N*-(1-pyridin-2-yl-cyclohexylméthyl)-propionamide ;
- le (S)3-(1*H*-indol-3-yl)-2-méthyl-*N*-(1-pyridin-2-yl-cyclohexylméthyl)-2-[3-(4-trifluorométhyl-phényl)-uréido]-propionamide ;
- l'ester éthylique de l'acide (S)4-(3-{2-(1*H*-indol-3-yl)-1-méthyl-1-[(1-pyridin-2-yl-cyclohexylméthyl)-carbamoyl]-éthyl}-uréido)-benzoïque ;
- le 2-[3-(2,6-diisopropyl-phényl)-uréido]-3-(1*H*-imidazol-4-yl)-*N*-(1-pyridin-2-yl-cyclohexylméthyl)-propionamide ;
- le 2-[3-(2,6-diisopropyl-phényl)-uréido]-2-méthyl-*N*-(1-pyridin-2-yl-cyclohexylméthyl)-3-(2-trifluorométhyl-phényl)-propionamide ;
- le 2-[3-(2,6-diisopropyl-phényl)-uréido]-2-méthyl-3-(2-nitro-phényl)-*N*-(1-pyridin-2-yl-cyclohexylméthyl)-propionamide ;
- le (S) 3-(1*H*-indol-3-yl)-*N*-[1-(5-méthoxy-pyridin-2-yl)-cyclohexylméthyl]-2-méthyl-2-[3-(4-nitro-phényl)-uréido]-propionamide ; et
- le *N*-cyclohexylméthyl-2-[3-(2,6-diisopropyl-phényl)-uréido]-2-méthyl-3-pyridin-2-yl-propionamide.

**24.** Utilisation selon l'une quelconque des revendications 1-3, dans laquelle l'antagoniste de récepteurs de la bombésine est un composé de formule (II) ou un sel pharmaceutiquement acceptable d'un tel composé :

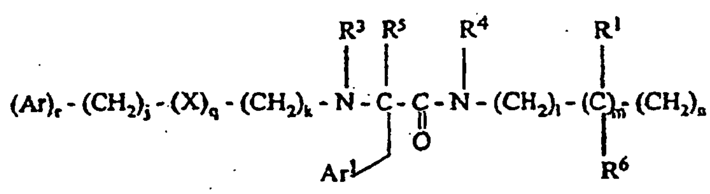

(II)

dans laquelle :

j représente 0, 1 ou 2 ;
k représente 0 ou 1 ;
l représente 0, 1, 2 ou 3 ;
m représente 0 ou 1 ;
n représente 0, 1 ou 2 ;
q représente 0 ou 1 ;
r représente 0 ou 1 ; lorsque r représente 0, Ar est remplacé par un hydrogène ;
Ar représente phényle, pyridyle, pyrimidyle, thiényle, furyle, imidazolyle, pyrrolyle ou thiazolyle, dont chacun est non-substitué ou substitué par de 1 à 3 substituants sélectionnés parmi acétyle, alcoxy, alkyle, amino, cyano, halogéno, hydroxy, nitro, sulfonamido, sulfonyle, $-CF_3$, $-OCF_3$ $-CO_2H$, $-CH_2CN$, $-SO_2CF_3$, $-CH_2CO_2H$

et $-(CH_2)_sNR^7R^8$ où s représente 0, 1, 2 ou 3, et $R^7$ et $R^8$ sont chacun indépendamment sélectionnés parmi H, les groupes alkyles à chaîne droite ou ramifiée ayant jusqu'à 6 atomes de carbone, ou $R^7$ et $R^8$ pris ensemble avec l'atome d'azote auquel ils sont liés peuvent former un noyau aliphatique ayant de 5 à 7 éléments qui peut contenir un ou deux atomes d'oxygène ;

$R^1$ représente l'hydrogène, alkyle à chaîne droite ou ramifiée ayant jusqu'à 6 atomes de carbone ou cycloalkyle ayant de 5 à 7 atomes de carbone qui peut contenir 1 ou 2 atomes d'azote ou d'oxygène ;

$R^6$ représente l'hydrogène, méthyl, ou forme, avec $R^1$, un noyau aliphatique ayant de 3 à 7 atomes qui peut contenir un atome d'oxygène ou d'azote, ou forme, avec $R^1$, un groupe carbonyle ;

$Ar^1$ est indépendamment sélectionné parmi Ar ou est indolyle ou pyridyl-*N*-oxyde ;

$R^3$, $R^4$ et $R^5$ sont chacun indépendamment sélectionnés parmi l'hydrogène et alkyle inférieur ;

$R^2$ est indépendamment sélectionné parmi Ar ou représente l'hydrogène, hydroxy, alcoxy, $-NMe_2$, $-CONR^9R^{10}$ où $R^9$ et $R^{10}$ sont chacun indépendamment sélectionnés parmi l'hydrogène, alkyle à chaîne droite ou ramifié ayant jusqu'à 6 atomes de carbone, ou $R^9$ et $R^{10}$, avec l'atome d'azote auquel ils sont liés, peuvent former un noyau aliphatique ayant de 5 à 7 éléments qui peut contenir 1 ou 2 atomes d'oxygène ou d'azote, ou $R^2$ représente

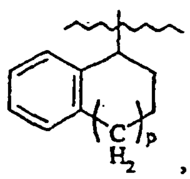 , 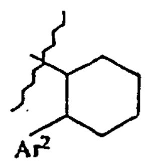 , 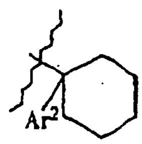 ,

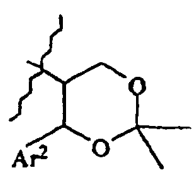 , ou 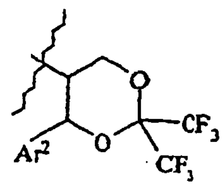

où p représente 0, 1 ou 2 et $Ar^2$ représente phényle ou pyridyle ;

X est un radical divalent dérivant de l'un quelconque des radicaux suivants

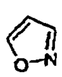 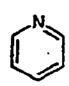 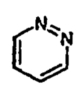 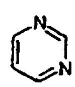 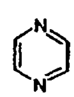 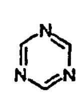

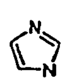 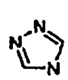 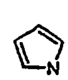 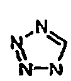 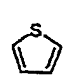 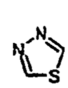

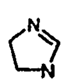 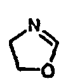 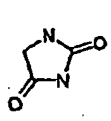 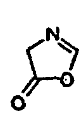 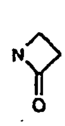 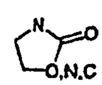

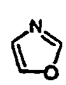 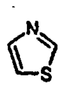 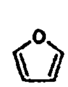 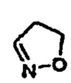 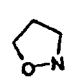 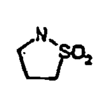

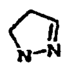 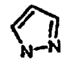 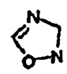 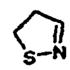 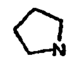 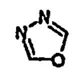

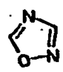 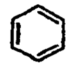 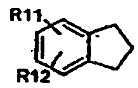 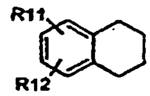 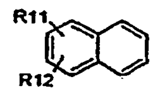

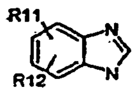 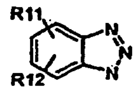 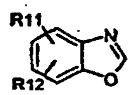 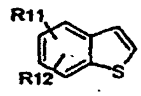 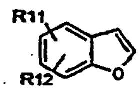

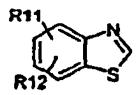 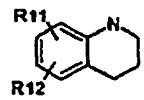 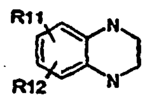 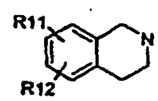

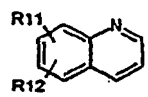 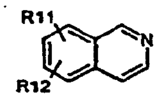

où des groupes alkyles inférieurs peuvent être fixés sur les atomes d'azote de cycle, $R^{11}$ et $R^{12}$ sont indépendamment sélectionnés parmi H, halogéno, hydroxy, alcoxy, acétyle, nitro, cyano, amino, $CF_3$ et $-(CH_2)_tNR^{13}R^{14}$ où t peut représenter 0 ou 1, $R^{13}$ et $R^{14}$ sont indépendamment sélectionnés parmi l'hydrogène, alkyle à chaîne droite ou ramifiée ayant jusqu'à 6 atomes de carbone ou cycloalkyle ayant de 5 à 7 atomes de carbone, contenant jusqu'à 2 atomes d'oxygène ou d'azote.

**25.** Utilisation selon l'une quelconque des revendications 1-3, dans laquelle l'antagoniste de récepteurs de la bombésine est un composé de formule (IIa) ou un sel pharmaceutiquement acceptable d'un tel composé :

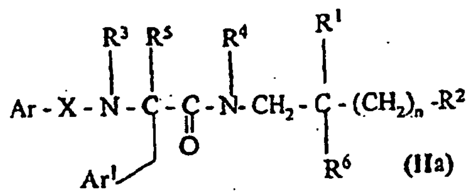

(IIa)

où :

n représente 0 ou 1 ;
Ar représente phényle ou pyridyle qui peut être non-substitué ou substitué par de 1 à 3 substituants sélectionnés parmi halogéno, alcoxy, nitro et cyano ;
$Ar^1$ est indépendamment sélectionné parmi Ar ou est pyridyl-N-oxyde ou indolyle ;
$R^6$ forme, avec $R^1$, un noyau aliphatique ayant de 3 à 7 atomes qui peut contenir un atome d'oxygène ou d'azote, ou forme, avec $R^1$, un groupe carbonyle ;
$R^2$ est indépendamment sélectionné parmi Ar ou représente l'hydrogène, hydroxy, alcoxy, diméthylamino, tétrazolyle ou $-CONR^9R^{10}$, où $R^9$ et $R^{10}$ sont chacun indépendamment sélectionnés parmi l'hydrogène ou méthyle, ou $R^2$ est l'un quelconque des groupes suivants

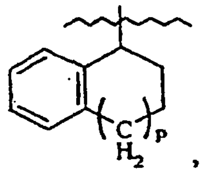 , 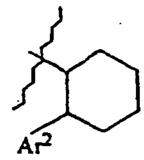 , 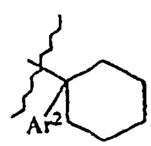 ,

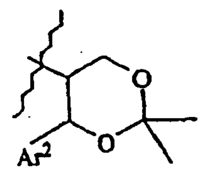 , ou 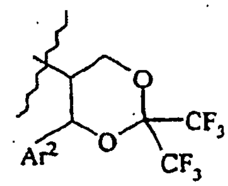

où p représente 0, 1 ou 2 et $Ar^2$ représente phényle ou pyridyle ;
$R^3$, $R^4$ et $R^5$ sont chacun indépendamment sélectionnés parmi l'hydrogène et méthyle ; et
X est sélectionné parmi les groupes :

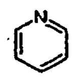 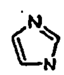 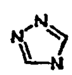 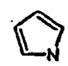 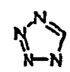 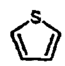

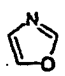 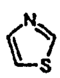 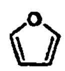 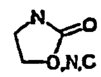 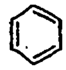 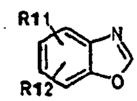

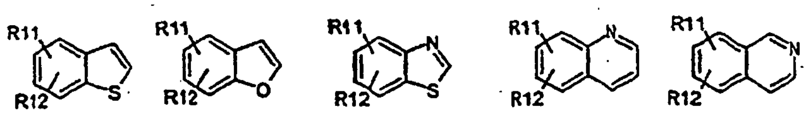

$R^{11}$ et $R^{12}$ étant indépendamment sélectionnés parmi H, halogéno, hydroxy, alcoxy, acétyle, nitro, cyano, amino, $CF_3$ et $(CH_2)_tNR^{13}R^{14}$ où t représente 0 ou 1 et $R^{13}$ et $R^{14}$ sont indépendamment sélectionnés parmi l'hydrogène et méthyle.

**26.** Utilisation selon l'une quelconque des revendications 1-3, dans laquelle l'antagoniste de récepteurs de la bombésine est un composé de formule (IIb) ou (IIc) ou un sel pharmaceutiquement acceptable d'un tel composé :

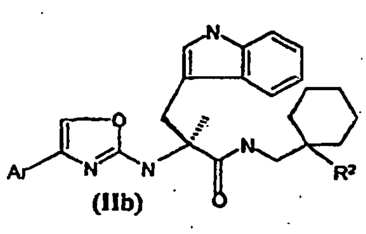
(IIb)

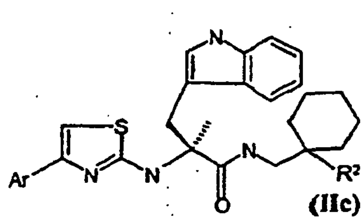
(IIc)

dans laquelle Ar et $R^2$ représentent indépendamment phényle ou pyridyle qui peuvent être non-substitués ou substitués par de 1 à 3 substituants sélectionnés parmi halogéno, alcoxy, nitro et cyano, et les sels pharmaceutiquement acceptables d'un tel composé.

**27.** Utilisation selon l'une quelconque des revendications 1 à 3, dans laquelle l'antagoniste de récepteurs de la bombésine est le (S)-3-(1*H*-indol-3-yl)-*N*-[1-(5-méthoxy-pyridin-2-yl)-cyclohexylméthyl]-2-méthyl-2-[4-(4-nitro-phényl)-oxazol-2-ylamino]-propionamide (également appelé Composé 2) ou un sel pharmaceutiquement acceptable d'un tel composé.

**28.** Utilisation selon l'une quelconque des revendications 1 à 3, dans laquelle l'antagoniste de récepteurs de la bombésine est l'un des composés suivants, ou un sel pharmaceutiquement acceptable d'un tel composé :

- le (S)-3-(1*H*-indol-3-yl)-*N*-(1-méthoxyméthyl-cyclohexylméthyl)-2-méthyl-2-[4-(4-nitro-phényl)-oxazol-2-ylamino]-propionamide ;
- le (S)-3-(1*H*-indol-3-yl)-2-méthyl-2-[4-(4-nitro-phényl)-oxazol-2-ylamino]-*N*-(2-oxo-2-phényl-éthyl)-propionamide ;
- le (S)-*N*-[1-(5-méthoxy-pyridin-2-yl)-cyclohexylméthyl]-2-méthyl-2-[4-(4-nitro-phényl)-oxazol-2-ylamino]-3-phényl-propionamide ;
- le (S)-2-[4-(4-cyano-phényl)-oxazol-2-ylamino]-3-(1*H*-indol-3-yl)-*N*-[1-(5-méthoxy-pyridin-2-yl)-cyclohexylméthyl]-2-méthyl-propionamide ;
- le (S)-3-(1*H*-indol-3-yl)-*N*-[1-(5-méthoxy-pyridin-2-yl)-cyclohexylméthyl]-2-méthyl-2-(4-phényl-oxazol-2-ylamino)-propionamide ;
- le (S)-2-(4-éthyl-oxazol-2-ylamino)-3-(1*H*-indol-3-yl)-*N*-[1-(5-méthoxy-pyridin-2-yl)-cyclohexylméthyl]-2-méthyl-propionamide ;
- le (S)-3-(1*H*-indol-3-yl)-*N*-[1-(5-méthoxy-pyridin-2-yl)-cyclohexylméthyl]-2-méthyl-2-[4-(4-nitro-phényl)-thiazol-2-ylamino)-propionamide ;
- le (S)-2-(benzooxazol-2-ylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-(pyridin-4-ylamino)-*N*-(1-pyridin-2-yl-cyclohexylméthyl)-propionamide ;
- le (S)-3-(1*H*-indol-3-yl)-2-(isoquinol-4-ylamino)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(pyrimidin-5-ylamino)-propionamide ;
- le (S)-2-(biphényl-2-ylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-*m*-tolylamino-propionamide ;
- le (S)-3-(1*H*-indol-3-yl)-2-méthyl-2-(6-phényl-pyridin-2-ylamino)-*N*-(1-pyridin-2-yl-cyclohexylméthyl)-propionamide ;
- le (R)-3-phényl-2-phénylamino-N-(1-pyridin-2-yl-cyclohexylméthyl)-propionamide ;
- le (S)-3-(1*H*-indol-3-yl)-2-méthyl-2-phényléthylamino-*N*-(1-pyridin-2-yl-cyclohexylméthyl)-propionamide ;
- le (S)-2-[(benzofuran-2-ylméthyl)-amino]-3-(1*H*-indol-3-yl)-2-méthyl-*N*-(1-pyridin-2-yl-cyclohexylméthyl)-propionamide ; et
- le (S)-3-(1*H*-indol-3-yl)-2-méthyl-2-(4-nitro-benzylamino)-*N*-(1-pyridin-2-yl-cyclohexylméthyl)-propionamide ;

**29.** Utilisation selon l'une quelconque des revendications 1-3, dans laquelle l'antagoniste de récepteurs de la bombésine est un composé de formule (III), ou un sel pharmaceutiquement acceptable d'un tel composé :

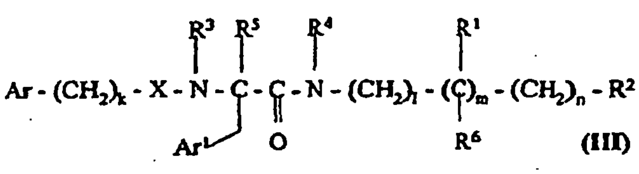

(III)

où :

k représente 0, 1 ou 2 ;
l représente 0, 1, 2 ou 3 ;
m représente 0 ou 1 ;
n représente 0, 1 ou 2 ;
X représente -CO-, -OCO, -SO- et $-SO_2-$ ;
Ar représente benzimidazolyle, benzofuryle, benzothiadiazolyle, benzothiazolyle, benzothiényle, benzopyrazinyle, benzotriazolyle, benzoxadiazolyle, furyle, imidazolyle, indanyle, indolyle, isoquinolyle, isoxazolyle, naphtyle, oxazolyle, phényle, pyrazinyle, pyrazolyle, pyridyle, pyridazinyle, pyrimidyle, pyrrolyle, quinolinyle, tétralinyle, tétrazolyle, thiazolyle, thiényle ou triazolyle, dont chacun est non-substitué ou substitué par de 1 à 3 substituants sélectionnés parmi amino, acétyle, alkyle (à chaîne droite ou ramifiée ayant de 1 à 6 atomes de carbone), alcoxy, cyano, halogéno, hydroxy, nitro, phényle, pyridyle, pyrrolyle, isoxazolyle, phénoxy, tolyloxy, $-CF_3$, $-OCF_3$, $-SO_2CF_3$, $-NHCONH_2$, $-CO_2H$, $-CH_2CO_2H$, $-CH_2CN$, $SO_2Me$, $SO_2NH_2$, $SO_2Ph$,

$-(CH_2)_qNR^7R^8$, $-CONR^9R^{10}$ et $CO_2R^{11}$, où q représente 0, 1 ou 2 et $R^7$, $R^8$, $R^9$, $R^{10}$ et $R^{11}$ sont chacun indépendamment sélectionnés parmi l'hydrogène et alkyle à chaîne droite ou ramifiée ayant jusqu'à 6 atomes de carbone ou alkyle cyclique ayant entre 5 et 7 atomes qui peuvent comporter 1 ou 2 atomes d'oxygène ou d'azote, ou $R^7$ et $R^8$, ou $R^9$ et $R^{10}$, ensemble avec l'atome d'azote auquel ils sont liés, peuvent former un noyau aliphatique ayant de 5 à 7 éléments pouvant contenir 1 ou 2 atomes d'oxygène ou d'azote ;

$Ar^1$ est indépendamment sélectionné parmi Ar et peut être également pyridyl-N-oxyde ;

$R^1$ représente l'hydrogène ou alkyle à chaîne droite ou ramifiée ayant jusqu'à 6 atomes de carbone ou cycloalkyle ayant de 5 à 7 atomes qui peuvent comporter 1 ou 2 atomes d'oxygène ou d'azote ;

$R^2$ est indépendamment sélectionné parmi Ar ou représente l'hydrogène, hydroxy, alcoxy, $-NMe_2$, $-CONR^{12}R^{13}$,

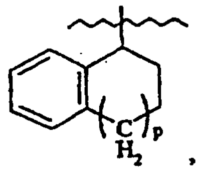 , 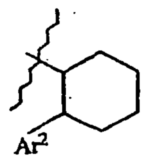 , 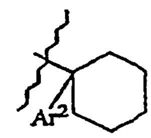 ,

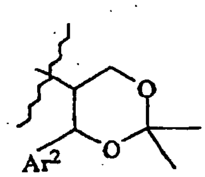 , ou 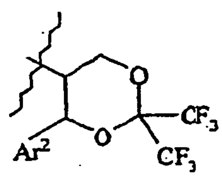 ,

où p représente 0, 1 ou 2, $Ar^2$ représente phényle ou pyridyle ; et $R^{12}$ et $R^{13}$ représentent chacun indépendamment l'hydrogène, alkyle à chaîne droite ou ramifiée ayant jusqu'à 6 atomes de carbone ou cycloalkyle ayant de 5 à 7 atomes de carbone ;

$R^3$, $R^4$ et $R^5$ sont chacun indépendamment sélectionnés parmi l'hydrogène et alkyle inférieur ; et

$R^6$ représente l'hydrogène, méthyle ou forme, avec $R^1$, un noyau ayant de 3 à 7 atomes de carbone qui peut contenir un atome d'oxygène ou d'azote, ou $R^1$ et $R^6$ peuvent former ensemble un groupe carbonyle.

**30.** Utilisation selon l'une quelconque des revendications 1-3, dans laquelle l'antagoniste de récepteurs de la bombésine est un composé de formule (III) dans laquelle :

k représente 0 ou 1 ;
l représente 1 ;
m représente 0 ou 1 ;
n représente 0 ou 1 ;
X représente -C(O)-, -OC(O)- ou $-SO_2-$ ;

Ar représente benzofuryle, furyle, indolyle, isoquinolyle, naphtyle, phényle, pyridyle, quinolyle ou thiényle, dont chacun est non-substitué ou substitué par 1 ou 2 substituants sélectionnés parmi alcoxy, cyano, halogéno, nitro, phényle, phénoxy, $-CF_3$, $-(CH_2)_qNR^7R^8$, où $R^7$ et $R^8$ peuvent former un noyau ayant 5 à 7 atomes qui peuvent comporter 1 ou 2 atomes d'oxygène ou d'azote, ou $R^7$ et $R^8$, peuvent être indépendamment sélectionnés parmi l'hydrogène, alkyle à chaîne droite ou ramifiée ayant jusqu'à 4 atomes de carbone ou cycloalkyle ayant 5 atomes de carbone ;

$Ar^1$ est indépendamment sélectionné parmi Ar, de préférence indolyle, et peut être également pyridyl-N-oxyde ;

$R^1$ et $R^6$ peuvent former un groupe cycloalkyle ayant de 5 à 7 atomes de carbone, ou $R^1$ et $R^6$ peuvent former ensemble un groupe carbonyle ;

$R^2$ est indépendamment sélectionné parmi pyridyle non-substitué ou substitué, ou représente l'hydrogène, hydroxy, alcoxy, $-NMe_2$, $-CONR^{12}R^{13}$ où $R^{12}$ et $R^{13}$ sont chacun indépendamment sélectionnés parmi H et $CH_3$ ;

$R^3$, $R^4$ et $R^5$ sont chacun indépendamment sélectionnés parmi l'hydrogène et méthyle.

**31.** Utilisation selon l'une quelconque des revendications 1-3, dans laquelle l'antagoniste de récepteurs de la bombésine est un composé de formule (III), dans laquelle :

l représente 1 ;
m représente 1 ;
n représente 0 ;
$R^2$ représente 2-pyridyle ;
$R^6$ forme un groupe cyclohexyl avec $R^1$.

**32.** Utilisation selon l'une quelconque des revendications 1-3, dans laquelle l'antagoniste de récepteurs de la bombésine est un composé de formule (IIIa) ou un sel d'un tel composé :

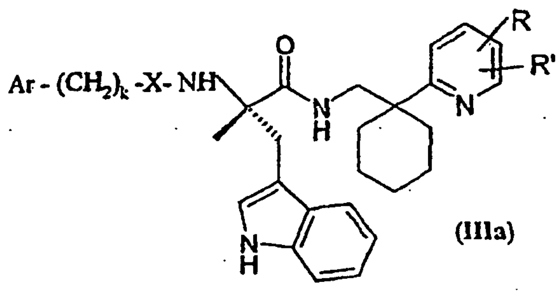

(IIIa)

où Ar, k et X ont les significations données ci-dessus en premier, et le noyau pyridine est facultativement substitué par 1 ou 2 substituants, R et R', indépendamment sélectionnés parmi alcoxy, cyano, halogéno, nitro, phényle, phénoxy, $-CF_3$, $-(CH_2)_qNR^7R^8$, où $R^7$ et $R^8$, pris ensemble avec l'atome d'azote auquel ils sont liés, peuvent former un noyau aliphatique ayant de 5 à 7 éléments qui peut contenir 1 ou 2 atomes d'oxygène ou d'azote, ou $R^7$ et $R^8$ peuvent être indépendamment sélectionné parmi l'hydrogène et cycloalkyle ayant entre 5 et 7 atomes de carbone, et leurs sels pharmaceutiquement acceptables.

**33.** Utilisation selon l'une quelconque des revendications 1-3, dans laquelle l'antagoniste de récepteurs de la bombésine est l'un des composés suivants, ou un sel de celui-ci :

- le *N*-{(S)-2-(1*H*-indol-3-yl)-1-méthyl-1-[(1-pyridin-2-yl-cyclohexylméthyl)-carbamoyl]-éthyl}-4-nitro-benzamide ;
- le *C*-diméthylamino-*N*-{(S)-2-(1*H*-indol-3-yl)-1-méthyl-1-[(1-pyridin-2-yl-cyclohexylméthyl)-carbamoyl]-éthyl}-benzamide ;
- le {(S)-2-(1*H*-indol-3-yl)-1-méthyl-1-[(1-pyridin-2-yl-cyclohexylméthyl)-carbamoyl]-éthyl}-amide de l'acide 1*H*-indole-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide benzo[b]thiophène-2-carboxylique ;
- le *N*-{(S)-2-(1*H*-indol-3-yl)-1-méthyl-1-[(1-pyridin-2-yl-cyclohexylméthyl)-carbamoyl]-éthyl}-2-pyrrol-1-yl-benzamide ;
- le {(S)-2-(1*H*-indol-3-yl)-1-méthyl-1-[(1-pyridin-2-yl-cyclohexylméthyl)-carbamoyl]-éthyl}-amide de l'acide 1*H*-indole-5-carboxylique ; et
- le ((S)-2-(1*H*-indol-3-yl)-1-{[1-(5-méthoxy-pyridin-2-yl)-cyclohexylméthyl]-carbamoyl}-1-méthyl-éthyl)-amide de l'acide 1*H*-indole-2-carboxylique.

**34.** Utilisation selon l'une quelconque des revendications 1-3, dans laquelle l'antagoniste de récepteurs de la bombésine est l'un des composés suivants, ou un sel de celui-ci :

- le *N*-{(S)-2-(1*H*-indol-3-yl)-1-méthyl-1-[(1-pyridin-2-yl-cyclohexylméthyl)-carbamoyl]-éthyl}-benzamide ;
- le *N*-{(S)-2-(1*H*-indol-3-yl)-1-méthyl-1-[(1-pyridin-2-yl-cyclohexylméthyl)-carbamoyl]-éthyl}-4-méthyl-benzamide ;
- le 4-chloro-*N*-{(S)-2-(1*H*-indol-3-yl)-1-méthyl-1-[(1-pyridin-2-yl-cyclohexylméthyl)-carbamoyl]-éthyl}-benzamide ;
- le *N*-{(S)-2-(1*H*-indol-3-yl)-1-méthyl-1-[(1-pyridin-2-yl-cyclohexylméthyl)-carbamoyl]-éthyl}-4-méthoxy-benzamide ;
- le *N*-{(S)-2-(1*H*-indol-3-yl)-1-méthyl-1-[(1-pyridin-2-yl-cyclohexylméthyl)-carbamoyl]-éthyl}-4-méthanesulfonyl-benzamide ;
- le 3-cyano-*N*-{(S)-2-(1*H*-indol-3-yl)-1-méthyl-1-[(1-pyridin-2-yl-cyclohexylméthyl)-carbamoyl]-éthyl}-benzamide ;
- le 3-chloro-*N*-{(S)-2-(1*H*-indol-3-yl)-1-méthyl-1-[(1-pyridin-2-yl-cyclohexylméthyl)-carbamoyl]-éthyl}-benzamide ;
- le *N*-{(S)-2-(1*H*-indol-3-yl)-1-méthyl-1-[(1-pyridin-2-yl-cyclohexylméthyl)-carbamoyl]-éthyl}-3-méthoxy-benzamide ;

- le *N*-{(S)-2-(1*H*-indol-3-yl)-1-méthyl-1-[(1-pyridin-2-yl-cyclohexylméthyl)-carbamoyl]-éthyl}-3-méthanesulfonyl-benzamide ;
- le diméthylamino-*N*-{(S)-2-(1*H*-indol-3-yl)-1-méthyl-1-[(1-pyridin-2-yl-cyclohexylméthyl)-carbamoyl]-éthyl}-benzamide ;
- le *N*-{(S)-2-(1*H*-indol-3-yl)-1-méthyl-1-[(1-pyridin-2-yl-cyclohexylméthyl)-carbamoyl]-éthyl}-3-méthyl-benzamide ;
- le 2-chloro-*N*-{(S)-2-(1*H*-indol-3-yl)-1-méthyl-1-[(1-pyridin-2-yl-cyclohexylméthyl)-carbamoyl]-éthyl}-benzamide ;
- le *N*-{(S)-2-(1*H*-indol-3-yl)-1-méthyl-1-[(1-pyridin-2-yl-cyclohexylméthyl)-carbamoyl]-éthyl}-2-nitro-benzamide ;
- le *N*-{(S)-2-(1*H*-indol-3-yl)-1-méthyl-1-[(1-pyridin-2-yl-cyclohexylméthyl)-carbamoyl]-éthyl}-2-méthoxy-benzamide ;
- le *N*-{(S)-2-(1*H*-indol-3-yl)-1-méthyl-1-[(1-pyridin-2-yl-cyclohexylméthyl)-carbamoyl]-éthyl}-2-méthyl-benzamide ;
- le 2-fluoro-*N*-{(S)-2-(1*H*-indol-3-yl)-1-méthyl-1-[(1-pyridin-2-yl-cyclohexylméthyl)-carbamoyl]-éthyl}-benzamide ;
- le (S)-3-(1*H*-indol-3-yl)-2-méthyl-*N*-(1-pyridin-2-yl-cyclohexylméthyl)-2-(2-p-tolyl-éthanoylamino)-propionamide ;
- le (S)-3-(1*H*-indol-3-yl)-2-méthyl-*N*-(1-pyridin-2-yl-cyclohexylméthyl)-2-(2-o-tolyl-éthanoylamino)-propionamide ;
- le (S)-2-[2-(4-hydroxy-phényl)-éthanoylamino]-3-(1*H*-indol-3-yl)-2-méthyl-*N*-(1-pyridin-2-yl-cyclohexylméthyl)-propionamide ;
- le (S)-2-[2-(3-hydroxy-phényl)-éthanoylamino]-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(2-m-tolyl-éthanoylamino)-propionamide ;
- le (S)-2-[2-(2-fluoro-phényl)-éthanoylamino]-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(2-thiophén-3-yl-éthanoylamino)-propionamide ;
- le {(S)-2-(1*H*-indol-3-yl)-1-méthyl-1-[(1-pyridin-2-yl-cyclohexylméthyl)-carbamoyl]-éthyl}-amide de l'acide pyridine-2-carboxylique ;
- le *N*-{(S)-2-(1*H*-indol-3-yl)-1-méthyl-1-[(1-pyridin-2-yl-cyclohexylméthyl)carbamoyl]-éthyl}-isonicotinamide ;
- le {(S)-2-(1*H*-indol-3-yl)-1-méthyl-1-[(1-pyridin-2-yl-cyclohexylméthyl)-carbamoyl]-éthyl}-amide de l'acide furan-3-carboxylique ;
- le {(S)-2-(1*H*-indol-3-yl)-1-méthyl-1-[(1-pyridin-2-yl-cyclohexylméthyl)-carbamoyl]-éthyl}-amide de l'acide furan-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 5-méthyl-isoxazole-3-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 1-méthyl-1*H*-pyrrole-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide thiophène-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide thiophène-3-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 1*H*-indole-6-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 1*H*-indole-5-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 1*H*-indole-4-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 1*H*-indole-7-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 1-méthyl-1*H*-indole-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide benzothiazole-6-carboxylique ;

- le {(S)-2-(1*H*-indol-3-yl)-1-méthyl-1-[(1-pyridin-2-yl-cyclohexylméthyl)-carbamoyl]-éthyl}-amide de l'acide 1*H*-benzotriazole-5-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 3-méthyl-thiophène-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 5-méthyl-thiophène-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 6-méthyl-pyridine-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide isoquinoline-3-carboxylique ;
- le {(S)-2-(1*H*-indol-3-yl)-1-méthyl-1-[(1-pyridin-2-yl-cyclohexylméthyl)-carbamoyl]-éthyl}-amide de l'acide quinoxaline-2-carboxylique ;
- le {(S)-2-(1*H*-indol-3-yl)-1-méthyl-1-[(1-pyridin-2-yl-cyclohexylméthyl)-carbamoyl]-éthyl}-amide de l'acide quinoline-8-carboxylique ;
- le {(S)-2-(1*H*-indol-3-yl)-1-méthyl-1-[(1-pyridin-2-yl-cyclohexylméthyl)-carbamoyl]-éthyl}-amide de l'acide 5-phényl-oxazole-4-carboxylique ;
- le (S)-3-(1*H*-indol-3-yl)-2-[2-(4-méthoxy-phényl)-éthanoylamino]-2-méthyl-N-(1-pyridin-2-yl-cyclohexylméthyl)-propionamide ;
- le (S)-2-[2-(4-diméthylamino-phényl)-éthanoylamino]-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-[2-(2-nitro-phényl)-éthanoylamino]-*N*-(1-pyridin-2-yl-cyclohexylméthyl)-propionamide ;
- le (S)-3-(1*H*-indol-3-yl)-2-[2-(2-méthoxy-phényl)-éthanoylamino]-2-méthyl-*N*-(1-pyridin-2-yl-cyclohexylméthyl)-propionamide ; et
- le *N*-{(S)-2-(1*H*-indol-3-yl)-1-méthyl-1-[(1-pyridin-2-yl-cyclohexylméthyl)-carbamoyl]-éthyl}-2-pyrrol-1-yl-benzamide.

**35.** Utilisation selon l'une quelconque des revendications 1-3, dans laquelle l'antagoniste de récepteurs de la bombésine est l'un des composés suivants, ou un sel de celui-ci

- l'ester naphtalén-1-ylméthylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 3,4-dichloro-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 3-nitro-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 3-trifluorométhyl-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester quinolin-6-ylméthylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 4-nitro-benzylique de l'acide {(S)-2-(1*H*-indol-3-yl)-1-méthyl-1-[(1-pyridin-2-yl-cyclohexylméthyl)-carbamoyl]-éthyl}-carbamique ; et
- l'ester 3-cyano-benzylique de l'acide {(S)-2-(1*H*-indol-3-yl)-1-méthyl-1-[(1-pyridin-2-yl-cyclohexylméthyl)-carbamoyl]-éthyl}-carbamique.

**36.** Utilisation selon l'une quelconque des revendications 1-3, dans laquelle l'antagoniste de récepteurs de la bombésine est l'un des composés suivants ou un sel de celui-ci :

- l'ester 3,4-diméthoxy-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester naphtalén-2-ylméthylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester indan-2-ylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 4-méthoxy-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 4-chloro-benzylique de l'acide {(S)-2-(1*H*-indol-3-yl)-1-méthyl-1-[(1-pyridin-2-yl-cyclohexylméthyl)-carbamoyl]-éthyl}-carbamique ;

- l'ester 2-fluoro-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 2-chloro-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 2-méthyl-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 4-tert-butyl-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 2-méthoxy-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 4-trifluorométhyl-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 3-éthoxy-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 2,4-dichloro-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 3-méthyl-benzylique de l'acide {(S)-2-(1*H*-indol-3-yl)-1-méthyl-1-[(1-pyridin-2-yl-cyclohexylméthyl)-carbamoyl]-éthyl}-carbamique ;
- l'ester 3-phénoxy-benzylique de l'acide {(S)-2-(1*H*-indol-3-yl)-1-méthyl-1-[(1-pyridin-2-yl-cyclohexylméthyl)-carbamoyl]-éthyl}-carbamique ; et
- l'ester 4-méthyl-benzylique de l'acide {(S)-2-(1*H*-indol-3-yl)-1-méthyl-1-[(1-pyridin-2-yl-cyclohexylméthyl)-carbamoyl]-éthyl}-carbamique.

**37.** Utilisation selon l'une quelconque des revendications 1-3, dans laquelle l'antagoniste de récepteurs de la bombésine est l'un des composés suivants ou un sel de celui-ci :

- le (S)-3-(1*H*-indol-3-yl)-2-méthyl-2-phénylméthanesulfonylamino-*N*-(1-pyridin-2-yl-cyclohexylméthyl)-propionamide ;
- le (S)-2-(2-chloro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-(naphtalène-1-sulfonylamino)-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(quinoline-8-sulfonylamino)-propionamide ;
- le (S)-3-(1*H*-indol-3-yl)-2-méthyl-*N*-(1-pyridin-2-yl-cyclohexylméthyl)-2-(2-trifluorométhyl-benzènesulfonylamino)-propionamide ;
- le (S)-2-(biphényl-2-sulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-(5-méthyl-2-phénoxy-benzènesulfonylamino)-*N*-(1-pyridin-2-yl-cyclohexylméthyl)-propionamide ; et
- le (S)-3-(1*H*-indol-3-yl)-2-méthyl-*N*-(1-pyridin-2-yl-cyclohexylméthyl)-2-(2-*p*-tolyloxy-benzènesulfonylamino)-propionamide.

**38.** Utilisation selon l'une quelconque des revendications 1-3, dans laquelle l'antagoniste de récepteurs de la bombésine est l'un des composés suivants ou un sel de celui-ci :

- le (S)-3-(1*H*-indol-3-yl)-2-méthyl-*N*-(1-pyridin-2-yl-cyclohexylméthyl)-2-(toluène-4-sulfonylamino)-propionamide ;
- le (S)-3-(1*H*-indol-3-yl)-2-méthanesulfonylamino-2-méthyl-*N*-(1-pyridin-2-yl-cyclohexylméthyl)-propionamide ;
- le (S)-2-(2-fluoro-benzènesulfonylamino)-3-(1*H*-indol-3-yl)-2-méthyl-*N*-(1-pyridin-2-yl-cyclohexylméthyl)-propionamide ;
- le (S)-2-(4-chloro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(2,2,2-trifluoro-éthanesulfonylamino)-propionamide ;
- le (S)-2-(5-diméthylamino-naphtalène-1-sulfonylamino)-3-(1*H*-indol-3-yl)-2-méthyl-*N*-(1-pyridin-2-yl-cyclohexylméthyl)-propionamide ;

- le (S)-3-(1*H*-indol-3-yl)-2-méthyl-2-(naphtalène-2-sulfonylamino)-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(thiophène-2-sulfonylamino)-propionamide ;
- le (S)-3-(1*H*-indol-3-yl)-2-méthyl-2-(3-nitro-benzènesulfonylamino)-*N*-(1-pyridin-2-yl-cyclohexylméthyl)-propionamide ;
- le (S)-2-(4-fluoro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-(4-nitro-benzènesulfonylamino)-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(3-trifluorométhyl-benzènesulfonylamino)-propionamide ;
- le (S)-2-(3,4-dichloro-benzènesulfonylamino)-3-(1*H*-indol-3-yl)-2-méthyl-*N*-(1-pyridin-2-yl-cyclohexylméthyl)-propionamide ;
- le (S)-2-(3-fluoro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(4-trifluorométhyl-benzènesulfonylamino)-propionamide ;
- le (S)-2-(5-chloro-thiophène-2-sulfonylamino)-3-(1*H*-indol-3-yl)-2-méthyl-*N*-(1-pyridin-2-yl-cyclohexylméthyl)-propionamide ;
- le (S)-2-(3-chloro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(toluène-3-sulfonylamino)-propionamide ;
- le (S)-2-(3,4-diméthoxy-benzènesulfonylamino)-3-(1*H*-indol-3-yl)-2-méthyl-*N*-(1-pyridin-2-yl-cyclohexylméthyl)-propionamide ;
- le (S)-2-(4-cyano-benzènesulfonylamino)-3-(1*H*-indol-3-yl)-2-méthyl-*N*-(1-pyridin-2-yl-cyclohexylméthyl)-propionamide ;
- le (S)-2-(2-cyano-benzènesulfonylamino)-3-(1*H*-indol-3-yl)-2-méthyl-*N*-(1-pyridin-2-yl-cyclohexylméthyl)-propionamide ;
- le (S)-2-(5-chloro-1,3-diméthyl-1*H*-pyrazole-4-sulfonylamino)-3-(1*H*-indol-3-yl)-2-méthyl-*N*-(1-pyridin-2-yl-cyclohexylméthyl)-propionamide ;
- le (S)-2-(3,5-diméthyl-isoxazole-4-sulfonylamino)-3-(1*H*-indol-3-yl)-2-méthyl-*N*-(1-pyridin-2-yl-cyclohexylméthyl)-propionamide ;
- le (S)-2-(benzo[1,2,5]thiadiazole-4-sulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-(1-méthyl-1*H*-imidazole-4-sulfonylamino)-*N*-(1-pyridin-2-yl-cyclohexylméthyl)-propionamide ;
- le (S)-2-(benzo[1,2,5]oxadiazole-4-sulfonylamino)-3-(1*H*-indol-3-yl)-2-méthyl-*N*-(1-pyridin-2-yl-cyclohexylméthyl)-propionamide ;
- l'ester méthylique de l'acide 3-{(S)-2-(1*H*-indol-3-yl)-1-méthyl-1-[(1-pyridin-2-yl-cyclohexylméthyl)-carbamoyl]-éthylsulfamoyl}-thiophène-2-carboxylique.
- le (S)-3-(1*H*-indol-3-yl)-2-(5-isoxazol-3-yl-thiophène-2-sulfonylamino)-2-méthyl-*N*-(1-pyridin-2-yl-cyclohexylméthyl)-propionamide ;
- le (S)-3-(1*H*-indol-3-yl)-2-méthyl-2-(2-nitro-phénylméthanesulfonylamino)-*N*-(1-pyridin-2-yl-cyclohexylméthyl)-propionamide ;
- le (S)-2-(3-cyano-benzènesulfonylamino)-3-(1*H*-indol-3-yl)-2-méthyl-*N*-(1-pyridin-2-yl-cyclohexylméthyl)-propionamide ;
- le (S)-2-(1,2-diméthyl-1*H*-imidazole-4-sulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-(3-méthoxy-benzènesulfonylamino)-2-méthyl-*N*-(1-pyridin-2-yl-cyclohexylméthyl)-propionamide ;
- le (S)-3-(1*H*-indol-3-yl)-2-méthyl-2-(8-nitro-naphtalène-1-sulfonylamino)-*N*-(1-pyridin-2-yl-cyclohexylméthyl)-propionamide ;
- le (S)-2-(2-chloro-5-nitro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(2,4,6-trichloro-benzènesulfonylamino)-propionamide ;

- le (S)-2-(4-chloro-2-nitro-benzènesulfonylamino)-3-(1*H*-indol-3-yl)-2-méthyl-*N*-(1-pyridin-2-yl-cyclohexylméthyl)-propionamide ;
- le (S)-2-(5-benzènesulfonyl-thiophène-2-sulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(4-trifluorométhoxy-benzènesulfonylamino)-propionamide ;
- l'ester méthylique de l'acide 2-{(S)-2-(1*H*-indol-3-yl)-1-méthyl-1-[(1-pyridin-2-yl-cyclohexylméthyl)-carbamoyl]-éthylsulfamoyl}-benzoïque.
- le (S)-2-(3-chloro-4-fluoro-benzènesulfonylamino)-3-(1*H*-indol-3-yl)-2-méthyl-*N*-(1-pyridin-2-yl-cyclohexylméthyl)-propionamide ;
- le (S)-2-(2,5-dichloro-thiophène-3-sulfonylamino)-3-(1*H*-indol-3-yl)-2-méthyl-*N*-(1-pyridin-2-yl-cyclohexylméthyl)-propionamide ;
- le (S)-2-(3-chloro-4-méthyl-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-(2-méthoxy-4-méthyl-benzènesulfonylamino)-2-méthyl-*N*-(1-pyridin-2-yl-cyclohexylméthyl)-propionamide ;
- le (S)-3-(1*H*-indol-3-yl)-2-méthyl-*N*-(1-pyridin-2-yl-cyclohexylméthyl)-2-(5-pyridin-2-yl-thiophène-2-sulfonylamino)-propionamide ;
- le (S)-2-(5-bromo-6-chloro-pyridine-3-sulfonylamino)-3-(1*H*-indol-3-yl)-2-méthyl-*N*-(1-pyridin-2-yl-cyclohexylméthyl)-propionamide ;
- le (S)-2-(2,4-dinitro-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-(4-méthanesulfonyl-benzènesulfonylamino)-2-méthyl-*N*-(1-pyridin-2-yl-cyclohexylméthyl)-propionamide ;
- le (S)-2-(4-*tert*-butyl-benzènesulfonylamino)-3-(1*H*-indol-3-yl)-2-méthyl-*N*-(1-pyridin-2-yl-cyclohexylméthyl)-propionamide ;
- le (S)-2-(2,4-dichloro-5-méthyl-benzènesulfonylamino)-3-(1*H*-indol-3-yl)-2-méthyl-*N*-(1-pyridin-2-yl-cyclohexylméthyl)-propionamide ;
- le (S)-2-(2-chloro-5-trifluorométhyl-benzènesulfonylamino)-3-(1*H*-indol-3-yl)-2-méthyl-*N*-(1-pyridin-2-yl-cyclohexylméthyl)-propionamide ;
- le (S)-3-(1*H*-indol-3-yl)-2-méthyl-2-(2-nitro-4-trifluorométhyl-benzènesulfonylamino)-*N*-(1-pyridin-2-yl-cyclohexylméthyl)-propionamide ; et
- le (S)-2-(4-butyl-benzènesulfonylamino)-3-(1*H*-indol-3-yl)-2-méthyl-*N*-(1-pyridin-2-yl-cyclohexylméthyl)-propionamide.

**39.** Utilisation, dans la préparation d'un médicament pour le traitement ou la prophylaxie d'une dysfonction sexuelle induite par un médicament chez l'homme, de la dysfonction érectile masculine, d'une dysfonction sexuelle induite par un médicament chez la femme, d'un trouble du désir sexuel hypoactif chez la femme, d'un trouble de l'excitation sexuelle chez la femme, de l'anorgasmie féminine ou de troubles douloureux sexuels féminins, d'une composition pharmaceutique (pour l'administration simultanée, séparée ou successive) d'un antagoniste de récepteurs de la bombésine et un inhibiteur de PDE 5.

**40.** Utilisation, dans la préparation d'un médicament pour le traitement ou la prophylaxie d'une dysfonction sexuelle induite par un médicament chez l'homme, de la dysfonction érectile masculine, d'une dysfonction sexuelle induite par un médicament chez la femme, d'un trouble du désir sexuel hypoactif chez la femme, d'un trouble de l'excitation sexuelle chez la femme, de l'anorgasmie féminine ou de troubles douloureux sexuels féminins, d'une composition pharmaceutique (pour l'administration simultanée, séparée ou successive) d'un antagoniste de récepteurs de la bombésine et un inhibiteur de NEP.

**41.** Utilisation, dans la préparation d'un médicament pour le traitement ou la prophylaxie d'une dysfonction sexuelle induite par un médicament chez l'homme, de la dysfonction érectile masculine, d'une dysfonction sexuelle induite par un médicament chez la femme, d'un trouble du désir sexuel hypoactif chez la femme, d'un trouble de l'excitation sexuelle chez la femme, de l'anorgasmie féminine ou de troubles douloureux sexuels féminins, d'une composition pharmaceutique (pour l'administration simultanée, séparée ou successive) d'un antagoniste de récepteurs de la bombésine et un ou plusieurs modulateurs de récepteur des oestrogènes (SERM) et/ou d'agonistes d'oestrogènes et/ou d'antagonistes d'oestrogènes.

**42.** Utilisation, dans la préparation d'un médicament pour le traitement ou la prophylaxie d'une dysfonction sexuelle

induite par un médicament chez l'homme, de la dysfonction érectile masculine, d'une dysfonction sexuelle induite par un médicament chez la femme, d'un trouble du désir sexuel hypoactif chez la femme, d'un trouble de l'excitation sexuelle chez la femme, de l'anorgasmie féminine ou de troubles douloureux sexuels féminins, d'une composition pharmaceutique (pour l'administration simultanée, séparée ou successive) d'un antagoniste de récepteurs de la bombésine et du lasofoxifène.

FIG. 1

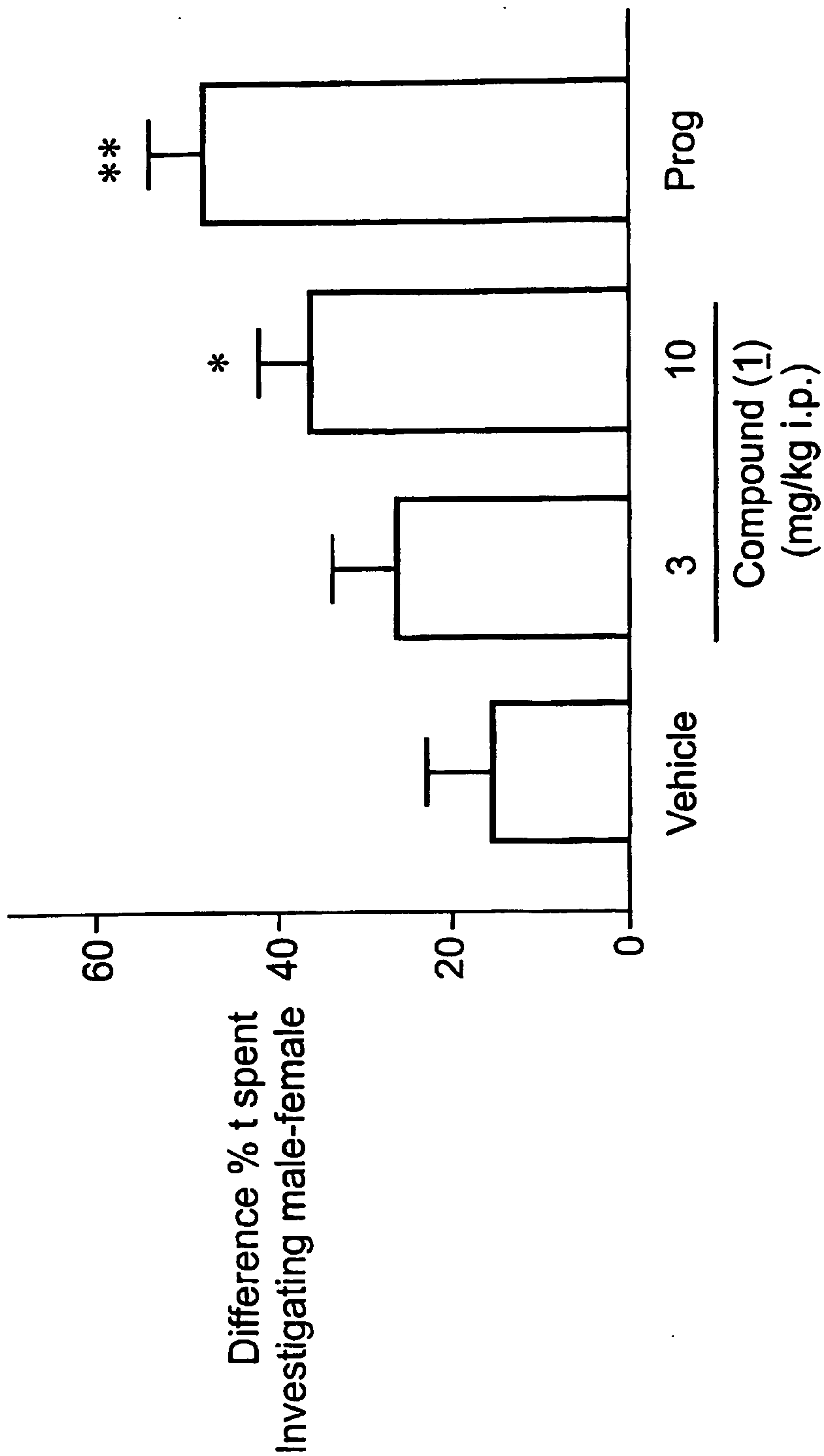
Difference % t spent
Investigating male-female
60
40
20
0
**
*
Vehicle
3
10
Prog
Compound (1)
(mg/kg i.p.)

FIG. 2

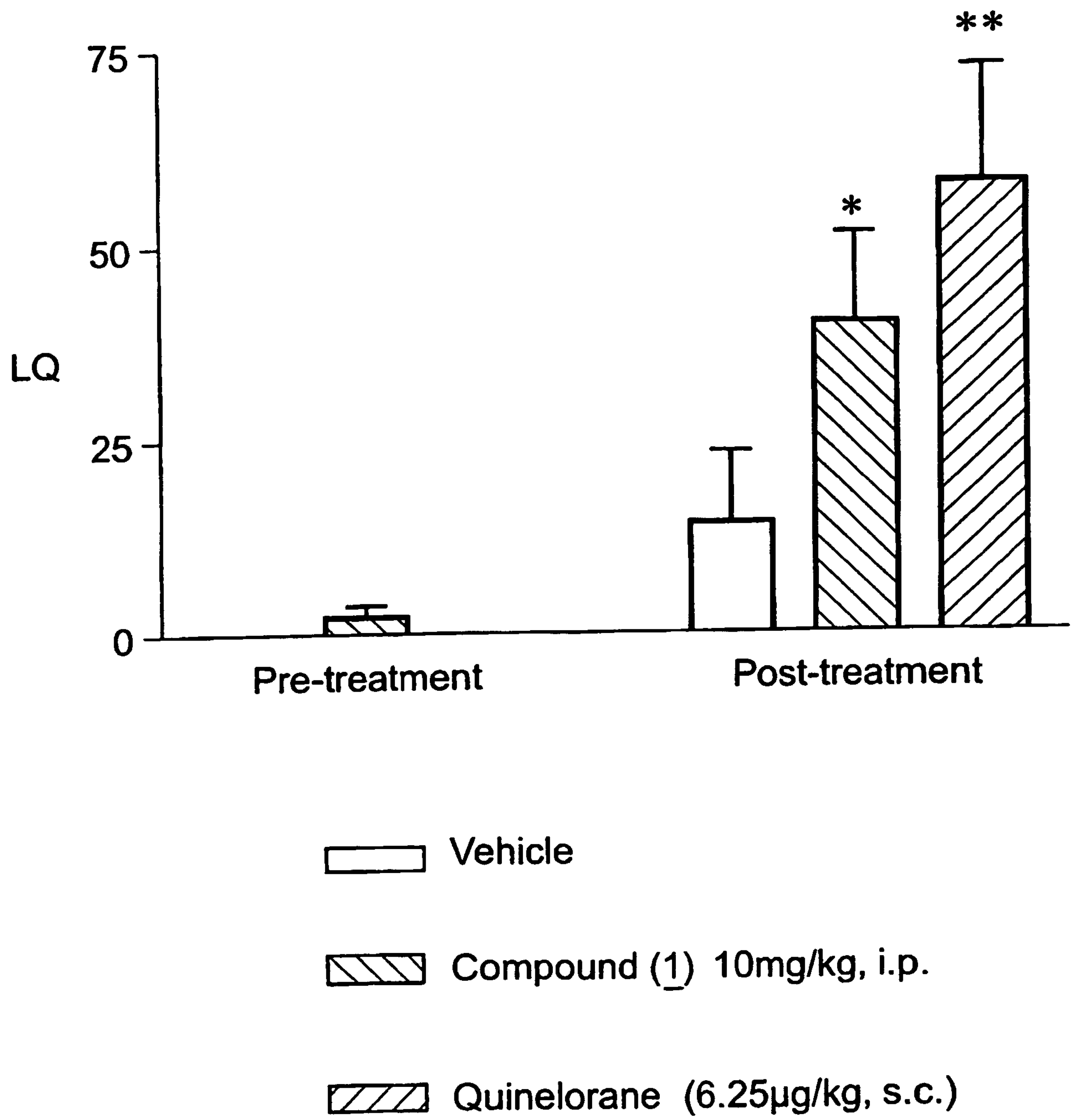

75
50
25
0
LQ
*
**
Pre-treatment
Post-treatment
Vehicle
Compound (1) 10mg/kg, i.p.
Quinelorane (6.25μg/kg, s.c.)

FIG. 3

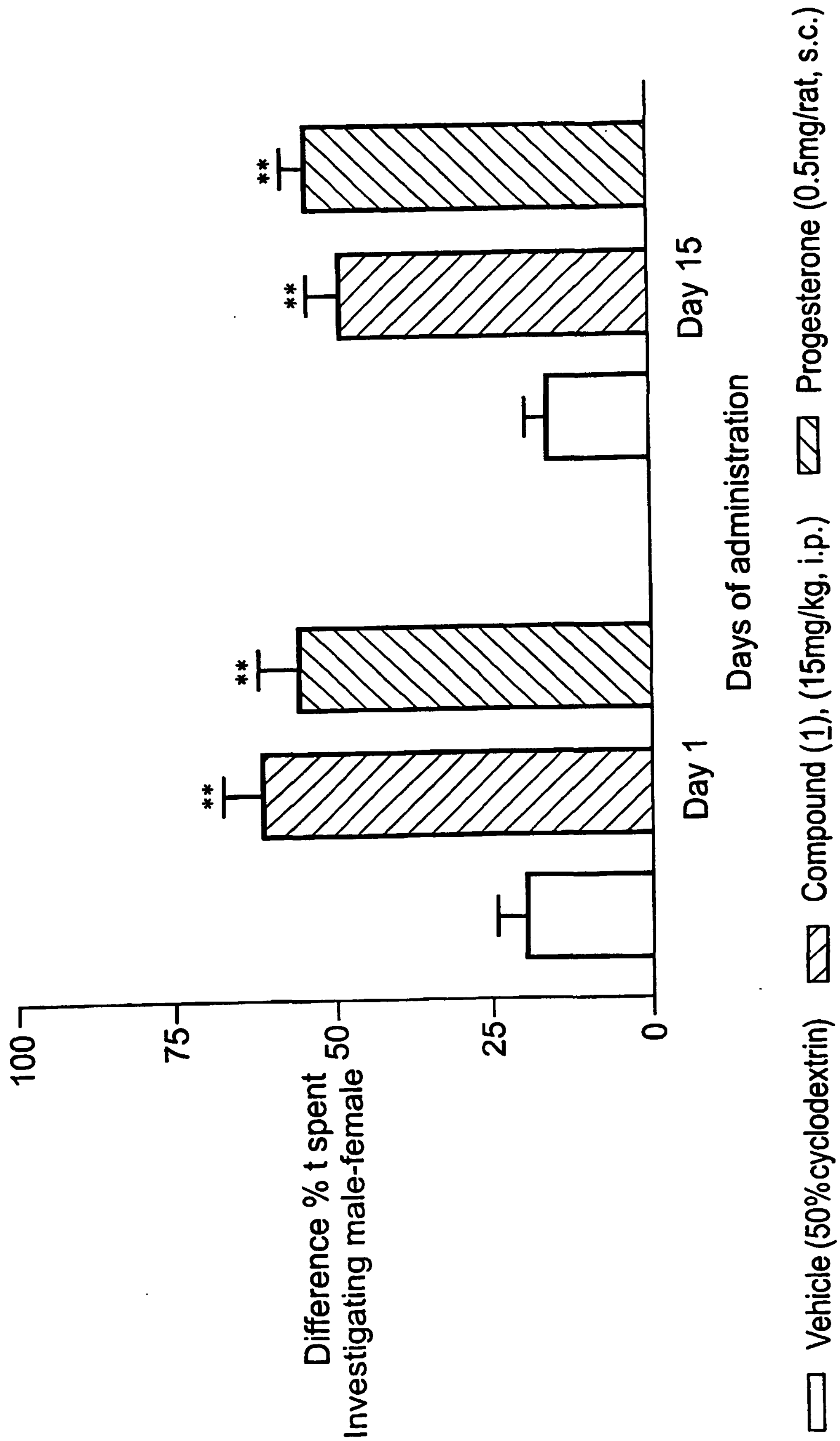
Difference % t spent
Investigating male-female
100
75
50
25
0
**
Day 1
Day 15
Days of administration
Vehicle (50%cyclodextrin)
Compound (1), (15mg/kg, i.p.)
Progesterone (0.5mg/rat, s.c.)

FIG. 4

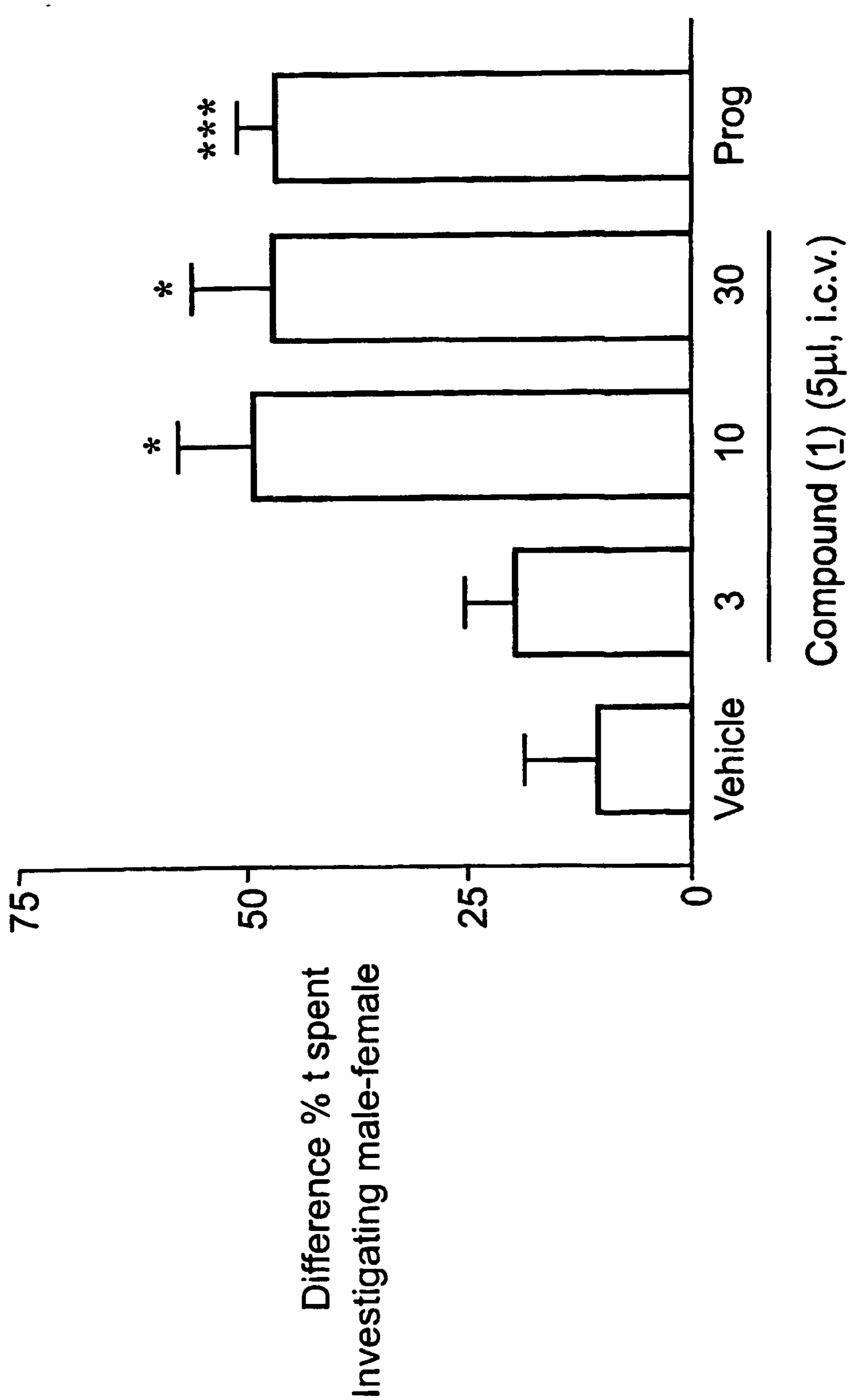
Difference % t spent
Investigating male-female
0
25
50
75
Vehicle
3
10
30
Prog
*
*
***
Compound (1) (5µl, i.c.v.)

FIG. 5

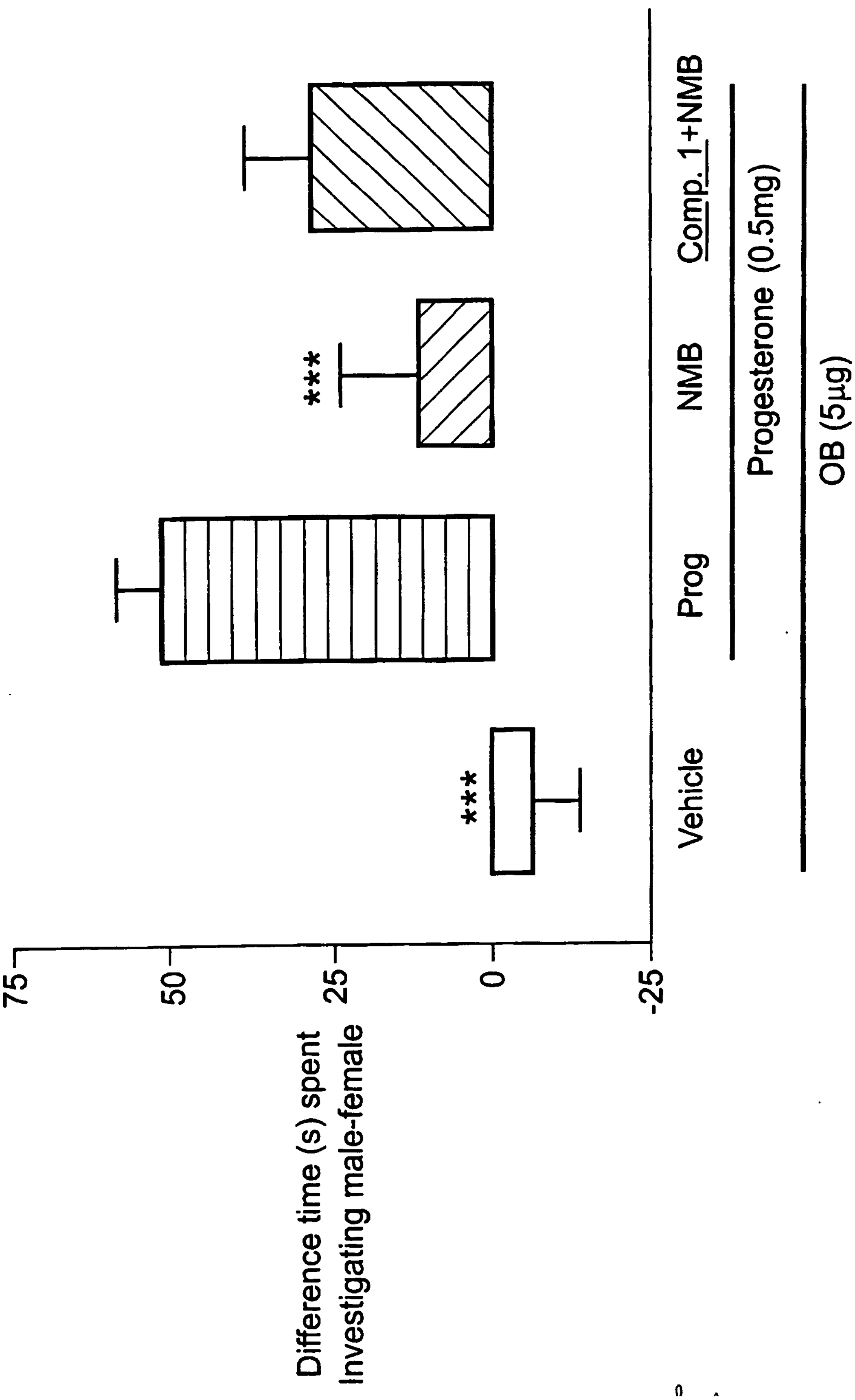
Difference time (s) spent
Investigating male-female
75
50
25
0
-25
***
***
Vehicle
Prog
NMB
Comp. 1+NMB
Progesterone (0.5mg)
OB (5μg)

FIG. 6

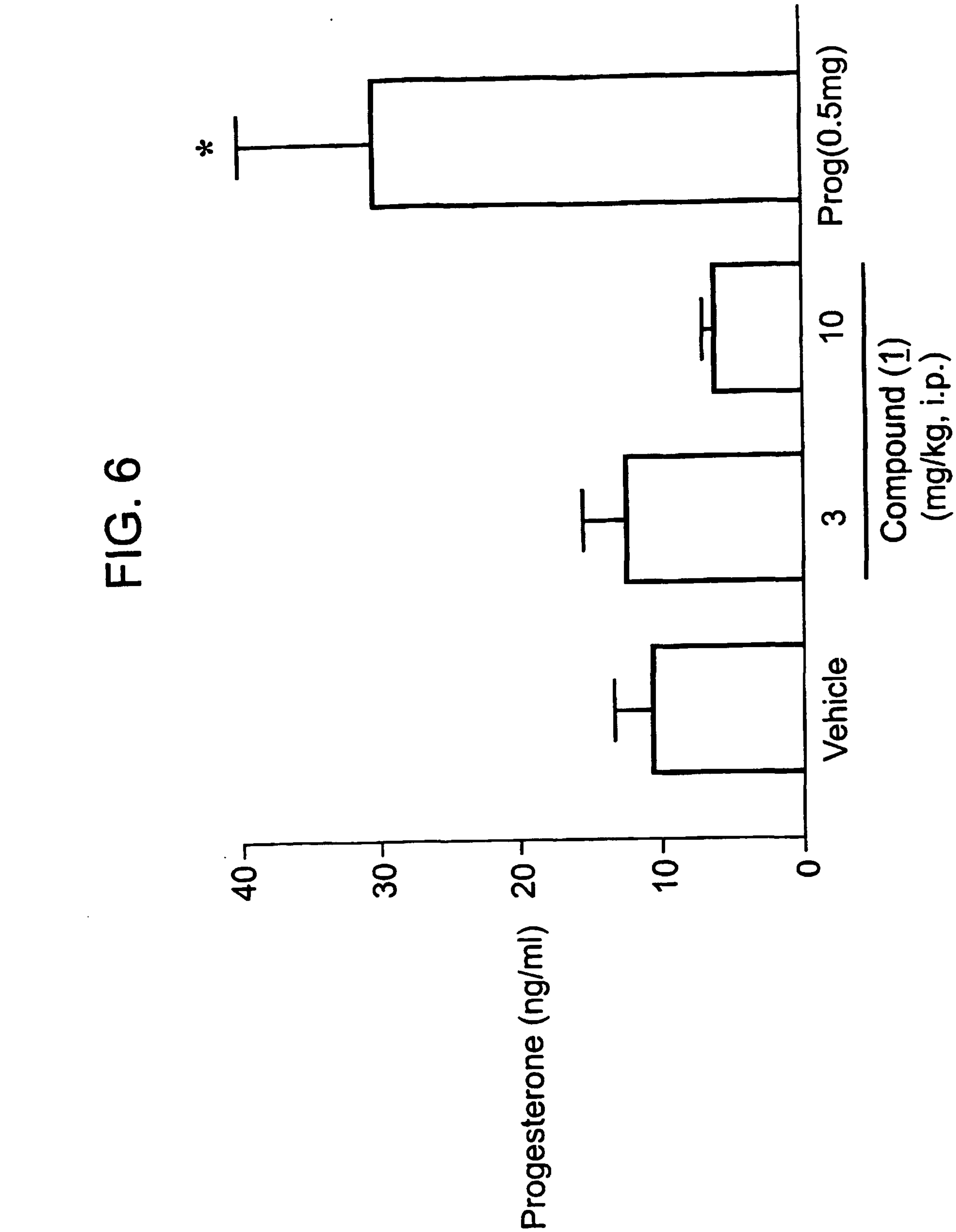
Progesterone (ng/ml)
0
10
20
30
40
Vehicle
3
10
Prog(0.5mg)
*
Compound (1)
(mg/kg, i.p.)

FIG. 7

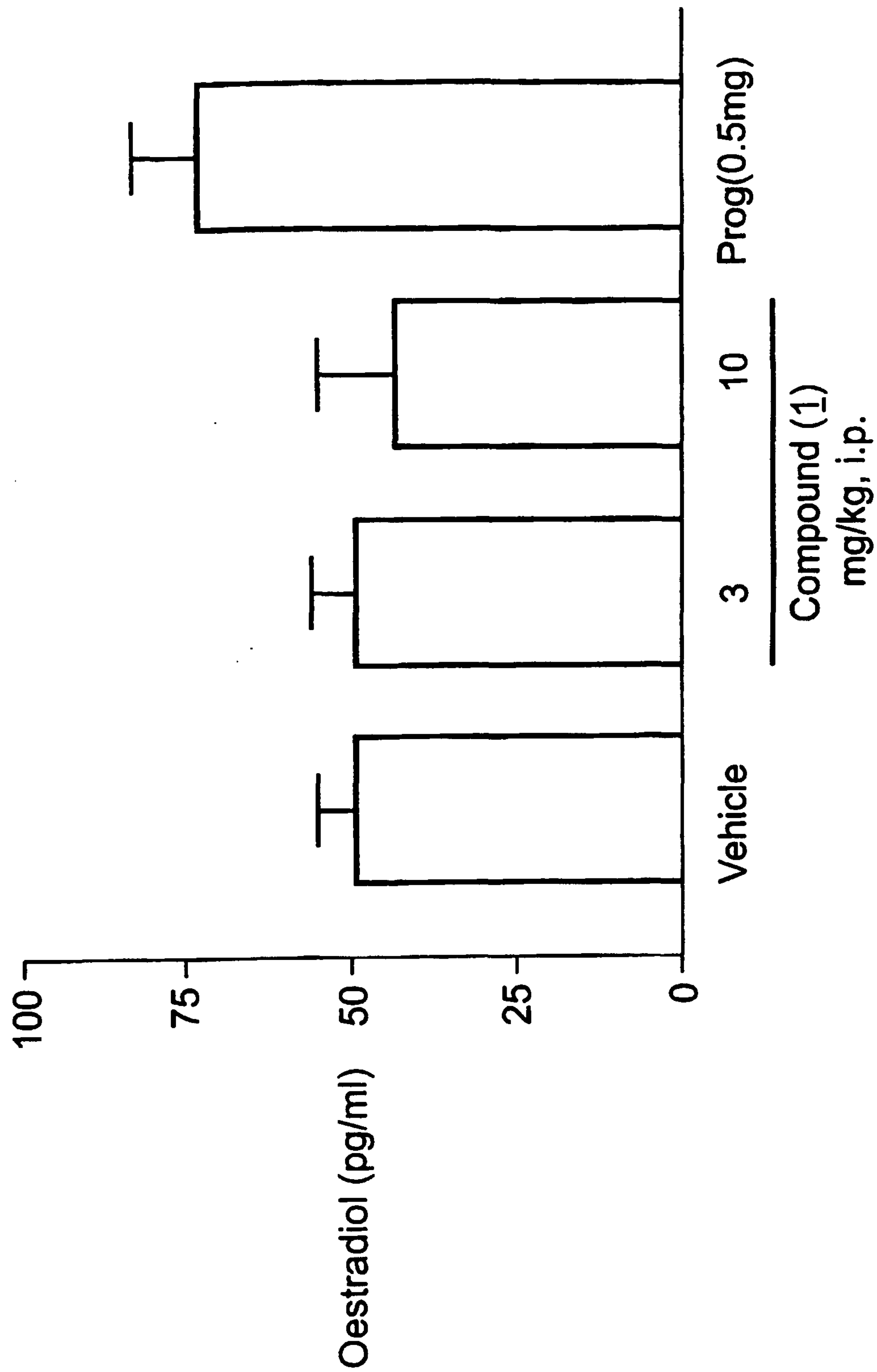
Oestradiol (pg/ml)
0
25
50
75
100
Vehicle
3
10
Prog(0.5mg)
Compound (1)
mg/kg, i.p.

FIG. 8

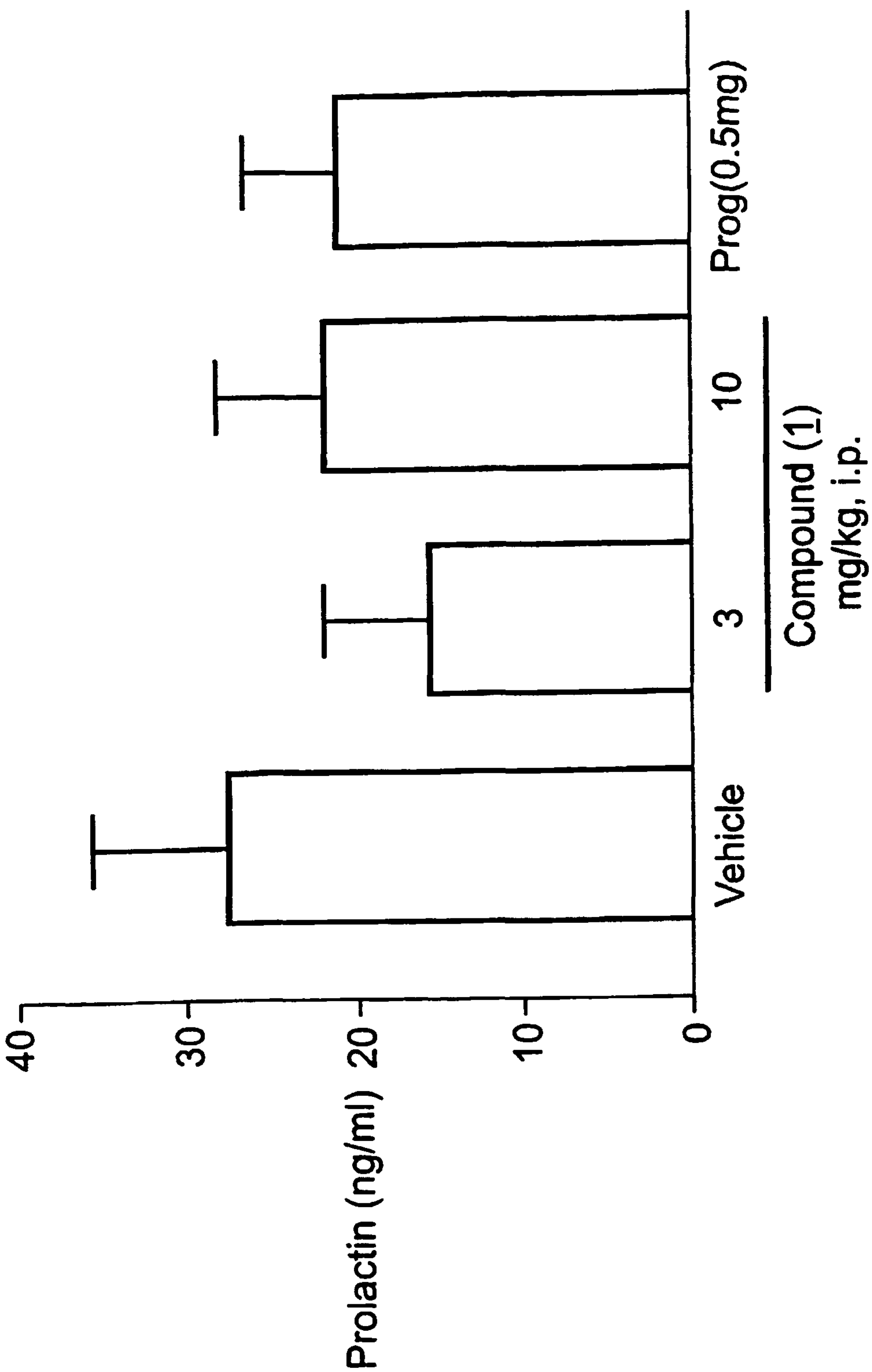
Prolactin (ng/ml)
0
10
20
30
40
Vehicle
3
10
Prog(0.5mg)
Compound (1)
mg/kg, i.p.

FIG. 9

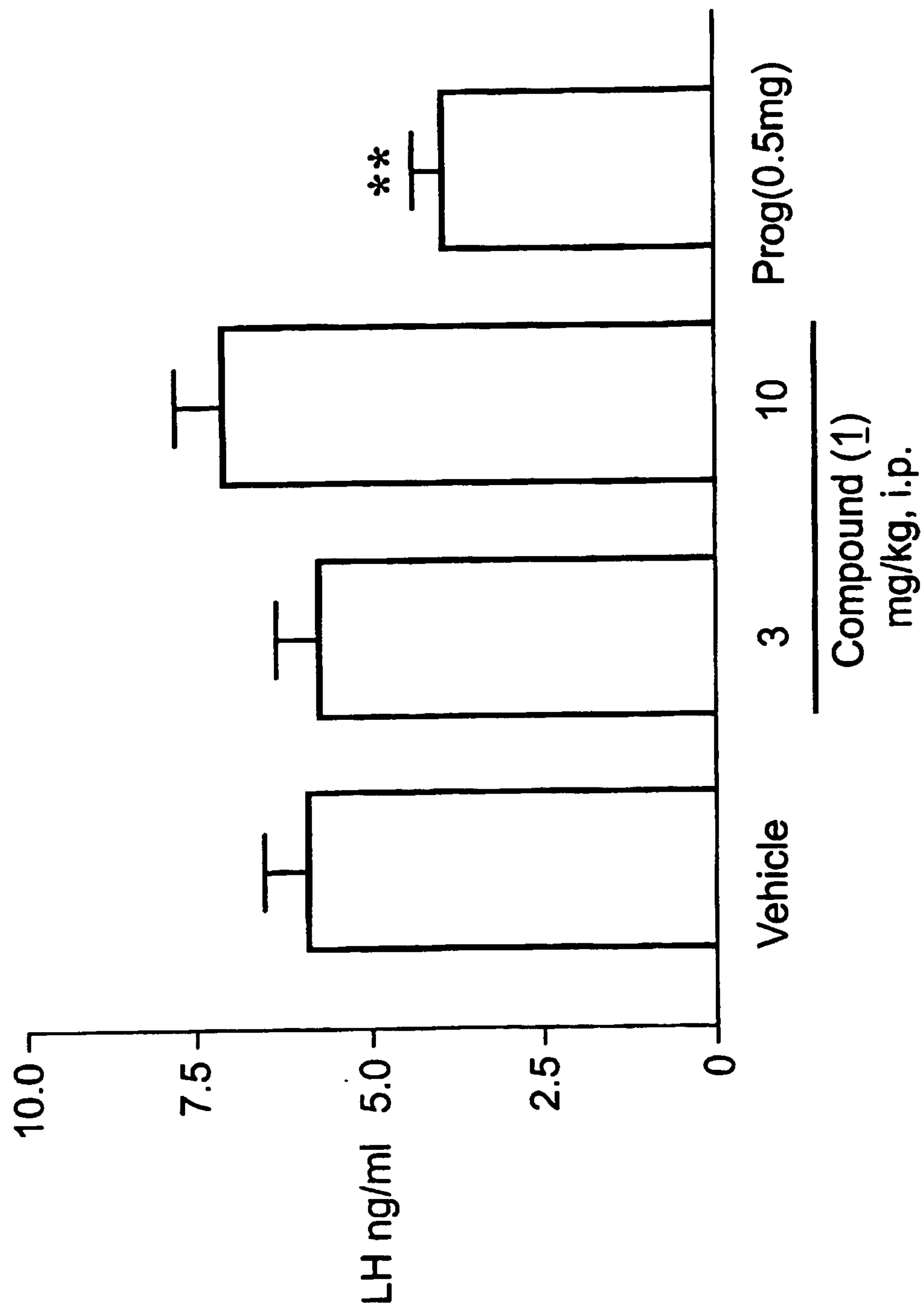

FIG. 10

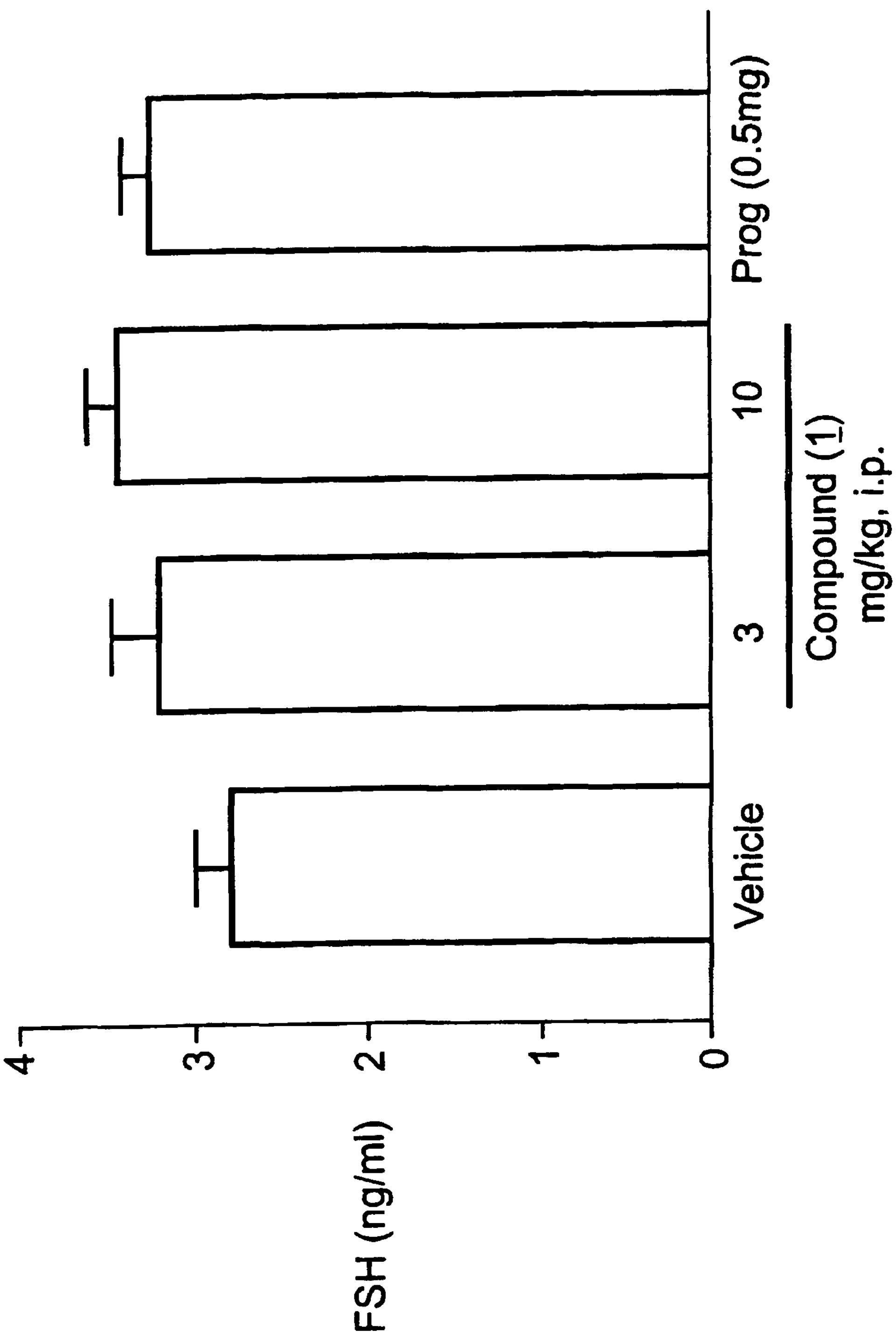

FIG. 11

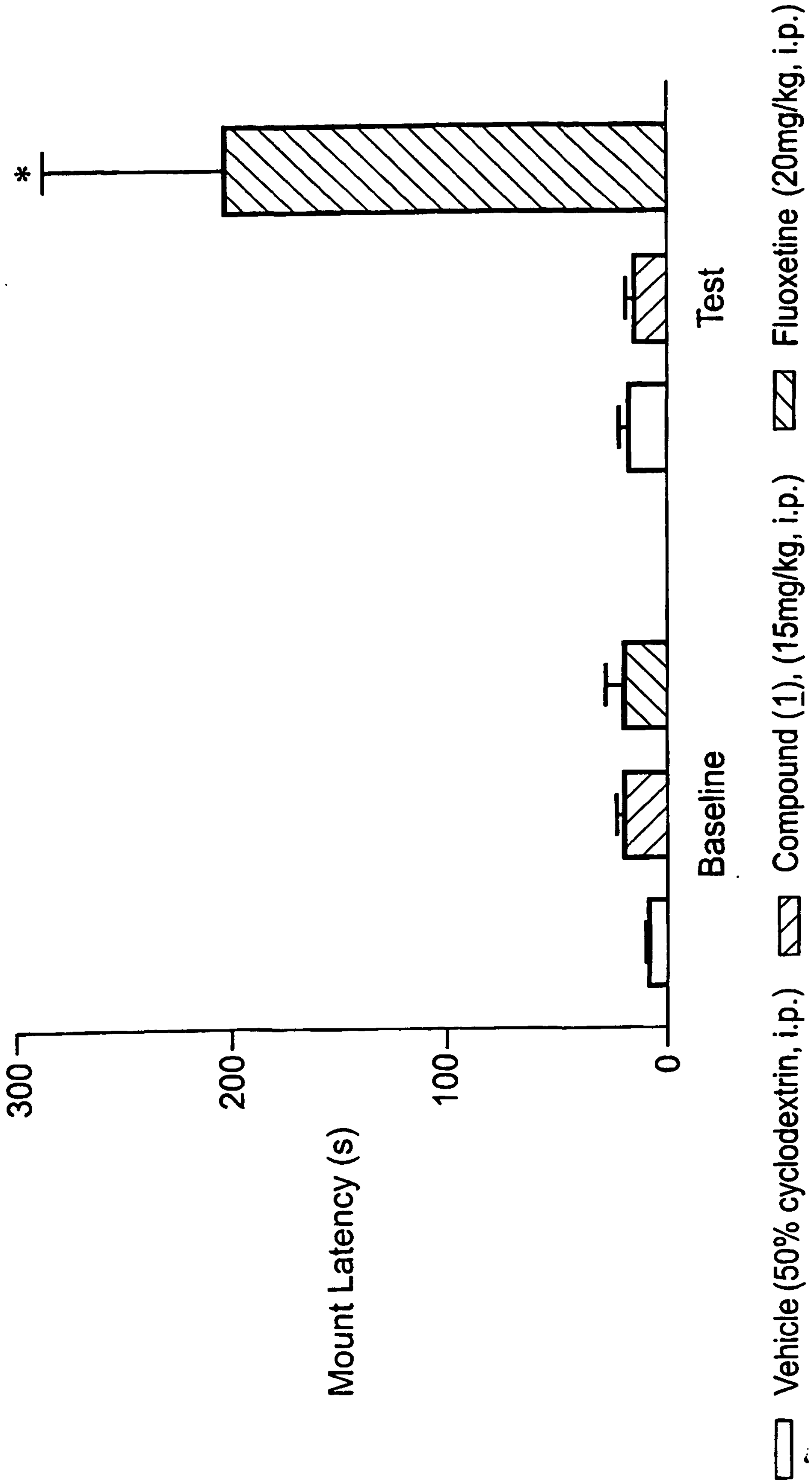
Mount Latency (s)
300
200
100
0
*
Baseline
Test
Vehicle (50% cyclodextrin, i.p.)
Compound (1), (15mg/kg, i.p.)
Fluoxetine (20mg/kg, i.p.)

FIG. 12

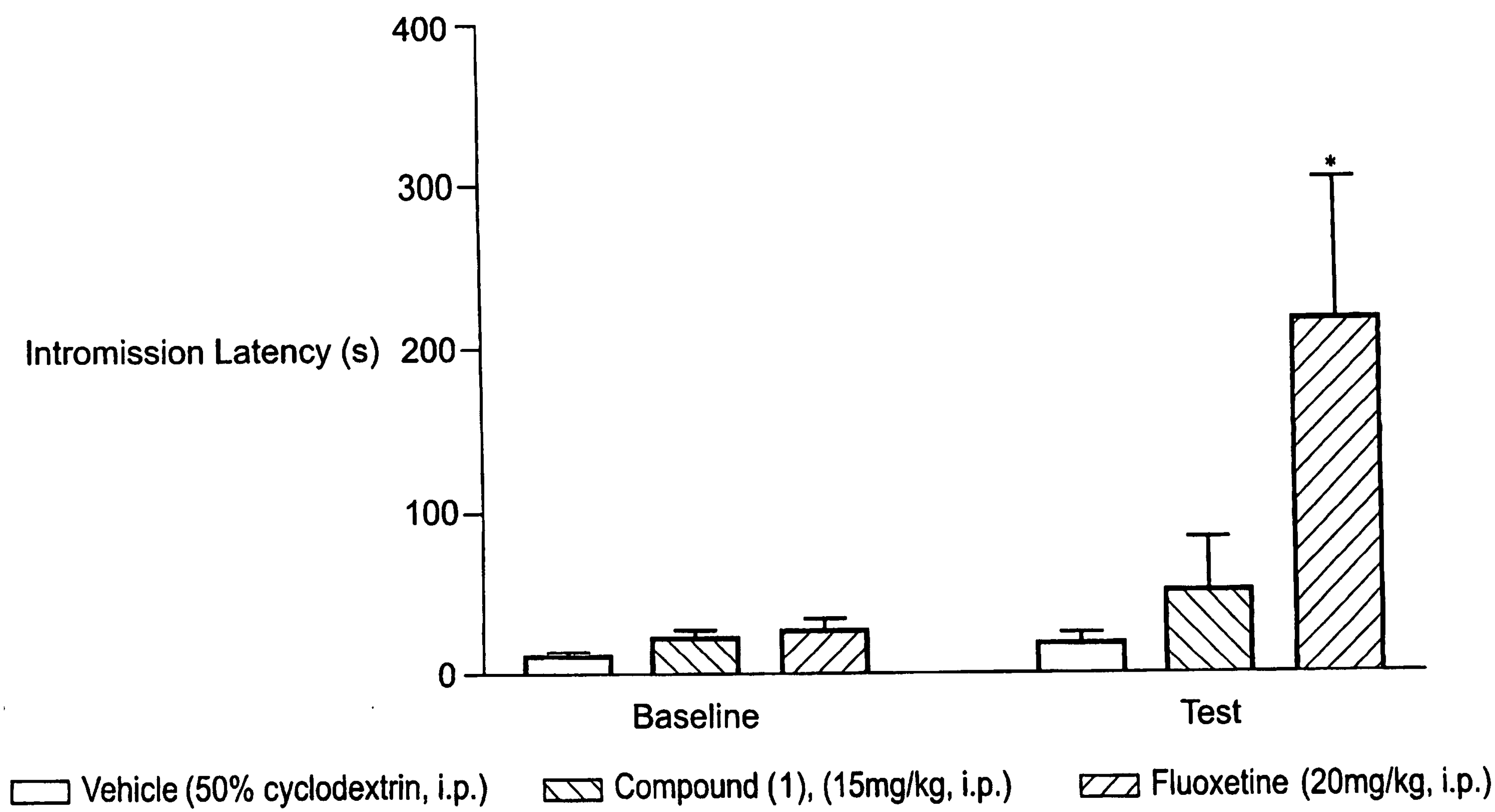
Intromission Latency (s)
400
300
200
100
0
*
Baseline
Test
Vehicle (50% cyclodextrin, i.p.)
Compound (1), (15mg/kg, i.p.)
Fluoxetine (20mg/kg, i.p.)

FIG. 13

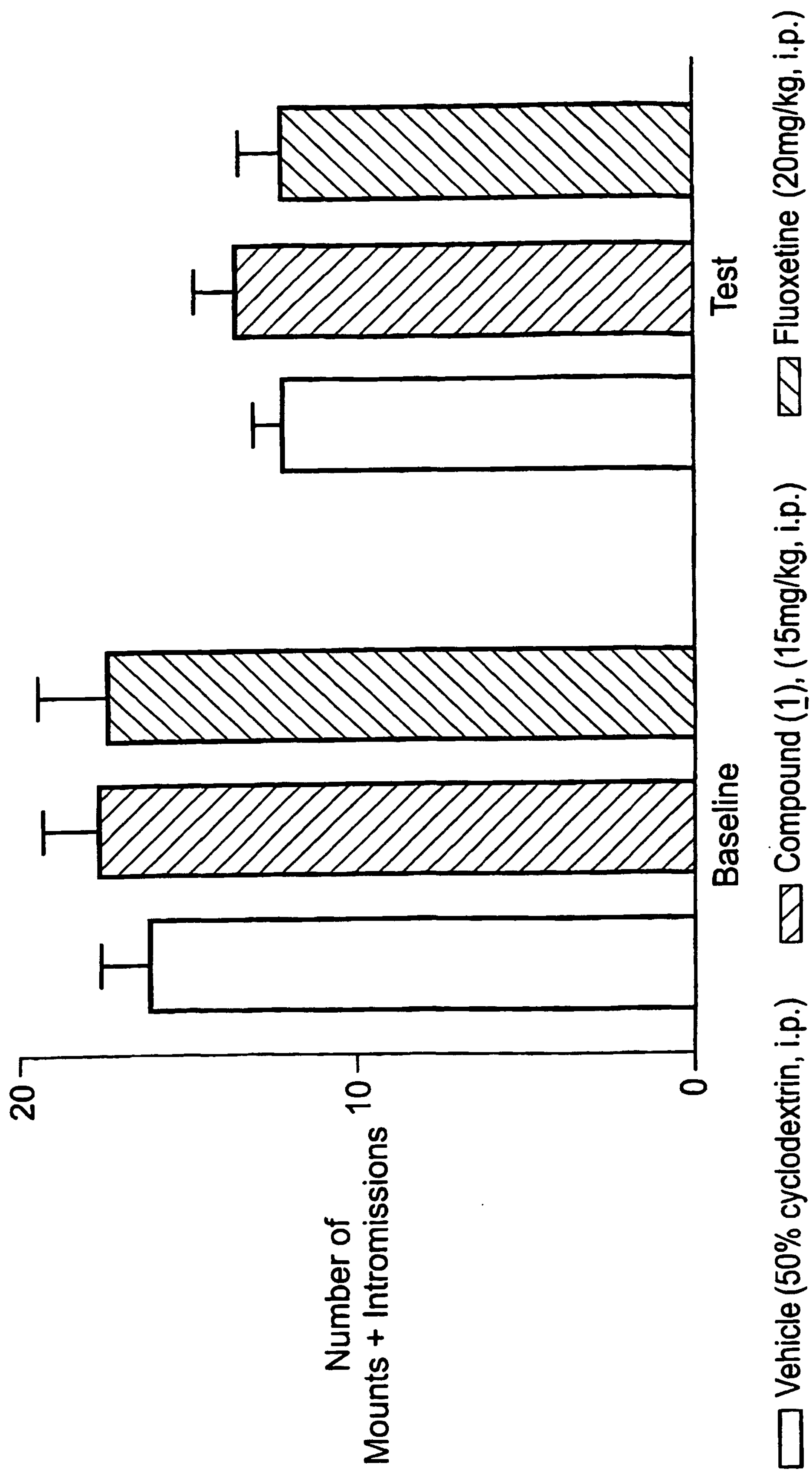
Number of
Mounts + Intromissions
20
10
0
Baseline
Test
Vehicle (50% cyclodextrin, i.p.)
Compound (1), (15mg/kg, i.p.)
Fluoxetine (20mg/kg, i.p.)

FIG. 14

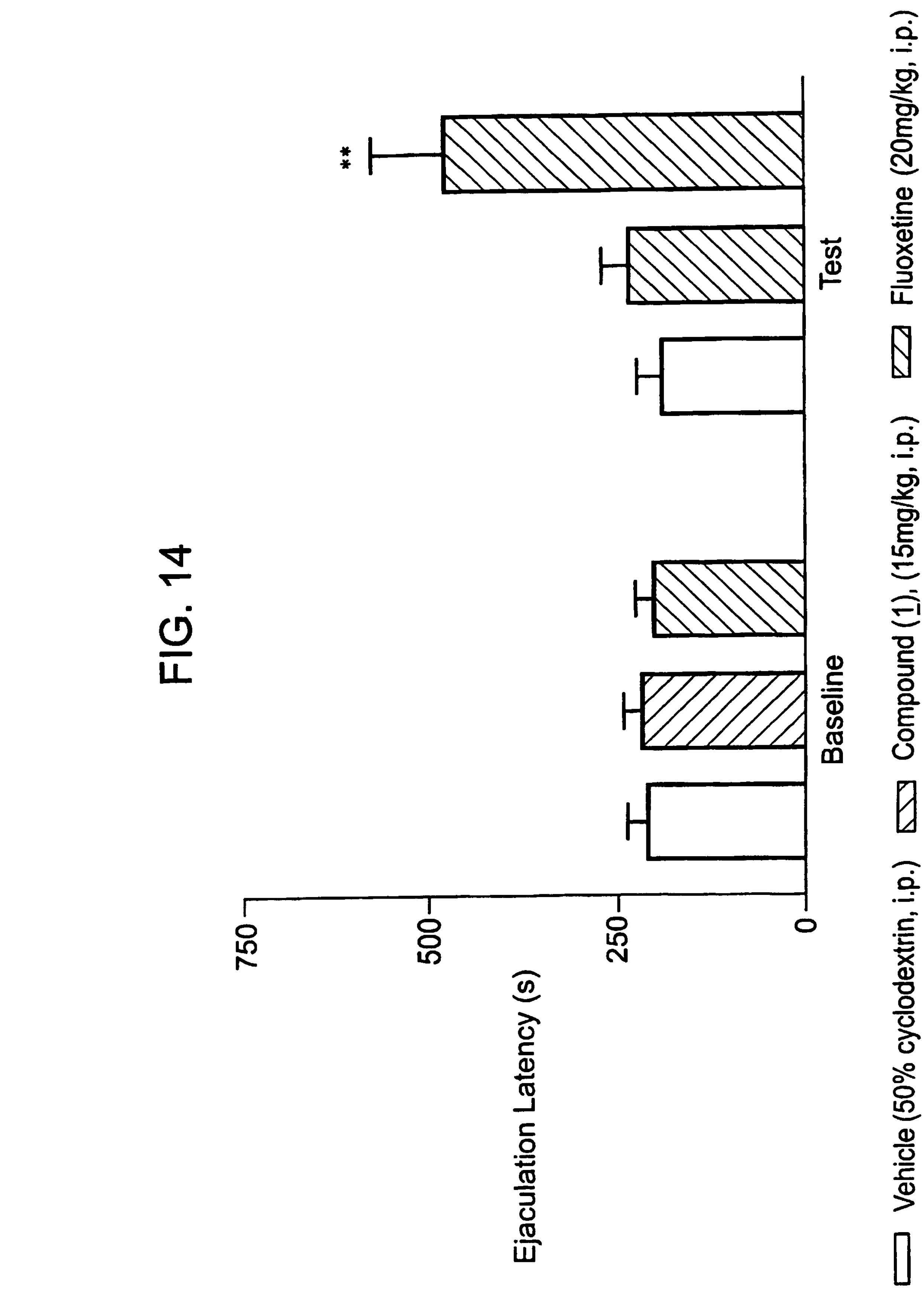
Ejaculation Latency (s)
750
500
250
0
**
Baseline
Test
Vehicle (50% cyclodextrin, i.p.)
Compound (1), (15mg/kg, i.p.)
Fluoxetine (20mg/kg, i.p.)

FIG. 15

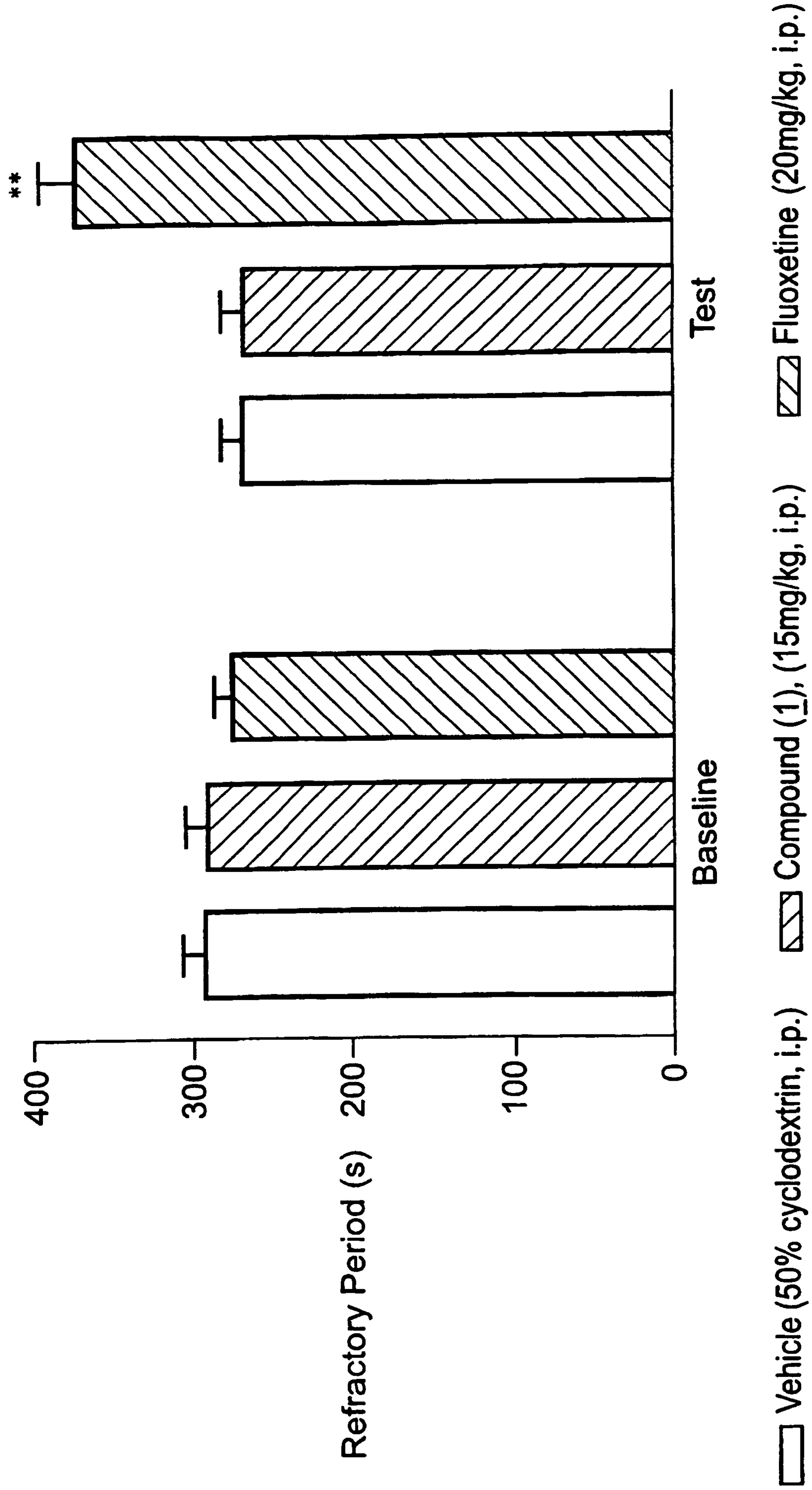

Refractory Period (s)
400
300
200
100
0
**
Baseline
Test
Vehicle (50% cyclodextrin, i.p.)
Compound (1), (15mg/kg, i.p.)
Fluoxetine (20mg/kg, i.p.)

FIG. 16

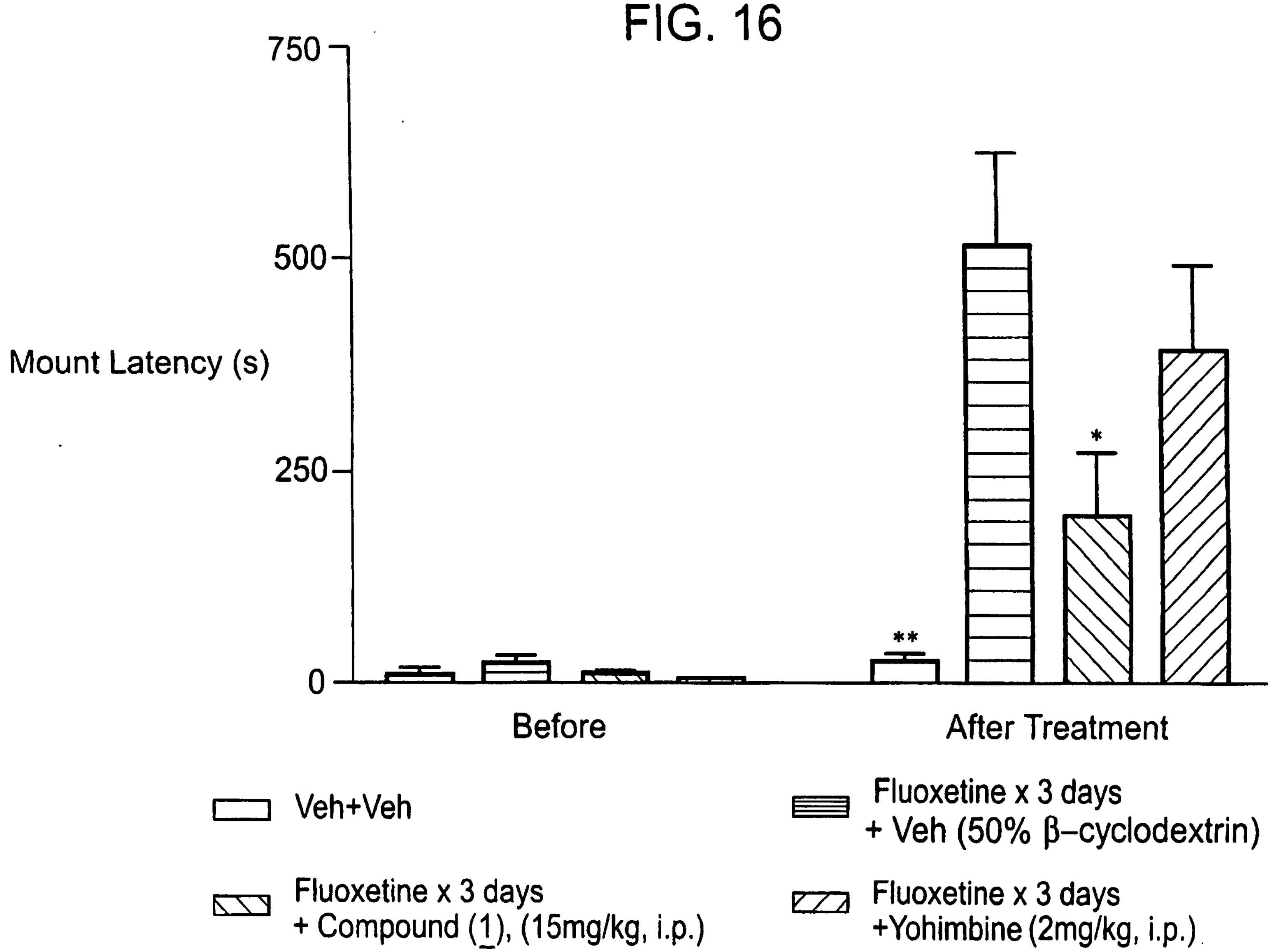
750
500
250
0
Mount Latency (s)
**
*
Before
After Treatment
Veh+Veh
Fluoxetine x 3 days
+ Compound (1), (15mg/kg, i.p.)
Fluoxetine x 3 days
+ Veh (50% β–cyclodextrin)
Fluoxetine x 3 days
+Yohimbine (2mg/kg, i.p.)

FIG. 17

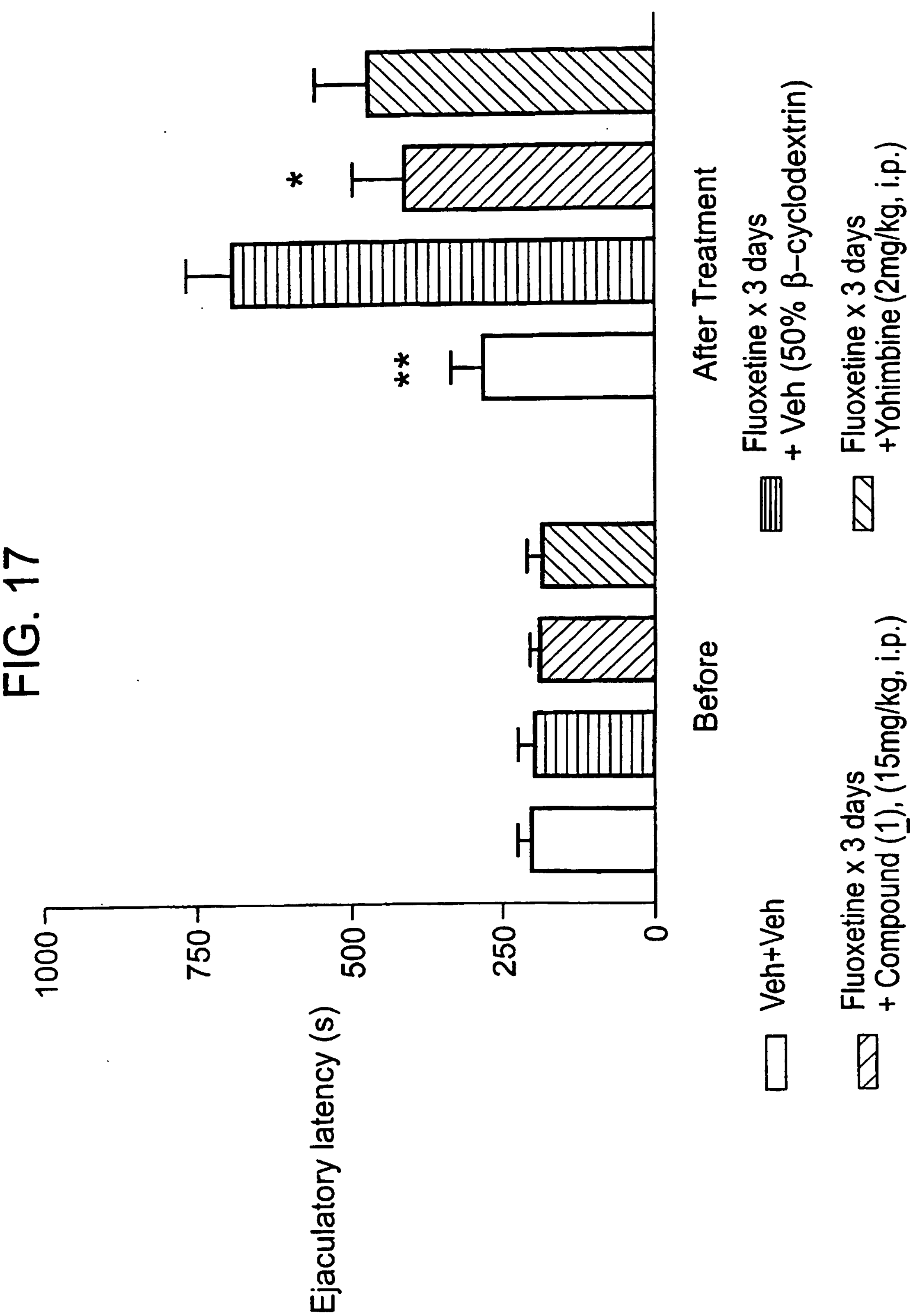
Ejaculatory latency (s)
1000
750
500
250
0
Before
After Treatment
**
*
Veh+Veh
Fluoxetine x 3 days
+ Veh (50% β–cyclodextrin)
Fluoxetine x 3 days
+ Compound (1), (15mg/kg, i.p.)
Fluoxetine x 3 days
+Yohimbine (2mg/kg, i.p.)

FIG. 18

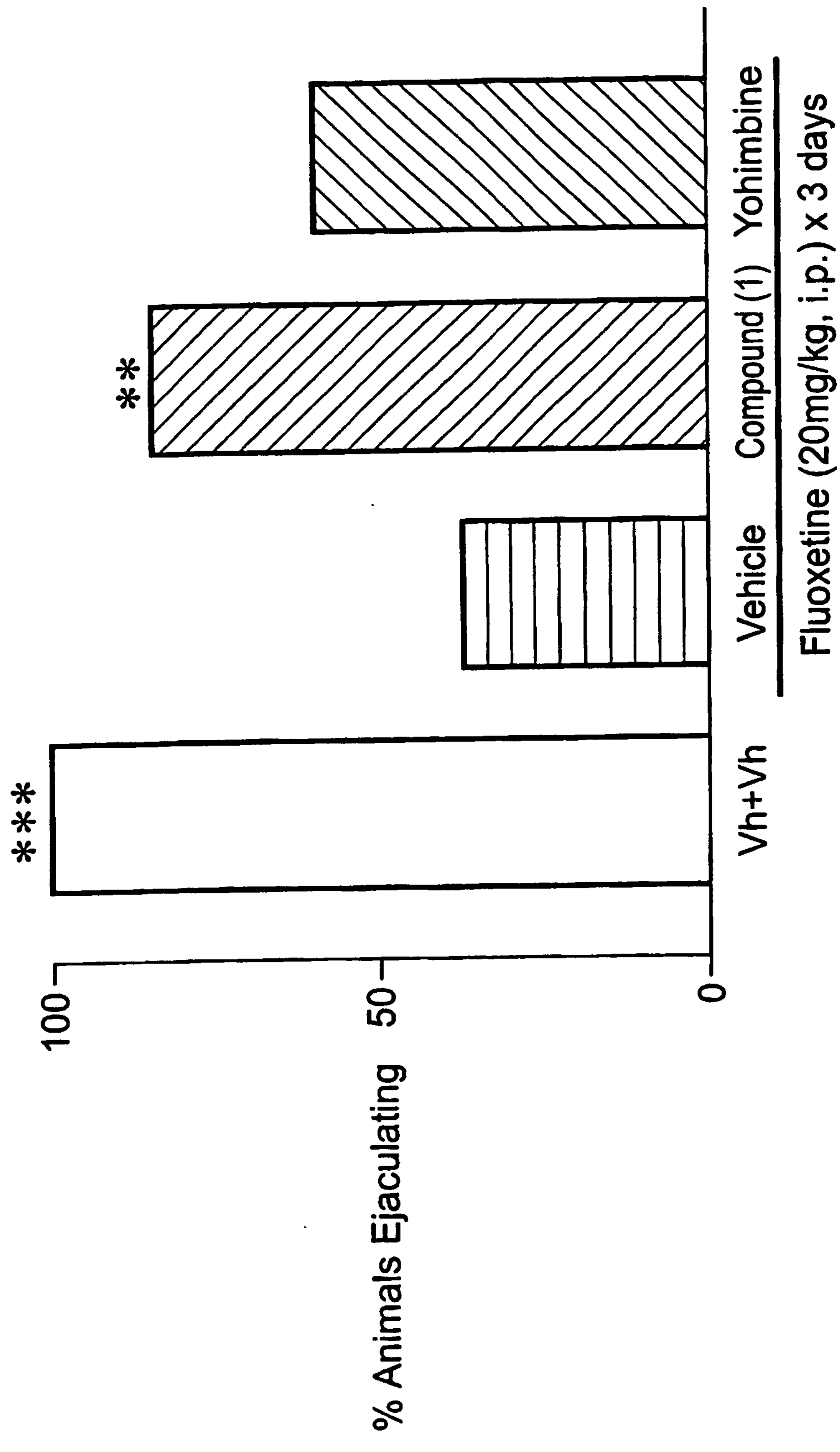
% Animals Ejaculating
100
50
0
***
**
Vh+Vh
Vehicle
Compound (1)
Yohimbine
Fluoxetine (20mg/kg, i.p.) x 3 days

FIG. 19

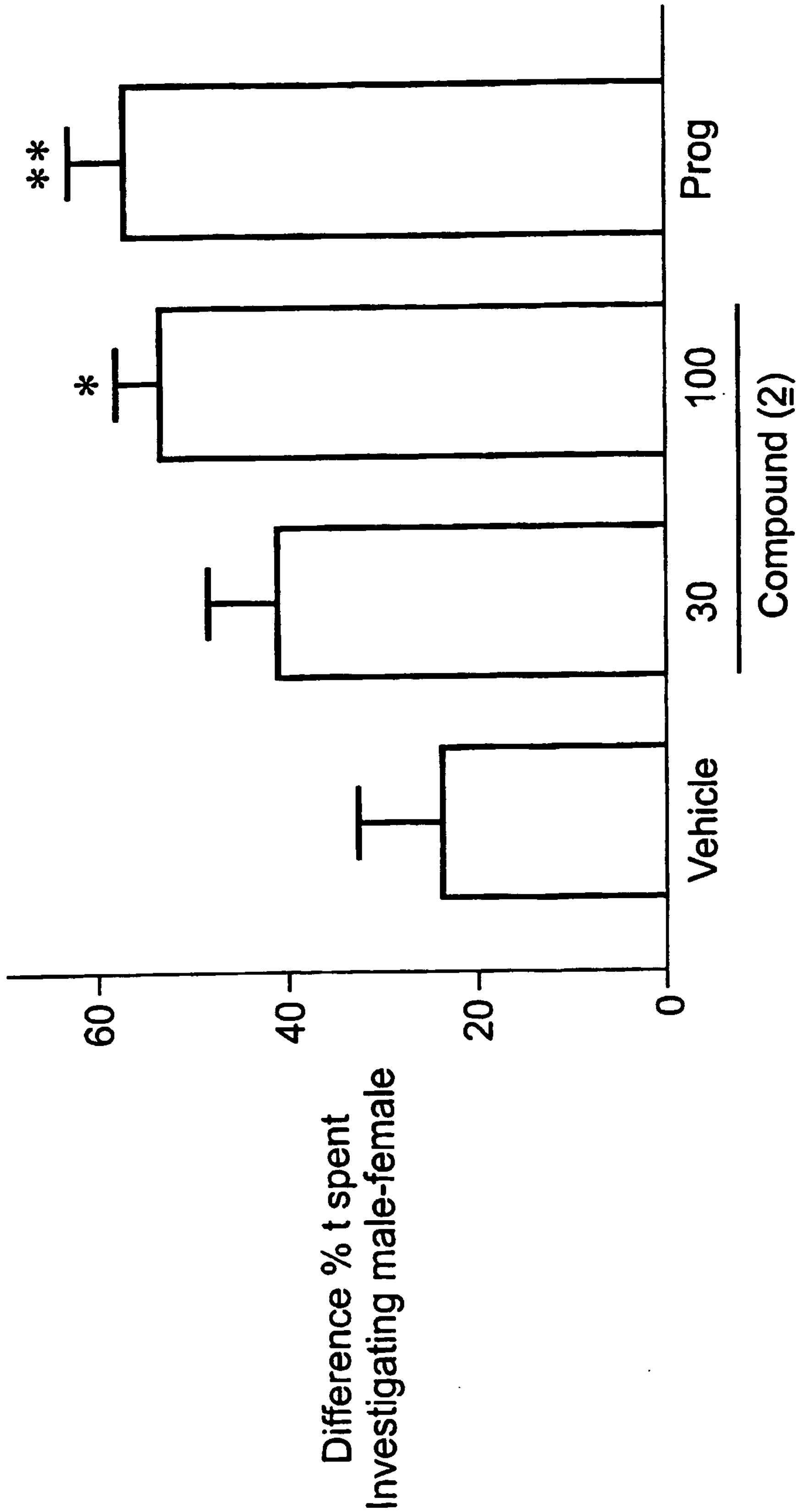
Difference % t spent
Investigating male-female
0
20
40
60
Vehicle
30
100
Prog
Compound (2)
*
**

FIG. 20

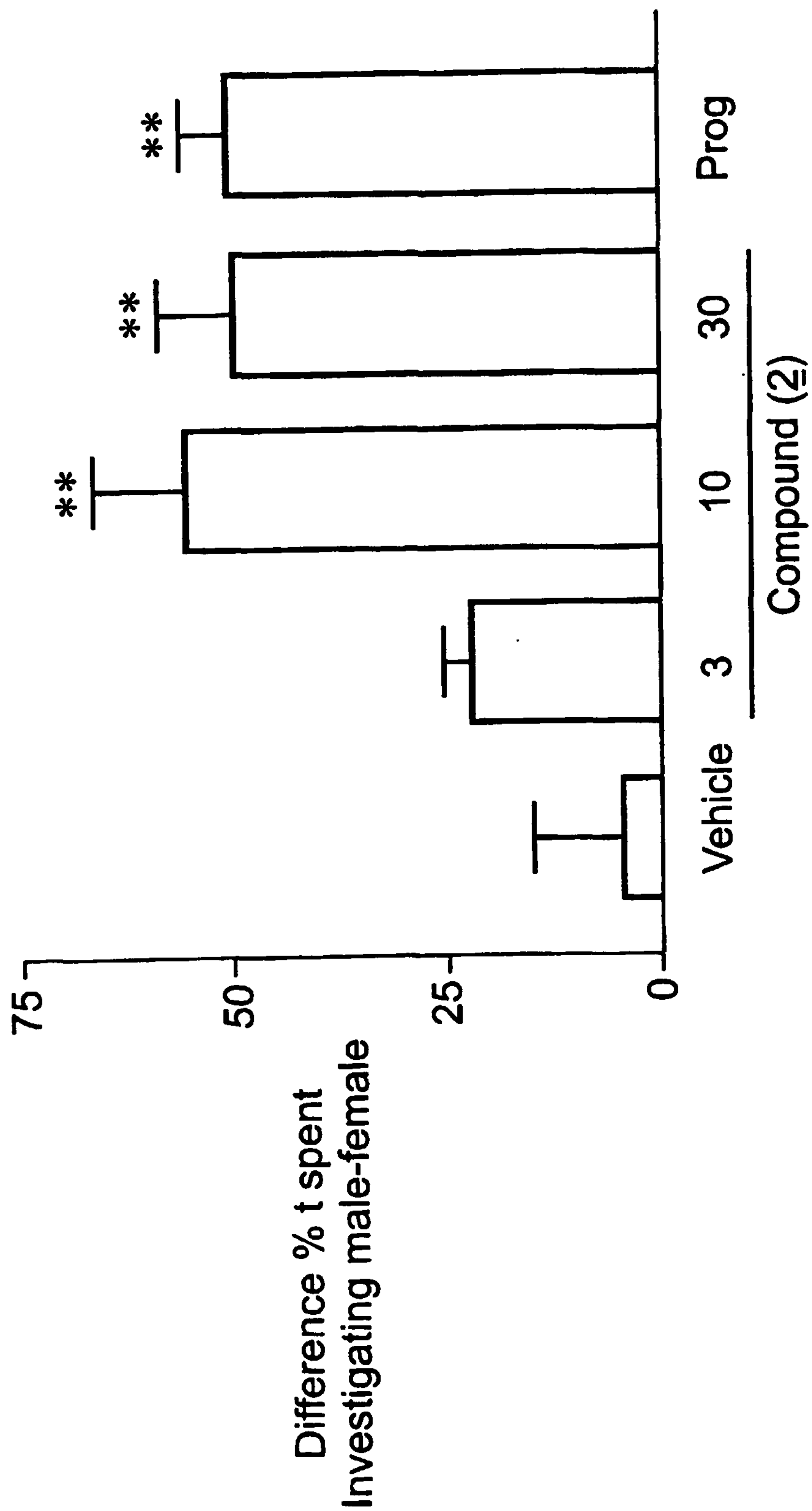
Difference % t spent
Investigating male-female
75
50
25
0
**
**
**
Vehicle
3
10
30
Prog
Compound (2)

FIG. 21

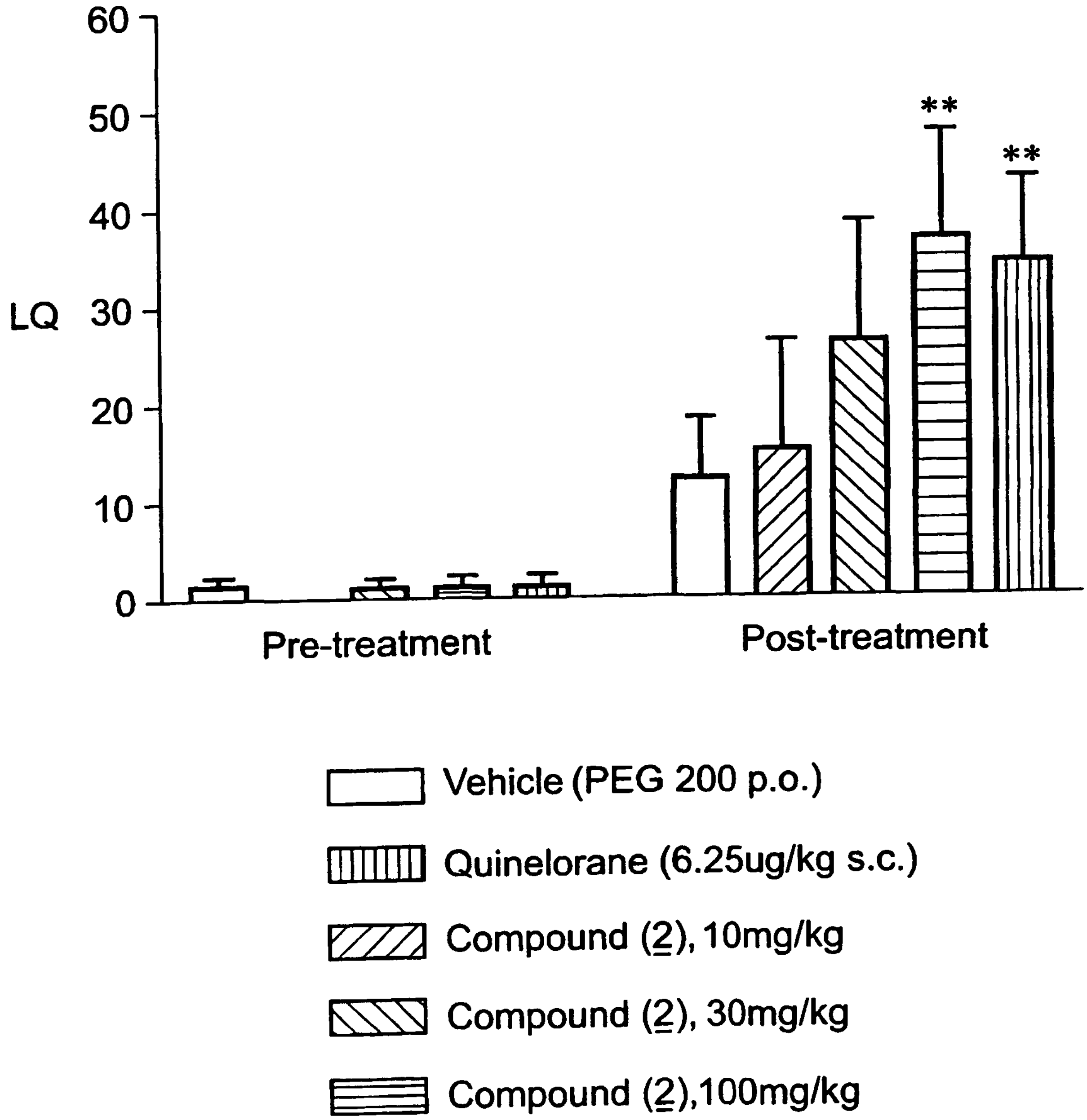
60
50
40
LQ
30
20
10
0
**
**
Pre-treatment
Post-treatment
Vehicle (PEG 200 p.o.)
Quinelorane (6.25ug/kg s.c.)
Compound (2), 10mg/kg
Compound (2), 30mg/kg
Compound (2),100mg/kg

FIG. 22

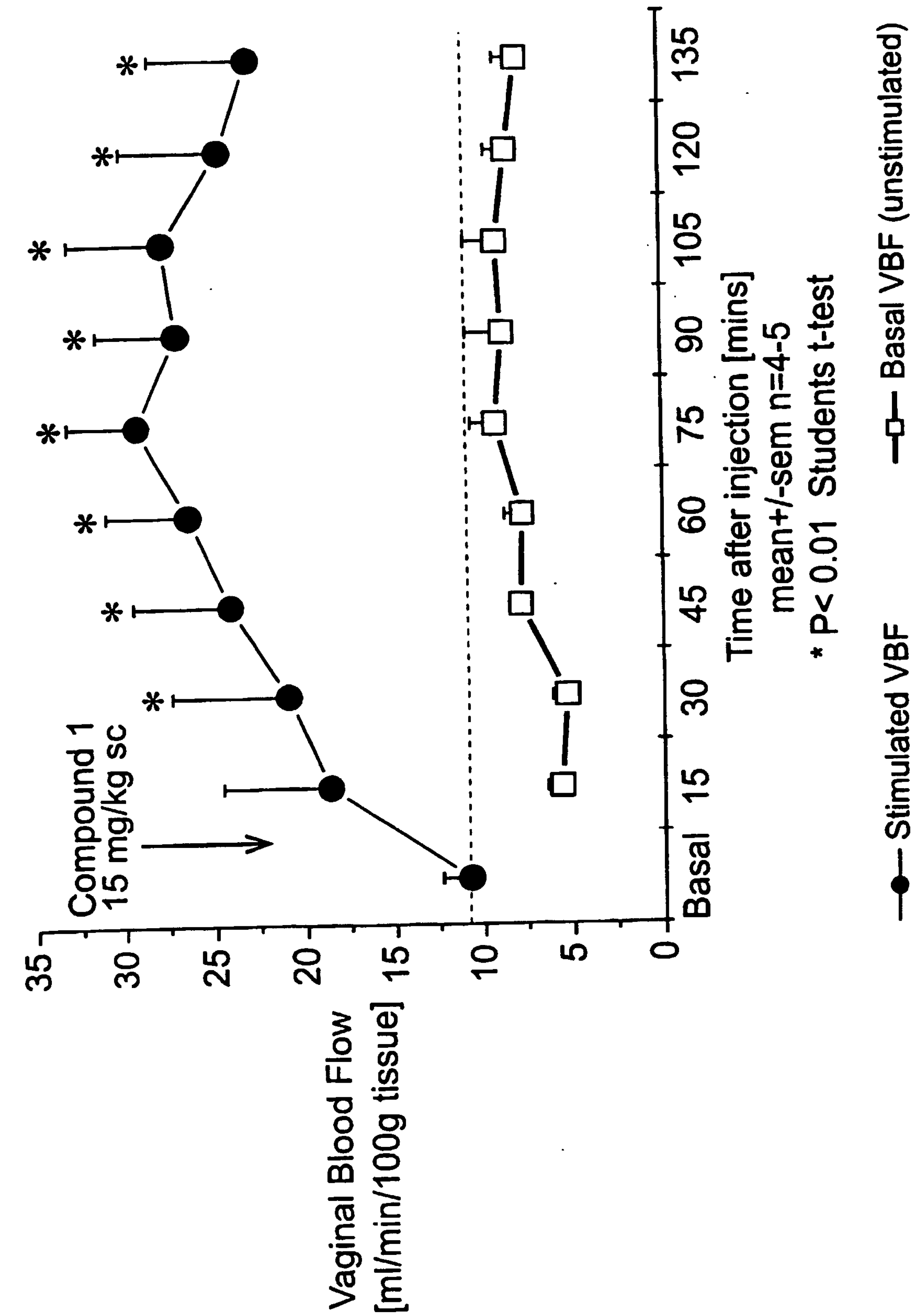
Vaginal Blood Flow
[ml/min/100g tissue]
35
30
25
20
15
10
5
0
Compound 1
15 mg/kg sc
*
Basal
15
30
45
60
75
90
105
120
135
Time after injection [mins]
mean+/-sem n=4-5
* P< 0.01 Students t-test
Stimulated VBF
Basal VBF (unstimulated)

FIG. 23

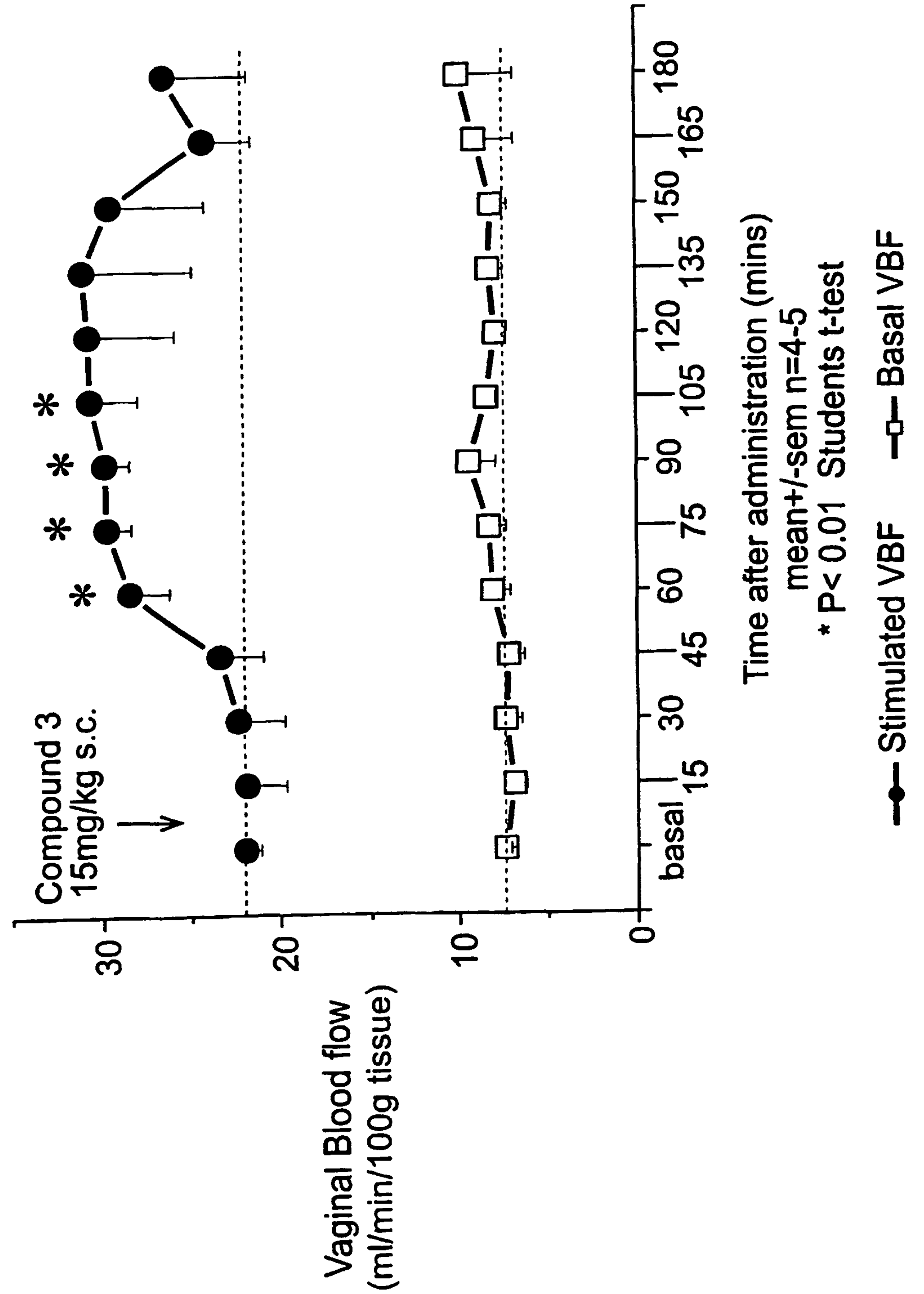
Compound 3
15mg/kg s.c.
*
*
*
*
Vaginal Blood flow
(ml/min/100g tissue)
0
10
20
30
basal
15
30
45
60
75
90
105
120
135
150
165
180
Time after administration (mins)
mean+/-sem n=4-5
* P< 0.01 Students t-test
Stimulated VBF
Basal VBF

FIG. 24

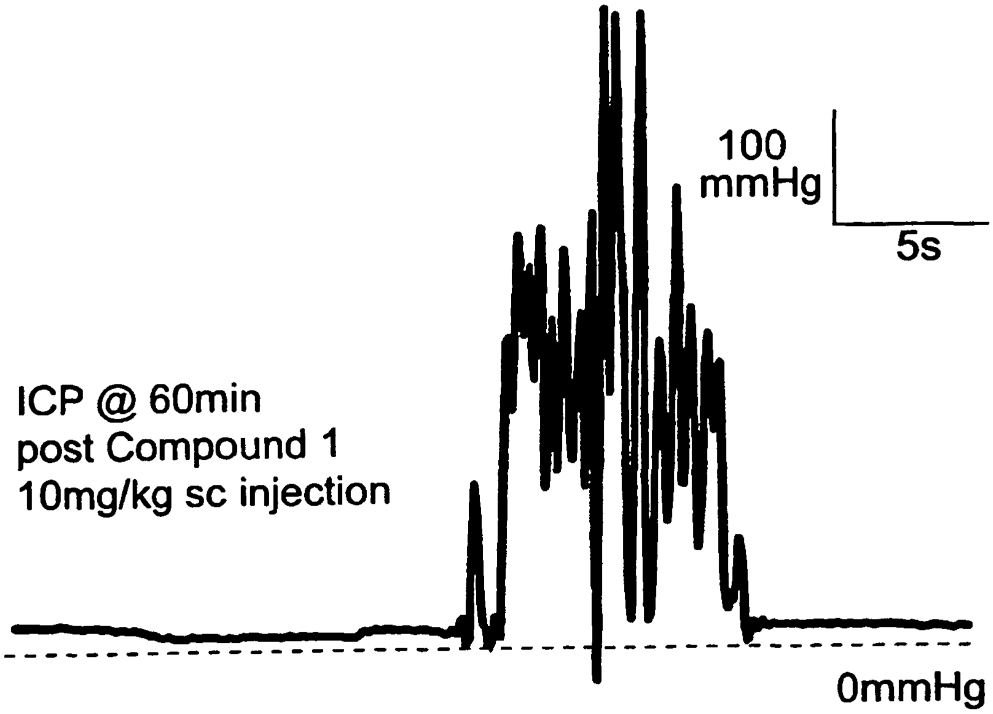
100
mmHg
5s
ICP @ 60min
post Compound 1
10mg/kg sc injection
0mmHg

FIG. 25

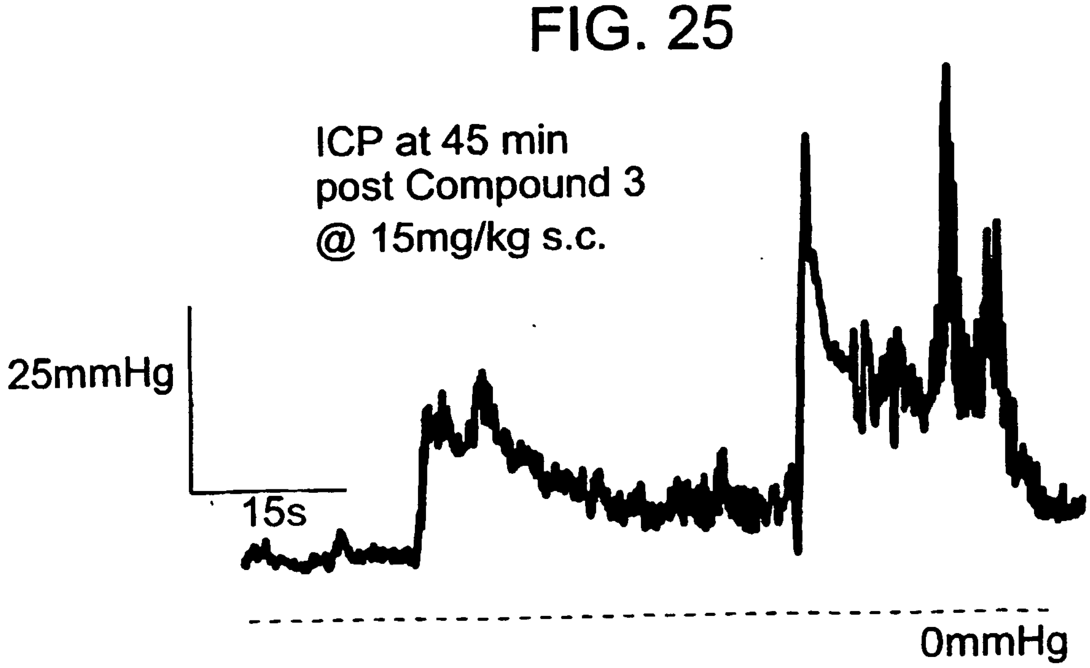
ICP at 45 min
post Compound 3
@ 15mg/kg s.c.
25mmHg
15s
0mmHg

FIG. 26

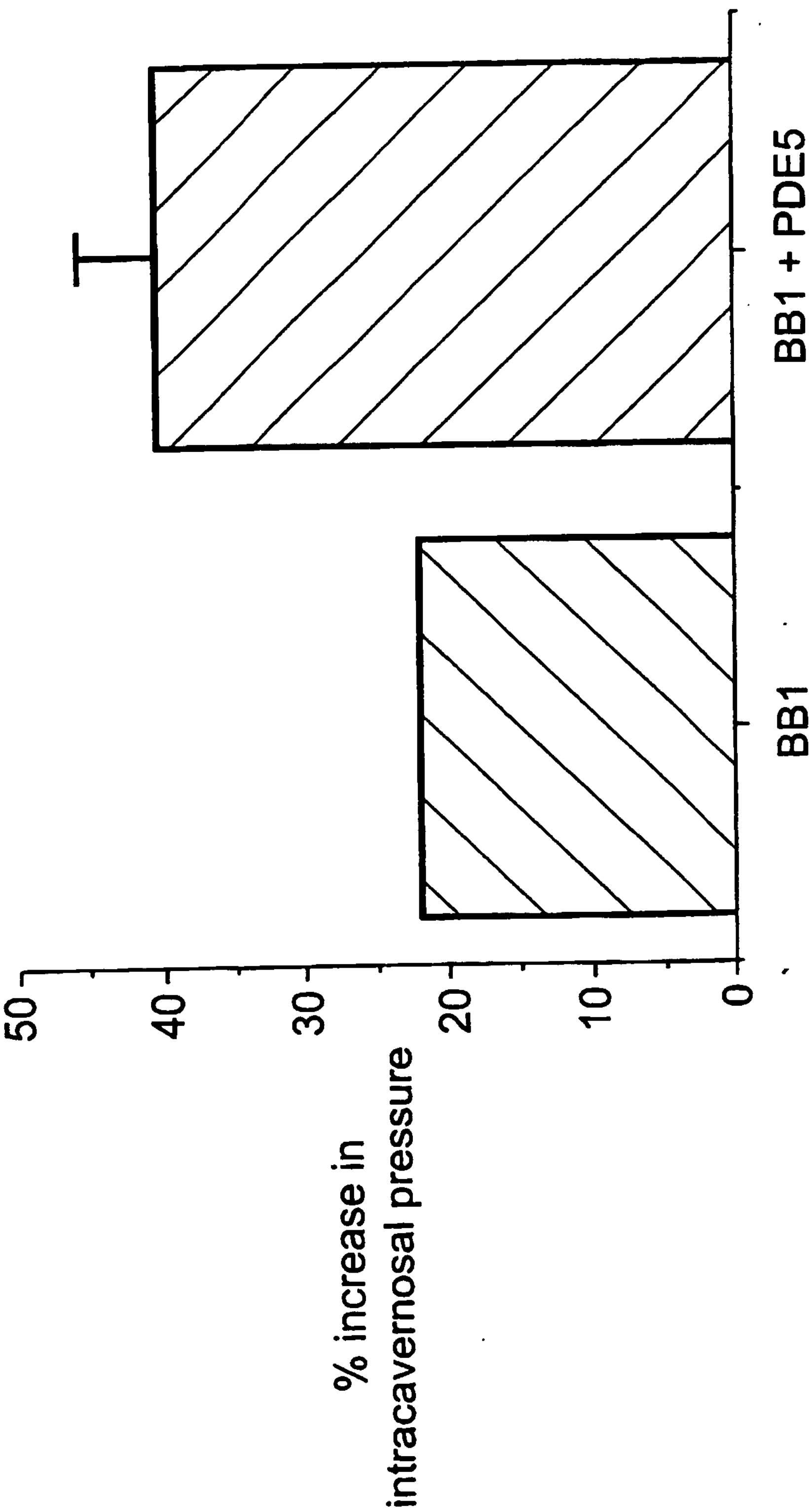
% increase in
intracavernosal pressure
50
40
30
20
10
0
BB1
BB1 + PDE5